# EUROPEAN PATENT SPECIFICATION

(11) **EP 2 566 477 B1**
(45) Date of publication and mention of the grant of the patent: **02.09.2015**
(21) Application number: 11778419.9
(22) Date of filing: 06.05.2011
(51) Int. Cl.: A61K 31/47, C07D 405/14, C07D 215/44, C07D 401/12, C07D 405/12, C07D 411/12, C07D 413/12, C07D 417/12, C07D 417/14, A61P 37/00

(54) **AMINO-QUINOLINES AS KINASE INHIBITORS**
AMINOCHINOLINE ALS KINASEHEMMER
AMINO-QUINOLÉINES EN TANT QU'INHIBITEURS DE KINASE

(30) Priority: 07.05.2010 US 332402 P
(43) Date of publication of application: 13.03.2013
(73) Proprietor: GlaxoSmithKline Intellectual Property Development Limited, Brentford Middlesex TW8 9GS (GB)
(72) Inventor: BURY, Michael, Jonathan, Collegeville, Pennsylvania 19426 (US); CASILLAS, Linda, N., Collegeville, PA 19426 (US); CHARNLEY, Adam, Kenneth, Collegeville, PA 19426 (US); DEMARTINO, Michael, P., Collegeville, PA 19426 (US); DONG, Xiaoyang, Collegeville, PA 19426 (US); HAILE, Pamela, A., Collegeville, Pennsylvania 19426 (US); HARRIS, Philip, Anthony, Collegeville, PA 19406 (US); LAKDAWALA SHAH, Ami, King of Prussia, PA 19406 (US); KING, Bryan, W., Collegeville, Pennsylvania 19406 (US); MARQUIS, Robert, W., Jr., Collegeville, PA 19426 (US); MEHLMANN, John, F., Collegeville, PA 19426 (US); ROMANO, Joseph, J., Collegeville, PA 19426 (US); SEHON, Clark, A., King of Prussia, PA 19406 (US); EIDAM, Patric, Collegeville, Pennsylvania 19426 (US)
(74) Representative: Price, Susanna Clare Hopley
(86) International application number: PCT/US2011/035521
(87) International publication number: WO 2011/140442

(56) References cited:
- EP-B1- 0 973 746
- WO-A1-02/068394
- US-A1- 2005 267 101
- US-A1- 2008 234 267
- GRETCHEN M ARGAST ET AL: "Inhibition of RIP2/RICK/CARDIAK activity by pyridinyl imidazole inhibitors of p38 MAPK", MOLECULAR AND CELLULAR BIOCHEMISTRY, KLUWER ACADEMIC PUBLISHERS, BO, vol. 268, no. 1-2, 1 January 2005 (2005-01-01), pages 129-140, XP019288634, ISSN: 1573-4919
- KUMAR ET AL.: 'Structure and Clinical Relevance of the Epidermal Growth Factor Receptor in Human Cancer' J CLIN ONCOL vol. 26, no. 10, 01 April 2008, pages 1742 - 1751, XP008133848

## Description

### BACKGROUND OF THE INVENTION

### Field of the Invention

The present invention relates to 4-amino-quinolines that inhibit RIP2 kinase and methods of making and using the same. Specifically, the present invention relates to substituted 4-amino-quinolines as RIP2 kinase inhibitors.

### Background of the Invention

Receptor interacting protein-2 (RIP2) kinase, which is also referred to as CARD3, RICK, CARDIAK, or RIPK2, is a TKL family serine/threonine protein kinase involved in innate immune signaling. RIP2 kinase is composed of an N-terminal kinase domain and a C-terminal caspase-recruitment domain (CARD) linked via an intermediate (IM) region ((1998) J. Biol. Chem. 273, 12296-12300; (1998) Current Biology 8, 885-889; and (1998) J. Biol. Chem. 273, 16968-16975). The CARD domain of RIP2 kinase mediates interaction with other CARD-containing proteins, such as NOD1 and NOD2 ((2000) J. Biol. Chem. 275, 27823-27831 and (2001) EMBO reports 2, 736-742). NOD1 and NOD2 are cytoplasmic receptors which play a key role in innate immune surveillance. They recognize both gram positive and gram negative bacterial pathogens and are activated by specific peptidoglycan motifs, diaminopimelic acid (i.e., DAP) and muramyl dipeptide (MDP), respectively ((2007) J Immunol 178, 2380-2386).

Following activation, RIP2 kinase associates with NOD 1 or NOD2 and appears to function principally as a molecular scaffold to bring together other kinases (TAK1, IKKα/β/γ) involved in NF-κB and mitogen-activated protein kinase activation ((2006) Nature Reviews Immunology 6, 9-20). RIP2 kinase undergoes a K63-linked polyubiquitination on lysine-209 which facilitates TAK1 recruitment ((2008) EMBO Journal 27, 373-383). This post-translational modification is required for signaling as mutation of this residue prevents NOD1/2 mediated NF-kB activation. RIP2 kinase also undergoes autophosphorylation on serine-176, and possibly other residues ((2006) Cellular Signalling 18, 2223-2229). Studies using kinase dead mutants (K47A) and non-selective small molecule inhibitors have demonstrated that RIP2 kinase activity is important for regulating the stability of RIP2 kinase expression and signaling ((2007) Biochem J 404, 179-190 and (2009) J. Biol. Chem. 284, 19183-19188).

Dysregulation of RIP2-dependent signaling has been linked to autoinflammatory diseases. Gain-of-function mutations in the NACHT-domain of NOD2 cause Blau Syndrome/Early-onset Sarcoidosis, a pediatric granulomateous disease characterized by uveitis, dermatitis, and arthritis((2001) Nature Genetic 29, 19-20; (2005) Journal of Rheumatology 32, 373-375; (2005) Current Rheumatology Reports 7, 427-433; (2005) Blood 105, 1195-1197; (2005) European Journal of Human Genetics 13, 742-747; (2006) American Journal of Ophthalmology 142, 1089-1092; (2006) Arthritis & Rheumatism 54, 3337-3344; (2009) Arthritis & Rheumatism 60, 1797-1803; and (2010) Rheumatology 49, 194-196). Mutations in the LRR-domain of NOD2 have been strongly linked to susceptibility to Crohn's Disease ((2002) Am. J. Hum. Genet. 70, 845-857; (2004) European Journal of Human Genetics 12, 206-212; (2008) Mucosal Immunology (2008) 1 (Suppl 1), S5-S9. 1, S5-S9; (2008) Inflammatory Bowel Diseases 14, 295-302; (2008) Experimental Dermatology 17, 1057-1058; (2008) British Medical Bulletin 87, 17-30; (2009) Inflammatory Bowel Diseases 15, 1145 - 1154 and (2009) Microbes and Infection 11, 912-918). Mutations in NOD1 have been associated with asthma ((2005) Hum. Mol. Genet. 14, 935-941) and early-onset and extra-intestinal inflammatory bowel disease ((2005) Hum. Mol. Genet. 14, 1245-1250). Genetic and functional studies have also suggested a role for RIP2-dependent signaling in a variety of other granulomateous disorders, such as sarcoidosis ((2009) Journal of Clinical Immunology 29, 78-89 and (2006) Sarcoidosis Vasculitis and Diffuse Lung Diseases 23, 23-29) and Wegner's Granulomatosis((2009) Diagnostic Pathology 4, 23).

Inhibition of RIP2/RICK/CARDIAK activity by pyridinyl inhibitors is disclosed in Mol. and Cell. Biochem. 268; 129-140 (2005).

A potent, selective, small molecule inhibitor of RIP2 kinase activity would block RIP2-dependent pro-inflammatory signaling and thereby provide a therapeutic benefit in autoinflammatory diseases characterized in increased and/or dysregulated RIP2 kinase activity.

### SUMMARY OF THE INVENTION

The invention is directed to novel 4-amino-quinolines. Specifically, the invention is directed to a compound according to Formula (I): 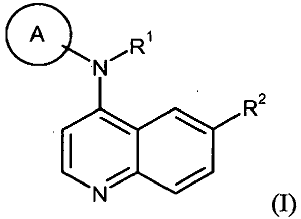 wherein:
R¹ is H, -SO₂(C₁-C₄alkyl), -CO(C₁-C₄alkyl), (C₁-C₄alkyl) or phenyl(C₁-C₄alkyl)-;
R² is -SR^{a}, -SOR^{a}, -SO₂R^{a}, -SO₂NH₂, -SO₂NR^{b}R^{c} or -CONR^{b}R^{c}, wherein
R^{a} is (C₁-C₆)alkyl, halo(C₁-C₄)alkyl, (C₂-C₆)alkenyl, (C₃-C₇)cycloalkyl, 4-7 membered heterocycloalkyl, aryl, or heteroaryl, wherein:
   said (C₁-C₆)alkyl is optionally substituted by one or two groups each independently selected from cyano, hydroxyl, (C₁-C₆)alkoxy, (C₁-C₆)alkoxy(C₂-C₆)alkoxy, -CO₂H, -CO₂(C₁-C₄)alkyl, -SO₂(C₁-C₄ alkyl), -CONH₂, -CONH(C₁-C₄ alkyl), -CON(C₁-C₄ alkyl)(C₁-C₄ alkyl), -SO₂NH₂, -SO₂NH(C₁-C₄ alkyl), -SO₂N(C₁-C₄ alkyl)(C₁-C₄ alkyl), amino, (C₁-C₄ alkyl)amino-, (C₁-C₄ alkyl)(C₁-C₄ alkyl)amino-, C₃-C₇cycloalkyl, phenyl, 5-6 membered heteroaryl, 9-10 membered heteroaryl, 4-7 membered heterocycloalkyl and (phenyl)(C₁-C₄ alkyl)amino-, wherein said C₃-C₇cycloalkyl, phenyl, (phenyl)(C₁-C₄ alkyl)amino-, 5-6 membered heteroaryl, 9-10 membered heteroaryl or 4-7 membered heterocycloalkyl is optionally substituted by 1-3 groups each independently selected from halogen, -CF₃, (C₁-C₄)alkyl, hydroxy(C₁-C₄)alkyl and (C₁-C₄)alkoxy,
   said (C₃-C₇)cycloalkyl or 4-7 membered heterocycloalkyl is optionally substituted by 1-3 groups each independently selected from halogen, -CF₃, hydroxyl, amino, (C₁-C₄)alkyl, phenyl(C₁-C₄)alkyl-, (C₁-C₄)alkoxycarbonyl-, hydroxy(C₁-C₄)alkyl-, oxo and (C₁-C₄)alkoxy, and
   said aryl or heteroaryl is optionally substituted by 1-3 groups each independently selected from halogen, -CF₃, hydroxyl, amino, (C₁-C₄)alkyl, phenyl(C₁-C₄)alkyl-, hydroxy(C₁-C₄)alkyl- and (C₁-C₄)alkoxy;
R^{b} is (C₁-C₆)alkyl, (C₃-C₇)cycloalkyl, 4-7 membered heterocycloalkyl, aryl, or heteroaryl, wherein:
   said (C₁-C₆)alkyl is optionally substituted by one or two groups each independently selected from cyano, hydroxyl, (C₁-C₆)alkoxy, (C₁-C₆)alkoxy(C₂-C₆)alkoxy, -CO₂H, -CO₂(C₁-C₄)alkyl, -SO₂(C₁-C₄ alkyl), -CONH₂, -CONH(C₁-C₄ alkyl), -CON(C₁-C₄ alkyl)(C₁-C₄ alkyl), -SO₂NH₂, -SO₂NH(C₁-C₄ alkyl), -SO₂N(C₁-C₄ alkyl)(C₁-C₄ alkyl), amino, (C₁-C₄ alkyl)amino-, (C₁-C₄ alkyl)(C₁-C₄ alkyl)amino-, C₃-C₇cycloalkyl, phenyl, 5-6 membered heteroaryl, 9-10 membered heteroaryl, 4-7 membered heterocycloalkyl and (phenyl)(C₁-C₄ alkyl)amino-, wherein said C₃-C₇cycloalkyl, phenyl, (phenyl)(C₁-C₄ alkyl)amino-, 5-6 membered heteroaryl, 9-10 membered heteroaryl or 4-7 membered heterocycloalkyl is optionally substituted by 1-3 groups each independently selected from halogen, -CF₃, (C₁-C₄)alkyl, hydroxy(C₁-C₄)alkyl and (C₁-C₄)alkoxy,
   said (C₃-C₇)cycloalkyl or 4-7 membered heterocycloalkyl is optionally substituted by 1-3 groups each independently selected from halogen, -CF₃, hydroxyl, amino, (C₁-C₄)alkyl, phenyl(C₁-C₄)alkyl-, (C₁-C₄)alkoxycarbonyl-, hydroxy(C₁-C₄)alkyl-, oxo and (C₁-C₄)alkoxy, and
   said aryl or heteroaryl is optionally substituted by 1-3 groups each independently selected from halogen, -CF₃, hydroxyl, amino, (C₁-C₄)alkyl, phenyl(C₁-C₄)alkyl-, hydroxy(C₁-C₄)alkyl- and (C₁-C₄)alkoxy;
R^{c} is H, (C₁-C₄)alkoxy or (C₁-C₆)alkyl;
or R^{b} and R^{c} taken together with the nitrogen atom to which they are attached form a 3-7 membered heterocycloalkyl or 5-6 membered heteroaryl group, optionally containing one or two additional ring heteroatoms each independently selected from nitrogen, oxygen and sulfur, wherein said 3-7 membered heterocycloalkyl or 5-6 membered heteroaryl is optionally substituted by 1-3 groups each independently selected from (C₁-C₄)alkyl, halo(C₁-C₄)alkyl, phenyl(C₁-C₄)alkyl-, hydroxyl, hydroxy(C₁-C₄)alkyl-, C₁-C₄ alkoxy, C₁-C₄ haloalkoxy, amino, (C₁-C₄ alkyl)amino-, (C₁-C₄ alkyl)(C₁-C₄ alkyl)amino-, -CO₂H, -CO₂(C₁-C₄)alkyl, -CO(C₁-C₄)alkyl, -CO(4-7 membered heterocycloalkyl), -CONH₂, -CONH(C₁-C₄alkyl), -CON(C₁-C₄alkyl)(C₁-C₄alkyl), -SO₂(C₁-C₄)alkyl, -SO₂(4-7 membered heterocycloalkyl), -SO₂NH₂, -SO₂NH(C₁-C₄alkyl) and -SO₂N(C₁-C₄alkyl)(C₁-C₄alkyl);
A is phenyl or aryl(C₁-C₄)alkyl-, substituted by R³, R⁴ and R⁵, wherein:
R³ is H, hydroxyl, halogen, -CF₃, hydroxy(C₁-C₄)alkyl, (C₁-C₄)alkyl or (C₁-C₄)alkoxy;
R⁴ is H, halogen, cyano, (C₁-C₄)alkyl, halo(C₁-C₄)alkyl, (C₁-C₄)alkoxy, phenoxy, phenyl(C₁-C₄)alkoxy, hydroxyl, hydroxy(C₁-C₄)alkyl-, or aminocarbonyl, wherein the phenyl moiety of said phenoxy or phenyl(C₁-C₄)alkoxy- is optionally substituted by 1-3 substituents each independently selected from halogen, -CF₃, (C₁-C₄)alkyl and (C₁-C₄)alkoxy; and
R⁵ is H, hydroxyl, halogen, -CF₃, hydroxy(C₁-C₄)alkyl, (C₁-C₄)alkyl or (C₁-C₄)alkoxy; or
A is phenyl, substituted by R⁶, R⁷ and R⁸, wherein:
   R⁶ and R⁷ are located on adjacent atoms and taken together with the atoms to which they are attached form a 5-membered heterocyclic group containing 1, 2 or 3 heteroatoms each independently selected from N, O and S, which 5-membered heterocyclic group is substituted by R⁹;
   wherein one of R⁸ or R⁹ is H, halogen, cyano, (C₁-C₄)alkyl, halo(C₁-C₄)alkyl, (C₁-C₄)alkoxy, phenoxy, phenyl(C₁-C₄)alkoxy, hydroxyl, hydroxy(C₁-C₄)alkyl-, or aminocarbonyl, where the phenyl moiety of said phenoxy or phenyl(C₁-C₄)alkoxy is optionally substituted by 1-3 substituents each independently selected from halogen, -CF₃, (C₁-C₄)alkyl and (C₁-C₄)alkoxy; and
   the other of R⁸ or R⁹ is H, hydroxyl, halogen, -CF₃, hydroxy(C₁-C₄)alkyl, (C₁-C₄)alkyl or (C₁-C₄)alkoxy; or
A is pyrazolyl, substituted by R¹⁰ and R¹¹, wherein:
   R¹⁰ and R¹¹ are located on adjacent carbon atoms and taken together with the atoms to which they are attached form a 6 membered carbocyclic or heterocyclic ring substituted by R¹² and R¹³;
   wherein R¹² is H, halogen, cyano, (C₁-C₄)alkyl, halo(C₁-C₄)alkyl, (C₁-C₄)alkoxy, phenoxy, phenyl(C₁-C₄)alkoxy, hydroxyl, hydroxy(C₁-C₄)alkyl-, or aminocarbonyl, wherein the phenyl moiety of said phenoxy or phenyl(C₁-C₄)alkoxy is optionally substituted by 1-3 substituents each independently selected from halogen, -CF₃, (C₁-C₄)alkyl and (C₁-C₄)alkoxy; and
   R¹³ is H, hydroxyl, halogen, -CF₃, hydroxy(C₁-C₄)alkyl, (C₁-C₄)alkyl or (C₁-C₄)alkoxy;
   or a salt, particularly a pharmaceutically acceptable salt, thereof.

In a particular embodiment, this invention excludes the following compounds:
2-fluoro-5[[6-(methylsulfonyl)-4-quinolinyl]amino-1,4-benzenediol;
N-(cyclopropylmethyl)-4-[(5-hydroxy-2-methylphenyl)amino]-6-quinolinesulfonamide;
4-methyl-3-[[6-(methylsulfonyl)-4-quinolinyl]amino]-phenol;
4-chloro-2-fluoro-5-[[6-(methylsulfonyl)-4-quinolinyl]amino]-phenol; and
4-[(5-hydroxy-2-methylphenyl)amino]-N-(1-methylethyl)-6-quinolinecarboxamide, or a salt thereof.

The present invention is also directed to a method of treating a RIP2 kinase-mediated disease in a patient (particularly, a human) which comprises administering to the patient a therapeutically effective amount of a compound according to Formula (I), or a pharmaceutically acceptable salt thereof.

The present invention is further directed to a method of inhibiting RIP2 kinase which comprises contacting the kinase with a compound according to Formula (I), or a salt, particularly a pharmaceutically acceptable salt, thereof.

The compounds of the invention (that is the compounds of Formula (I) and salts thereof), are inhibitors of RIP2 kinase and can be useful for the treatment of RIP2 kinase-mediated diseases and disorders, particularly uveitis, dermatitis, arthritis Crohn's disease, asthma, early-onset and extra-intestinal inflammatory bowel disease and granulomateous disorders, such as adult sarcoidosis, Blau syndrome, early-onset sarcoidosis and Wegner's Granulomatosis. Accordingly, the invention is further directed to pharmaceutical compositions comprising a compound of the invention.

The invention is still further directed to methods of inhibiting RIP2 kinase and treatment of conditions associated therewith using a compound of the invention or a pharmaceutical composition comprising a compound of the invention.

### DETAILED DESCRIPTION OF THE INVENTION

The alternative definitions for the various groups and substituent groups of Formula I provided throughout the specification are intended to particularly describe each compound species disclosed herein, individually, as well as groups'of one or more compound species. The scope of this invention includes any combination of these group and substituent group definitions. The compounds of the invention are only those which are contemplated to be "chemically stable" as will be appreciated by those skilled in the art.

The invention is further directed to a compound of Formula (I), wherein:
R¹ is H, -SO₂(C₁-C₄alkyl), -CO(C₁-C₄alkyl), (C₁-C₄alkyl) or phenyl(C₁-C₄alkyl)-;
R² is -SR^{a}, -SOR^{a}, -SO₂R^{a}, -SO₂NH₂, -SO₂NR^{b}R^{c} or -CONR^{b}R^{c}, wherein
R^{a} or R^{b} is (C₁-C₆)alkyl, (C₃-C₇)cycloalkyl, 4-7 membered heterocycloalkyl, aryl, or heteroaryl, wherein:
   said (C₁-C₆)alkyl is optionally substituted by one or two groups each independently selected from cyano, hydroxyl, (C₁-C₆)alkoxy, (C₁-C₆)alkoxy(C₂-C₆)alkoxy, -CO₂H, -CO₂(C₁-C₄)alkyl, -SO₂(C₁-C₄ alkyl), -CONH₂, -CONH(C₁-C₄ alkyl), -CON(C₁-C₄ alkyl)(C₁-C₄ alkyl), -SO₂NH₂, -SO₂NH(C₁-C₄ alkyl), -SO₂N(C₁-C₄ alkyl)(C₁-C₄ alkyl), amino, (C₁-C₄ alkyl)amino-, (C₁-C₄ alkyl)(C₁-C₄ alkyl)amino-, C₃-C₇cycloalkyl, phenyl, 5-6 membered heteroaryl, 9-10 membered heteroaryl, 4-7 membered heterocycloalkyl and (phenyl)(C₁-C₄ alkyl)amino-, wherein said C₃-C₇cycloalkyl, phenyl, (phenyl)(C₁-C₄ alkyl)amino-, 5-6 membered heteroaryl, 9-10 membered heteroaryl or 4-7 membered heterocycloalkyl is optionally substituted by 1-3 groups each independently selected from halogen, -CF₃, (C₁-C₄)alkyl, hydroxy(C₁-C₄)alkyl and (C₁-C₄)alkoxy,
   said (C₃-C₇)cycloalkyl or 4-7 membered heterocycloalkyl is optionally substituted by 1-3 groups each independently selected from halogen, -CF₃, hydroxyl, amino, (C₁-C₄)alkyl, phenyl(C₁-C₄)alkyl-, hydroxy(C₁-C₄)alkyl-, oxo and (C₁-C₄)alkoxy, and
   said aryl of heteroaryl is optionally substituted by 1-3 groups each independently selected from halogen, -CF₃, hydroxyl, amino, (C₁-C₄)alkyl, phenyl(C₁-C₄)alkyl-, hydroxy(C₁-C₄)alkyl- and (C₁-C₄)alkoxy;
R^{c} is H, (C₁-C₄)alkoxy or (C₁-C₆)alkyl;
   or R^{b} and R^{c} taken together with the nitrogen atom to which they are attached form a 5-7 membered heterocycloalkyl or 5-6 membered heteroaryl group, optionally containing one additional ring heteroatom selected from nitrogen, oxygen and sulfur, wherein said 5-7 membered heterocycloalkyl or 5-6 membered heteroaryl is optionally substituted by 1-3 groups each independently selected from (C₁-C₄)alkyl, halo(C₁-C₄)alkyl, phenyl(C₁-C₄)alkyl-, hydroxy(C₁-C₄)alkyl-, C₁-C₄ alkoxy, C₁-C₄ haloalkoxy, amino, (C₁-C₄ alkyl)amino-, (C₁-C₄ alkyl)(C₁-C₄ alkyl)amino-, -CO₂H, -CO₂(C₁-C₄)alkyl, -CO(C₁-C₄)alkyl, -CO(4-7 membered heterocycloalkyl), -CONH₂, -CONH(C₁-C₄alkyl), -CON(C₁-C₄alkyl)(C₁-C₄alkyl), -SO₂(C₁-C₄)alkyl, -SO₂(4-7 membered heterocycloalkyl), -SO₂NH₂, -SO₂NH(C₁-C₄alkyl) and -SO₂N(C₁-C₄alkyl)(C₁-C₄alkyl);
A is phenyl or aryl(C₁-C₄)alkyl-, substituted by R³, R⁴ and R⁵, wherein:
   R³ is H, hydroxyl, halogen, -CF₃, hydroxy(C₁-C₄)alkyl, (C₁-C₄)alkyl or (C₁-C₄)alkoxy;
   R⁴ is H, halogen, cyano, (C₁-C₄)alkyl, halo(C₁-C₄)alkyl, (C₁-C₄)alkoxy, phenoxy, phenyl(C₁-C₄)alkoxy, hydroxyl, hydroxy(C₁-C₄)alkyl-, or aminocarbonyl, wherein the phenyl moiety of said phenoxy or phenyl(C₁-C₄)alkoxy- is optionally substituted by 1-3 substituents each independently selected from halogen, -CF₃, (C₁-C₄)alkyl and (C₁-C₄)alkoxy; and
   R⁵ is H, hydroxyl, halogen, -CF₃, hydroxy(C₁-C₄)alkyl, (C₁-C₄)alkyl or (C₁-C₄)alkoxy; or
A is phenyl substituted by R⁶, R⁷ and R⁸, wherein:
   R⁶ and R⁷ are located on adjacent atoms and taken together with the atoms to which they are attached form a 5-membered heterocyclic group containing 1, 2 or 3 heteroatoms each independently selected from N, O and S, which 5-membered heterocyclic group is substituted by R⁹;
   wherein one of R⁸ or R⁹ is H, halogen, cyano, (C₁-C₄)alkyl, halo(C₁-C₄)alkyl, (C₁-C₄)alkoxy, phenoxy, phenyl(C₁-C₄)alkoxy, hydroxyl, hydroxy(C₁-C₄)alkyl-, or aminocarbonyl, where the phenyl moiety of said phenoxy or phenyl(C₁-C₄)alkoxy is optionally substituted by 1-3 substituents each independently selected from halogen, -CF₃, (C₁-C₄)alkyl and (C₁-C₄)alkoxy; and
   the other of R⁸ or R⁹ is H, hydroxyl, halogen, -CF₃, hydroxy(C₁-C₄)alkyl, (C₁-C₄)alkyl or (C₁-C₄)alkoxy; or
A is pyrazolyl, substituted by R¹⁰ and R¹¹, wherein:
   R¹⁰ and R¹¹ are located on adjacent carbon atoms and taken together with the atoms to which they are attached form a 6 membered carbocyclic ring or heterocyclic ring substituted by R¹² and R¹³;
   wherein R¹² is H, halogen, cyano, (C₁-C₄)alkyl, halo(C₁-C₄)alkyl, (C₁-C₄)alkoxy, phenoxy, phenyl(C₁-C₄)alkoxy, hydroxyl, hydroxy(C₁-C₄)alkyl-, or aminocarbonyl, wherein the phenyl moiety of said phenoxy or phenyl(C₁-C₄)alkoxy is optionally substituted by 1-3 substituents each independently selected from halogen, -CF₃, (C₁-C₄)alkyl and (C₁-C₄)alkoxy; and
   R¹³ is H, hydroxyl, halogen, -CF₃, hydroxy(C₁-C₄)alkyl, (C₁-C₄)alkyl or (C₁-C₄)alkoxy;
   or a salt thereof.

In one embodiment of this invention, R¹ is H. In other embodiments, R¹ is -SO₂(C₁-C₄alkyl) or -CO(C₁-C₄alkyl); specifically, -SO₂CH₃ or -COCH₃. In other embodiments, R¹ is (C₁-C₂)alkyl or phenyl(C₁-C₂alkyl)-; specifically, -CH₃ or benzyl.

In yet another embodiment, R² is -SR^{a}, -SOR^{a} or -SO₂R^{a}. In other embodiments, R² is -CONR^{b}R^{c}, -SO₂NH₂ or -SO₂NR^{b}R^{c}. In a further embodiment, R² is -SR^{a}, -SOR^{a}, -SO₂R^{a}, or -SO₂NR^{b}R^{c}. In more specific embodiments, R² is -SOR^{a} or -SO₂R^{a}.

In a further embodiment, R^{a} is (C₁-C₆)alkyl, (C₂-C₆)alkenyl, hato(C₁-C₆)alkyl, C₃-C₆cycloalkyl, 4-6-membered heterocycloalkyl, 5-6-membered heteroaryl or phenyl;
wherein said (C₁-C₆)alkyl is optionally substituted by 1 or 2 substituents each independently selected from hydroxyl, (C₁-C₄)alkoxy, (C₁-C₄)alkoxy(C₂-C₄)alkoxy-, amino, (C₁-C₄ alkyl)amino-, (C₁-C₄ alkyl)(C₁-C₄ alkyl)amino-, (phenyl)(C₁-C₄ alkyl)amino-, -CO₂(C₁-C₄)alkkyl, -CONH₂, -SO₂(C₁-C₄)alkyl, and a C₃-C₆cycloalkyl, phenyl, 4-6-membered heterocycloalkyl, 5-6-membered heteroaryl, or 9-10-membered heteroaryl, where said C₃-C₆cycloalkyl, phenyl, 4-6-membered heterocycloalkyl, 5-6-membered heteroaryl, or 9-10-membered heteroaryl is optionally substituted by 1-3 groups each independently selected from halogen, -CF₃, hydroxyl, amino, (C₁-C₄)alkyl, phenyl(C₁-C₄)alkyl-, hydroxy(C₁-C₄)alkyl- and (C₁-C₄)alkoxy; and
wherein said C₃-C₆cycloalkyl, 4-6-membered heterocycloalkyl, 5-6-membered heteroaryl or phenyl is optionally substituted by 1-3 groups each independently selected from halogen, -CF₃, hydroxyl, amino, (C₁-C₄)alkyl, phenyl(C₁-C₄)alkyl-, (C₁-C₄)alkoxycarbonyl-, hydroxy(C₁-C₄)alkyl- and (C₁-C₄)alkoxy.

In a further embodiment, R^{a} or R^{b} is (C₁-C₆)alkyl, C₃-C₆cycloalkyl, 4-6-membered heterocycloalkyl, 5-6-membered heteroaryl or phenyl;
wherein said (C₁-C₆)alkyl is optionally substituted by 1 or 2 substituents each independently selected from hydroxyl, (C₁-C₄)alkoxy, (C₁-C₄)alkoxy(C₂-C₄)alkoxy-, amino, (C₁-C₄ alkyl)amino-, (C₁-C₄ alkyl)(C₁-C₄ alkyl)amino-, (phenyl)(C₁-C₄ alkyl)amino-, -CO₂(C₁-C₄)alkyl, -CONH₂, -SO₂(C₁-C₄)alkyl, and a C₃-C₆cycloalkyl, phenyl, 4-6-membered heterocycloalkyl, 5-6-membered heteroaryl, or 9-10-membered heteroaryl, where said C₃-C₆cycloalkyl, phenyl, 4-6-membered heterocycloalkyl, 5-6-membered heteroaryl, or 9-10-membered heteroaryl is optionally substituted by 1-3 groups each independently selected from halogen, -CF₃, hydroxyl, amino, (C₁-C₄)alkyl, phenyl(C₁-C₄)alkyl-, hydroxy(C₁-C₄)alkyl- and (C₁-C₄)alkoxy; and
wherein said C₃-C₆cycloalkyl, 4-6-membered heterocycloalkyl, 5-6-membered heteroaryl or phenyl is optionally substituted by 1-3 groups each independently selected from halogen, -CF₃, hydroxyl, amino, (C₁-C₄)alkyl, phenyl(C₁-C₄)alkyl-, (C₁-C₄)alkoxycarbonyl-, hydroxy(C₁-C₄)alkyl- and (C₁-C₄)alkoxy.

When R^{a} is a heterocycloalkyl or heteroaryl group, it is to be understood that the heterocycloalkyl or heteroaryl group is bonded to the sulfur or nitrogen atom of the -SR^{a}, -SOR^{a}, -SO₂R^{a}, -SO₂NR^{b}R^{c} or -CONR^{b}R^{c} moiety by a ring carbon atom.

In a still further embodiment, R^{a} is (C₁-C₆)alkyl, C₃-C₆cycloalkyl, 4-6-membered heterocycloalkyl, 5-6-membered heteroaryl or phenyl, wherein:
said (C₁-C₆)alkyl is optionally substituted by 1 or 2 substituents each independently selected from hydroxyl, (C₁-C₁)alkoxy, (C₁-C₂)alkoxy(C₂-C₃)alkoxy-, amino, (C₁-C₃ alkyl)amino-, (C₁-C₃ alkyl)(C₁-C₂ alkyl)amino-, (phenyl)(C₁-C₂ alkyl)amino-, -CO₂(C₁-C₂)alkyl, -CONH₂, -SO₂(C₁-C₂)alkyl, and a C₃-C₆cycloalkyl (optionally substituted by (C₁-C₄)alkyl or hydroxy(C₁-C₄)alkyl), 4-6-membered heterocycloalkyl (optionally substituted by (C₁-C₄)alkyl), 5-6-membered heteroaryl (optionally substituted by (C₁-C₄)alkyl), phenyl, or 9-10-membered heteroaryl,
said 4-6-membered heterocycloalkyl is optionally substituted by (C₁-C₄)alkyl, (C₁-C₄)alkoxycarbonyl-, or benzyl,
said 5-6-membered heteroaryl is optionally substituted by (C₁-C₄)alkyl or hydroxy(C₁-C₄)alkyl, and
said phenyl is optionally substituted by amino.

In one embodiment of this invention, R^{a} is -CH₃, -CF₃, -CH₂CH₃, -CH₂CF₃, -CH₂CH₂CH₃, -CH₂CH=CH₂, -CH(CH₃)₂, -CH(CH₃)CH₂CH₃, -C(CH₃)₃, -CH₂CH₂OH, -CH₂CH₂OCH₃, -CH₂CH₂OCH₂CH₂OCH₃, -CH₂CH₂CH₂OH, -CH₂CH₂CH₂CH₂OH, -CH(CH₃)CH₂OH, -C(CH₃)₂CH₂OH, -C(CH₃)₂CO₂H, -C(CH₃)₂CH₂CH₂OH, -C(CH₃)₂CH₂CH₂OCH₃, -CH₂CH₂NH₂, -CH₂CH₂N(CH₃)₂, -CH₂CH₂N(CH₂CH₃)₂, -CH₂CH₂SO₂CH₃, -CH₂CONH₂, -CH₂CH₂CONH₂, -C(CH₃)₂CO₂CH₃, [1-(2-hydroxyethyl)cyclopropyl]methyl-, cyclopropyl, cyclopentyl, cyclohexyl, oxetan-3-yl, 3-methyl-oxetan-3-yl, tetrahydro-2*H*-pyran-4-yl, tetrahydro-2*H*-pyran-3-yl, -CH₂-tetrahydro-2*H*-pyran-4-yl, tetrahydrofuran-3-yl, 2-methyl-tetrahydrofuran-3-yl, -CH₂- tetrahydrofuran-2-yl, 1*H*-imidazol-4-yl, 1*H*-1,2,4-triazol-3-yl, phenyl, benzyl, -CH₂CH₂CH₂-phenyl, 4-amino-phenyl-, pyridin-4-yl, -CH₂-(6-methyl-pyridin-2-yl), piperidin-4-yl, 1-methyl-piperidin-4-yl-, 1-((CH₃)₃C-O-CO-piperidin-4-yl-, -CH₂-piperidin-4-yl, -CH₂CH₂-morpholin-4-yl, -CH₂CH₂CH₂-morpholin-4-yl, pyrimidin-2-yl, -CH₂CH₂-indol-3-yl, 4,5-dimethyl-thiazol-2-yl, (3R)-1-benzyl-pyrrolidin-3-yl-, -CH₂CH₂-pyrrolidin-1-yl, -CH₂-benzimidazol-2-yl, -CH₂CH₂CH₂-imidazol-1-yl, -CH₂CH₂-imidazol-4-yl, -CH₂CH₂-(3,5-dimethyl-isoxazol-4-yl), (2*S*)-1-hydroxy-3-(1*H*-imidazol-4-yl)prop-2-yl, 3-[methyl(phenyl)amino]prop-1-yl tetrahydro-2*H*-thiopyran-4-yl, or 1,1-dioxidotetrahydro-2*H*-thiopyran-4-yl.

In another embodiment of this invention, R^{b} is -CH₃, -CH₂CH₃, -CH₂CH₂CH₃, -CH(CH₃)₂, -CH(CH₃)CH₂CH₃, -C(CH₃)₃, -CH₂CH₂OH, -CH₂CH₂OCH₃, -CH₂CH₂OCH₂CH₂OCH₃, -CH₂CH₂CH₂OH, -CH₂CH₂CH₂CH₂OH, -CH(CH₃)CH₂OH, -C(CH₃)₂CH₂OH, -C(CH₃)₂CO₂H, -C(CH₃)₂CH₂CH₂OH, -C(CH₃)₂CH₂CH₂OCH₃, -CH₂CH₂NH₂, -CH₂CH₂N(CH₃)₂, -CH₂CH₂N(CH₂CH₃)₂, -CH₂CH₂SO₂CH₃, -CH₂CONH₂, -CH₂CH₂CONH₂, -C(CH₃)₂CO₂CH₃, cyclopentyl, cyclohexyl, oxetan-3-yl, 3-methyl-oxetan-3-yl, tetrahydro-2*H*-pyran-4-yl, tetrahydro-2*H*-pyran-3-yl, -CH₂-tetrahydro-2*H*-pyran-4-yl, tetrahydrofuran-3-yl, 2-methyl-tetrahydrofuran-3-yl, -CH₂- tetrahydrofuran-2-yl, 1*H*-imidazol-4-yl, 1*H*-1,2,4-triazol-3-yl, phenyl, benzyl, -CH₂CH₂CH₂-phenyl, 4-amino-phenyl-, pyridin-4-yl, -CH₂-(6-methyl-pyridin-2-yl), piperidin-4-yl, 1-methyl-piperidin-4-yl-, 1-((CH₃)₃C-O-CO-piperidin-4-yl-, -CH₂-piperidin-4-yl, -CH₂CH₂-morpholin-4-yl, -CH₂CH₂CH₂-morpholin-4-yl, pyrimidin-2-yl, -CH₂CH₂-indol-3-yl, 4,5-dimethyl-thiazol-2-yl, (3R)-1-benzyl-pyrrolidin-3-yl-, -CH₂CH₂-pyrrolidin-1-yl, -CH₂-benzimidazol-2-yl, -CH₂CH₂CH₂-imidazol-1-yl, -CH₂CH₂-imidazol-4-yl, -CH₂CH₂-(3,5-dimethyl-isoxazol-4-yl), (2*S*)-1-hydroxy-3-(1*H*-imidazol-4-yl)prop-2-yl, 3-[methyl(phenyl)amino]prop-1-yl tetrahydro-2*H*-thiopyran-4-yl, or 1,1-dioxidotetrahydro-2*H*-thiopyran-4-yl.

In another embodiment, R^{a} or R^{b} is -CH₃, -CH₂CH₃, -CH₂CH₂CH₃, -CH(CH₃)₂, -CH(CH₃)CH₂CH₃, -C(CH₃)₃, -CH₂CH₂OH -CH₂CH₂OCH₃, -CH₂CH₂OCH₂CH₂OCH₃, -CH₂CH₂CH₂OH, -CH₂CH₂CH₂CH₂OH, -CH(CH₃)CH₂OH, -C(CH₃)₂CH₂OH, -C(CH₃)₂CH₂CH₂OH, -C(CH₃)₂CH₂CH₂OCH₃, -CH₂CH₂NH₂, -CH₂CH₂N(CH₃)₂, -CH₂CH₂SO₂CH₃, -CH₂CONH₂, -CH₂CH₂CONH₂, -C(CH₃)₂CO₂CH₃, [1-(2-hydroxyethyl)cyclopropyl]methyl-, cyclopropyl, cyclopentyl, cyclohexyl, oxetan-3-yl, 3-methyl-oxetan-3-yl, tetrahydro-2H-pyran-4-yl, -CH₂-tetrahydro-2H-pyran-4-yl, phenyl, benzyl, -CH₂CH₂CH₂-phenyl, 4-amino-phenyl-, pyridin-4-yl, -CH₂-(6-methyl-pyridin-2-yl), piperidin-4-yl, 1-methyl-piperidin-4-yl-, -CH₂-piperidin-4-yl, -CH₂CH₂CH₂-morpholin-4-yl, pyrimidin-2-yl, -CH₂CH₂-indol-3-yl, 4,5-dimethyl-thiazol-2-yl, (3R)-1-benzyl-pyrrolidin-3-yl-, -CH₂CH₂-pyrrolidin-1-yl, -CH₂-benzimidazol-2-yl, -CH₂CH₂CH₂-imidazol-1-yl, -CH₂CH₂-imidazol-4-yl, (2S)-1-hydroxy-3-(1H-imidazol-4-yl)prop-2-yl, 3-[methyl(phenyl)amino]prop-1-yl or -CH₂CH₂CH2-morpholin-4-yl.

In more specific embodiments, R^{a} is -CH₃, -CF₃, -CH₂CH₃, -CH₂CF₃, -CH₂CH₂CH₃, -CH₂CH=CH₂, -CH(CH₃)₂, -CH(CH₃)CH₂CH₃, -C(CH₃)₃, -CH₂CH₂OH, -CH₂CH₂OCH₃, -CH₂CH₂CH₂OH, -CH₂CH₂CH₂CH₂OH, -CH(CH₃)CH₂OH, -C(CH₃)₂CH₂OH, -C(CH₃)₂CO₂H, -C(CH₃)₂CH₂CH₂OH, -C(CH₃)₂CH₂CH₂OCH₃, -CH₂CH₂NH₂, -CH₂CH₂N(CH₂CH₃)₂, -C(CH₃)₂CO₂CH₃, [1-(2-hydroxyethyl)cyclopropyl]methyl-, cyclopropyl, cyclopentyl, cyclohexyl, tetrahydro-2*H*-pyran-4-yl, tetrahydro-2*H*-pyran-3-yl, tetrahydrofuran-3-yl, 2-methyl-tetrahydrofuran-3-yl, -CH₂- tetrahydrofuran-2-yl, 1*H*-imidazol-4-yl, 1*H*-1,2,4-triazol-3-yl, phenyl, 4-amino-phenyl-, pyridin-4-yl, piperidin-4-yl, 1-methyl-piperidin-4-yl-, 1-((CH₃)₃C-O-CO-piperidin-4-yl-, -CH₂CH₂-morpholin-4-yl, pyrimidin-2-yl, -CH₂CH₂-(3,5-dimethyl-isoxazol-4-yl), tetrahydro-2*H*-thiopyran-4-yl, or 1,1-dioxidotetrahydro-2*H*-thiopyran-4-yl.

In another more specific embodiment, R^{b} is -CH₃, -CH(CH₃)₂, -CH₂CH₂OH, -CH₂CH₂OCH₃, -CH₂CH₂OCH₂CH₂OCH₃, -CH₂CH₂N(CH₃)₂, -CH₂CH₂SO₂CH₃, -CH₂CONH₂, -CH₂CH₂CONH₂, cyclohexyl, oxetan-3-yl, 3-methyl-oxetan-3-yl, tetrahydro-2H-pyran-4-yl, -CH₂-tetrahydro-2H-pyran-4-yl, benzyl, -CH₂CH₂CH₂-phenyl, -CH₂-(6-methyl-pyridin-2-yl), piperidin-4-yl, -CH₂-piperidin-4-yl, -CH₂CH₂CH₂-morpholin-4-yl, -CH₂CH₂-indol-3-yl, 4,5-dimethyl-thiazol-2-yl, (3R)-1-benzyl-pyrrolidin-3-yl-, -CH₂CH₂-pyrrolidin-1-yl, -CH₂-benzimidazol-2-yl, -CH₂CH₂CH₂-imidazol-1-yl, -CH₂CH₂-imidazol-4-yl, (2*S*)-1-hydroxy-3-(1*H*-imidazol-4-yl)prop-2-yl, or 3-[methyl(phenyl)amino]prop-1-yl.

In another specific embodiment, R^{c} is H. In a further embodiment, R^{c} is (C₁-C₄)alkoxy or (C₁-C₄)alkyl. Specifically, R^{c} is -OCH₃or -CH₃, more specifically R^{c} is -CH₃.

In a further embodiment, or R^{b} and R^{c} taken together with the nitrogen atom to which they are attached form a 5-7 membered heterocycloalkyl group, optionally containing one additional ring heteroatom selected from nitrogen, oxygen and sulfur, which 5-7 membered heterocycloalkyl is optionally substituted by 1-3 groups each independently selected from (C₁-C₄)alkyl, (C₁-C₄)haloalkyl, hydroxyl, hydroxy(C₁-C₄)alkyl-, phenyl(C₁-C₄)alkyl-, C₁-C₄ alkoxy, C₁-C₄ haloalkoxy, amino, (C₁-C₄ alkyl)amino-, (C₁-C₄ alkyl)(C₁-C₄ alkyl)amino-, -CO₂H, -CO₂(C₁-C₄)alkyl, -CO(C₁-C₄)alkyl, -CO(4-7 membered heterocycloalkyl), -CONH₂, -CONH(C₁-C₄alkyl), -CON(C₁-C₄alkyl)(C₁-C₄alkyl), -SO₂(C₁-C₄)alkyl, -SO₂(4-7 membered heterocycloalkyl), -SO₂NH₂, -SO₂NH(C₁-C₄alkyl) and -SO₂N(C₁-C₄alkyl)(C₁-C₄alkyl).

In another embodiment, R^{b} and R^{c} taken together with the nitrogen atom to which they are attached form a 4-6-membered heterocycloalkyl, optionally containing 1 or 2 additional heteroatoms each independently selected from N, O and S, and optionally substituted by 1 or 2 groups each independently selected from hydroxyl, (C₁-C₄)alkyl, hydroxy(C₁-C₄)alkyl-, phenyl(C₁-C₄)alkyl- and-CO₁(C₁-C₄)alkyl.

In a further embodiment, R^{b} and R^{c} taken together with the nitrogen atom to which they are attached form a 3-6-membered heterocycloalkyl, optionally containing 1 or 2 additional heteroatoms each independently selected from N, O and S, and optionally substituted by 1 or 2 groups each independently selected from hydroxyl, (C₁-C₄)alkyl, hydroxy(C₁-C₄)alkyl- and-CO₂(C₁-C₄)alkyl;

In a still further embodiment, R^{b} and R^{c} taken together with the nitrogen atom to which they are attached form a 5-6-membered heterocycloalkyl, optionally containing 1 additional heteroatom selected from N, O and S, and optionally substituted by 1 or 2 groups each independently selected from hydroxyl, (C₁-C₄)alkyl, hydroxy(C₁-C₄)alkyl-and-CO₂(C₁-C₄)alkyl;

In another embodiment, R^{b} and R^{c} taken together with the nitrogen atom to which they are attached form a 5-6-membered heteroaryl, optionally containing 1 or 2 additional heteroatoms each independently selected from N, O and S, and optionally substituted by 1 or 2 groups each independently selected from hydroxyl, (C₁-C₄)alkyl, hydroxy(C₁-C₄)alkyl-, phenyl(C₁-C₄)alkyl- and-CO₂(C₁-C₄)alkyl.

Specifically, R^{b} and R^{c} taken together with the nitrogen atom to which they are attached form a morpholin-4-yl, piperidin-1-yl, piperazin-1-yl, 4-methyl-piperazin-1-yl, pyrrolidin-1-yl, 3-hydroxy-pyrrolidin-1-yl, (2S)-2-[(methyloxy)carbonyl]-1-pyrrolidin-1-yl, (3S,6R)-6-methyl-3-[(methyloxy)carbonyl]-piperidin-1-yl, 2-methyl-morpholin-4-yl, 2,2-dimethyl-morpholin-4-yl, 3-methyl-morpholin-4-yl, (3R)-3-methyl-morpholin-4-yl, (3S)-3-methyl-morpholin-4-yl, 2-hydroxymethyl-morpholin-4-yl, (2S,5R)-2-hydroxymethyl-5-methyl-morpholin-4-yl, (2S,5R)-2-hydroxymethyl-5-ethyl-morpholin-4-yl, thiomorpholin-4-yl, or a 1,1dioxoido-thiomorpholin-4-yl group.

In another embodiment, A is phenyl or phenyl(C₁-C₄)alkyl-, wherein any phenyl (including the phenyl moiety of phenyl(C₁-C₄)alkyl-) is substituted by R³, R⁴ and R⁵, wherein:
R³ is H, hydroxyl, halogen, hydroxy(C₁-C₄)alkyl, (C₁-C₄)alkyl or (C₁-C₄)alkoxy;
R⁴ is H, halogen, cyano, (C₁-C₄)alkyl, halo(C₁-C₄)alkyl, (C₁-C₄)alkoxy, phenoxy, phenyl(C₁-C₄)alkoxy, hydroxyl, hydroxy(C₁-C₄)alkyl- or aminocarbonyl, wherein the phenyl moiety of said phenoxy or phenyl(C₁-C₄)alkoxy- is optionally substituted by 1-3 substituents each independently selected from halogen, -CF₃, (C₁-C₄)alkyl and (C₁-C₄)alkoxy; and
R⁵ is H, hydroxyl, halogen, hydroxy(C₁-C₄)alkyl, (C₁-C₄)alkyl or (C₁-C₄)alkoxy.

Specifically, A is phenyl, unsubstituted or substituted by 1, 2 or 3 substituents each independently selected from hydroxyl, halogen, -CF₃, hydroxy(C₁-C₄)alkyl, (C₁-C₄)alkyl and (C₁-C₄)alkoxy, or A is phenyl, substituted by phenoxy or benzyloxy, wherein the phenyl moiety of said phenoxy or benzyloxy- is optionally substituted by 1-3 substituents each independently selected from halogen, -CF₃, (C₁-C₄)alkyl and (C₁-C₄)alkoxy.

In other specific embodiments, A is phenyl, 2-hydroxy-5-chloro-phenyl, 2-hydroxy-5-fluoro-phenyl, 2-hydroxy-4-fluoro-phenyl, 3-hydroxy-4-fluoro-phenyl, 3-hydroxy-4-chloro-phenyl, 2-methyl-4-hydroxy-phenyl, 2-fluoro-4-hydroxy-phenyl, 2-fluoro-5-hydroxy-phenyl, 3-fluoro-4-hydroxy-phenyl, 2-methyl-6-hydroxy-phenyl, 3-methyl-5-hydroxy-phenyl, 2-chloro-5-hydroxy-phenyl, 2-chloro-4-hydroxy-phenyl, 3-chloro-4-hydroxy-phenyl, 4-hydroxy-phenyl, 2-fluoro-phenyl, 4-fluoro-phenyl, 3-chloro-phenyl, 4-chloro-phenyl, 2,5-difluoro-phenyl, 2,6-difluoro-phenyl, 3,5-difluoro-phenyl, 3,4,5-trifluoro-phenyl, 2,4-dichloro-phenyl, 3,4,5-trichloro-phenyl, 2,4-dimethyl-phenyl, 2-methyl-5-fluoro-phenyl, 2-chloro-3-hydroxy-4-methyl-phenyl, 2-methyl-phenyl, 3-methoxy-phenyl, 2-methyl-3-hydroxy-phenyl, 2-methyl-5-hydroxy-phenyl, 3-methoxy-4-methyl-phenyl, 2,4-dimethyl-5-hydroxy-phenyl, 2,4-dimethyl-5-methoxy-phenyl, 3-hydroxy-4-methyl-phenyl, 2-chloro-5-methoxy-phenyl, 3-methoxy-4-fluoro-phenyl, 3-methoxy-4-chloro-phenyl, 3-methoxy-4-bromo-phenyl, 3-hydroxymethyl-phenyl, 2-methyl-5-hydroxymethyl-phenyl, 4-phenoxy-phenyl or 4-[(2-chlorophenyl)methoxy]phenyl.

In another embodiment, the invention is directed to method of inhibiting RIP2 kinase comprising contacting the kinase with a compound according to Formula (II): 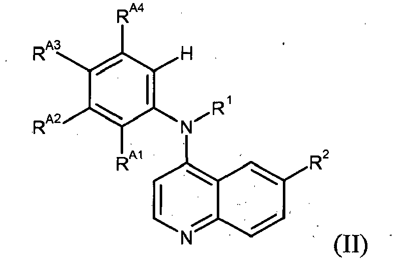 wherein:
R¹ is H, -SO₂(C₁-C₄alkyl) or -CO(C₁-C₄alkyl); particularly, R¹ is H;
R² is -SR^{a}, -SOR^{a}, -SO₂R^{a} or -SO₂NR^{b}R^{c}, wherein R^{b} and R^{c} are as defined herein;
R^{A1} is H, halogen, -CF₃, (C₁-C₄)alkyl or (C₁-C₄)alkoxy;
R^{A2} is H, halogen, -CF₃, (C₁-C₄)alkyl or (C₁-C₄)alkoxy; particularly, R^{A2} is H;
R^{A3} is H, halogen, cyano, (C₁-C₄)alkyl, halo(C₁-C₄)alkyl, (C₁-C₄)alkoxy, phenoxy, phenyl(C₁-C₄)alkoxy, hydroxyl, hydroxy(C₁-C₄)alkyl-, or aminocarbonyl, wherein the phenyl moiety of said phenoxy or phenyl(C₁-C₄)alkoxy- is optionally substituted by 1-3 substituents each independently selected from halogen, -CF₃, (C₁-C₄)alkyl and (C₁-C₄)alkoxy; and
R^{A4} is hydroxyl or hydroxy(C₁-C₄)alkyl;
or a salt, particularly a pharmaceutically acceptable salt, thereof.

In a further embodiment, the invention is directed to method of treating a RIP2 kinase-mediated disease in a human comprising administering a therapeutically effective amount of a compound according to Formula (II), or a pharmaceutically acceptable salt thereof, to said human.

In a still further embodiment, the invention is directed to a compound of Formula (II), or a salt, particularly a pharmaceutically acceptable salt, thereof, provided that the compound is not:
2-fluoro-5[[6-(methylsulfonyl)-4-quinolinyl]amino-1,4-benzenediol;
N-(cyclopropylmethyl)-4-[(5-hydroxy-2-methylphenyl)amino]-6-quinolinesulfonamide;
4-methyl-3-[[6-(methylsulfonyl)-4-quinolinyl]amino]-phenol;
4-chloro-2-fluoro-5-[[6-(methylsulfonyl)-4-quinolinyl]amino]-phenol; or
4-[(5-hydroxy-2-methylphenyl)amino]-N-(1-methylethyl)-6-quinolinecarboxamide

In yet another embodiment, A is phenyl, substituted by R⁶, R⁷ and R⁸, wherein R⁶ and R⁷ are located on adjacent atoms and taken together with the atoms to which they are attached form a 5-membered heterocyclic group containing 1, 2 or 3 heteroatoms each independently selected from N, O and S, which 5-membered heterocyclic group is substituted by R⁹;
wherein one of R⁸ or R⁹ is H, halogen, cyano, (C₁-C₄)alkyl, -CF₃, (C₁-C₄)alkoxy, phenoxy, phenyl(C₁-C₄)alkoxy, hydroxyl, hydroxy(C₁-C₄)alkyl-, or aminocarbonyl, where the phenyl moiety of said phenoxy or phenyl(C₁-C₄)alkoxy is optionally substituted by 1-3 substituents each independently selected from halogen, -CF₃, (C₁-C₄)alkyl and (C₁-C₄)alkoxy; and
the other of R⁸ or R⁹ is H, hydroxyl, halogen, -CF₃, hydroxy(C₁-C₄)alkyl, (C₁-C₄)alkyl or (C₁-C₄)alkoxy.

Specifically, A is phenyl, substituted on adjacent carbon atoms by R^{A6} and R^{A7}, wherein R^{A6} and R^{A7}, taken together with the phenyl to which they are attached form a 9-membered bicyclic heteroaryl, wherein said heteroaryl is an optionally substituted indolyl, indazolyl, 1*H*-1,2,3-benzotriazolyl, 1,2,3-benzothiadiazolyl, 2,1,3-benzoxadiazolyl, 1,3-benzodioxolyl, 1,3-benzoxathiol-2-on-yl, benzofuranyl, benzothiazolyl, benzoxazolyl, benzoisoxazolyl or benzoisothiazolyl, optionally substituted by hydroxyl, halogen, -CF₃, cyario, hydroxy(C₁-C₄)alkyl, (C₁-C₄)alkyl, (C₁-C₄)alkoxy or aminocarbonyl.

In a still further embodiment, A is pyrazolyl, substituted by R¹⁰ and R¹¹ wherein:
R¹⁰ and R¹¹ are located on adjacent carbon atoms and taken together with the atoms to which they are attached form a 6 membered carbocyclic ring or heterocyclic ring substituted by R¹² and R¹³;
wherein R¹² is H, halogen, cyano, (C₁-C₄)alkyl, -CF₃, (C₁-C₄)alkoxy, phenoxy, phenyl(C₁-C₄)alkoxy, hydroxyl, hydroxy(C₁-C₄)alkyl-, or aminocarbonyl, wherein the phenyl moiety of said phenoxy or phenyl(C₁-C₄)alkoxy is optionally substituted by 1-3 substituents each independently selected from halogen, -CF₃, (C₁-C₄)alkyl and (C₁-C₄)alkoxy; and
R¹³ is H, hydroxyl, halogen, -CF₃, hydroxy(C₁-C₄)alkyl, (C₁-C₄)alkyl or (C₁-C₄)alkoxy.

In one embodiment, A is an optionally substituted indazolyl, pyrazolopyridinyl or thiazolopyridinyl, optionally substituted by one substituent selected from halogen, cyano, (C₁-C₄)alkyl, halo(C₁-C₄)alkyl (specifically, -CF₃), (C₁-C₄)alkoxy, hydroxyl, hydroxy(C₁-C₄)alkyl-, or aminocarbonyl, and is further optionally substituted by a second substituent selected from hydroxyl, halogen, -CF₃, (C₁-C₄)alkyl and (C₁-C₄)alkoxy.

In another embodiment, A is an optionally substituted indazolyl, optionally substituted by halogen, cyano, (C₁-C₄)alkyl, halo(C₁-C₄)alkyl, (C₁-C₄)alkoxy, or aminocarbonyl or A is an optionally substituted 1*H*-pyrazolo[3,4-*b*]pyridinyl or [1,3]thiazolo[5,4-*b*]pyridinyl, optionally substituted by one or two substituents each independently selected from halogen, (C₁-C₄)alkyl, halo(C₁-C₄)alkyl (specifically, -CF₃), and (C₁-C₄)alkoxy.

In a further embodiment, A is an optionally substituted indazolyl, 1*H*-pyrazolo[3,4-*b*]pyridinyl or [1,3]thiazolo[5,4-*b*]pyridinyl, optionally substituted by one or two substituents each independently selected from halogen, (C₁-C₄)alkyl, halo(C₁-C₄)alkyl, and (C₁-C₄)alkoxy.

More specifically, A is an optionally substituted indol-6-yl, indazol-3-yl, indazol-6-yl, 1*H*-1,2,3-benzotriazol-4-yl, 1*H*-1,2,3-benzotriazol-5-yl, 1,2,3-benzothiadiazol-5-yl, 2,1,3-benzoxadiazol-5-yl, 1,3-benzodioxol-4-yl, 1,3-benzodioxol-4-yl, 1,3-benzoxathiol-2-on-5-yl, benzofuran-4-yl, benzothiazol-4-yl, benzothiazol-5-yl, benzothiazol-6-yl, benzoxazol-5-yl, or 1,2-benzoisoxazol-6-yl, optionally substituted by hydroxyl, bromo, chloro, fluoro, -CF₃, cyano, hydroxymethyl-, methyl, methoxy or aminocarbonyl or A is an optionally substituted 1*H*-pyrazolo[3,4-*b*]pyridin-3-yl or [1,3]thiazolo[5,4-*b*]pyridin-6-yl, optionally substituted by one or two substituents each independently selected from fluoro and methyl.

In other embodiments, A is indol-6-yl, indazol-3-yl, indazol-6-yl, 5-methoxy-indazol-3-yl, 5-fluoro-indazol-3-yl, 4-chloro-indazol-3-yl, 5-chloro-indazol-3-yl, 6-chloro-indazol-3-yl, 7-trifluoromethyl-indazol-3-yl, 7-chloro-indazol-3-yl, 1-methyl-indazol-3-yl, 5-cyano-indazol-6-yl, 7-methyl-indazol-6-yl, 5-aminocarbonyl-indazol-6-yl, 3-fluoro-indazol-6-yl, 3-methyl-indazol-6-yl, 1*H*-1,2,3-benzotriazol-4-yl, 1*H*-1,2,3-benzotriazol-5-yl, 4-methyl-1*H*-1,2,3-benzotriazol-6-yl, 5-methyl-1*H*-1,2,3-benzotriazol-6-yl, 5-fluoro-1*H*-1,2,3-benzotriazol-6-yl, 1,2,3-benzothiadiazol-5-yl, 2,1,3-benzoxadiazol-5-yl, 1,3-benzodioxol-4-yl, 5-chloro-1,3-benzodioxol-4-yl, 2-oxo-1,3-benzoxathiol-5-yl, benzofuran-4-yl, benzothiazol-5-yl, benzothiazol-6-yl, 6-methyl-benzothiazol-5-yl, 6-fluorobenzothiazol-5-yl, 4-methyl-benzothiazol-5-yl, 4-fluoro-benzothiazol-5-yl, 4-chloro-benzothiazol-5-yl, 4-bromo-benzothiazol-5-yl, benzoxazol-5-yl, 1,2-benzoisoxazol-6-yl, 2,1-benzisothiazol-6-yl, 5-fluoro-1*H*-pyrazolo[3,4-*b*]pyridine-3-yl, 5-methyl-1*H*-pyrazolo[3,4-*b*]pyridine-3-yl, 5-fluoro-6-methyl-1*H*-pyrazolo[3,4-*b*]pyridine-3-yl, or [1,3]thiazolo[5,4-*b*]pyridin-6-yl.

In another embodiment, the invention is directed to a compound according to Formula (III): 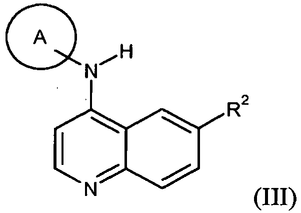 wherein A is a 9-membered bi-cyclic heteroaryl group, optionally by one substituent selected from halogen, cyano, (C₁-C₄)alkyl, hato(C₁-C₄)alkyl (specifically, - CF₃), (C₁-C₄)alkoxy, hydroxyl, hydroxy(C₁-C₄)alkyl-, or aminocarbonyl, and further optionally substituted by a second substituent selected from, halogen, (C₁-C₄)alkyl and (C₁-C₄)alkoxy;
said 9-membered bi-cyclic heteroaryl group is an optionally substituted indazolyl bonded to the amino (NH) moiety via any substitutable carbon ring atom of the indazolyl group, or
said 9-membered bi-cyclic heteroaryl group is an optionally substituted indolyl, 1H-1,2,3-benzotriazolyl, 1,2,3-benzothiadiazolyl, 2,1,3-benzoxadiazolyl, 1,3-benzodioxolyl, benzofuranyl, benzothiazolyl, benzoxazolyl, 1H-pyrazolo[3,4-*b*]pyridinyl, or [1,3]thiazolo[5,4-*b*]pyridinyl, bonded to the amino (NH) moiety via any substitutable carbon ring atom of the 6-membered ring moiety of said indolyl, 1H-1,2,3-benzotriazolyl, 1,2,3-benzothiadiazolyl, 2,1,3-benzoxadiazolyl, 1,3-benzodioxolyl, benzofuranyl, benzothiazolyl, benzoxazolyl, 1*H*-pyrazolo[3,4-*b*]pyridinyl, or [1,3]thiazolo[5,4-*b*]pyridinyl group;
or a salt, particularly a pharmaceutically acceptable salt, thereof.

In a further embodiment, A is an optionally substituted indol-6-yl, indazol-3-yl, indazol-6-yl, 1*H*-pyrazolo[3,4-*b*]pyridine-3-yl, 1*H*-1,2,3-benzotriazol-4-yl, 1*H*-1,2,3-benzotriazol-5-yl, 1,2,3-benzothiadiazol-5-yl, 2,1,3-benzoxadiazol-5-yl, 1,3-benzodioxol-4-yl, benzofuran-4-yl, benzothiazol-4-yl, benzothiazol-5-yl, [1,3]thiazolo[5,4-*b*]pyridin-6-yl, benzothiazol-6-yl or benzoxazol-5-yl, optionally substituted by one group selected from hydroxyl, chloro, bromo, fluoro, -CF₃, cyano, hydroxymethyl-, methyl, methoxy and aminocarbonyl and further optionally substituted by a second group selected from chloro, fluoro, and methyl.

In another embodiment, A is an optionally substituted indol-6-yl, indazol-3-yl, indazol-6-yl, 1*H*-1,2,3-benzotriazol-4-yl, 1*H*-1,2,3-benzotriazol-5-yl, 1,2,3-benzothiadiazol-5-yl, 2,1,3-benzoxadiazol-5-yl, 1,3-benzodioxol-4-yl, benzofuran-4-yl, benzothiazol-4-yl, benzothiazol-5-yl, benzothiazol-6-yl or benzoxazol-5-yl, optionally substituted by hydroxyl, chloro, fluoro, -CF₃, cyano, hydroxymethyl-, methyl, methoxy or aminocarbonyl.

Accordingly, a compound of this invention includes a compound of Formula (I), (II) or (III), or a salt thereof, particularly a pharmaceutically acceptable salt thereof.

In another embodiment, the invention is directed to method of inhibiting RIP2 kinase comprising contacting the kinase with a compound according to Formula (III), or a salt, particularly a pharmaceutically acceptable salt, thereof.

In a further embodiment, the invention is directed to method of treating a RIP2 kinase-mediated disease or condition in a human comprising administering a therapeutically effective amount of a compound according to Formula (III), or a pharmaceutically acceptable salt thereof, to said human.

The invention is further directed to a compound of Formula (I), wherein:
R¹ is H, -SO₂CH₃ or -COCH₃;
R² is -SR^{a}, -SOR^{a}, -SO₂R^{a}, -SO₂NH₂, -SO₂NR^{b}R^{c} or -CONR^{b}R^{c}, wherein:
R^{a} is (C₁-C₆)alkyl, halo(C₁-C₄)alkyl, (C₂-C₆)alkenyl, (C₃-C₇)cycloalkyl, 4-7 membered heterocycloalkyl, aryl, or heteroaryl, wherein:
said (C₁-C₆)alkyl is optionally substituted by 1 or 2 substituents each independently selected from hydroxyl, (C₁-C₄)alkoxy, (C₁-C₄)alkoxy(C₂-C₄)alkoxy-, amino, (C₁-C₄ alkyl)amino-, (C₁-C₄ alkyl)(C₁-C₄ alkyl)amino-, (phenyl)(C₁-C₄ alkyl)amino-, -CO₂(C₁-C₄)alkyl, -CONH₂, -SO₂(C₁-C₄)alkyl, and a C₃-C₆cycloalkyl, phenyl, 4-6-membered heterocycloalkyl, 5-6-membered heteroaryl, or 9-10-membered heteroaryl, where said C₃-C₆cycloalkyl, phenyl, 4-6-membered heterocycloalkyl, 5-6-membered heteroaryl, or 9-10-membered heteroaryl is optionally substituted by 1-3 groups each independently selected from halogen, -CF₃, hydroxyl, amino, (C₁-C₄)alkyl, phenyl(C₁-C₄)alkyl-, hydroxy(C₁-C₄)alkyl- and (C₁-C₄)alkoxy;
wherein said C₃-C₆cycloalkyl, 4-6-membered heterocycloalkyl, 5-6-membered heteroaryl or phenyl is optionally substituted by 1-3 groups each independently selected from halogen, -CF₃, hydroxyl, amino, (C₁-C₄)alkyl, phenyl(C₁-C₄)alkyl-, (C₁-C₄)alkoxycarbonyl-, hydroxy(C₁-C₄)alkyl-, oxo and (C₁-C₄)alkoxy;
R^{c} is H or (C₁-C₄)alkyl;
or R^{b} and R^{c} taken together with the nitrogen atom to which they are attached form a 5-6-membered heterocycloalkyl, optionally containing 1 additional heteroatom selected from N, O and S, and optionally substituted by 1 or 2 groups each independently selected from hydroxyl, (C₁-C₄)alkyl, hydroxy(C₁-C₄)alkyl- and-CO₂(C₁-C₄)alkyl;

A is phenyl, substituted by 1, 2 or 3 substituents each independently selected from hydroxyl, halogen, -CF₃, hydroxy(C₁-C₄)alkyl, (C₁-C₄)alkyl and (C₁-C₄)alkoxy, or
A is phenyl, substituted by phenoxy, or
A is phenyl, substituted on adjacent carbon atoms by R^{A6} and R^{A7}, wherein R^{A6} and R^{A7}, taken together with the phenyl to which they are attached form a 9-membered bicyclic heteroaryl, wherein said heteroaryl is an optionally substituted indolyl, indazolyl, 1*H*-1,2,3-benzotriazolyl, 1,2,3-benzothiadiazolyl, 2,1,3-benzoxadiazolyl, 1,3-benzodioxolyl, benzofuranyl, benzothiazolyl, benzoxazolyl, 1*H*-pyrazolo[3,4-*b*]pyridine-3-yl or [1,3]thiazolo[5,4-*b*]pyridin-6-yl, optionally substituted by one or two substituents each independently selected from hydroxyl, halogen, -CF₃, cyano, hydroxy(C₁-C₄)alkyl, (C₁-C₄)alkyl, (C₁-C₄)alkoxy and aminocarbonyl; or
A is indazolyl, optionally substituted by halogen, cyano, (C₁-C₄)alkyl, halo(C₁-C₄)alkyl, (C₁-C₄)alkoxy, or aminocarbonyl; or
A is 1*H*-pyrazolo[3,4-*b*]pyridinyl or [1,3]thiazolo[5,4-*b*]pyridinyl, optionally substituted by one or two substituents each independently selected from halogen, (C₁-C₄)alkyl, halo(C₁-C₄)alkyl, and (C₁-C₄)alkoxy;
or a salt, particularly a pharmaceutically acceptable salt, thereof.

In another embodiment, the invention is directed to a compound of Formula (I) wherein:
R¹ is H;
R² is -CONR^{b}R^{c}, -SR^{a}, -SOR^{a}, -SO₂R^{a}, -SO₂NH₂ or -SO₂NR^{b}R^{c},
R^{a} is -CH₃, -CF₃, -CH₂CH₃, -CH₂CF₃, -CH₂CH₂CH₃, -CH₂CH=CH₂, -CH(CH₃)₂, -CH(CH₃)CH₂CH₃, -C(CH₃)₃, -CH₂CH₂OH, -CH₂CH₂OCH₃, -CH₂CH₂CH₂OH, -CH₂CH₂CH₂CH₂OH, -CH(CH₃)CH₂OH, -C(CH₃)₂CH₂OH, -C(CH₃)₂CO₂H, -C(CH₃)₂CH₂CH₂OH, -C(CH₃)₂CH₂CH₂OCH₃, -CH₂CH₂NH₂, -CH₂CH₂N(CH₂CH₃)₂, -C(CH₃)₂CO₂CH₃, [1-(2-hydroxyethyl)cyclopropyl]methyl-, cyclopropyl, cyclopentyl, cyclohexyl, tetrahydro-2*H*-pyran-4-yl, tetrahydro-2*H*-pyran-3-yl, tetrahydrofuran-3-yl, 2-methyl-tetrahydrofuran-3-yl, -CH₂- tetrahydrofuran-2-yl, 1*H*-imidazol-4-yl, 1*H*-1,2,4-triazol-3-yl, phenyl, 4-amino-phenyl-, pyridin-4-yl, piperidin-4-yl, 1-methyl-piperidin-4-yl-, 1-((CH₃)₃C-O-CO-piperidin-4-yl-, -CH₂CH₂-morpholin-4-yl, pyrimidin-2-yl, -CH₂CH₂-(3,5-dimethyl-isoxazol-4-yl), tetrahydro-2*H*-thiopyran-4-yl, or 1,1-dioxidotetrahydro-2*H*-thiopyran-4-yl;
R^{b} is -CH₃, -CH(CH₃)₂, -CH₂CH₂OH, -CH₂CH₂OCH₃, -CH₂CH₂OCH₂CH₂OCH₃, -CH₂CH₂N(CH₃)₂, -CH₂CH₂SO₂CH₃, -CH₂CONH₂, -CH₂CH₂CONH₂, cyclohexyl, oxetan-3-yl, 3-methyl-oxetan-3-yl, tetrahydro-2H-pyran-4-yl, -CH₂-tetrahydro-2H-pyran-4-yl, benzyl, -CH₂CH₂CH₂-phenyl, -CH₂-(6-methyl-pyridin-2-yl), piperidin-4-yl, -CH₂-piperidin-4-yl, -CH₂CH₂CH₂-morpholin-4-yl, -CH₂CH₂-indol-3-yl, 4,5-dimethyl-thiazol-2-yl, (3R)-1-benzyl-pyrrolidin-3-yl-, -CH₂CH₂-pyrrolidin-1-yl, -CH₂-benzimidazol-2-yl, -CH₂CH₂CH₂-imidazol-1-yl, -CH₂CH₂-imidazol-4-yl, (2*S*)-1-hydroxy-3-(1*H*-imidazol-4-yl)prop-2-yl, or 3-[methyl(phenyl)amino]prop-1-yl;
R^{c} is H or -CH₃;
or R^{b} and R^{c} taken together with the nitrogen atom to which they are attached form a morpholin-4-yl, 2-methyl-morpholin-4-yl, 2,2-dimethyl-morpholin-4-yl, piperidin-1-yl, piperazin-1-yl, 4-methyl-piperazin-1-yl, pyrrolidin-1-yl, 3-hydroxy-pyrrolidin-1-yl, (2S)-2-[(methyloxy)carbonyl]-1-pyrrolidin-1-yl, (3S,6R)-6-methyl-3-[(methyloxy)carbonyl]-piperidin-1-yl, 3-methyl-morpholin-4-yl, (3R)-3-methyl-morpholin-4-yl, (3S)-3-methyl-morpholin-4-yl, 2-hydroxymethyl-morpholin-4-yl, (2S,5R)-2-hydroxymethyl-5-methyl-morpholin-4-yl, (2S,5R)-2-hydroxymethyl-5-ethyl-morpholin-4-yl, thiomorpholin-4-yl, or a 1,1 dioxoido-thiomorpholin-4-yl group;
A is phenyl, 2-hydroxy-5-chloro-phenyl, 2-hydroxy-5-fluoro-phenyl, 2-hydroxy-4-fluoro-phenyl, 3-hydroxy-4-fluoro-phenyl, 3-hydroxy-4-chloro-phenyl, 2-methyl-4-hydroxy-phenyl, 2-fluoro-4-hydroxy-phenyl, 2-fluoro-5-hydroxy-phenyl, 3-fluoro-4-hydroxy-phenyl, 2-methyl-6-hydroxy-phenyl, 3-methyl-5-hydroxy-phenyl, 2-chloro-5-hydroxy-phenyl, 2-chloro-4-hydroxy-phenyl, 3-chloro-4-hydroxy-phenyl, 4-hydroxy-phenyl, 2-fluoro-phenyl, 4-fluoro-phenyl, 3-chloro-phenyl, 4-chloro-phenyl, 2,5-difluoro-phenyl, 2,6-difluoro-phenyl, 3,5-difluoro-phenyl, 3,4,5-trifluoro-phenyl, 2,4-dichloro-phenyl, 3,4,5-trichloro-phenyl, 2,4-dimethyl-phenyl, 2-methyl-5-fluoro-phenyl, 2-chloro-3-hydroxy-4-methyl-phenyl, 2-methyl-phenyl, 3-methoxy-phenyl, 2-methyl-3-hydroxy-phenyl, 2-methyl-5-hydroxy-phenyl, 3-methoxy-4-methyl-phenyl, 2,4-dimethyl-5-hydroxy-phenyl, 2,4-dimethyl-5-methoxy-phenyl, 3-hydroxy-4-methyl-phenyl, 2-chloro-5-methoxy-phenyl, 3-methoxy-4-fluoro-phenyl, 3-methoxy-4-chloro-phenyl, 3-methoxy-4-bromo-phenyl, 3-hydroxymethyl-phenyl, 2-methyl-5-hydroxymethyl-phenyl, 4-phenoxy-phenyl, 4-[(2-chlorophenyl)methoxy]phenyl, indol-6-yl, indazol-3-yl, indazol-6-yl, 1-methyl-indazol-3-yl, 5-methoxy-indazol-3-yl, 5-methyl-indazol-3-yl, 5-fluoro-indazol-3-yl, 4-chloro-iridazol-3-yl, 5-chloro-indazol-3-yl, 6-chloro-indazol-3-yl, 7-chloro-indazol-3-yl, 7-trifluoromethyl-indazol-3-yl, 5-cyano-indazol-6-yl, 7-methyl-indazol-6-yl, 5-aminocarbonyl-indazol-6-yl, 3-fluoro-indazol-6-yl, 3-methyl-indazol-6-yl, 1*H*-1,2,3-benzotriazol-4-yl, 1*H*-1,2,3-benzotriazol-5-yl, 4-methyl-1*H*-1,2,3-benzotriazol-6-yl, 5-methyl-1*H*-1,2,3-benzotriazol-6-yl, 5-fluoro-1*H*-1,2,3-benzotriazol-6-yl, 1,2,3-benzothiadiazol-5-yl, 2,1,3-benzoxadiazol-5-yl, 1,3-benzodioxol-4-yl, 5-chloro-1,3-benzodioxol-4-yl, benzofuran-4-yl, benzothiazol-5-yl, 4-methyl-benzothiazol-5-yl, 4-bromo-benzothiazol-5-yl, 4-chloro-benzothiazol-5-yl, benzothiazol-6-yl, benzoxazol-5-yl, 5-fluoro-1*H*-pyrazolo[3,4-*b*]pyridin-3-yl, 5-fluoro-6-methyl-1*H*-pyrazolo[3,4-*b*]pyridin-3-yl or [1,3]thiazolo[5,4-*b*]pyridin-6-yl;
or a salt, particularly a pharmaceutically acceptable salt, thereof.

As used herein, the term "alkyl" represents a saturated, straight or branched hydrocarbon moiety, which may be unsubstituted or substituted by one, or more of the substituents defined herein. Exemplary alkyls include, but are not limited to methyl (Me), ethyl (Et), propyl, isopropyl, butyl, isobutyl, t-butyl and pentyl. The term "C₁-C₄ alkyl" refers to an alkyl group or moiety containing from 1 to 4 carbon atoms.

When the term "alkyl" is used in combination with other substituent groups, such as "haloalkyl" or "hydroxyalkyl" or "arylalkyl", the term "alkyl" is intended to encompass a divalent straight or branched-chain hydrocarbon radical. For example, "arylalkyl" is intended to mean the radical -alkylaryl, wherein the alkyl moiety thereof is a divalent straight or branched-chain carbon radical and the aryl moiety thereof is as defined herein, and is represented by the bonding arrangement present in a benzyl group (-CH₂-phenyl); "halo(C₁-C₄)alkyl" is intended to mean a radical having one or more halogen atoms, which may be the same or different, at one or more carbon atoms of an alkyl moiety containing from 1 to 4 carbon atoms, which a is straight or branched-chain carbon radical, and is represented by a trifluoromethyl group (-CF₃).

As used herein, the term "cycloalkyl" refers to a non-aromatic, saturated, cyclic hydrocarbon ring. The term "(C₃-C₈)cycloalkyl" refers to a non-aromatic cyclic hydrocarbon ring having from three to eight ring carbon atoms. Exemplary "(C₃-C₈)cycloalkyl" groups useful in the present invention include cyclopropyl, cyclobutyl, cyclopentyl, cyclohexyl, cycloheptyl, and cyclooctyl.

"Alkoxy" refers to a group containing an alkyl radical attached through an oxygen linking atom. The term "(C₁-C₄)alkoxy" refers to a straight- or branched-chain hydrocarbon radical having at least 1 and up to 4 carbon atoms attached through an oxygen linking atom. Exemplary "(C₁-C₄)alkoxy" groups useful in the present invention include, but are not limited to, methoxy, ethoxy, *n*-propoxy, isopropoxy, *n*-butoxy, *s*-butoxy, and *t*-butoxy.

"Aryl" represents a group or moiety comprising an aromatic, monovalent monocyclic or bicyclic hydrocarbon radical containing from 6 to 10 carbon ring atoms, which may be unsubstituted or substituted by one or more of the substituents defined herein, and to which may be fused one or more cycloalkyl rings, which may be unsubstituted or substituted by one or more substituents defined herein.

Generally, in the compounds of this invention, aryl is phenyl.

Heterocyclic groups may be heteroaryl or heterocycloalkyl groups.

"Heterocycloalkyl" represents a group or moiety comprising a non-aromatic, monovalent monocyclic or bicyclic radical, which is saturated or partially unsaturated, containing 3 to 10 ring atoms, unless otherwise specified, which includes 1 to 4 heteroatoms selected from nitrogen, oxygen and sulfur, and which may be unsubstituted or substituted by one or more of the substituents defined herein. Illustrative examples of heterocycloalkyls include, but are not limited to, azetidinyl, oxetanyl, pyrrolidyl (or pyrrolidinyl), piperidinyl, piperazinyl, morpholinyl, tetrahydro-2H-1,4-thiazinyl, tetrahydrofuryl (or tetrahydrofuranyl), dihydrofuryl, oxazolinyl, thiazolinyl, pyrazolinyl, tetrahydropyranyl, dihydropyranyl, 1,3-dioxolanyl, 1,3-dioxanyl, 1,4-dioxanyl, 1,3-oxathiolanyl, 1,3-oxathianyl, 1,3-dithianyl, azabicylo[3.2.1]octyl, azabicylo[3.3.1]nonyl, azabicylo[4.3.0]nonyl, oxabicylo[2.2.1]heptyl and 1,5,9-triazacyclododecyl.

In some of the compounds of this invention, heterocycloalkyl groups include 4-membered heterocycloalkyl groups containing one heteroatom, such as oxetanyl, thietanyl and azetidinyl.

In other compounds of this invention, heterocycloalkyl groups include 5-membered heterocycloalkyl groups containing one heteroatom selected from nitrogen, oxygen and sulfur and optionally containing one or two an additional nitrogen atoms, or optionally containing one additional oxygen or sulfur atom, such as pyrrolidyl (or pyrrolidinyl), tetrahydrofuryl (or tetrahydrofuranyl), tetrahydrothienyl, dihydrofuryl, oxazolinyl, thiazolinyl, imidazolinyl, pyrazolinyl, 1,3-dioxolanyl, and 1,3-oxathiolan-2-on-yl.

In other compounds of this invention, heterocycloalkyl groups are 6-membered heterocycloalkyl groups containing one heteroatom selected from nitrogen, oxygen and sulfur and optionally containing one or two an additional nitrogen atoms, such as piperidyl (or piperidinyl), piperazinyl, morpholinyl, thiomorpholinyl, 1,1dioxoidothiomorpholin-4-yl, tetrahydropyranyl, dihydropyranyl, tetrahydro-2H-1,4-thiazinyl, 1,4-dioxanyl, 1,3-oxathianyl, and 1,3-dithianyl.

"Heteroaryl" represents a group or moiety comprising an aromatic monovalent monocyclic or bicyclic radical, containing 5 to 10 ring atoms, including 1 to 4 heteroatoms selected from nitrogen, oxygen and sulfur, which may be unsubstituted or substituted by one or more of the substituents defined herein. This term also encompasses bicyclic heterocyclic-aryl compounds containing an aryl ring moiety fused to a heterocycloalkyl ring moiety, containing 5 to 10 ring atoms, including 1 to 4 heteroatoms selected from nitrogen, oxygen and sulfur, which may be unsubstituted or substituted by one or more of the substituents defined herein. Illustrative examples of heteroaryls include, but are not limited to, thienyl, pyrrolyl, imidazolyl, pyrazolyl, furyl (or furanyl), isothiazolyl, furazanyl, isoxazolyl, oxazolyl, oxadiazolyl, thiazolyl, pyridyl (or pyridinyl), pyrazinyl, pyrimidinyl, pyridazinyl, triazinyl, tetrazinyl, triazolyl, tetrazolyl, benzo[b]thienyl, isobenzofuryl, 2,3-dihydrobenzofuryl, chromenyl, chromanyl, indolizinyl, isoindolyl, indolyl, indazolyl, purinyl, isoquinolyl, quinolyl, phthalazinyl, naphthridinyl, quinzolinyl, benzothiazolyl, benzimidazolyl, tetrahydroquinolinyl, cinnolinyl, pteridinyl, isothiazolyl.

In some embodiments, the heteroaryl groups present in the compounds of this invention are 5-membered and/or 6-memebred monocyclic heteroaryl groups. Selected 5-membered heteroaryl groups contain one nitrogen, oxygen or sulfur ring heteroatom, and optionally contain 1, 2 or 3 additional nitrogen ring atoms. Selected 6-membered heteroaryl groups contain 1, 2, 3 or 4 nitrogen ring heteroatoms. Selected 5- or 6-membered heteroaryl groups include thienyl, pyrrolyl, imidazolyl, pyrazolyl, furyl (furanyl), isothiazolyl, furazanyl, isoxazolyl, oxazolyl, oxadiazolyl, thiazolyl, triazolyl and tetrazolyl or pyridyl, pyrazinyl, pyrimidinyl, pyridazinyl and triazinyl.

In other embodiments, the heteroaryl groups present in the compounds of this invention are 9-membered or 10-membered monocyclic heteroaryl groups. Selected 9-10 membered heteroaryl groups contain one nitrogen, oxygen or sulfur ring heteroatom, and optionally contain 1, 2, 3 or 4 additional nitrogen ring atoms.

In some of the compounds of this invention, heteroaryl groups include 9-membered heteroaryl groups include benzothienyl, benzofuranyl, indolyl, indolinyl, isoindolyl, isoindolinyl, indazolyl, indolizinyl, isobenzofuryl, 2,3-dihydrobenzofuryl, benzoxazolyl, benzthiazolyl, benzimidazolyl, benzoxadiazolyl, benzthiadiazolyl, benzotriazolyl, 1,3-benzoxathiol-2-on-yl (2-oxo-1,3-benzoxathiolyl), purinyl and imidazopyridinyl.

In some of the compounds of this invention, heteroaryl groups include 10-membered heteroaryl groups include chromenyl, chromanyl, quinolyl, isoquinolyl, phthalazinyl, naphthridinyl, quinazolinyl, quinoxalinyl, 4H-quinolizinyl, tetrahydroquinolinyl, cinnolinyl, and pteridinyl.

It is to be understood that the terms heterocycle, heterocyclic, heteroaryl, heterocycloalkyl, are intended to encompass stable heterocyclic groups where a ring nitrogen heteroatom is optionally oxidized (e.g., heterocyclic groups containing an N-oxide, such as pyridine-N-oxide) or where a ring sulfur heteroatom is optionally oxidized (e.g., heterocyclic groups containing sulfones or sulfoxide moieties, such as tetrahydrothienyl-1-oxide (a tetramethylene sulfoxide) or tetrahydrothienyl-1,1-dioxide (a tetramethylene sulfone)).

"Oxo" represents a double-bonded oxygen moiety; for example, if attached directly to a carbon atom forms a carbonyl moiety (C=O). The terms "halogen" and "halo" represent chloro, fluoro, bromo or iodo substituents. "Hydroxy" or "hydroxyl" is intended to mean the radical -OH.

As used herein, the term "compound(s) of the invention" means a compound of Formulas (I), (II) or (III), as defined above, in any form, i.e., any salt or non-salt form (e.g., as a free acid or base form, or as a pharmaceutically acceptable salt thereof) and any physical form thereof (e.g., including non-solid forms (e.g., liquid or semi-solid forms), and solid forms (e.g., amorphous or crystalline forms, specific polymorphic forms, solvates, including hydrates (e.g., mono-, di- and hemi- hydrates)), and mixtures of various forms.

As used herein, the term "optionally substituted" means unsubstituted groups or rings (e.g., cycloalkyl, heterocycle, and heteroaryl rings) and groups or rings substituted with one or more specified substituents.

Specific compounds of this invention are:
*N*-1,3-Benzothiazol-5-yl-6-(methylsulfonyl)-4-quinolinamine,
N-(2-methylphenyl)-6-(methylsulfonyl)-4-quinolinamine,
4-chloro-2-{[6-(methylsulfonyl)-4-quinolinyl]amino} phenol,
4-fluoro-2- {[6-(methylsulfonyl)-4-quinolinyl]amino} phenol,
N-1H-1,2,3-benzotriazol-5-yl-6-(methylsulfonyl)-4-quinolinamine,
3-fluoro-4-{[6-(methylsulfonyl)-4-quinolinyl]amino} phenol,
2-chloro-4-{[6-(methylsulfonyl)-4-quinolinyl]amino} phenol,
3-methyl-2-{[6-(methylsulfonyl)-4-quinolinyl]amino} phenol,
4-chloro-3-{[6-(methylsulfonyl)-4-quinolinyl]amino} phenol,
3-chloro-4-{[6-(methylsulfonyl)-4-quinolinyl]amino} phenol,
4-{[6-(methylsulfonyl)-4-quinolinyl] amino}phenol,
2-fluoro-4-{[6-(methylsulfonyl)-4-quinolinyl]amino} phenol,
N-1H-indol-6-yl-6-(methylsulfonyl)-4-quinolinamine,
5-fluoro-2-{[6-(methylsulfonyl)-4-quinolinyl]amino} phenol
2-chloro-6-methyl-3-{[6-(methylsulfonyl)-4-quinolinyl]amino} phenol,
6-(methylsulfonyl)-N-phenyl-4-quinolinamine,
N-(2-fluorophenyl)-6-(methylsulfonyl)-4-quinolinamine,
N-(2,5-difluorophenyl)-6-(methylsulfonyl)-4-quinolinamine,
N-(2,6-difluorophenyl)-6-(methylsulfonyl)-4-quinolinamine,
N-(2,4-dichlorophenyl)-6-(methylsulfonyl)-4-quinolinamine,
N-(2,4-dimethylphenyl)-6-(methylsulfonyl)-4-quinolinamine,
N-(3,5-difluorophenyl)-6-(methylsulfonyl)-4-quinolinamine,
N-(5-fluoro-2-methylphenyl)-6-(methylsulfonyl)-4-quinolinamine,
N-(3-chlorophenyl)-6-(methylsulfonyl)-4-quinolinamine,
N-(4-fluorophenyl)-6-(methylsulfonyl)-4-quinolinamine,
N-(4-chlorophenyl)-6-(methylsulfonyl)-4-quinolinamine,
(4-methyl-3-{[6-(methylsulfonyl)-4-quinolinyl]amino}phenyl)methanol,
N-[4-methyl-3-(methyloxy)phenyl]-6-(methylsulfonyl)-4-quinolinamine,
(3-{[6-(methylsulfbnyl)-4-quinolinyl]amino}phenyl)methanol,
N-1,3-benzodioxol-4-yl-6-(methylsulfonyl)-4-quinolinamine,
N-(4-{[(2-chlorophenyl)methyl]oxy}phenyl)-6-(methylsulfonyl)-4-quinolinamine,
2-fluoro-5-{[6-(methylsulfonyl)-4-quinolinyl]amino} phenol,
4-fluoro-3-{[6-(methylsulfonyl)-4-quinolinyl]amino} phenol,
2,4-dimethyl-5-{[6-(methylsulfonyl)-4-quinolinyl]amino} phenol,
3-methyl-5-{[6-(methylsulfonyl)-4-quinolinyl]amino} phenol,
N-1H-1,2,3-benzotriazol-4-yl-6-(methylsulfonyl)-4-quinolinamine,
5-{[6-(methylsulfonyl)-4-quinolinyl]amino}-1,3-benzoxathiol-2-one,
N-(4-methyl-1H-1,2,3-benzotriazol-6-yl)-6-(methylsulfonyl)-4-quinolinamine,
N-(5-fluoro-1H-1,2,3-benzotriazol-6-yl)-6-(methylsulfonyl)-4-quinolinamine
N-(5-methyl-1H-1,2,3-benzotriazol-6-yl)-6-(methylsulfonyl)-4-quinolinamine,
N-1,2,3-benzothiadiazol-5-yl-6-(methylsulfonyl)-4-quinolinamine,
N-1,2-benzisoxazol-6-yl-6-(methylsulfonyl)-4-quinolinamine,
N-[2,4-dimethyl-5-(methyloxy)phenyl]-6-(methylsulfonyl)-4-quinolinamine,
6-(methylsulfonyl)-N-[4-(phenyloxy)phenyl]-4-quinolinamine,
N-[3-(methyloxy)phenyl]-6-(methylsulfonyl)-4-quinolinamine,
2-methyl-3-{[6-(methylsulfonyl)-4-quinolinyl]amino} phenol,
2-methyl-5{[6-(methylsulfonyl)-4-quinolinyl]amino} phenol,
N-[2-chloro-5-(methyloxy)phenyl]-6-(methylsulfonyl)-4-quinolinamine,
N-[4-fluoro-3-(methyloxy)phenyl]-6-(methylsulfonyl)-4-quinolinamine,
3-methyl-4-{[6-(methylsulfonyl)-4-quinolinyl]amino} phenol,
6-(methylsulfonyl)-N-phenyl-4-quinolinamine,
N-1-benzofuran-4-yl-6-(methylsulfonyl)-4-quinolinamine,
N-[4-bromo-3-(methyloxy)phenyl]-6-(methylsulfonyl)-4-quinolinamine,
2-chloro-5-{[6-(methylsulfonyl)-4-quinolinyl]amino}phenol,
N-1,3-benzothiazol-6-yl-6-(methylsulfonyl)-4-quinolinamine,
N-2H-indazol-3-yl-6-(methylsulfonyl)-4-quinolinamine,
N-1,3-benzothiazol-5-yl-6-[(1-methylethyl)sulfonyl]-4-quinolinamine,
N-1H-indazol-6-yl-6-[(1-methylethyl)sulfonyl]-4-quinolinamine,
N-1,3-benzothiazol-5-yl-6-(ethylsulfonyl)-4-quinolinamine,
6-(ethylsulfonyl)-N-1H-indazol-6-yl-4-quinolinamine,
6-({6-[(1-methylethyl)sulfonyl]-4-quinolinyl}amino)-1H-indazole-5-carboxamide,
6-[(1-methylethyl)sulfonyl]-N-(7-methyl-1H-indazol-6-yl)-4-quinolinamine,
N-[4-chloro-3-(methyloxy)phenyl]-6-[(1-methylethyl) sulfonyl]-4-quinolinamine,
6-({6-[(1-methylethyl)sulfonyl]-4-quinolinyl}amino)-1H-indazole-5-carbonitrile,
N-1H-indazol-3-yl-6-[(1-methylethyl) sulfonyl]-4-quinolinamine,
6-[(1-methylethyl)sulfonyl]-N-[5-(methyloxy)-1H-indazol-3-yl]-4-quinolinamine,
N-(5-fluoro-1H-indazol-3-yl)-6-[(1-methylethyl)sulfonyl]-4-quinolinamine,
N-(3-methyl-1H-indazol-6-yl)-6-(methylsulfonyl)-4-quinolinamine,
N-[4-chloro-3-(methyloxy)phenyl]-6-(methylsulfonyl)-4-quinolinamine,
N-(3-fluoro-1H-indazol-6-yl)-6-(methylsulfonyl)-4-quinolinamine,
N-(4-chloro-1H-indazol-3-yl)-6-(methylsulfonyl)-4-quinolinamine,
N-[5-(methyloxy)-1H-indazol-3-yl]-6-(methylsulfonyl)-4-quinolinamine,
N-(5-fluoro-1H-indazol-3-yl)-6-(methylsulfonyl)-4-quinolinamine,
N-(5-chloro-1H-indazol-3-yl)-6-(methylsulfonyl)-4-quinolinamine,
N-(6-chloro-1H-indazol-3-yl)-6-(methylsulfonyl)-4-quinolinamine,
N-1H-indazol-6-yl-6-(methylsulfonyl)-4-quinolinamine,
6-[(1,1-dimethylethyl)sulfonyl]-*N*-(5-fluoro-1*H*-pyrazolo[3,4-6]pyridin-3-yl)-4-quinolinamine,
*N*-(5-methyl-1*H*-pyrazolo[3,4-*b*]pyridin-3-yl)-6-(methylsulfonyl)-4-quinolinamine,
*N*-(4-methyl-1,3-benzothiazol-5-yl)-6-(methylsulfonyl)-4-quinolinamine,
*N*-(4-bromo-1,3-benzothiazol-5-yl)-6-(methylsulfonyl)-4-quinolinamine,
*N*-(4-chloro-1,3-benzothiazol-5-yl)-6-(methylsulfonyl)-4-quinolinamine,
*N*-[(1,1-dimethylethyl)sulfonyl]-*N*-(5-fluoro-1*H*-pyrazolo[3,4-*b*]pyridin-3-yl)-4-quinolinamine,
*N*-[(1,1-dimethylethyl)sulfonyl]-*N*-(5-fluoro-6-methyl-1*H*-pyrazolo[3,4-*b*]pyridin-3-yl)-4-quinolinamine,
6-[(1-methylethyl)sulfonyl]-N-(1-methyl-1H-indazol-3-yl)-4-quinolinamine,
6-[(1,1-dimethylethyl)sulfonyl]-N-1H-indazol-6-yl-4-quinolinamine,
6-[(1,1-dimethylethyl)sulfonyl]-N-(3-fluoro-1H-indazol-6-yl)-4-quinolinamine,
6-[(1,1-dimethylethyl)sulfonyl]-N-(3-methyl-1H-indazol-6-yl)-4-quinolinamine,
6-[(1,1-dimethylethyl)sulfonyl]-N-1H-indazol-3-yl-4-quinolinamine,
6-[(1,1-dimethylethyl)sulfonyl]-N-(5-fluoro-1H-indazol-3-yl)-4-quinolinamine,
N-[4-chloro-3-(methyloxy)phenyl]-6-[(1,1-dimethylethyl)sulfonyl]-4-quinolinamine,
N-(3-fluoro-1H-indazol-6-yl)-6-[(1-methylethyl)sulfonyl]-4-quinolinamine,
6-[(1-methylethyl)sulfonyl]-N-(3-methyl-1H-indazol-6-yl)-4-quinolinamine,
6-[(1-methylethyl)sulfonyl]-N-[7-(trifluoromethyl)-1H-indazol-3-yl]-4-quinolinamine,
N-(5-fluoro-1H-indazol-3-yl)-6-[(trifluoromethyl)sulfonyl]-4-quinolinamine,
N-(6-(((tetrahydrofuran-2-yl)methyl)sulfonyl)quinolin-4-yl)benzo[d]thiazol-5-amine,
4-(1,3-benzothiazol-5-ylamino)-6-[(trifluoromethyl)sulfonyl]quinoline,
N-(6-(tert-butylsulfonyl)quinolin-4-yl)thiazolo[5,4-b]pyridin-6-amine,
6-[(1-methyl)ethyl)sulfonyl]-*N*-[1,3]thiazolo[5,4-*b*]pyridin-6-yl-4-quinolinamine,
*N*-2,1,3-Benzoxadiazol-5-yl-6-(methylsulfonyl)-4-quinolinamine,
3-{[4-(1,3-Benzothiazol-5-ylamino)-6-quinolinyl]sulfonyl}-3-methyl-1-butanol,
N-1,3-benzothiazol-5-yl-6-(phenylsulfonyl)-4-quinolinamine,
N-1,3-benzothiazol-5-yl-6-(cyclopropyl sulfonyl)-4-quinolinamine,
6-(methylsulfonyl)-N-(2-phenylethyl)-4-quinolinamine,
6-[(4-aminophenyl)sulfonyl]-N-1,3-benzothiazol-5-yl-4-quinolinamine,
*N*³-methylidene-4-(methylthio)-*N*¹-[6-(propylsulfonyl)-4-quinolinyl]-1,3-benzenediamine,
N-1,3-benzothiazol-5-yl-6-(cyclohexylsulfonyl)-4-quinolinamine,
N-1,3-benzothiazol-5-yl-6-(4-pyridinylsulfonyl)-4-quinolinamine,
3-{[4-(1,3-benzothiazol-5-ylamino)-6-quinolinyl]thio}-3-methyl-1-butanol,
N-1,3-benzothiazol-5-yl-6-(ethylsulfonyl)-4-quinolinamine,
3-{[4-(1,3-benzothiazol-5-ylamino)-6-quinolinyl]sulfonyl}-1-propanol,
4-{[4-(1,3-benzothiazol-5-ylamino)-6-quinolinyl]sulfonyl}-1-butanol,
2-{[4-(1,3-benzothiazol-5-ylamino)-6-quinolinyl]sulfonyl}ethanol,
N-1,3-benzothiazol-5-yl-6-[(1,1-dimethylethyl)sulfonyl]-4-quinolinamine,
N-1,3-benzothiazol-5-yl-6-[(1,1-dimethylethyl)thio]-4-quinolinamine,
N-1,3-benzothiazol-5-yl-6-[(1-methylpropyl)thio]-4-quinolinamine,
N-1,3-benzothiazol-5-yl-6-(ethylthio)-4-quinolinamine,
6-[(2-aminoethyl)thio]-N-1,3-benzothiazol-5-yl-4-quinolinamine,
N-1,3-benzothiazol-5-yl-6-(cyclopentylthio)-4-quinolinamine,
N-1,3-benzothiazol-5-yl-6-(cyclopentyl sulfonyl)-4-quinolinamine,
3-{[4-(1H-indazol-6-ylamino)-6-quinolinyl]thio}-3-methyl-1-butanol,
3-{[4-(1H-indazol-6-ylamino)-6-quinolinyl]sulfonyl}-3-methyl-1-butanol,
N-1,3-benzothiazol-5-yl-6-[(1-methyl-4-piperidinyl)thio]-4-quinolinamine,
N-1,3-benzothiazol-5-yl-6-(2-pyrimidinylthio)-4-quinolinamine,
2-{[4-(1,3-benzothiazol-5-ylamino)-6-quinolinyl]thio}-1-propanol,
2-{[4-(1,3-benzothiazol-5-ylamino)-6-quinolinyl]sulfonyl}-1-propanol,
2-[1-({[4-(1,3-benzothiazol-5-ylamino)-6-quinolinyl]thio}methyl)cyclopropyl]ethanol,
2-[1-({[4-(1,3-benzothiazol-5-ylamino)-6-quinolinyl]sulfonyl}methyl)cyclopropyl]ethanol,
2-{[4-(1,3-benzothiazol-5-ylamino)-6-quinolinyl]sulfonyl}-2-methyl-1-propanol,
2-{[4-(1,3-benzothiazol-5-ylamino)-6-quinolinyl]thio}-2-methyl-1-propanol,
2-{[4-(1,3-benzothiazol-5-ylamino)-6-quinolinyl]sulfinyl}-2-methyl-1-propanol
2-{[4-(1H-indazol-6-ylamino)-6-quinolinyl]sulfonyl}-2-methyl-1-propanol,
methyl 2-{[4-(1,3-benzothiazol-5-ylamino)-6-quinolinyl]thio}-2-methylpropanoate,
methyl 2-{[4-(1,3-benzothiazol-5-ylamino)-6-quinolinyl]sulfonyl}-2-methylpropanoate,
N-1,3-benzothiazol-5-yl-6-{[1,1-dimethyl-3-(methyloxy)propyl]thio}-4-quinolinamine,
N-(5-fluoro-1H-indazol-3-yl)-6-(tetrahydro-2H-pyran-4-ylsulfonyl)-4-quinolinamine,
2-({4-[(5-fluoro-1H-indazol-3-yl)amino]-6-quinolinyl}sulfonyl)ethanol,
N-1,3-benzothiazol-5-yl-6-(1H-1,2,4-triazol-3-ylsulfonyl)-4-quinolinamine,
N-(5-fluoro-1H-indazol-3-yl)-6-(1H-imidazol-4-ylsulfonyl)-4-quinolinamine,
N-(5-fluoro-1H-indazol-3-yl)-6-(1H-1,2,4-triazol-3-ylsulfonyl)-4-quinolinamine,
N-(5-fluoro-1H-indazol-3-yl)-6-(tetrahydro-3-furanylsulfonyl)-4-quinolinamine,
N-1,3-benzothiazol-5-yl-6-[(2-methyltetrahydro-3-furanyl)sulfonyl]-4-quinolinamine,
N-1,3-benzothiazol-5-yl-6-{[2-(methyloxy)ethyl]sulfonyl}-4-quinolinamine,
N-1,3-benzothiazol-5-yl-6-(methylthio)-4-quinolinamine,
2-{[4-(1,3-benzothiazol-5-ylamino)-6-quinolinyl]thio}ethanol,
N-1,3-benzothiazol-5-yl-6-{[2-(diethylamino)ethyl]thio}-4-quinolinamine,
N-1,3-benzothiazol-5-yl-6-{[2-(diethylamino)ethyl]sulfonyl}-4-quinolinamine,
N-1,3-benzothiazol-5-yl-6-(tetrahydro-3-furanylthio)-4-quinolinamine,
N-1,3-benzothiazol-5-yl-6-(tetrahydro-3-furanylsulfonyl)-4-quinolinamine,
N-1,3-benzothiazol-5-yl-6-(tetrahydro-2H-pyran-4-ylthio)-4-quinolinamine,
N-1,3-benzothiazol-5-yl-6-(tetrahydro-2H-pyran-4-ylsulfonyl)-4-quinolinamine,
N-1,3-benzothiazol-5-yl-6-(2-propen-1-ylsulfonyl)-4-quinolinamine,
N-1,3-benzothiazol-5-yl-6-{[2-(4-morpholinyl)ethyl]thio}-4-quinolinamine,
N-1,3-benzothiazol-5-yl-6-{[2-(3,5-dimethyl-4-isoxazolyl)ethyl]thio}-4-quinolinamine,
N-1,3-benzothiazol-5-yl-6-{[2-(3,5-dimethyl-4-isoxazolyl)ethyl]sulfonyl}-4-quinolinamine,
N-(6-((1-methylpiperidin-4-yl)sulfonyl)quinolin-4-yl)benzo[d]thiazol-5-amine,
1,1-dimethylethyl4-{[4-(1,3-benzothiazol-5-ylamino)-6-quinolinyl]thio}-1-piperidinecarboxylate,
1,1-dimethylethyl4-{[4-(1,3-benzothiazol-5-ylamino)-6-quinolinyl]sulfonyl}-1-piperidinecarboxylate,
N-1,3-benzothiazol-5-yl-6-[(2,2,2-trifluoroethyl)thio]-4-quinolinamine,
N-1,3-benzothiazol-5-yl-6-[(2,2,2-trifluoroethyl)sulfonyl]-4-quinolinamine,
N-1,3-benzothiazol-5-yl-6-(tetrahydro-2H-thiopyran-4-ylthio)-4-quinolinamine,
2-{[4-(1,3-benzothiazol-5-ylamino)-6-quinolinyl]sulfinyl} ethanol,
N-(6-(((3*S*)-tetrahydro-2H-pyran-3-yl)sulfonyl)quinolin-4-yl)benzo[d]thiazol-5-amine,
N-(6-(((3*R*)-tetrahydro-2H-pyran-3-yl)sulfonyl)quinolin-4-yl)benzo[d]thiazol-5-amine,
2-{[4-(1,3-benzothiazol-5-ylamino)-6-quinolinyl]thio} ethanol,
*N*-1,3-benzothiazol-5-yl-6-[(1-methylethyl)thio]-4-quinolinamine,
2-{[4-(1,3-benzothiazol-5-ylamino)-6-quinolinyl]thio}-2-methylpropanoic acid,
2-{[4-(1,3-benzothiazol-5-ylamino)-6-quinolinyl]sulfonyl}-2-methylpropanoic acid,
N-1,3-benzothiazol-5-yl-6-[(1,1-dioxidotetrahydro-2H-thiopyran-4-yl)sulfonyl]-4-quinolinamine,
N-1,3-benzothiazol-5-yl-6-(4-piperidinylsulfonyl)-4-quinolinamine,
2-{[4-(1,3-benzothiazol-5-ylamino)-6-quinolinyl]sulfinyl} ethanol,
N-1,3-benzothiazol-5-yl-6-((*R*)methylsulfinyl)-4-quinolinamine,
N-1,3-benzothiazol-5-yl-6-((*S*)methylsulfinyl)-4-quinolinamine,
N-1,3-benzothiaaol-5-yl-6-[(1-methylethyl)sulfinyl]-4-quinolinamine,
4-[(5-Hydroxy-2-methylphenyl)amino]-*N*-(phenylmethyl)-6-quinolinecarboxamide,
N-cyclohexyl-4-[(5-hydroxy-2-methylphenyl)amino]-6-quinolinecarboxamide,
4-[(5-hydroxy-2-methylphenyl)amino]-N-[3-(4-morpholinyl)propyl]-6-quinolinecarboxamide,
4-[(5-hydroxy-2-methylphenyl)amino]-N-(tetrahydro-2H-pyran-4-ylmethyl)-6-quinolinecarboxamide,
4-[(5-hydroxy-2-methylphenyl)amino]-N-(3-phenylpro.pyl)-6-quinolinecarboxamide,
4-[(5-hydroxy-2-methylphenyl)amino]-N-[(3R)-1-(phenylmethyl)-3-pyrrolidinyl]-6-quinolinecarboxamide,
4-[(5-hydroxy-2-methylphenyl)amino]-N-{3-[methyl(phenyl) amino]propyl}-6-quinolinecarboxamide,
4-[(5-hydroxy-2-methylphenyl)amino]-N-[3-(1H-imidazol-1-yl)propyl]-6-quinolinecarboxamide,
4-[(5-hydroxy-2-methylphenyl)amino]-N-[2-(1H-imidazol-4-yl)ethyl]-6-quinolinecarboxamide,
N-[(1S)-2-hydroxy-1-(1H-imidazol-4-ylmethyl)ethyl]-4-[(5-hydroxy-2-methylphenyl)amino]-6-quinolinecarboxamide,
N-(1H-benzimidazol-2-ylmethyl)-4-[(5-hydroxy-2-methylphenyl)amino]-6-quinolinecarboxamide,
4-[(5-hydroxy-2-methylphenyl)amino]-N-[2-(1-pyrrolidinyl)ethyl]-6-quinolinecarboxamide,
4-[(5-hydroxy-2-methylphenyl)amino]-N-[2-(methylsulfonyl) ethyl]-6-quinolinecarboxamide,
4-[(5-hydroxy-2-methylphenyl)amino]-N-[2-(1H-indol-3-yl)ethyl]-6-quinolinecarboxamide,
4-[(5-hydroxy-2-methylphenyl)amino]-N-[(6-methyl-2-pyridinyl)methyl]-6-quinolinecarboxamide,
N-(4,5-dimethyl-1,3-thiazol-2-yl)-4-[(5-hydroxy-2-methylphenyl)amino]-6-quinolinecarboxamide,
4-(1,3-benzothiazol-5-ylamino)-N-(phenylmethyl)-6-quinolinecarboxamide,
N-1,3-Benzothiazol-5-yl-6-(4-morpholinylsulfonyl)-4-quinolinamine,
4-(1,3-Benzothiazol-5-ylamino)-N-(3-methyl-3-oxetanyl)-6-quinolinesulfonamide,
4-(1,3-benzothiazol-5-ylamino)-N-(1-methylethyl)-6-quinoline sulfonamide,
N-1,3-benzothiazol-5-yl-6-(1-piperidinylsulfonyl)-4-quinolinamine,
4-(1,3-benzothiazol-5-ylamino)-N,N-dimethyl-6-quinolinesulfonamide,
4-(1,3-benzothiazol-5-ylamino)-N-methyl-N-(methyloxy)-6-quinolinesulfonamide,
4-(1,3-benzothiazol-5-ylamino)-N-(2-hydroxyethyl)-N-methyl-6-quinolinesulfonamide,
N-(2-hydroxyethyl)-4-(1H-indazol-6-ylamino)-N-methyl-6-quinolinesulfonamide,
4-{[4-chloro-3-(methyloxy)phenyl]amino}-N-(2-hydroxyethyl)-N-methyl-6-quinolinesulfonamide,
4-{[4-chloro-3-(methyloxy)phenyl]amino}-6-quinolinesulfonamide,
4-(1H-indazol-6-ylamino)-6-quinolinesulfonamide,
4-(1,3-benzothiazol-5-ylamino)-N-4-piperidinyl-6-quinolinesulfonamide,
4-(1,3-benzothiazol-5-ylamino)-N-(4-piperidinylmethyl)-6-quinolinesulfonamide,
N-1,3-benzothiazol-5-yl-6-(4-morpholinylsulfonyl)-4-quinolinairiine,
4-(1,3-benzothiazol-5-ylamino)-N-[(6-methyl-2-pyridinyl)methyl]-6-quinolinesulfonamide,
4-(1,3-benzothiazol-5-ylamino)-N-[2-(dimethylamino)ethyl]-6-quinolinesulfonamide,
4-(1,3-benzothiazol-5-ylamino)-N-[2-(methyloxy)ethyl]-6-quinolinesulfonamide,
4-(1,3-benzothiazol-5-ylamino)-N-[3-(4-morpholinyl)propyl]-6-quinolinesulfonamide,
4-(1,3-benzothiazol-5-ylamino)-N-(tetrahydro-2H-pyran-4-ylmethyl)-6-quinolinesulfonamide,
4-(1,3-benzothiazol-5-ylamino)-N-(tetrahydro-2H-pyran-4-yl)-6-quinolinesulfonamide,
4-( 1,3-benzothiazol-5-ylamino)-N-cyclohexyl-6-quinolinesulfonamide,
4-(1,3-benzothiazol-5-ylamino)-N-[2-(methylsulfonyl)ethyl]-6-quinolinesulfonamide,
4-(1,3-benzothiazol-5-ylamino)-N-(phenylmethyl)-6-quinolinesulfonamide,
N∼2∼{[4-(1,3-benzothiazol-5-ylamino)-6-quinolinyl]sulfonyl}glycinamide,
N∼3∼-{[4-(1,3-benzothiazol-5-ylamino)-6-quinolinyl]sulfonyl}-beta-alaninamide,
4-(1,3-benzothiazol-5-ylamino)-N-(2-hydroxyethyl)-6-quinolinesulfonamide,
4-(1,3-benzothiazol-5-ylamino)-N-3-oxetanyl-6-quinolinesulfonamide,
4-(1,3-benzothiazol-5-ylamino)-N-(2-{[2-(methyloxy)ethyl]oxy}ethyl)-6-quinolinesulfonamide,
4-(1,3-benzothiazol-5-ylamino)-N-methyl-N-(1-methylethyl)-6-quinolinesulfonamide,
4-(1H-indazol-6-ylamino)-N,N-dimethyl-6-quinolinesulfonamide,
4-{[4-chloro-3-(methyloxy)phenyl]amino}-N,N-dimethyl-G-quiriolinesulfonamide,
N-1H-indazol-6-yl-6-(4-morpholinylsulfonyl)-4-quinolinamine,
N-[4-chloro-3-(methyloxy)phenyl]-6-(4-morpholinylsulfonyl)-4-quinolinamine,
4-(1H-indazol-6-ylamino)-N-(1-methylethyl)-6-quinolinesulfonamide,
4-{[4-chloro-3-(methyloxy)phenyl]amino}-N-(1-methylethyl)-6-quinolinesulfonamide,
N-1,3-benzothiazol-5-yl-6-(1-pyrrolidinylsulfonyl)-4-quinolinamine,
methyl 1-{[4-(1,3-benzothiazol-5-ylamino)-6-quinolinyl]sulfonyl}-L-prolinate,
methyl (3S,6R)-1-{[4-(1,3-benzothiazol-5-ylamino)-6-quinolinyl]sulfonyl}-6-methyl-3-piperidinecarboxylate,
1-{[4-(1,3-benzothiazol-5-ylamino)-6-quinolinyl]sulfonyl}-3-pyrrolidinol,
N-1,3-benzothiazol-5-yl-6-[(3-methyl-4-morpholinyl)sulfonyl]-4-quinolinamine,
N-1,3-benzothiazol-5-yl-6-[(4-methyl-1-piperazinyl)sulfonyl]-4-quinolinamine,
N-1,3-benzothiazol-5-yl-6-(4-thiomorpholinylsulfonyl)-4-quinolinamine,
N-1,3-benzothiazol-5-yl-6-[(1,1-dioxido-4-thiomorpholinyl)sulfonyl]-4-quinolinamine,
N-1,3-benzothiazol-5-yl-6-{[(3R)-3-methyl-4-morpholinyl]sulfonyl}-4-quinolinamine,
((2S,5R)-4-{[4-(1,3-benzothiazol-5-ylamino)-6-quinolinyl]sulfonyl}-5-ethyl-2-morpholinyl)methanol,
N-1,3-benzothiazol-5-yl-6-{[(3S)-3-methyl-4-morpholinyl]sulfonyl}-4-quinolinamine,
N-1,3-benzothiazol-5-yl-6-(1-piperazinylsulfonyl)-4-quinolinamine,
((2S,5R)-4-{[4-(1,3-benzothiazol-5-ylamino)-6-quinolinyl]sulfonyl)-5-methyl-2-morpholinyl)methanol,
(4-{[4-(1,3-benzothiazol-5-ylamino)-6-quinolinyl]sulfonyl}-2-morpholinyl)methanol,
4-(1,3-benzothiazol-5-ylamino)-N-methyl-N-[2-(methyloxy)ethyl]-6-quinolinesulfonamide,
4-(1,3-benzothiazol-5-ylamino)-N-[2-(methyloxy)ethyl]-6-quinolinesulfonamide,
N-(5-fluoro-1H-indazol-3-yl)-6-(4-morpholinylsulfonyl)-4-quinolinamine,
N-1,3-benzothiazol-5-yl-6-[(2,2-dimethyl-4-morpholinyl)sulfonyl]-4-quinolinamine,
N-1,3-benzothiazol-5-yl-6-[(2-methyl-4-morpholinyl)sulfonyl]-4-quinolinamine,
4-[(7-chloro-1H-indazol-3-yl)amino]-N-[2-(methyloxy)ethyl]-6-quinolinesulfonamide,
N-1,3-Benzothiazol-5-yl-N-[6-(methylsulfonyl)-4-quinolinyl]acetamide,
N-1,3-benzothiazol-5-yl-N-[6-(methylsulfonyl)-4-quinolinyl]methanesulfonamide,
N 1,3-Benzoxazol-5-yl-6-(methylsulfonyl)-4-quinolinamine,
N-l,3-benzoxazol-5-yl-6-[(l-methylethyl)sulfonyl]-4-quinolinamine,
N-1,3-benzoxazol-5-yl-6-(4-morpholinylsulfonyl)-4-quinolinamine,
N-[4-chloro-3-(methyloxy)phenyl]-6-(tetrahydro-2H-pyran-4-ylsulfonyl)-4-quinolinamine,
N-[4-chloro-3-(methyloxy)phenyl]-6-(tetrahydro-2H-pyran-4-ylsulfinyl)-4-quinolinamine,

in free base form, or in the form of a salt, particularly a pharmaceutically acceptable salt, thereof.

Representative compounds of this invention include the compounds of Examples 1-252.

Compound names were generated using the software naming program ACD/Name Pro V6.02 available from Advanced Chemistry Development, Inc., 110 Yonge Street, 14^{th} Floor, Toronto, Ontario, Canada, M5C 1T4 (http://www.acdlabs.com/). It will be appreciated by those skilled in the art that many of the compounds of this invention, as well as compounds used in the preparation of the compounds of Formula (I), (II) or (III), may exist in tautomeric forms. The program used to name the compounds of this invention,will only name one of such tautomeric forms at a time. It is to be understood that any reference to a named compound or a structurally depicted compound is intended to encompass all tautomers of such compounds and any mixtures of tautomers thereof.

The compounds according to Formula (I), (II) or (III) may contain one, or more asymmetric center (also referred to as a chiral center) and may, therefore, exist as individual enantiomers, diastereomers, or other stereoisomeric forms, or as mixtures thereof. Chiral centers, such as chiral carbon atoms, may also be present in a substituent such as an alkyl group. Where the stereochemistry of a chiral center present in a compound of this invention, or in any chemical structure illustrated herein, is not specified the structure is intended to encompass all individual stereoisomers and all mixtures thereof. Thus, compounds according to Formula (I), (II) or (III) containing one or more chiral center may be used as racemic mixtures, enantiomerically enriched mixtures, or as enantiomerically pure individual stereoisomers.

Individual stereoisomers of a compound according to according to Formula (I), (II) or (III) which contain one or more asymmetric center may be resolved by methods known to those skilled in the art. For example, such resolution may be carried out (1) by formation of diastereoisomeric salts, complexes or other derivatives; (2) by selective reaction with a stereoisomer-specific reagent, for example by enzymatic oxidation or reduction; or (3) by gas-liquid or liquid chromatography in a chiral environment, for example, on a chiral support such as silica with a bound chiral ligand or in the presence of a chiral solvent. The skilled artisan will appreciate that where the desired stereoisomer is converted into another chemical entity by one of the separation procedures described above, a further step is required to liberate the desired form. Alternatively, specific stereoisomers may be synthesized by asymmetric synthesis using optically active reagents, substrates, catalysts or solvents, or by converting one enantiomer to the other by asymmetric transformation. When a disclosed compound or its salt is named or depicted by structure, it is to be understood that the compound or salt, including solvates (particularly, hydrates) thereof, may exist in crystalline forms, non-crystalline forms or a mixture thereof. The compound or salt, or solvates (particularly, hydrates) thereof, may also exhibit polymorphism (i.e. the capacity to occur in different crystalline forms). These different crystalline forms are typically known as "polymorphs." It is to be understood that when named or depicted by structure, the disclosed compound, or solvates (particularly, hydrates) thereof, also include all polymorphs thereof. Polymorphs have the same chemical composition but differ in packing, geometrical arrangement, and other descriptive properties of the crystalline solid state. Polymorphs, therefore, may have different physical properties such as shape, density, hardness, deformability, stability, and dissolution properties. Polymorphs typically exhibit different melting points, IR spectra, and X-ray powder diffraction patterns, which may be used for identification. One of ordinary skill in the art will appreciate that different polymorphs may be produced, for example, by changing or adjusting the conditions used in crystallizing/recrystallizing the compound.

Because of their potential use in medicine, the salts of the compounds of Formula (I), (II) or (III) are preferably pharmaceutically acceptable salts. Suitable pharmaceutically acceptable salts include those described by Berge, Bighley and Monkhouse J.Pharm.Sci (1977) 66, pp 1-19. Salts encompassed within the term "pharmaceutically acceptable salts" refer to non-toxic salts of the compounds of this invention.

When a compound of the invention is a base (contain a basic moiety), a desired salt form may be prepared by any suitable method known in the art, including treatment of the free base with an inorganic acid, such as hydrochloric acid, hydrobromic acid, sulfuric acid, nitric acid, phosphoric acid, and the like, or with an organic acid, such as acetic acid, trifluoroacetic acid, maleic acid, succinic acid, mandelic acid, fumaric acid, malonic acid, pyruvic acid, oxalic acid, glycolic acid, salicylic acid, and the like, or with a pyranosidyl acid, such as glucuronic acid or galacturonic acid, or with an alpha-hydroxy acid, such as citric acid or tartaric acid, or with an amino acid, such as aspartic acid or glutamic acid, or with an aromatic acid, such as benzoic acid or cinnamic acid, or with a sulfonic acid, such as p-toluenesulfonic acid, methanesulfonic acid, ethanesulfonic acid or the like.

Suitable addition salts are formed from acids which form non-toxic salts and examples include acetate, p-aminobenzoate, ascorbate, aspartate, benzenesulfonate, benzoate, bicarbonate, bismethylenesalicylate, bisulfate, bitartrate, borate, calcium edetate, camsylate, carbonate, clavulanate, citrate, cyclohexylsulfamate, edetate, edisylate, estolate, esylate, ethanedisulfonate, ethanesulfonate, formate, fumarate, gluceptate, gluconate, glutamate, glycollate, glycollylarsanilate, hexylresorcinate, hydrabamine, hydrobromide, hydrochloride, dihydrochloride, hydrofumarate, hydrogen phosphate, hydroiodide, hydromaleate, hydrosuccinate, hydroxynaphthoate, isethionate, itaconate, lactate, lactobionate, laurate, malate, maleate, mandelate, mesylate, methylsulfate, monopotassium maleate, mucate, napsylate, nitrate, N-methylglucamine, oxalate, oxaloacetate, pamoate (embonate), palmate, palmitate, pantothenate, phosphate/diphosphate, pyruvate, polygalacturonate, propionate, saccharate, salicylate, stearate, subacetate, succinate, sulfate, tannate, tartrate, teoclate, tosylate, triethiodide, trifluoroacetate and valerate.

Other exemplary acid addition salts include pyrosulfate, sulfite, bisulfite, decanoate, caprylate, acrylate, isobutyrate, caproate, heptanoate, propiolate, oxalate, malonate, suberate, sebacate, butyne-1,4-dioate, hexyne-1,6-dioate, chlorobenzoate, methylbenzoate, dinitrobenzoate, hydroxybenzoate, methoxybenzoate, phthalate, phenylacetate, phenylpropionate, phenylbutrate, lactate, γ-hydroxybutyrate, mandelate, and sulfonates, such as xylenesulfonate, propanesulfonate, naphthalene-1-sulfonate and naphthalene-2-sulfonate.

If an inventive basic compound is isolated as a salt, the corresponding free base form of that compound may be prepared by any suitable method known to the art, including treatment of the salt with an inorganic or organic base, suitably an inorganic or organic base having a higher pKₐ than the free base form of the compound.

When a compound of the invention is an acid (contains an acidic moiety), a desired salt may be prepared by any suitable method known to the art, including treatment of the free acid with an inorganic or organic base, such as an amine (primary, secondary, or tertiary), an alkali metal or alkaline earth metal hydroxide, or the like. Illustrative examples of suitable salts include organic salts derived from amino acids such as glycine and arginine, ammonia, primary, secondary, and tertiary amines, and cyclic amines, such as N-methyl-D-glucamine, diethylamine, isopropylamine, trimethylamine, ethylene diamine, dicyclohexylamine, ethanolamine, piperidine, morpholine, and piperazine, as well as inorganic salts derived from sodium, calcium, potassium, magnesium, manganese, iron, copper, zinc, aluminum, and lithium.

Certain of the compounds of this invention may form salts with one or more equivalents of an acid (if the compound contains a basic moiety) or a base (if the compound contains an acidic moiety). The present invention includes within its scope all possible stoichiometric and non-stoichiometric salt forms.

Compounds of the invention having both a basic and acidic moiety may be in the form of zwitterions, acid-addition salt of the basic moiety or base salts of the acidic moiety. This invention also provides for the conversion of one pharmaceutically acceptable salt of a compound of this invention, e.g., a hydrochloride salt, into another pharmaceutically acceptable salt of a compound of this invention, e.g., a sodium salt.

For solvates of the compounds of the invention, or salts thereof that are in crystalline form, the skilled artisan will appreciate that pharmaceutically-acceptable solvates may be formed wherein solvent molecules are incorporated into the crystalline lattice during crystallization. Solvates may involve nonaqueous solvents such as ethanol, isopropanol, DMSO, acetic acid, ethanolamine, and ethyl acetate, or they may involve water as the solvent that is incorporated into the crystalline lattice. Solvates wherein water is the solvent that is incorporated into the crystalline lattice are typically referred to as "hydrates." Hydrates include stoichiometric hydrates as well as compositions containing variable amounts of water. The invention includes all such solvates.

The subject invention also includes isotopically-labeled compounds which are identical to those recited in according to Formula (I), (II) or (III) but for the fact that one or more atoms are replaced by an atom having an atomic mass or mass number different from the atomic mass or mass number most commonly found in nature. Examples of isotopes that can be incorporated into compounds of the invention include isotopes of hydrogen, carbon, nitrogen, oxygen, fluorine, iodine and chlorine such as ³H, ¹¹C, ¹⁴C, ¹⁸F, ¹²³I or ¹²⁵I.

Compounds of the present invention and pharmaceutically acceptable salts of said compounds that contain the aforementioned isotopes and/or other isotopes of other atoms are within the scope of the present invention. Isotopically labeled compounds of the present invention, for example those into which radioactive isotopes such as ³H or ¹⁴C have been incorporated, are useful in drug and/or substrate tissue distribution assays. Tritiated, i.e., ³H, and carbon-14, i.e., ¹⁴C, isotopes are particularly preferred for their ease of preparation and detectability. ¹¹C and ¹⁸F isotopes are particularly useful in PET (positron emission tomography).

Because the compounds of Formula (I), (II) or (III) are intended for use in pharmaceutical compositions it will readily be understood that they are each preferably provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions.

### GENERAL SYNTHETIC METHODS

The compounds of Formula (I), (II) or (III) may be obtained by using synthetic procedures illustrated in the Schemes below or by drawing on the knowledge of a skilled organic chemist. The synthesis provided in these Schemes are applicable for producing compounds of the invention having a variety of different R¹ and R² groups employing appropriate precursors, which are suitably protected if needed, to achieve compatibility with the reactions outlined herein. Subsequent deprotection, where needed, affords compounds of the nature generally disclosed. While the Schemes are shown with compounds only of Formula (I), (II) or (III), they are illustrative of processes that may be used to make the compounds of the invention.

Intermediates (compounds used in the preparation of the compounds of the invention) may also be present as salts. Thus, in reference to intermediates, the phrase "compound(s) of formula (number)" means a compound having that structural formula or a pharmaceutically acceptable salt thereof.

4-Chloroquinoline intermediates were synthesized via condensation of an aniline with an enol ether followed by cyclization, hydrolysis of the resultant ester, and decarboxylation. The sulfide was then oxidized to the sulfone followed by conversion of the hydroxyquinoline to the chloroquinoline.

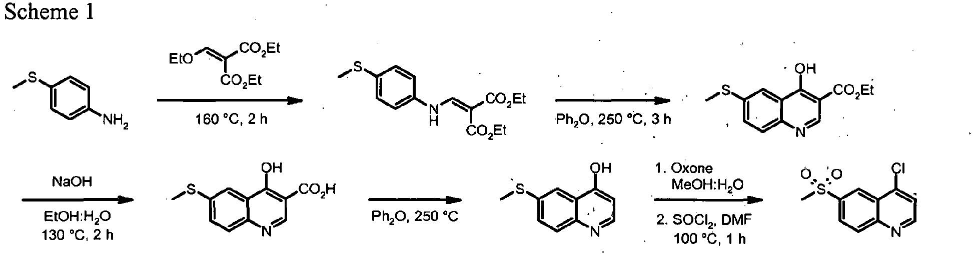

Alternatively, chloroquinolines could be made through condensation of the appropriate aniline with Meldrum's acid followed by cyclization and chlorination.

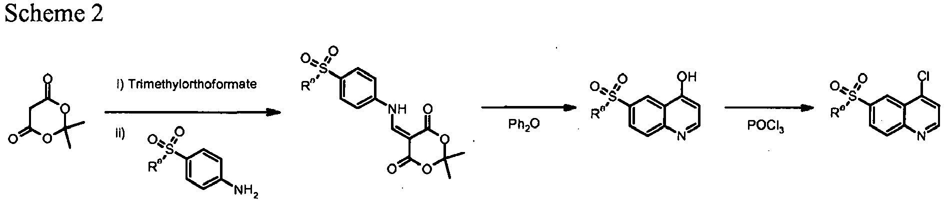

4-Chloro-6-iodoquinoline was made in an analogous manner to that in Scheme 2 beginning with the *p*-iodoaniline.

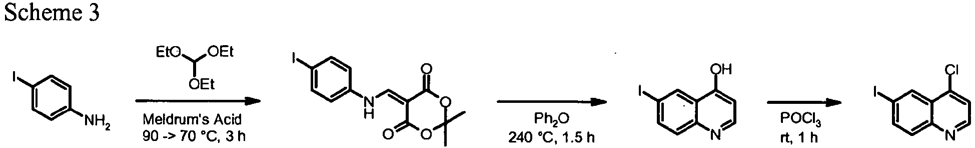

4-Chloro-6-sulfonylquinolines were synthesized from 4-chloro-6-iodoquinoline via a palladium catalyzed coupling with a thiol followed by oxidation to the sulfone.

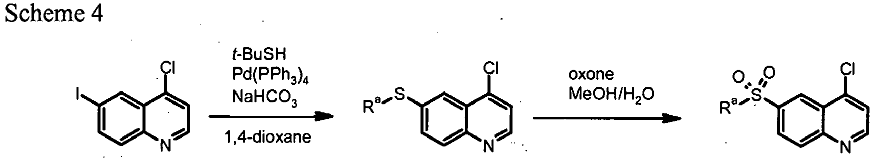

Various alkyl sulfones at C6 could be installed via a palladium coupling with the 4-hydroxy-6-iodoquinoline and the sodium salt of the appropriate thiol.

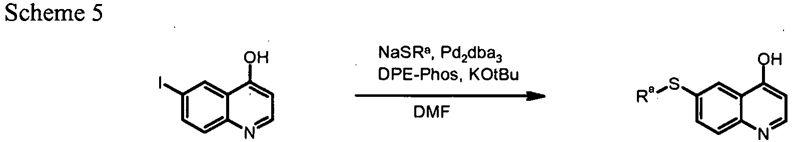

An alternative installation of alkyl sulfones occurs via a copper catalyzed addition of alkylsulfinic acid sodium salt followed by chlorination.

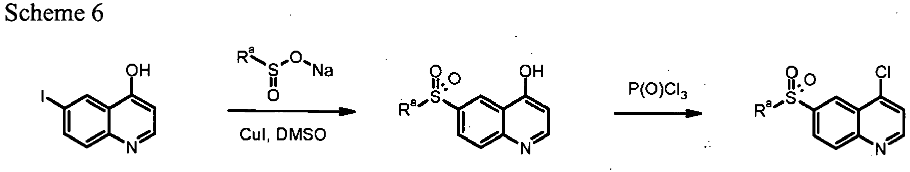

Construction of 1,2,3-benzothiadiazol-5-amine was achieved via sulfur displacement of the chloride of chloronitroaniline followed by diazonium ion formation with sodium nitrate and cylcization.

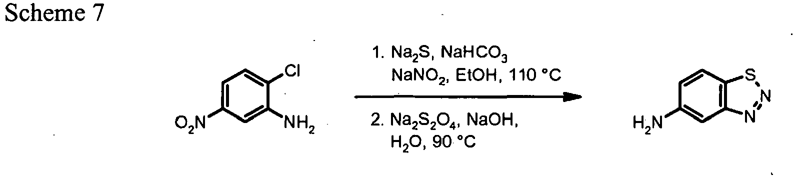

Construction of substituted benzotriazoles began with bromination of the appropriate nitroaniline followed by reduction of the nitro group to the bisaniline. Formation of the diazonium ion followed by copper mediated ring closure afforded the 5-iodobenzotriazole which was then converted to the 5-aminobenzotriazole via imine formation.

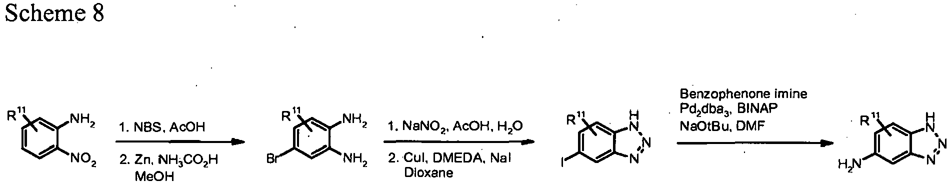

3-Fluoro-1*H*-indazol-6-amine was formed through fluorination of the 6-nitroindazole followed by reduction to the amine.

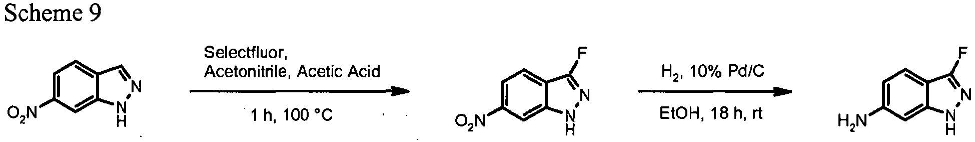

1-Benzofuran-4-amine was constructed from the benzofuranone via the oximine.

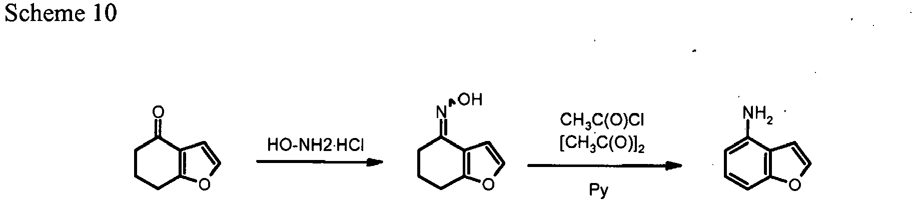

Select β-hydroxythiols were synthesized via reduction of commercially available carboxylic acids.

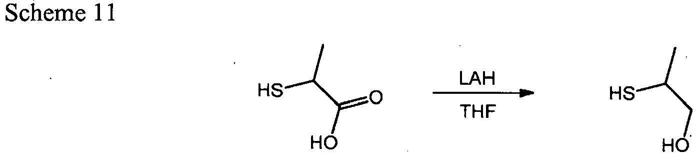

Aniline backpocket groups were reacted with 4-chloro-6-sulfonyl-quinolines under microwave or thermal conditions to afford final compounds.

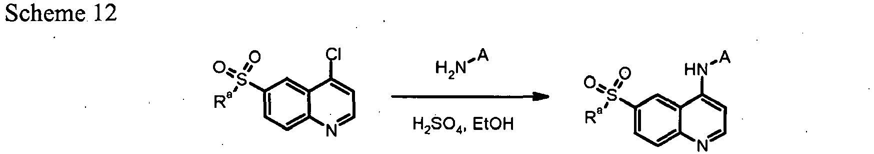

Alternatively, aniline backpocket groups were reacted with 4-chloro-6-sulfonyl-quinolines under palladium catalyzed conditions to afford final compounds.

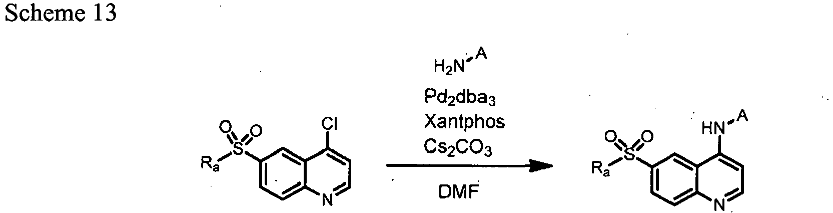

The benzoxadiazole backpocket group was constructed via a palladium catalyzed coupling to the 4-aminoquinoline.

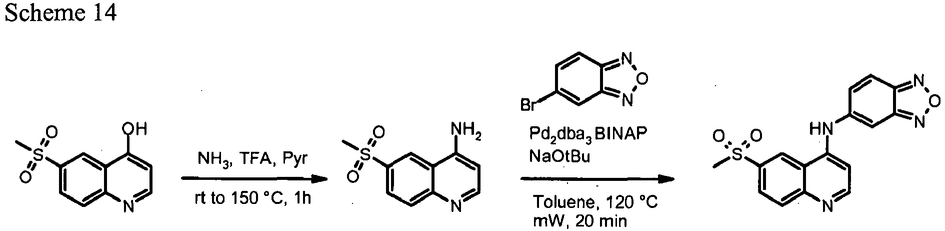

Additional sulfones were constructed with the backpocket group already in place. Addition of an aniline to the 4-chloroquinoline followed by a palladium catalyzed coupling of a thiol led to thioethers which were then oxidized to sulfones.

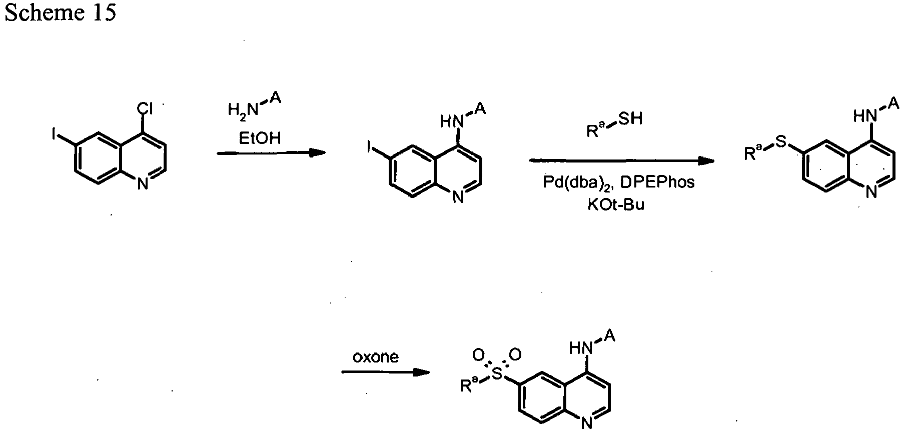

β-carboxylic acid sulfides and sulfones were generated via hydrolysis of the corresponding ester.

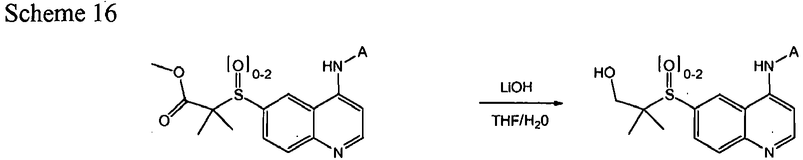

Bis-sulfides were oxidized with excess oxone to give bis-sulfones.

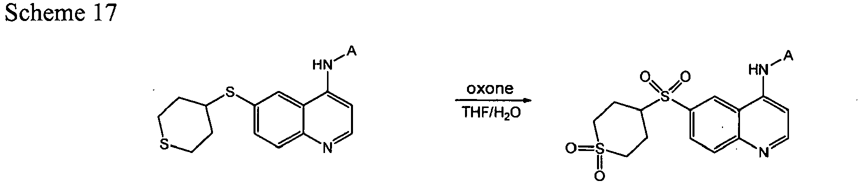

*N*-Boc groups were removed under acidic conditions.

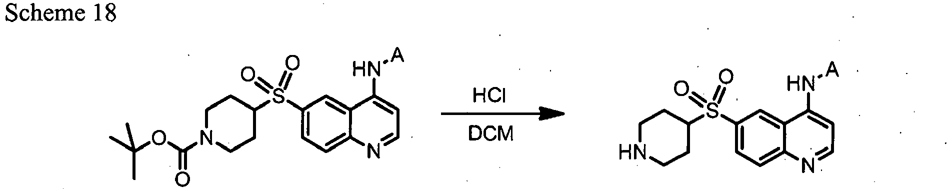

Sulfides can be oxidized to sulfoxides by addition of half and equivalent of oxone.

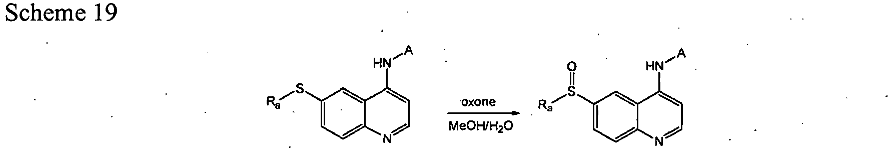

C6 amides were formed by addition of an aniline to the 4-chloroquinoline followed by a palladium catalyzed coupling with an amine to the aryl iodide in the presence of a carbon monoxide source.

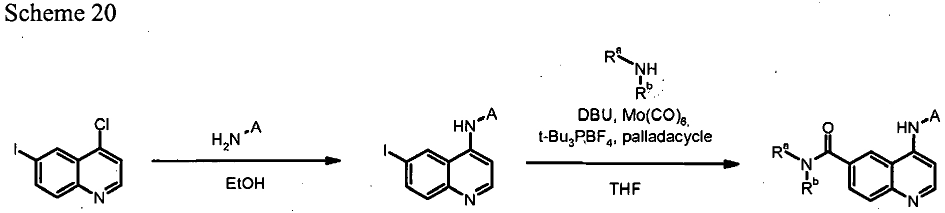

Sulfonamides were generated via the 4-chloro-6-iodoquinoline. A Suzuki coupling provided the benzylthioether which was then converted to the sulfonylchloride. Displacement of the chloride with an amine provides the sulfonylchloride. The backpocket group may be installed under thermal conditions in a variety of solvents. Some substrates required the addition of acid.

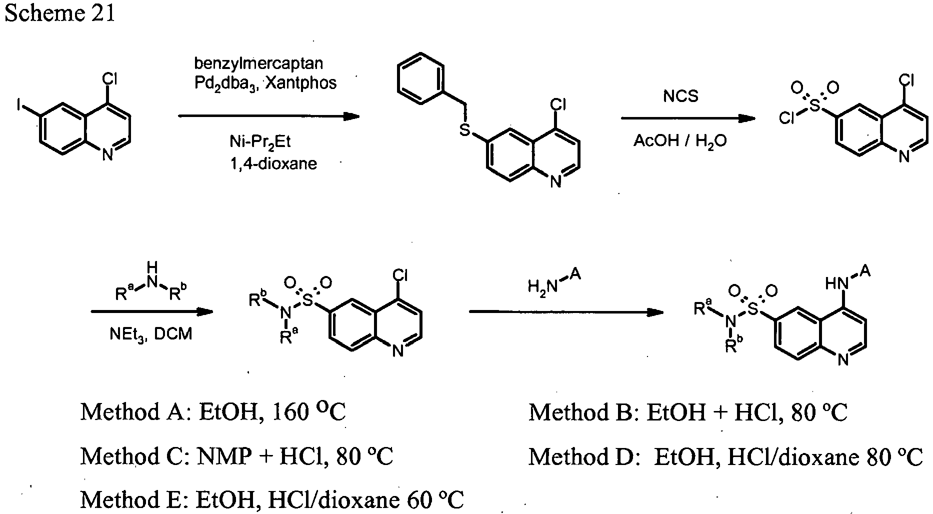

The benzothiazole of 4-(1,3-benzothiazol-5-ylamino)-*N*-(3-methyl-3-oxetanyl)-6-quinolinesulfonamide required a palladium catalyzed coupling for installation.

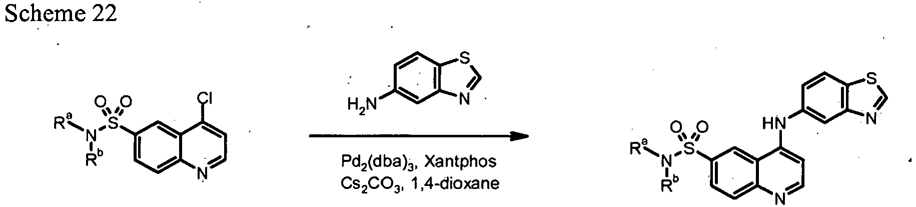

Installation of substitution on the aniline nitrogen was achieved via deprotonation with sodium hydride and reaction with the appropriate chloride.

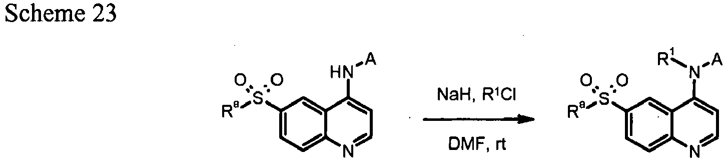

The benzoxazole backpocket group required construction on the quinoline core. Following addition of 4-amino-2-nitrophenol to the chloroquinoline, the nitro group was reduced, and formation of the five-membered ring occurred upon addition of triethylorthoformate.

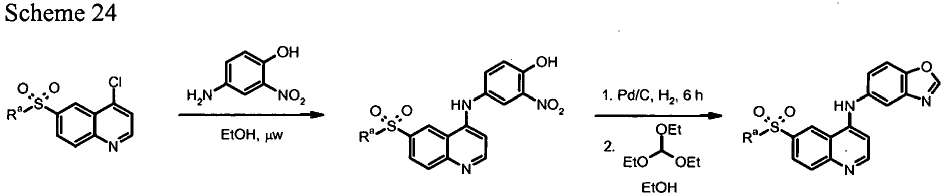

6-Sulfonylquinolines can also be synthesized by arrangement of the steps described in previous schemes. Following the palladium catalyzed coupling to install the sulfide, the appropriate aniline/amine is installed under acidic conditions. Oxidation to the sulfone is then achieved upon reaction with oxone.

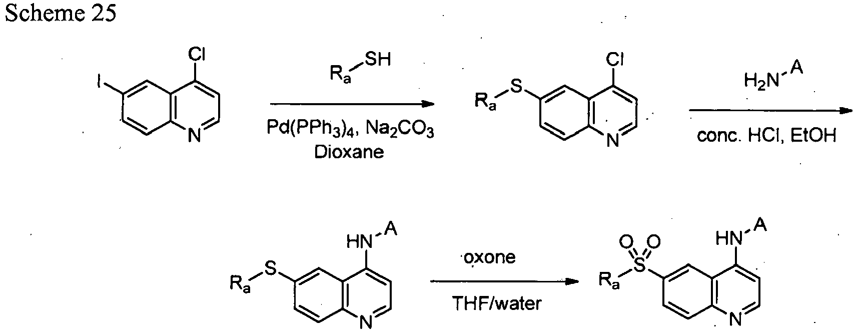

In addition to oxidation with substochiometric amounts of oxone, oxidation of sulfides to sulfoxides occurs with periodic acid in the presence of catalytic FeCl₃.

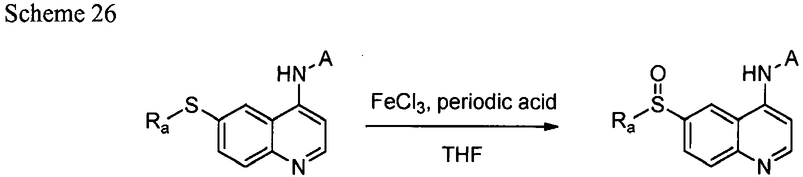

The present invention is also directed to a method of inhibiting RIP2 kinase which comprises contacting the kinase with a compound according to Formula (I), (II) or (III), or a salt, particularly a pharmaceutically acceptable salt, thereof. This invention is also directed to a method of treatment of a RIP2-mediated disease or disorder comprising administering a therapeutically effective amount of a compound according to Formula (I), (II) or (III), or a salt thereof, particularly a pharmaceutically acceptable salt thereof, to a patient, specifically a human, in need thereof. As used herein, "patient" refers to a human or other mammal. The invention is still further directed to the use of a compound according to Formula (I), (II) or (III), or a salt thereof, particularly a pharmaceutically acceptable salt thereof, or a pharmaceutical composition comprising a compound according to Formula (I), (II) or (III), or a salt thereof, particularly a pharmaceutically acceptable salt thereof, to inhibit RIP2 kinase and/or treat a RIP2 kinase-mediated disease or disorder.

The compounds of this invention may be particularly useful for treatment of the following RIP2-mediated diseases or disorders, particularly, uveitis, interleukin-1 converting enzyme (ICE, also known as Caspase-1) associated fever syndrome, dermatitis, type 2 diabetes mellitus, acute lung injury, arthritis (specifically rheumatoid arthritis), inflammatory bowel disorders (such as ulcerative colitis and Crohn's disease), prevention of ischemia reperfusion injury in solid organ transplant, liver diseases (non-alcohol steatohepatitis, alcohol steatohepatitis, autoimmune hepatitis), allergic diseases (such as asthma), autoimmune diseases (such as systemic lupus erythematosus and Multiple Sclerosis), transplant reactions (such as graft versus host disease) and granulomateous disorders, such as adult sarcoidosis, Blau syndrome, early-onset sarcoidosis, cutaneous sarcoidosis, Wegner's granulomatosis, and interstitial pulmonary disease. The compounds of this invention may be particularly useful in the treatment of uveitis, ICE fever, Blau Syndrome/early-onset sarcoidosis, ulcerative colitis, Crohn's disease, Wegener's granulamatosis and sarcoidosis.

Treatment of RIP2-mediated disease conditions, or more broadly, treatment of immune mediated disease, such as, but not limited to, allergic diseases, autoimmune diseases, prevention of transplant rejection and the like, may be achieved using a compound of this invention of as a monotherapy, or in dual or multiple combination therapy, particularly for the treatment of refractory cases, such as in combination with other anti-inflammatory and/or anti-TNF agents, which may be administered in therapeutically effective amounts as is known in the art. For example, the compounds of this invention may be administered in combination with corticosteroids and/or anti-TNF agents to treat Blau syndrome/early-onset sarcoidosis; or in combination with anti-TNF biologies or other anti-inflammatory biologies to treat Crohn's Disease; or in combination with low-dose corticosteroids and/or methotrexate to treat Wegener's granulamatosis or sarcoidosis or interstitial pulmonary disease; or in combination with a biologic (e.g. anti-TNF, anti-IL-6, etc.) to treat rheumatoid arthritis; or in combination with anti-IL6 and or methotrexate to treat ICE fever.

Examples of suitable anti-inflammatory agents include corticosteroids, particularly low-dose corticosteroids (such as Deltasone® (prednisone)) and anti-inflammatory biologies (such as Acterma® (anti-IL6R mAb) and Rituximab® (anti-CD20 mAb)). Examples of suitable anti-TNF agents include anti-TNF biologies (such as Enbrel® (etanecerpt)), Humira® (adalimumab), Remicade® (infliximab) and Simponi® (golimumab)).

This invention also provides a compound according to Formula (I), (II) or (III), or a salt thereof, particularly a pharmaceutically acceptable salt thereof, for use in the treatment or prophylaxis of RIP2-mediated diseases or disorders, for example those diseases and disorders mentioned hereinabove.

The invention also provides the use of a compound according to Formula (I), (II) or (III), or a salt thereof, particularly a pharmaceutically acceptable salt thereof, in the manufacture of a medicament for the treatment or prophylaxis of RIP2-mediated diseases or disorders, for example those diseases and disorders mentioned hereinabove. Accordingly, the present invention is also directed to pharmaceutical compositions comprising a compound according to Formula (I), (II) or (III), or a salt thereof, particularly a pharmaceutically acceptable salt thereof.

A therapeutically "effective amount" is intended to mean that amount of a compound that, when administered to a patient in need of such treatment, is sufficient to effect treatment, as defined herein. Thus, e.g., a therapeutically effective amount of a compound of Formula (I), (II) or (III), or a pharmaceutically acceptable salt thereof, is a quantity of an inventive agent that, when administered to a human in need thereof, is sufficient to modulate or inhibit the activity of RIP2 kinase such that a disease condition which is mediated by that activity is reduced, alleviated or prevented. The amount of a given compound that will correspond to such an amount will vary depending upon factors such as the particular compound (e.g., the potency (pIC₅₀), efficacy (EC₅₀), and the biological half-life of the particular compound), disease condition and its severity, the identity (e.g., age, size and weight) of the patient in need of treatment, but can nevertheless be routinely determined by one skilled in the art. Likewise, the duration of treatment and the time period of administration (time period between dosages and the timing of the dosages, e.g., before/with/after meals) of the compound will vary according to the identity of the mammal in need of treatment (e.g., weight), the particular compound and its properties (e.g., pharmaceutical characteristics), disease or condition and its severity and the specific composition and method being used, but can nevertheless be determined by one of skill in the art.

"Treating" or "treatment" is intended to mean at least the mitigation of a disease condition in a patient. The methods of treatment for mitigation of a disease condition include the use of the compounds in this invention in any conventionally acceptable manner, for example for prevention, retardation, prophylaxis, therapy or cure of a mediated disease. Specific diseases and conditions that may be particularly susceptible to treatment using a compound of this invention are described herein.

The compounds of the invention may be administered by any suitable route of administration, including both systemic administration and topical administration. Systemic administration includes oral administration, parenteral administration, transdermal administration, rectal administration, and administration by inhalation. Parenteral administration refers to routes of administration other than enteral, transdermal, or by inhalation, and is typically by injection or infusion. Parenteral administration includes intravenous, intramuscular, and subcutaneous injection or infusion. Inhalation refers to administration into the patient's lungs whether inhaled through the mouth or through the nasal passages. Topical administration includes application to the skin.

The compounds of the invention may be administered once or according to a dosing regimen wherein a number of doses are administered at varying intervals of time for a given period of time. For example, doses may be administered one, two, three, or four times per day. Doses may be administered until the desired therapeutic effect is achieved or indefinitely to maintain the desired therapeutic effect. Suitable dosing regimens for a compound of the invention depend on the pharmacokinetic properties of that compound, such as absorption, distribution, and half-life, which can be determined by the skilled artisan. In addition, suitable dosing regimens, including the duration such regimens are administered, for a compound of the invention depend on the condition being treated, the severity of the condition being treated, the age and physical condition of the patient being treated, the medical history of the patient to be treated, the nature of concurrent therapy, the desired therapeutic effect, and like factors within the knowledge and expertise of the skilled artisan. It will be further understood by such skilled artisans that suitable dosing regimens may require adjustment given an individual patient's response to the dosing regimen or over time as individual patient needs change.

For use in therapy, the compounds of the invention will be normally, but not necessarily, formulated into a pharmaceutical composition prior to administration to a patient. Accordingly, the invention is also directed to pharmaceutical compositions comprising a compound of the invention and a pharmaceutically-acceptable excipient.

The pharmaceutical compositions of the invention may be prepared and packaged in bulk form wherein an effective amount of a compound of the invention can be extracted and then given to the patient such as with powders, syrups, and solutions for injection. Alternatively, the pharmaceutical compositions of the invention may be prepared and packaged in unit dosage form. For oral application, for example, one or more tablets or capsules may be administered. A dose of the pharmaceutical composition contains at least a therapeutically effective amount of a compound of this invention (i.e., a compound of Formula (I), (II) or (III) or a salt, particularly a pharmaceutically acceptable salt, thereof). When prepared in unit dosage form, the pharmaceutical compositions may contain from 1 mg to 1000 mg of a compound of this invention.

The pharmaceutical compositions of the invention typically contain one compound of the invention. However, in certain embodiments, the pharmaceutical compositions of the invention contain more than one compound of the invention. In addition, the pharmaceutical compositions of the invention may optionally further comprise one or more additional pharmaceutically active compounds.

As used herein, "pharmaceutically-acceptable excipient" means a material, composition or vehicle involved in giving form or consistency to the composition. Each excipient must be compatible with the other ingredients of the pharmaceutical composition when commingled such that interactions which would substantially reduce the efficacy of the compound of the invention when administered to a patient and interactions which would result in pharmaceutical compositions that are not pharmaceutically-acceptable are avoided. In addition, each excipient must of course be of sufficiently high purity to render it pharmaceutically-acceptable.

The compounds of the invention and the pharmaceutically-acceptable excipient or excipients will typically be formulated into a dosage form adapted for administration to the patient by the desired route of administration. Conventional dosage forms include those adapted for (1) oral administration such as tablets, capsules, caplets, pills, troches, powders, syrups, elixirs, suspensions, solutions, emulsions, sachets, and cachets; (2) parenteral administration such as sterile solutions, suspensions, and powders for reconstitution; (3) transdermal administration such as transdermal patches; (4) rectal administration such as suppositories; (5) inhalation such as aerosols and solutions; and (6) topical administration such as creams, ointments, lotions, solutions, pastes, sprays, foams, and gels.

Suitable pharmaceutically-acceptable excipients will vary depending upon the particular dosage form chosen. In addition, suitable pharmaceutically-acceptable excipients may be chosen for a particular function that they may serve in the composition. For example, certain pharmaceutically-acceptable excipients may be chosen for their ability to facilitate the production of uniform dosage forms. Certain pharmaceutically-acceptable excipients may be chosen for their ability to facilitate the production of stable dosage forms. Certain pharmaceutically-acceptable excipients may be chosen for their ability to facilitate the carrying or transporting the compound or compounds of the invention once administered to the patient from one organ, or portion of the body, to another organ, or portion of the body. Certain pharmaceutically-acceptable excipients may be chosen for their ability to enhance patient compliance.

Suitable pharmaceutically-acceptable excipients include the following types of excipients: diluents, fillers, binders, disintegrants, lubricants, glidants, granulating agents, coating agents, wetting agents, solvents, co-solvents, suspending agents, emulsifiers, sweeteners, flavoring agents, flavor masking agents, coloring agents, anti-caking agents, humectants, chelating agents, plasticizers, viscosity increasing agents, antioxidants, preservatives, stabilizers, surfactants, and buffering agents. The skilled artisan will appreciate that certain pharmaceutically-acceptable excipients may serve more than one function and may serve alternative functions depending on how much of the excipient is present in the formulation and what other ingredients are present in the formulation.

Skilled artisans possess the knowledge and skill in the art to enable them to select suitable pharmaceutically-acceptable excipients in appropriate amounts for use in the invention. In addition, there are a number of resources that are available to the skilled artisan which describe pharmaceutically-acceptable excipients and may be useful in selecting suitable pharmaceutically-acceptable excipients. Examples include Remington's Pharmaceutical Sciences (Mack Publishing Company), The Handbook of Pharmaceutical Additives (Gower Publishing Limited), and The Handbook of Pharmaceutical Excipients (the American Pharmaceutical Association and the Pharmaceutical Press).

The pharmaceutical compositions of the invention are prepared using techniques and methods known to those skilled in the art. Some of the methods commonly used in the art are described in Remington's Pharmaceutical Sciences (Mack Publishing Company).

In one aspect, the invention is directed to a solid oral dosage form such as a tablet or capsule comprising an effective amount of a compound of the invention and a diluent or filler. Suitable diluents and fillers include lactose, sucrose, dextrose, mannitol, sorbitol, starch (e.g. corn starch, potato starch, and pre-gelatinized starch), cellulose and its derivatives (e.g. microcrystalline cellulose), calcium sulfate, and dibasic calcium phosphate. The oral solid dosage form may further comprise a binder. Suitable binders include starch (e.g. corn starch, potato starch, and pre-gelatinized starch), gelatin, acacia, sodium alginate, alginic acid, tragacanth, guar gum, povidone, and cellulose and its derivatives (e.g. microcrystalline cellulose). The oral solid dosage form may further comprise a disintegrant. Suitable disintegrants include crospovidone, sodium starch glycolate, croscarmelose, alginic acid, and sodium carboxymethyl cellulose. The oral solid dosage form may further comprise a lubricant. Suitable lubricants include stearic acid, magnesium stearate, calcium stearate, and talc.

### EXAMPLES

The following examples illustrate the invention. These examples are not intended to limit the scope of the present invention, but rather to provide guidance to the skilled artisan to prepare and use the compounds, compositions, and methods of the present invention. While particular embodiments of the present invention are described, the skilled artisan will appreciate that various changes and modifications can be made without departing from the spirit and scope of the invention.

Names for the intermediate and final compounds described herein were generated using a commercially available software naming program. It will be appreciated by those skilled in the art that in certain instances such programs will name a structurally depicted compound as a single tautomer of that compound. It is to be understood that any reference to a named compound or a structurally depicted compound is intended to encompass all tautomers of such compounds and any mixtures of tautomers thereof.

In the following experimental descriptions, the following abbreviations may be used:

| **Abbreviation** | **Meaning** |
|---|---|
| AcOH | acetic acid |
| aq | aqueous |
| brine | saturated aqueous NaCl |
| CH₂Cl₂, DCM | methylene chloride |
| CH₃CN or MeCN | acetonitrile |
| CH₃NH₂ | methylamine |
| d | day |
| DMF | *N,N-*dimethylformamide |
| DMSO | dimethylsulfoxide |
| EDC | 1-ethyl-3-(3-dimethylaminopropyl) carbodiimide |
| equiv | equivalents |
| Et | ethyl |
| Et₃N | triethylamine |
| Et₂O | diethyl ether |
| EtOAc | ethyl acetate |
| h, hr | hour |
| HATU | *O*-(7-Azab6nzotriazol-1yl)-*N,N,N',N'*-tetramethylyronium hexafluorophosphate |
| HCl | hydrochloric acid |
| *i*-Pr₂NEt | *N,N'*-diisopropylethylamine |
| KO*t*-Bu | potassium *tert*-butoxide |
| LCMS | liquid chromatography-mass spectroscopy |
| Me | methyl |
| MeOH or CH₃OH | methanol |
| MgSO₄ | magnesium sulfate |
| min | minute |
| MS | mass spectrum |
| µw | microwave |
| NaBH₄ | sodium borohydride |
| Na₂CO₃ | sodium carbonate |
| NaHCO₃ | sodium bicarbonate |
| NaOH | sodium hydroxide |
| Na₂SO₄ | sodium sulfate |
| NH₄Cl | ammonium chloride |
| NiCl₂•6H₂O | nickel (II) chloride hexahydrate |
| NMP | *N*-methyl-2-pyrrolidone |
| Ph | phenyl |
| rt | room temperature |
| satd | saturated |
| SCX | strong cation exchange |
| SPE | solid phase extraction |
| TFA | trifluoroacetic acid |
| THF | tetrahydrofuran |
| *t*_{R} | retention time |

### Preparation 1

### 4-Chloro-6-(methylsulfonyl)quinolines

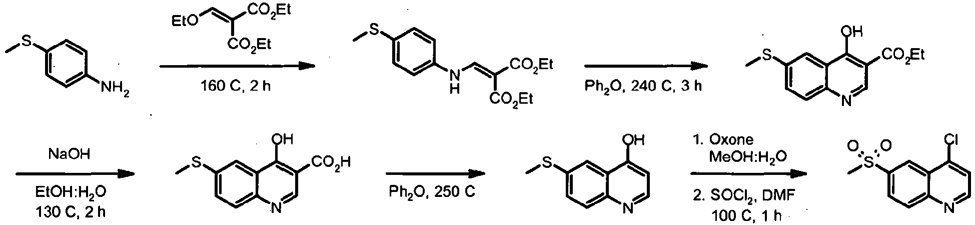

Step 1. Diethyl ({[4-(methylthio)phenyl]amino}methylidene)propanedioate: 4-(methylthio)aniline (42.3 ml, 340 mmol) and diethyl [(ethyloxy)methylidene]-propanedioate (110 ml, 540 mmol) were combined and heated to 160 °C for 2 hours in a round bottom flask with a reflux condenser attached. The condenser was then removed, and the mixture was heated for an additional hour. The reaction mixture was then cooled to room temperature where it solidified overnight. The solid mass was broken up, suspended in hexanes, and filtered. The cake was rinsed with hexanes three times. A pale yellow solid was obtained (92 g, 83 %). MS (m/z) 310.1 (M+H⁺).

Step 2. Ethyl 4-hydroxy-6-(methylthio)-3-quinolinecarboxylate: To diphenyl ether (62 ml, 390 mmol) at 250 °C was added diethyl ({[4-(methylthio)phenyl]amino} methylidene)propanedioate (10 g, 32 mmol) in a steady addition. Reaction was vigorously stirred and heated for the next 3 hours before cooling to room temperature overnight. In the morning, the solid mass was transferred to a beaker, broken up, and suspended in 500 mL of hexanes before being filtered. The solid was rinsed with hexanes to afford the desired product (8.5 g, 100 %). MS (m/z) 264.1 (M+H⁺).

Step 3. 4-Hydroxy-6-(methylthio)-3-quinolinecarboxylic acid: Ethyl 4-hydroxy-6-(methylthio)-3-quinolinecarboxylate (8.5 g, 19 mmol) was dissolved in ethanol (16 ml) before NaOH (3.9 g, 97 mmol) and water (32 ml) were added. The suspension was heated to 130 °C for 2 hours. The reaction was cooled to room temperature and stirred for 72 hours until reaction was complete. The residual ethanol was removed by rotary evaporation and the aqueous solution was acidified using conc HCl. The solid that formed was filtered and was washed with water and ether and was then air dried. The solid was then triturated with acetone and filtered to give a tan solid (4.43 g, 97 %). MS (m/z) 236.0 (M+H⁺).

Step 4. 6-(Methylthio)-4-quinolinol: 4-Hydroxy-6-(methylthio)-3-quinolinecarboxylic acid (49.8 g, 212 mmol) was steadily added to diphenyl ether (500 ml, 3.2 mol) at 250 °C. Once the reaction was complete (solution becomes homogeneous) it was cooled to room temperature. 500 mL of hexanes was added to the reaction which was then filtered and the cake was rinsed with hexanes and dried. Title compound was obtained as a brown solid (25.8 g, 60 %). MS (m/z) 192.1 (M+H⁺).

Step 5. 6-(Methylsulfonyl)-4-quinolinol: 6-(Methylthio)-4-quinolinol (1.6 g, 8.6 mmol) and oxone (5.8 g, 9.4 mmol) were suspended in methanol (34 ml) and water (34 ml) and the mixture was stirred at room temperature for 4 hours. Upon filtration the cake was washed with methanol and the filtrate was concentrated. The residue was suspended in methanol, filtered, and concentrated. The residue was triturated with acetone and concentrated to a yellow solid. MS (m/z) 224.1 (M+H⁺).

Step 6. 4-Chloro-6-(methylsulfonyl)quinoline: 6-(Methylsulfonyl)-4-quinolinol (3.4 g, 9.1 mmol) was suspended in thionyl chloride (26 ml, 360 mmol) before DMF (0.035 ml, 0.45 mmol) was added and the reaction was heated to 100 °C for 1 hour. After cooling to room temperature the mixture was concentrated. Reaction was incomplete and was resubjected to reaction conditions. Complete conversion was observed after 1 hour. The reaction mixture was cooled and concentrated to a yellow solid (1.99 g, 91 %). MS (m/z) 242.0, 244.0 (M+H⁺).

### Preparation 2

### 4-Chloro-6-(methylsulfonyl)quinoline

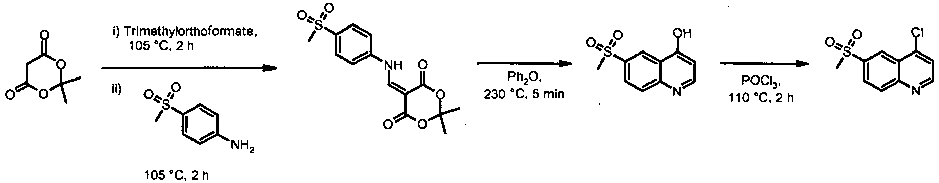

Step 1: 2,2-Dimethyl-5-({[4-(methylsulfonyl)phenyl]amino}methylidene)-1,3-dioxane-4,6-dione: A mixture of 2,2-dimethyl-1,3-dioxane-4,6-dione (51 g, 350 mmol) and trimethyl orthoformate (500 mL) was heated at reflux for 2h at which time 4-(methylsulfonyl)aniline (50 g, 290 mmol) was added. The reaction was stirred at 105 °C for 2h, cooled to room temperature, and filtered. The filter cake was washed with methanol and dried to provide pure 2,2-dimethyl-5-({[4-(methylsulfonyl)phenyl]amino}methylidene)-1,3-dioxane-4,6-dione in quantitative yield. ¹H NMR(400 MHz, DMSO-*d*₆) δ 11.36 (d, *J* = 14.4 Hz, 1H), 8.68 (d, *J* = 14.4 Hz, 1H), 7.91 - 8.00 (m, 2H), 7.84 (d, *J* = 8.8 Hz, 2H), 3.25 (s, 3H), 1.69 (s, 6H); MS (m/z) 326 (M+H⁺).

Step 2: 6-(Methylsulfonyl)-4-quinolinol: To a 3-neck round bottom flask containing diphenylether heated to 245 °C (internal temperature) was added 2,2-dimethyl-5-({[4-(methylsulfonyl)phenyl]amino}methylidene)-1,3-dioxane-4,6-dione (21 g, 12.6 mmol) over 5 minutes. The internal temperature dropped to 230 °C over the course of the addition. The reaction was allowed to cool to 60 °C and the mixture was diluted with hexanes (300 mL) and filtered to provide the desired product (∼15g) which contained some residual diphenylether. The reaction was repeated 3 additional times. The 4 batches were combined to provide the desired product (50 g) w/ some diphenylether present. The crude product was suspended in refluxing methanol (1.5 L), diluted with hexanes (500 mL) and filtered to provide pure 6-(methylsulfonyl)-4-quinolinol (47.3 g, 212 mmol, 80 % yield). ¹H NMR (400 MHz, DMSO-*d*₆) δ 12.14 (br. s., 1H), 8.58 (d, *J* = 2.3 Hz, 1H), 8.11 (dd, *J* = 8.8, 2.3 Hz, 1H), 8.03 (d, *J* = 7.6 Hz, 1H), 7.75 (d, *J* = 8.6 Hz, 1H), 6.17 (d, *J* = 7.3 Hz, 1H), 3.25 (s, 3H); MS (m/z) 224 (M+H⁺).

Step 3: 4-Chloro-6-(methylsulfonyl)quinoline: 6-(Methylsulfonyl)-4-quinolinol (23 g, 103 mmol) and phosphorus oxychloride (380 ml, 4.1 mol) were combined and heated at 110 °C for 2 h. The reaction was concentrated to dryness. The residue was treated with saturated sodium carbonate (CAUTION: gas evolution) to quench any residual POCl₃. The suspension was diluted with water and filtered to provide pure 4-chloro-6-(methylsulfonyl)quinoline (23 g, 95 mmol, 92 % yield). ¹H NMR (500 MHz, DMSO-*d*₆) δ 9.05 (d, *J* = 4.9 Hz, 1H), 8.74 (s, 1H), 8.29 - 8.40 (m, 2H), 7.98 (d, *J* = 4.6 Hz, 1H), 3.39 (s, 3H); MS (m/z) 242 (M+H⁺).

The following intermediates can also be made in an analogous manner beginning with the appropriate commercial aniline:

| Prep # | Structure | Name | MS (M+H)⁺ | NMR |
|---|---|---|---|---|
| 3 | 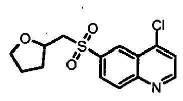 | 4-chloro-6-[(tetrahydro-2-furanylmethyl) sulfonyl]quinoline | 312 | ¹H NMR (CHLOROFORM-d) δ: 8.97 (d, J = 4.5 Hz, 1H), 8.93 (s, 1H), 8.28 - 8.36 (m, J = 8.8 Hz,1H), 8.24 (d, J = 8.8, 2.0 Hz, 1H), 7.66 (d, J = 4.8 Hz, 1H), 4.29 - 4.45 (m, 1H), 3.63 - 3.82 (m, 2H), 3.46 - 3.60 (m, 1H), 3.30 - 3.42 (m, 1H), 2.10-2.26 (m, 1H), 1.83 - 1.97 (m, 2H), 1.70 - 1.75 (m, 1H) |
| 4 | 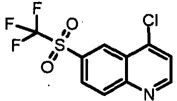 | 4-chloro-6-[(trifluoromethyl) sulfonyl]quinoline | 296 | ¹H NMR (CHLOROFORM-d) δ: 9.05 (m, 2 H), 8.43 (d, J = 8.8 Hz, 1H), 8.28 (dd, J = 8.8, 1.5 Hz, 1H), 7.73 (d, J = 4.5 Hz, 1H) |

### Preparation 5

### 4-Chloro-6-iodoquinoline

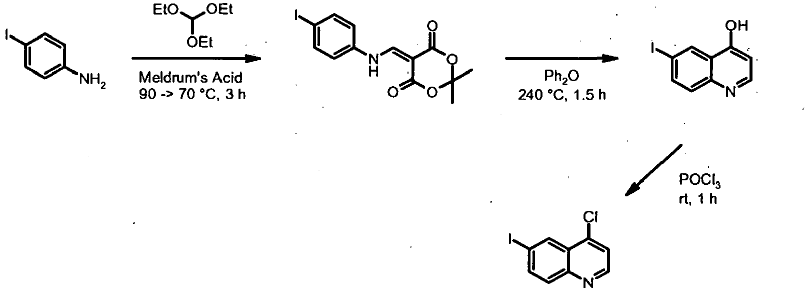

Step 1. 5- {[(4-Iodophenyl)amino]methylidene}-2,2-dimethyl-1,3-dioxane-4,6-dione: A mixture of Meldrum's acid (227 g, 1.58 mol) and triethyl orthoformate (262 mL, 1.58 mol) were heated to 90 °C for 1.5 hours before being cooled to 70 °C where 4-iodoaniline (300 g, 1.37 mol) was added in portions. In order for the reaction to be continually stirred via mechanical stirrer, MeOH was added (500 mL). Once the addition was complete, the reaction was stirred at 70 °C for another 1 hour before it was diluted with MeOH (1.5 L) and the suspension was filtered. The cake was broken up and washed with MeOH (2 x 1 L) and dried under vacuum overnight to afford the title compound as a tan solid (389 g, 75 %). ¹H NMR (400 MHz, DMSO-d₆) δ 11.22 (d, *J* = 14.4 Hz, 1H), 8.55 (d, *J* = 14.7 Hz, 1H), 7.76 (d, *J* = 8.8 Hz, 2H), 7.41 (d, *J* = 8.8 Hz, 2H), 1.67 (s, 6H).

Step 2. 6-Iodo-4-quinolinol: To diphenyl ether (1.3 ml, 8.0 mol) at 240 °C was added 5-{[(4-iodophenyl)amino]methylidene}-2,2-dimethyl-1,3-dioxane-4,6-dione (120 g, 322 mmol) portion-wise. The reaction was heated for 1.5 hours before being cooled to room temperature and poured into 1.5 L of hexanes. The resulting suspension was then filtered. The cake was broken up and rinsed with hexanes (2 x 500 mL). The solid was dried under vacuum to afford the title compound as a brown solid (80 g, 82 %). ¹H NMR (400 MHz, DMSO-d₆) δ 11.89 (d, *J*= 4.0 Hz, 1H), 8.36 (d, *J*= 2.3 Hz, 1H), 7.89 - 7.98 (m, 2H), 7.37 (d, *J*= 8.6 Hz, 1H), 6.07 (dd, *J*= 7.5, 1.1 Hz, 1H); MS (m/z) 272.0 (M+H⁺).

Step 3. 4-Chloro-6-iodoquinoline: 4-Hydroxy-6-iodoquinoline (100 g, 369 mmol) was suspended in POCl₃ (340 ml, 3.7 mol) at room temperature. After 1 hour it was concentrated and the resulting residue was placed in an ice water bath and carefully neutralized using saturated aqueous Na₂CO₃. The resulting brown suspension was filtered and the solid was rinsed with water (2 x 500 mL) and dried under vacuum overnight. 4-chloro-6-iodoquinoline was obtained as a brown solid (103 g, 92 %). ¹H NMR (400 MHz, DMSO-d₆) δ 8.88 (d, *J* = 4.8 Hz, 1H), 8.54 (d, *J* = 1.8 Hz, 1H), 8.15 (dd, *J* = 8.8, 2.0 Hz, 1H), 7.88 (d, *J* = 8.8 Hz, 1H), 7.82 (d, *J* = 4.8 Hz, 1H); MS (m/z) 289.9 (M+H⁺).

### Preparation 6

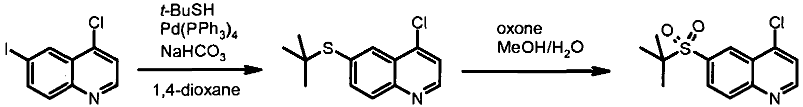

Step 1: 4-chloro-6-[(1,1-dimethylethyl)thio]quinoline: To a flask was added quinoline (25 g, 86 mmol), tetrakis(triphenylphosphonium)palladium(0) (5.0 g, 4.3 mmol), and sodium carbonate (23 g, 216 mmol). The flask was then evacuated and backfilled with nitrogen three times. 1,4-Dioxane (200 ml) was then added followed by thiol. The reaction was then heated to 50 °C overnight. Reaction was not complete and heating was continued at 70 °C for an additional 20 hours. Upon completion, the reaction was cooled to rt and poured into 200 mL of 2M aq 5:1 Na₂S₂O₃:NaHCO₃. The organics were collected and the aqueous layer was backextracted with EtOAc (2 x 200 mL). The combined organics were dried over sodium sulfate, filtered, and concentrated. The crude material was purified by flash chromatography (0 -> 20 % EtOAc in hexanes) and desired fractions were combined and concentrated to an oil which solidified upon standing to provide 9.4 g (43%) of the desired product. MS (m/z) 252.1 (M+H⁺).

Step 2: 4-chloro-6-[(1,1-dimethylethyl)sulfonyl]quinoline: 4-Chloro-6-[(1,1-dimethylethyl)thio]quinoline (9.4 g, 37 mmol) was suspended in methanol (100 ml) and water (100 ml) before oxone (25.2 g, 41.1 mmol) was added and the reaction was stirred at rt until complete by LCMS (3 hours). The methanol was removed in vacuo and the heterogeneous aqueous solution was extracted 3x with 100 mL EtOAc. The combined organics were concentrated to provide 8.5 g (80%) of a yellow powder. ¹H NMR (400 MHz, DMSO-d₆) δ ¹H NMR (DMSO-d₆) δ: 9.08 (d, J = 4.8 Hz, 1H), 8.63 (d, J = 2.0 Hz, 1H), 8.36 (d, J = 8.8 Hz, 1H), 8.20 (dd, J = 8.8, 2.0 Hz, 1H), 8.00 (d, J = 4.8 Hz, 1H), 1.32 (s, 9H); MS (m/z) 284.1 (M+H⁺).

The following intermediates can also be made in an analogous manner:

| Prep # | Structure | Name | MS (M+H)⁺ | NMR |
|---|---|---|---|---|
| 7 | 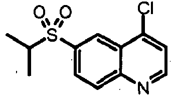 | 4-chloro-6-[(1-methylethyl)thio] quinoline | 270 | ¹H NMR (DMSO-d₆) d: 9.06 (d, J = 4.8 Hz, 1H), 8.67 (d, J = 1.8 Hz, 1H), 8.36(d, J = 8.8Hz, 1H), 8.24 (dd, J = 8.6, 2.0 Hz, 1H), 7.99 (d, J = 4.5 Hz, 1H), 3.65 (spt, J = 6.8 Hz, 1H), 1.22 (d, 6H) |
| 8 | 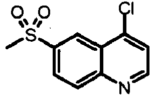 | 4-chloro-6-(methylsulfonyl) quinoline | 242 | ¹H NMR (500 MHz, DMSO-*d*₆) δ 9.05 (d, *J*= 4.9 Hz, 1H), 8.74 (s, 1H), 8.29-8.40 (m, 2H), 7.98 (d, *J*= 4.6 Hz, 1H), 3.39 (s, 3H) |
| 9 | 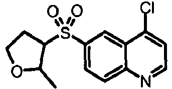 | 4-chloro-6-[(2-methyltetrahydro-3-furanyl)sulfonyl] quinoline (cis/trans relationship unknown; racemic mixture) | 312 | ¹H NMR (400 MHz, DMSO-*d*₆) δ ppm 9.07 (d, *J*=4.8 Hz, 1H), 8.71 (d, *J*=1.8 Hz, 1H), 8.21 - 8.50 (m, 2 H), 8.00 (d, *J*=4.5 Hz, 1H), 4.29-4.45 (m, 1H), 4.21 (t, *J*=6.7 Hz, 1H), 3.96 (td, *J*=8.4, 3.4 Hz, 1H), 3.44-3.60 (m, 1 H), 2.11 - 2.29 (m, 1H), 2.01 (s, 1H), 1.50(d, 3H) |

### Preparation 10 6-[(1-Methylethyl)thio]-4-quinolinol

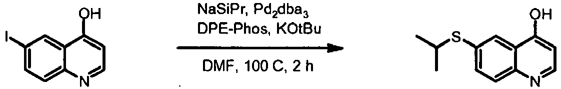

To a flask was added 4-hydroxy-6-iodoquinoline (5.0 g, 18 mmol), sodium isopropylthiolate (1.8 g, 18 mmol), Pd₂dba₃ (0.84 g, 0.92 mmol), DPE-Phos (0.50 g, 0.92 mmol), and KOtBu (4.6 g, 41 mmol). The flask was evacuated and backfilled with nitrogen three times. N,N-Dimethylformamide (DMF) (62 ml) was added. The mixture was stirred at room temperature for 10 min before being heated to 100 °C for 2 hours. It was complete by LCMS so it was cooled to room temperature and concentrated. The crude was taken up in a mixture of DCM and EtOAc and filtered through celite. The pad of celite was rinsed with copious amounts of EtOAc and the mother liquor was concentrated. This residue was dissolved in EtOAc and washed three times with aq 5:1 Na₂S₂O₃:NaHCO₃. The combined aqueous layers were back-extracted once with EtOAc. The combined organics were dried over magnesium sulfate, filtered, and concentrated. The material was then purified by flash chromatography (0 -> 10 % 2M NH₃/MeOH in DCM). Desired fractions were combined and concentrated to a brown oil that eventually became a foam (1.8 g, 41 %). ¹H NMR (400 MHz, DMSO-d₆) δ 11.85 (br. S., 1H), 8.03 (d, *J* = 2.0 Hz, 1H), 7.90 (d, *J* = 7.3 Hz, 1H), 7.65 (dd, *J* = 8.6, 2.0 Hz, 1H), 7.52 (d, *J* = 8.6 Hz, 1H), 6.05 (d, *J* = 7.3 Hz, 1H), 3.47 (m, 1H), 1.24 (d, *J* = 6.8 Hz, 6H); MS (m/z) 220.1 (M+H⁺). This material can be carried forward to 4-chloro-6-[(1-methylethyl)sulfonyl]quinoline in an analogous manner to the methyl sulfone in preparation 1.

### Preparation 11

### 4-Chloro-6-(ethylsulfonyl)quinoline

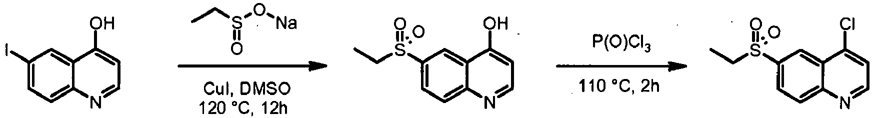

Step 1. 6-(Ethylsulfonyl)-4-quinolinol: To a solution of 6-iodo-4-quinolinol (250 mg, 0.92 mmol) in DMSO (3.7 mL) was added copper(I) iodide (350 mg, 1.85 mmol) and sodium isopropanesulfinate (240 mg, 1.85 mmol). The mixture was evacuated and purged with N2 three times and then heated at 120 °C overnight. After cooling to r.t., the reaction mixture was loaded onto an SCX cartridge, washed with MeOH, and the product was then eluted with 2N NaOH/MeOH. Some product eluted with the MeOH wash and was loaded onto another SCX cartridge and the process was repeated to give 140 mg (64%) of 6-(ethylsulfonyl)-4-quinolinol. MS (m/z) 238.1 (M+H)

Step 2. 4-Chloro-6-(ethylsulfonyl)quinoline: 6-(Ethylsulfonyl)-4-quinoliriol (50 mg, 0.21 mmol) was suspended in POCl₃ (1 mL, 11 mmol) and heated to 110 °C for 2 hours. Upon reaction completion it was cooled to room temperature and concentrated azeotroping with toluene. The residue was taken up in saturated aqueous NaHCO₃ slowly. A precipitate did not from so the aqueous mixture was extracted with DCM. The organic was concentrated to provide 4-chloro-6-(ethylsulfonyl)quinoline. MS (m/z) 256.0 (M+H)

### Preparation 12

### 1,2,3-Benzothiadiazol-5-amine

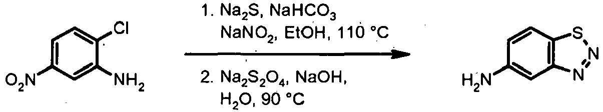

2-Chloro-5-nitroaniline (5.0 g, 29 mmol) was dissolved in ethanol (72 ml) and heated to 110 °C. An aqueous solution (30 mL) of sodium bicarbonate (3.7 g, 44 mmol) and sodium sulfide (3.4 g, 44 mmol) was added dropwise. The reaction was heated for 1 hour before cooling to room temperature where it was poured over 400 mL of water with several large chucks of ice. The resulting suspension was filtered and sodium nitrite (2.0 g, 29 mmol) was added to the filtrate. The filtrate was then poured into 10 mL of H₂SO₄ with several more pieces of dry ice. The mixture was allowed to warm to room temperature where it was stirred for 1 hour before being filtered. The collected solid was air-dried overnight (3.3 g, 64 %). ¹H NMR (400 MHz, DMSO-d₆) δ 9.57 (d, *J* = 2.3 Hz, 1H), 8.68 (d, *J* = 9.0 Hz, 2H), 8.59 (dd, *J* = 9.0, 2.3 Hz, 2H). 5-nitro-1,2,3-benzothiadiazole (3.34 g, 18.44 mmol) was suspended in 2M aqueous NaOH (100 mL) at heated to 90 °C. Sodium dithionate (12 g, 58.2 mmol) was then added in small portions before further heating the mixture to boiling. After 2 hours of heating the reaction was cooled to room temperature and extracted with ether (3 x 150 mL). The combined organics were dried over sodium sulfate, filtered, and concentrated to obtain pure desired product (78 mg, 3 %). ¹H NMR (400 MHz, DMSO-d₆) δ 8.00 (d, *J=* 8.8 Hz, 1H), 7.60 (d, *J=* 2.0 Hz, 1H), 7.15 (dd, *J* = 8.7, 2.1 Hz, 1H), 5.77 (s, 2H).

### Preparation 13

### 5-Fluoro-1H-1,2,3-benzotriazol-6-amine

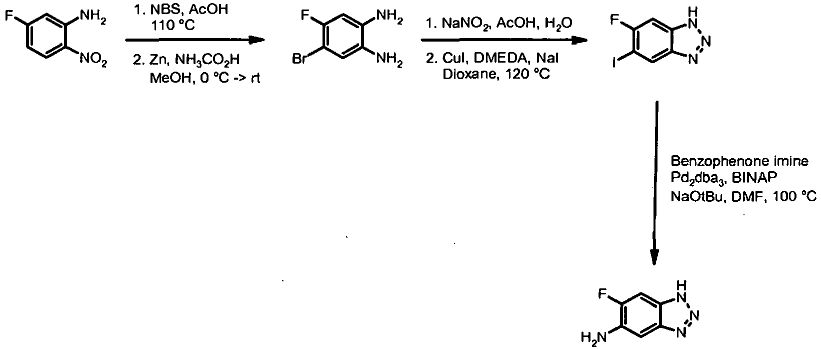

Step 1. 4-Bromo-5-fluoro-1,2-benzenediamine: To a flask was added 5-fluoro-2-nitroaniline (8.9 g, 57 mmol), NBS (10 g, 56 mmol), and acetic acid (500 mL), and the reaction was heated to 110 °C. Once complete it was cooled to room temperature and poured into 3 L of water. The resulting suspension was filtered and the solid was washed with 500 mL of water. Solid was air dried overnight (12.7 g, 91 %). ¹H NMR (400 MHz, DMSO-d₆) δ 8.27 (d, *J* = 7.3 Hz, 1H), 7.73 (br. S., 2H), 6.94 (d, *J* = 11.0 Hz, 1H). 4-bromo-5-fluoro-2-nitroaniline (0.53 g, 2.255 mmol), zinc (0.89 g, 13.5 mmol), and methanol (32 ml) were added to the flask and argon was bubbled through the solution for 1 hour. Ammonium formate (1.42 g, 22.6 mmol) was then added in several portions. The reaction was then stirred at room temperature overnight and was complete by LCMS in the morning. It was filtered through a pad of celite and the filtrate was concentrated. The residue was partitioned between water and EtOAc and the organic layer was collected. The aqueous layer was back-extracted using EtOAc (2 x 50 mL). The combined organics were dried over sodium sulfate, filtered, and concentrated (378 mg, 82 %). MS (m/z) 205.0, 207.0 (M⁺, M+2⁺).

Step 2. 5-Fluoro-6-iodo-1*H*-1,2,3-benzotriazole: 2-Amino-4-bromo-5-fluoroaniline (9.2 g, 45 mmol) was dissolved in acetic acid (190 ml) and water (260 ml) before sodium nitrite (3.1 g, 45 mmol) in 10 mL of water was added dropwise. The reaction was stirred at room temperature for 1 hour before the solution was cooled to 0 °C and ammonium hydroxide was added to adjust the pH to ~11. The mixture was extracted with EtOAc (3 x 500 mL), and the combined organics were dried over sodium sulfate, filtered, and concentrated to a brown solid (9.0 g, 71 %). MS (m/z) 215.9, 217.9 (M⁺, M+2⁺). 5-bromo-6-fluoro-1H-1,2,3-benzotriazole (1.0 g, 4.6 mmol), copper(I) iodide (88 mg, 0.46 mmol), and sodium iodide (2.8 g, 19 mmol) were added to a flask which was subsequently purged with argon. 1,4-Dioxane (7.0 ml) was added followed by N,N'-ethylenediamine (0.10 ml, 0.93 mmol). The reaction was heated to 120 °C overnight. It was cooled to room temperature and partitioned between water and EtOAc. The organics were collected and the aqueous layer was back-extracted with EtOAc. The combined organics were dried over sodium sulfate, filtered, and concentrated (420 mg, 35 %). MS (m/z) 263.9 (M+H⁺).

Step 3. 5-Fluoro-1H-1,2,3-benzotriazol-6-amine: To a vial was added 5-fluoro-6-iodo-1H-1,2,3-benzotriazole (400 mg, 1.5 mmol), sodium tert-butoxide (290 mg, 3.0 mmol), Pd₂dba₃ (280 mg, 0.30 mmol), benzophenone imine (383 µL, 2.28 mmol), and BINAP (380 mg, 0.61 mmol). The vial was purged with argon before N,N-Dimethylformamide (DMF) (7.6 ml) was added. The mixture was heated at 100 °C overnight. It was cooled to room temperature and quenched with saturated aqueous NH₄Cl. The mixture was extracted with EtOAc three times and the combined organics were washed with water, dried over sodium sulfate, filtered, and concentrated. The imine was removed by taking the residue and dissolving in EtOH before adding 2M aqueous NaOH and stirring overnight at room temperature. This mixture was neutralized with 2M HCl, extracted with EtOAc, and the organics were concentrated.

The following intermediates, used for the preparation of named example compounds, were synthesized using methods analogous to the one described above.

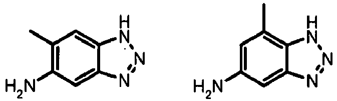

### Preparation 14

### 3-Fluoro-1H-indazol-6-amine

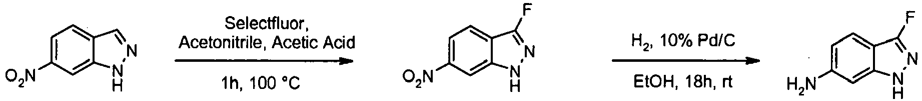

Step 1: 3-Fluoro-6-nitro-1H-indazole: 6-Nitroindazole (300 mg, 1.8 mmol), acetonitrile (3.0 ml), acetic acid (613 µl), and Selectfluor (847 mg, 2.4 mmol) were added to a microwave vial and irradiated at 100 °C for 1h. The reaction was concentrated, suspended in DCM and pipetted directly onto a 25g biotage snap column and purified via column chromatography (Biotage SP-1, 25g, 0-70% ethyl acetate/hexane) to yield 3-fluoro-6-nitro-1H-indazole (130 mg, 0.718 mmol, 39.0 % yield). ¹H NMR (400 MHz, DMSO-*d*₆) δ 13.33 (br. S., 1H), 8.42 (s, 1H), 7.92 - 8.06 (m, 2H).

Step 2: 3-Fluoro-1H-indazol-6-amine: To a solution of palladium on carbon (38 mg, 36 µmol) in ethyl acetate (300 µL) was added a solution of 3-fluoro-6-nitro-1H-indazole (130 mg, 0.72 mmol) in ethanol (3.6 mL). The reaction was flushed with nitrogen (3x), then flushed with hydrogen (2x) and stirred under a hydrogen atmosphere at room temperature for 18h. The reaction was filtered through celite and concentrated to provide 3-fluoro-1H-indazol-6-amine (119 mg, 0.709 mmol, 99 % yield). ¹H NMR (400 MHz, DMSO-*d*₆) δ 11.65 (s, 1H), 7.28 (d, *J* = 8.6 Hz, 1H), 6.52 (dd, *J* = 8.8, 1.8 Hz, 1H), 6.39 (s, 1H), 5.48 (s, 2H); MS (m/z) 224 (M+H⁺).

### Preparation 15

### 1-Benzofuran-4-amine

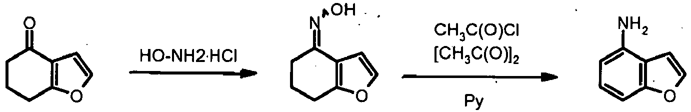

Step 1. 3-({4-[(5-Fluoro-1H-indazol-3-yl)amino]-2-pyrimidinyl}amino)-N,N-dimethyl-benzenesulfonamide: 6,7-Dihydro-1-benzofuran-4(5H)-one (3.5 g, 26 mmol) was dissolved in ethanol (20 ml), then a solution of hydroxylamine hydrochloride (4.5 g, 64 mmol) in water (3 ml) was added to the ethanol solution. The mixture was stirred at 78 °C for 16 h. The reaction mixture was concentrated to remove ethanol. The residue was partitioned between water and dichloromethane. An aqueous solution of saturated NaHCO₃ was slowly added to the stirring mixture to neutralize the acidic mixture. The organic layer was separated and the aqueous layer was washed with dichloromethane. The combined organic phases were dried over Na₂SO₄ and concentrated to a white solid. The crude solid was purified by Isco Combiflash (5%-20% EtOAc/CH2Cl2; 120g column). Collected fractions were combined and concentrated to give the desired product as a white solid. The product is a mixture of two E/Z-isomers.

Step 2. 1-Benzofuran-4-amine: (4Z)-6,7-Dihydro-1-benzofuran-4(5H)-one oxime (3.62g, 24 mmol) was dissolved in acetic anhydride (16 ml, 170 mmol), then pyridine (3.4 ml, 42 mmol) was added at 0 °C. The mixture was stirred at room temperature for 15 min. The clear solution turned into a white mixture (The acetate intermediate was observed by LCMS). Acetyl chloride (2.6 ml, 36 mmol) was added to mixture. The mixture was maintained at 120 °C for 18 h. The white mixture turned into brown solution upon heating. The reaction mixture was partitioned between water and dichloromethane. Solid NaHCO₃ was slowly added to the stirring mixture to neutralize the acidic mixture. The organic layer was separated and the aqueous layer was washed with DCM. The combined organic phases were dried over Na₂SO₄ and concentrated to a brown residue. The residue was purified by Isco Combiflash (5%-20% EtOAc/CH2Cl2; 12g column). ¹H NMR (500 MHz, DMSO-*d*₆) δ 6.64 (d, *J* = 1.3 Hz, 1H), 7.08 (d, *J* = 7.0 Hz, 1H), 7.39 (t, *J* = 8.0 Hz, 1H), 7.60 (d, *J* = 8.3 Hz, 1H), 7.70 (d, *J* = 2.3 Hz, 1H).

### Preparation 16

### 2-mercapto-1-propanol

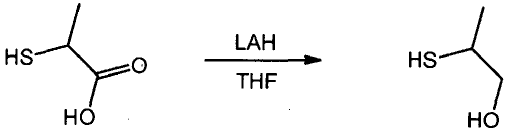

To a solution of 2-mercaptopropanoic acid (2 g, 18.8 mmol), at 0°C in 150 mL THF was added LAH (37.7 mL, 37.7 mmol, 1M in THF). After stirring for 1h, the reaction was slowly poured into 6N HCl and EtOAc, the organic layer separated, dried over MgSO₄, and concentrated *in vacuo* to provide 2-mercapto-1-propanol as a colorless oil (1.4g, 81% yield). ¹H NMR (CHLOROFORM-d) δ: 3.70 (m, 1H), 3.44 (m, 1H), 2.95 - 3.16 (m, 1H), 1.32 - 1.40 (d, *J* = 6.8 Hz, 3H)
The following compound was prepared using procedures analogous to those described above.

| Prep. # | Structure | Name | MS (M+H)⁺ | NMR |
|---|---|---|---|---|
| 17 | 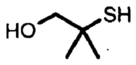 | 2-mercapto-2-methyl-1-propanol | NA | ¹H NMR (CHLOROFORM-d) δ: 3.45 (s, 2H), 1.38 (s, 6H) |

### Example 1

### N-1,3-Benzothiazol-5-yl-6-(methylsulfonyl)-4-quinolinamine

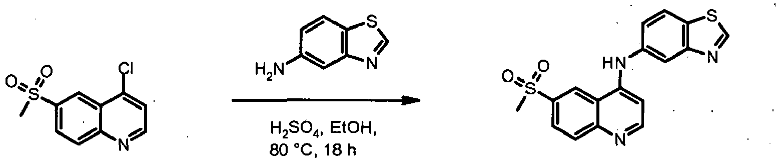

A mixture of 4-chloro-6-(methylsulfonyl)quinoline (9.0 g, 37 mmol), 1,3-benzothiazol-5-amine (5.6 g, 37 mmol), sulfuric acid (0.16 ml, 1.9 mmol) and ethanol (370 ml) was heated at 80 °C for 18 h. The reaction was cooled to room temperature, diluted with diethyl ether and filtered. The material was dissolved in 10 % methanol/ethyl acetate and washed with saturated sodium bicarbonate solution. The aqueous layer was extracted with 10% methanol/ethyl acetate (2x) and the combined organics were washed with brine (1x) and concentrated to yield the desired product along with minor amounts of methanol. The material was suspended in 2:1 acetonitrile/water (50 mL), filtered, and the resulting solid was dried overnight. The reaction was repeated on a 7g scale and the 2 batches were combine to yield *N*-1,3-benzothiazol-5-yl-6-(methylsulfonyl)-4-quinolinamine (16 g, 44.6 mmol, 67 % yield). ¹H NMR (400 MHz, CHLOROFORM-*d*) δ 9.11 (s, 1H), 8.88 (s, 1H), 8.67 (d, *J* = 5.6 Hz, 1H), 8.24 (d, *J* = 8.8 Hz, 1H), 8.09 - 8.18 (m, 2H), 8.05 (d, *J* = 8.6 Hz, 1H), 7.48 (dd, *J* = 8.6, 2.0 Hz, 1H), 7.09 (d, *J* = 5.6 Hz, 1H), 3.20 (s, 3H); MS (m/z) 356 (M+H⁺). Alternatively, this reaction can be done with a drop of con. HCl (or catalytic 4M HCl in dioxane) or in a microwave reactor in the absence of acid at 80 °C - 150 °C for 5 to 15 minutes. NMP may also be used as the solvent in select cases. Select compounds were purified by reverse phase HPLC.

The following compounds were prepared using procedures analogous to those described above.

| Ex # | Structure | Name | MS (M+H) + | NMR |
|---|---|---|---|---|
| 2 | 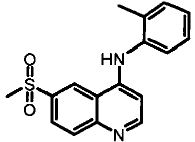 | N-(2-methylphenyl)-6-(methylsulfonyl)-4-quinolinamine | 313 | ¹H NMR (DMSO-d6) δ 9.30 (s, 1H), 9.12 (s, 1H), 8.47 (d, J = 5.1 Hz, 1H), 8.10 (d, J = 8.8 Hz, 1H), 8.03 (d, J = 8.8 Hz, 1H), 7.41 (d, J = 6.8 Hz, 1H), 7.25 - 7.37 (m, 3H), 6.15 (d, J = 4.9 Hz, 1H), 3.32 (s, 3H), 2.18 (s, 3H) |
| 3 | 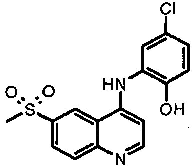 | 4-chloro-2-{[6-(methylsulfonyl)-4-quinolinyl] amino} phenol trifluoroacetate | 349, 351 | ¹H NMR (DMSO-d6) δ 11.14 (s, 1H), 10.51 (s, 1H), 9.36 (s, 1H), 8.61 (d, J = 7.1 Hz, 1H), 8.46 (dd, J = 8.9, 1.6 Hz, 1H), 8.17 (d, J = 8.8 Hz, 1H), 7.45 (d, J = 2.4 Hz, 1H), 7.39 (dd, J = 8.8, 2.4 Hz, 1H), 7.12 (d, J = 8.8 Hz, 1H), 6.52 (d, J = 6.8 Hz, 1H), 3.38 (s, 3H) |
| 4 | 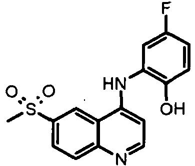 | 4-fluoro-2-{[6-(methylsulfonyl)-4-quinolinyl]amino} phenol trifluoroacetate | 333 | ¹H NMR (DMSO-d6) δ 11.17 (s, 1H), 10.16 (br. s., 1H), 9.38 (d, J = 1.2 Hz, 1H), 8.61 (d, J = 7.1 Hz, 1H), 8.46 (dd, J = 8.9, 1.6 Hz, 1H), 8.17 (d, J = 9.0 Hz, 1H), 7.28 (dd, J = 9.0, 2.9 Hz, 1H), 7.21 (td, J = 8.7, 3.2 Hz, 1H), 7.10 (dd, J = 9.0, 5.4 Hz, 1H), 6.78 - 6.87 (m, 1H), 6.54 (d, J = 7.1 Hz, 1H), 3.39 (s, 3H) |
| 5 | 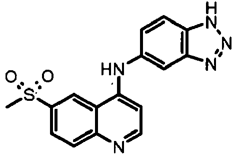 | N-1H-1,2,3-benzotriazol-5-yl-6-(methylsulfonyl)-4-quinolinamine | 340 | ¹H NMR (DMSO-d6) δ 9.58 (br. s., 1H), 9.13 (s, 1H), 8.52 (d, J = 5.0 Hz, 1H), 8.07 - 8.13 (m, 1H), 8.00 - 8.06 (m, 1H), 7.83 (d, J = 8.8 Hz, 1H), 7.65 (s, 1H), 7.15 (dd, J = 8.6, 1.5 Hz, 1H), 6.88 (d, J = 5.3 Hz, 1H), 3.33 (s, 3H) |
| 6 | 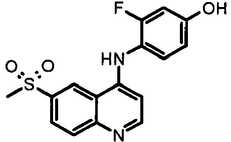 | 3-fluoro-4-{[6-(methylsulfonyl)-4-quinolinyl]amino} phenol trifluoroacetate | 333 | ¹H NMR (DMSO-d6) δ 11.13 (s, 1H), 10.36 (br. s., 1H), 9.36 (s, 1H), 8.63 (d, J = 6.8 Hz, 1H), 8.47 (dd, J = 8.9, 1.6 Hz, 1H), 8.18 (d, J = 8.8 Hz, 1H), 7.37 (t, J = 8.9 Hz, 1H), 6.77 - 6.93 (m, 2H), 6.61 (dd, J = 7.0, 1.6 Hz, 1H), 3.38 (s, 3H) |
| 7 | 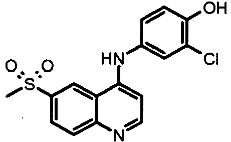 | 2-chloro-4-{[6-(methylsulfonyl)-4-quinolinyl]amino} phenol trifluoroacetate | 349, 351 | ¹H NMR (DMSO-d6) δ 11.23 (s, 1H), 10.65 (br. s., 1H), 9.32 (s, 1H), 8.58 (d, J = 7.1 Hz, 1H), 8.45 (dd, J = 8.9, 1.6 Hz, 1H), 8.15 (d, J = 8.8 Hz, 1H), 7.51 (d, J = 2.4 Hz, 1H), 7.28 (dd, J = 8.7, 2.6 Hz, 1H), 7.16 (d, J = 8.5 Hz, 1H), 6.83 (d, J = 7.1 Hz, 1H), 3.37 (s, 3H) |
| 8 | 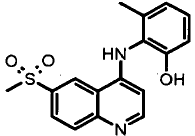 | 3-methyl-2-{[6-(methylsulfonyl)-4-quinolinyl]amino} phenol | 329 | NA |
| 9 | 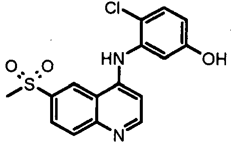 | 4-chloro-3-{[6-(methylsulfonyl)-4-quinolinyl]amino} phenol | 349, 351 | NA |
| 10 | 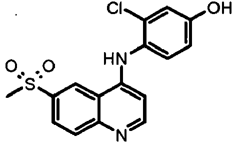 | 3-chloro-4-{[6-(methylsulfonyl)-4-quinolinyl]amino} phenol | 349, 351 | NA |
| 11 | 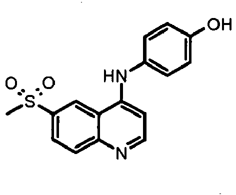 | 4-{[6-(methylsulfonyl)-4-quinolinyl] amino}phenol | 315 | ¹H NMR (DMSO-d6) δ 9.28 (br. s., 1H), 9.06 (d, J = 1.7 Hz, 1H), 8.47 (d, J = 5.4 Hz, 1H), 8.07 (dd, J = 8.8, 1.7 Hz, 1H), 7.99 (d, J = 8.8 Hz, 1H), 7.15 (d, J = 8.3 Hz, 2H), 6.84 (d, J = 8.3 Hz, 2H), 6.65 (d, J = 5.4 Hz, 1H), 3.30 (s, 3H) |
| 12 | 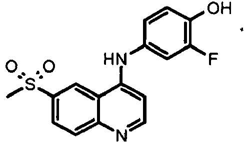 | 2-fluoro-4-{[6-(methylsulfonyl)-4-quinolinyl]amino} phenol | 333 | ¹H NMR (DMSO-d6) δ 9.87 (br. s., 1H), 9.34 (s, 1H), 9.04 (s, 1H), 8.53 (d, J = 5.4 Hz, 1H), 8.05 - 8.14 (m, 1H), 7.99 - 8.05 (m, 1H), 7.14 - 7.21 (m, 1H), 6.99 - 7.07 (m, 1H), 6.79 (d, J = 5.4 Hz, 1H), 3.31 (s, 3H) |
| 13 | 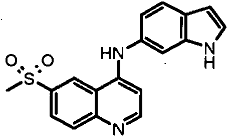 | N-1H-indol-6-yl-6-(methylsulfonyl)-4-quinolinamine | 338 | ¹H NMR (DMSO-d6) δ 11.13 (br. s., 1H), 9.57 (br. s., 1H), 9.13 (s, 1H), 8.49 (d, J = 5.4 Hz, 1 H), 8.11 (d, J = 8.8 Hz, 1H), 8.02 (d, J = 9.0 Hz, 1H), 7.62 (d, J = 8.3 Hz, 1H), 7.39 (s, 1H), 7.37 (t, J = 2.7 Hz, 1H), 7.03 (dd, J = 8.3, 1.5 Hz, 1H), 6.85 (d, J = 5.6 Hz, 1H), 6.47 (br. s., 1H), 3.32 (s, 3H) |
| 14 | 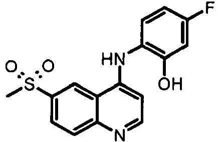 | 5-fluoro-2-{[6-(methylsulfonyl)-4-quinotinyl]amino} phenol | 333 | ¹H NMR (DMSO-d6) δ 9.07 (d, J = 1. 5 Hz, 1H), 8.44 (d, J = 5.5 Hz, 1H), 8.02 - 8.08 (m, 1H), 7.99 (s, 1H), 7.11 - 7.20 (m, 1H), 6.64 (br. s., 1H), 6.39 - 6.50 (m, 1H), 6.37 (d, J = 5.3 Hz, 1H), 3.17 (s, 3H) |
| 15 | 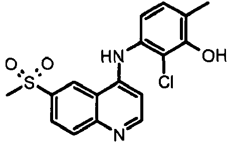 | 2-chloro-6-methyl-3-{[6-(methylsulfonyl)-4-quinolinyl]amino} phenol | 363, 365 | ¹H NMR (DMSO-d6) δ 9.38 (br. s., 1H), 9.13 (s, 1H), 8.46 (br. s., 1H), 8.07 - 8.12 (m, 1H), 8.03 (br. s., 1H), 7.08 (d, J = 7.8 Hz, 1H), 6.63 (br. s., 1H), 6.21 (br. s., 1H), 3.34 (s, 3H), 2.21 (s, 3H) |
| 16 | 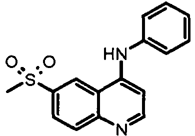 | 6-(methylsulfonyl)-N-phenyl-4-quinolinamine | 299 | ¹H NMR (DMSO-d6) 8 9.47 (br. s., 1H), 9.08 (s, 1H), 8.58 (d, J = 5.1 Hz, 1H), 8.09 - 8.14 (m, 1H), 8.03 - 8.07 (m, 1H), 7.43 - 7.49 (m, 2H), 7.37 - 7.42 (m, 2H), 7.20 (t, J = 7.1 Hz, 1H), 7.00 (d, J = 5.1 Hz, 1H), 3.32 (m, 3H) |
| 17 | 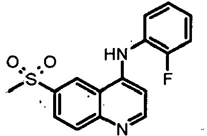 | N-(2-fluorophenyl)-6-(methylsulfonyl)-4-quinolinamine | 317 | ¹H NMR (DMSO-d6) δ 9.44 (br. s., 1H), 9.10 (br. s., 1H), 8.57 (br. s., 1H), 8.01 - 8.19 (m, 2H), 7.25 - 7.56 (m, 4H), 6.48 (br. s., 1H), 3.32 (s, 3H) |
| 18 | 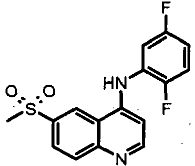 | N-(2,5-difluorophenyl)-6-(methylsulfonyl)-4-quinolinamine | 335 | ¹H NMR (DMSO-d6) 8 9.39 (br. s., 1H), 9.10 (br. s., 1H), 8.57 (br. s., 1H), 7.99 - 8.17 (m, 2H), 7.20 - 7.35 (m, 2H), 7.15 (br. s., 1H), 6.48 (br. s., 1H), 3.32 (s, 3H) |
| 19 | 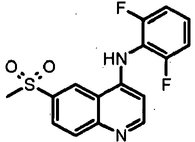 | N-(2,6-difluorophenyl)-6-(methylsulfonyl)-4-quinolinamine | 335 | ¹H NMR (DMSO-d6) δ 9.42 (br. s., 1H), 9.16 (br. s., 1H), 8.59 (br. s., 1H), 8.03 - 8.22 (m, 2H), 7.42 - 7.59 (m, 1H), 7.25 - 7.41 (m, 2H), 6.36 (br. s., 1H), 3.32 (s, 3H) |
| 20 | 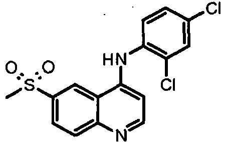 | N-(2,4-dichlorophenyl)-6-(methylsulfonyl)-4-quinolinamine | 367, 369 | ¹H NMR (DMSO-d6) δ 9.51 (br. s., 1H), 9.04 (br. s., 1H), 8.61 (br. s., 1H), 7.99 - 8.23 (m, 2H), 7.06 - 7.58 (m, 3H), 6.61 (br. s., 1H), 3.31 (s, 3H) |
| 21 | 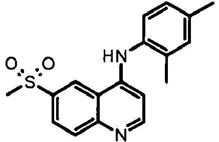 | N-(2,4-dimethylphenyl)-6-(methylsulfonyl)-4-quinolinamine | 327 | ¹H NMR (DMSO-d6) δ 9.23 (br. s., 1H), 9.12 (s, 1H), 8.45 (d, J = 5.1 Hz, 1H), 8.06 - 8.12 (m, 1H), 7.99 - 8.04 (m, 1H), 7.22 (br. s., 1H), 7.14 (br. s., 2H), 6.12 (d, J = 5.1 Hz, 1H), 3.32 (s, 3H), 2.34 (s, 3H), 2.13 (s, 3H) |
| 22 | 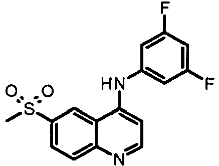 | N-(3,5-difluorophenyl)-6-(methylsulfonyl)-4-quinolinamine | 335 | ¹H NMR (DMSO-d6) δ 9.66 (br. s., 1H), 8.98 (br. s., 1H), 8.71 (br. s., 1H), 8.04 - 8.24 (m, 2H), 7.32 (br. s., 1H), 7.07 (br. s., 2H), 6.87 - 6.99 (m, 1H), 3.31 (s, 3H) |
| 23 | 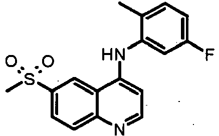 | N-(5-fluoro-2-methylphenyl)-6-(methylsulfonyt)-4-quinolinamine | 331 | ¹H NMR (DMSO-d6) δ 9.33 (br. s., 1H), 9.09 (br. s., 1H), 8.52 (br. s., 1H), 8.10 - 8.14 (m, 1H), 8.05 (dq, 1H), 7.39 - 7.46 (m, 1H), 7.08 - 7.20 (m, 2H), 6.25 (br. s., 1H), 3.32 (s, 3H), 2.14 (s, 3H) |
| 24 | 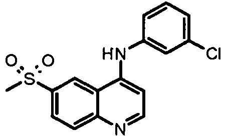 | N-(3-chlorophenyl)-6-(methylsulfonyl)-4-quinolinamine | 333, 335 | ¹H NMR (DMSO-d6) δ 9.55 (br. s., 1H), 9.03 (br. s., 1H), 8.65 (br. s., 1H), 8.03 - 8.20 (m, 2H), 7.33 - 7.52 (m, 3H), 7.21 (d, J = 7.6 Hz, 1H), 7.13 (br. s., 1H), 3.31 (s, 3H) |
| 25 | 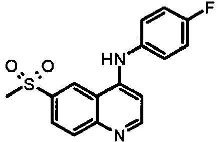 | N-(4-fluorophenyl)-6-(methylsulfonyl)-4-quinolinamine | 317 | ¹H NMR (DMSO-d6) δ 9.45 (br. s., 1H), 9.06 (br. s., 1H), 8.56 (d, J = 4.9 Hz, 1H), 8.09 - 8.13 (m, 1H), 8.03 - 8.07 (m, 1H), 7.38 - 7.45 (m, 2H), 7.26 - 7.33 (m, 2H), 6.86 (d, J = 4.9 Hz, 1H), 3.31 (s, 3H) |
| 26 | 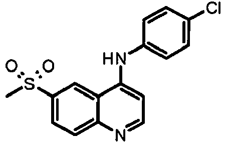 | N-(4-chlorophenyl)-6-(methylsulfonyl)-4-quinolinamine | 333, 335 | ¹H NMR (DMSO-d6) δ 9.51 (br. s., 1 H), 9.04 (br. s., 1H), 8.61 (br. s., 1H), 8.10 - 8.14 (m, 1H), 8.05 - 8.09 (m, 1H), 7.49 (d, J = 8.1 Hz, 2H), 7.39 - 7.44 (m, 2H), 7.02 - 7.06 (m, 1H), 3.29 (s, 3H) |
| 27 | 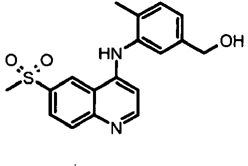 | (4-methyl-3-{[6-(methylsulfonyl)-4-quinolinyl]amino} phenyl)methanol | 343 | ¹H NMR (DMSO-d6) δ 9.31 (br. s., 1H), 9.13 (br. s., 1H), 8.47 (d, J = 5.1 Hz, 1H), 8.07 - 8.12 (m, 1 H), 8.03 (d, J = 8.8 Hz, 1H), 7.34 (d, J = 7.6 Hz, 1H), 7.20 - 7.24 (m, 2H), 6.16 (d, J = 5.1 Hz, 1H), 5.13 - 5.22 (m, 1H), 4.51 (d, J = 4.9 Hz, 2H), 3.33 (s, 3H), 2.15 (s, 3H) |
| 28 | 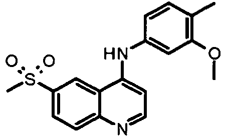 | N-[4-methyl-3-(methyloxy)phenyl]-6-(methylsulfonyl)-4-quinolinamine | 343 | ¹H NMR (DMSO-d6) δ 9.41 (br. s., 1H), 9.07 (br. s., 1H), 8.55 (d, J = 5.4 Hz, 1H), 8.07 - 8.12 (m, 1 H), 8.04 (d, J = 8.8 Hz, 1H), 7.20 (d, J = 7.6 Hz, 1H), 6.99 (d, J = 5.4 Hz, 1H), 6.94 (br. s., 1H), 6.85 - 6.92 (m, 1H), 3.80 (s, 3H), 3.31 (s, 3H), 2.17 (s, 3H) |
| 29 | 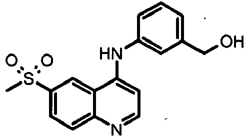 | (3-{[6-(methylsulfonyl)-4-quinolinyl] amino} phenyl)methanol | 329 | ¹H NMR (DMSO-d6) δ 9.47 (s, 1H), 9.09 (s, 1 H), 8.58 (d, J = 5.4 Hz, 1H), 8.09 - 8.12 (m, 1H), 8.03 - 8.07 (m, 1H), 7.38 - 7.42 (m, 1H), 7.37 (s, 1H), 7.26 (d, J = 7.8 Hz, 1H), 7.13 (d, J = 7.6 Hz, 1H), 7.02 (d, J = 5.4 Hz, 1H), 5.23 (t, J = 5.6 Hz, 1H), 4.54 (d, J = 5.4 Hz, 2H), 3.31 (s, 3H) |
| 30 | 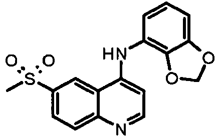 | N-1,3-benzodioxol-4-yl-6-(methylsulfonyl)-4-quinolinamine | 343 | ¹H NMR (DMSO-d6) δ 9.47 (br. s., 1H), 9.07 (br. s., 1H), 8.59 (d, J = 4.6 Hz, 1H), 8.08 - 8.13 (m, 1H), 8.02 - 8.08 (m, 1H), 6.92 - 6.99 (m, 1H), 6.89 (br. s., 2H), 6.55 (d, J = 4.9 Hz, 1H), 6.04 (s, 2H), 3.31 (s, 3H) |
| 31 | 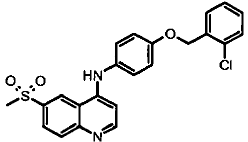 | N-(4-{[(2-chlorophenyl)methyl] oxy}phenyl)-6-(methylsulfonyl)-4-quinolinamine | 439, 441 | ¹H NMR (DMSO-d6) δ 9.07 (br. s., 1H), 8.52 (d, J = 5.4 Hz, 1H), 8.09 (d, J = 6.8 Hz, 1H), 7.99 - 8.05 (m, 1H), 7.64 (dd, J = 5.5, 3.8 Hz, 1H), 7.54 (dd, J = 5.6, 3.7 Hz, 1H), 7.42 (dd, J = 5.7, 3.5 Hz, 2H), 7.33 (d, J = 8.5 Hz, 2H), 7.10 - 7.18 (m, 2H), 6.77 (d, J = 5.4 Hz, 1H), 5.20 (s, 2H), 3.31 (s, 3H) |
| 32 | 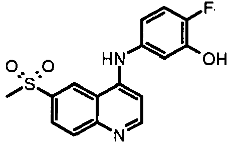 | 2-fluoro-5-{[6-(methylsulfonyl)-4-quinolinyl]amino} phenol | 333 | ¹H NMR (DMSO-d6) δ 11.24 (br. s., 1H), 10.44 (s, 1H), 9.34 (d, J = 1.5 Hz, 1H), 8.62 (d, J = 7.0 Hz, 1H), 8.45 (dd, J = 9.0, 1.8 Hz, 1H), 8.16 (d, J = 8.8 Hz, 1H), 7.38 (dd, J = 11.0, 8.8 Hz, 1H), 7.05 (dd, J = 8.0, 2.5 Hz, 1H), 6.86 - 6.96 (m, 2H), 3.39 (s, 3H) |
| 33 | 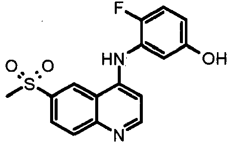 | 4-fluoro-3-{[6-(methylsulfonyl)-4-quinolinyl]amino} phenol | 333 | ¹H NMR (DMSO-d6) δ 10.02 (br. s., 1H), 9.32 (s, 1H), 8.61 (d, J = 6.3 Hz, 1H), 8.26 - 8.30 (m, 1 H), 8.18 (d, J = 8.8 Hz, 1H), 7.19 - 7.28 (m, 1H), 6.91 (dd, J = 6.5, 3.0 Hz, 1 H), 6.81 (dt, J = 8.8, 3.4 Hz, 1H), 6.57 (dd, J = 6.0, 3.0 Hz, 1H), 3.39 (s, 3H) |
| 34 | 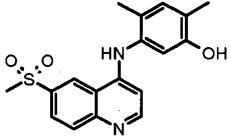 | 2,4-dimethyl-5-{[6-(methylsulfonyl)-4-quinolinyl]amino} phenol | 343 | ¹H NMR (DMSO-d6) δ 9.26 (s, 1H), 9.17 (s, 1H), 9.09 (d, J = 1.5 Hz, 1H), 8.45 (d, J = 5.4 Hz, 1H), 8.06 - 8.10 (m, 1H), 7.99 - 8.03 (m, 1H), 7.06 (s, 1H), 6.67 (s, 1H), 6.17 (d, J = 5.4 Hz, 1H), 3.32 (s, 3H), 2.14 (s, 3H), 2.01 (s, 3H) |
| 35 | 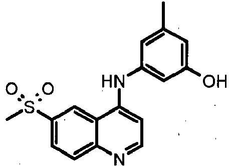 | 3-methyl-5-{[6-(methylsulfonyl)-4-quinolinyl]amino} phenol | 329 | ¹H NMR (DMSO-d6) δ 9.42 (br. s., 1H), 9.30 (s, 1H), 9.05 (s, 1H), 8.58 (d, J = 5.4 Hz, 1H), 8.07 - 8.13 (m, 1H), 8.01 - 8.07 (m, 1H), 7.04 (d, J = 5.4 Hz, 1H), 6.64 (s, 1H), 6.60 (s, 1H), 6.43 (s, 1H), 3.30 (s, 3H), 2.25 (s, 3H) |
| 36 | 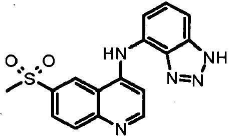 | N-1H-1,2,3-benzotriazol-4-yl-6-(methylsulfonyl)-4-quinolinamine | 340 | ¹H NMR (DMSO-d6) δ 15.75 (br. s., 1H), 9.94 (s, 1H), 9.21 (s, 1H), 8.57 (d, J = 5.3 Hz, 1H), 8.05 - 8.23 (m, 2H), 7.76 (br. s., 1H), 7.52 (t, J = 7.8 Hz, 1H), 7.40 (d, J = 7.0 Hz, 1H), 6.60 (br, s., 1H), 3.34 (s, 3H) |
| 37 | 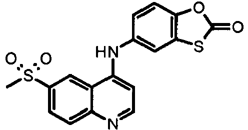 | 5-{[6-(methylsulfonyl)-4-quinolinyl]amino}-1,3-benzoxathiol-2-one hydrochloride | 373 | ¹H NMR (DMSO-d6) δ 11.56 (br. s., 1H), 9.47 (d, J = 1.5 Hz, 1H), 8.66 (d, J = 7.0 Hz, 1H), 8.47 (dd, J = 9.0, 1.8 Hz, 1H), 8.27 (d, J = 9.0 Hz, 1H), 7.96 (d, J = 2.3 Hz, 1H), 7.72 (d, J = 8.8 Hz, 1H), 7.55 (dd, J = 8.7, 2.4 Hz, 1H), 6.92 (d, J = 7.0 Hz, 1H), 3.43 (s, 3H) |
| 38 | 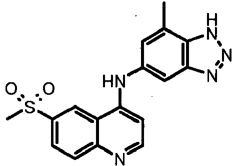 | N-(4-methyl-1H-1,2,3-benzotriazol-6-yl)-6-(methylsulfonyl)-4-quinolinamine | 354 | NA |
| 39 | 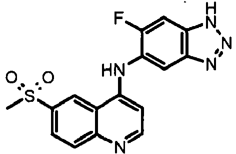 | N-(5-fluoro-1H-1,2,3-benzotriazol-6-yl)-6-(methylsulfonyl)-4-quinolinamine | 358 | ¹H NMR (DMSO-d6) δ 8.92 (d, J = 5.3 Hz, 1H), 8.69 (s, 1H), 8.06 - 8.25 (m, 3H), 7.21 (d, J = 5.5 Hz, 1H), 6.55 (s, 1H), 3.33 (s, 3H) |
| 40 | 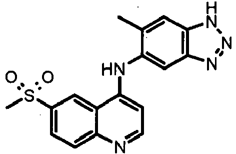 | N-(5-methyl-1H-1,2,3-benzotriazol-6-yl)-6-(methylsulfonyl)-4-quinolinamine | 354 | ¹H NMR (DMSO-d6) δ 15.81 (br. s., 1H), 10.13 (br, s., 1H), 9.17 - 9.34 (m, 1H), 8.44 - 8.53 (m, 1H), 8.23 (d, J = 8.5 Hz, 1H), 8.11 (d, J = 9.0 Hz, 1H), 7.85 - 8.03 (m, 1H), 6.24 (d, J = 4.8 Hz, 1H), 3.38 (s, 3H), 2.34 (s, 3H) |
| 41 | 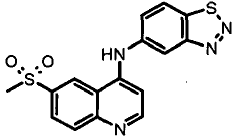 | N-1,2,3-benzothiadiazol-5-yl-6-(methylsulfonyt)-4-quinolinamine | 357 | ¹H NMR (DMSO-d6) δ 9.90 (s, 1H), 9.12 (s, 1H), 8.66 - 8.71 (m, 1H), 8.62 (br. s., 1H), 8.47 (d, J = 8.8 Hz, 1H), 8.15 - 8.22 (m, 1H), 8.10 - 8.15 (m, 1H), 7.91 (d, J = 8.5 Hz, 1H), 7.20 - 7.35 (m, 1H), 3.35 (s, 3H) |
| 42 | 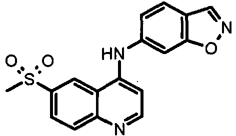 | N-1,2-benzisoxazol-6-yl-6-(methylsulfonyl)-4-quinolinamine | 340 | ¹H NMR (DMSO-d6) δ 11.49 (br. s., 1H), 9.26 (s, 1H), 8.73 (d, J = 6.3 Hz, 1H), 8.40 . (d, J = 8.5 Hz, 1H), 8.20 (d, J = 8.5 Hz, 1H), 7.76 (d, J = 8.3 Hz, 1H), 7.24 (d, J = 5.9 Hz, 1H), 7.10 (s, 1H), 7.05 (d, J = 8.3 Hz, 1H), 3.37.(s, 3H) |
| 43 | 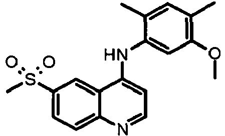 | N-[2,4-dimethyl-5-(methyloxy)phenyl]-6-(methylsulfonyl)-4-quinolinamine | 357 | ¹H NMR (DMSO-d6) δ 11.06 (br. s., 1H), 9.36 (s, 1H), 8.54 (d, J = 7.1 Hz, 1H), 8.43 (dd, J = 8.8, 1.5 Hz; 1H), 8.16 (d, J = 8.8 Hz, 1H), 7.25 (s, 1H), 6.95 (s, 1H), 6.37 (d, J = 6.8 Hz, 1H), 3.76 (s, 3H), 3.39 (s, 3H), 2.20 (s, 3H), 2.10 (s, 3H) |
| 44 | 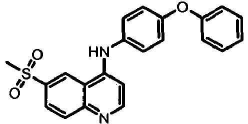 | 6-(methylsulfonyl)-N-[4-(phenyloxy) phenyl]-4-quinolinamine | 391 | ¹H NMR (DMSO-d6) δ 9.45 (br. s., 1H), 9.07 (br. s., 1H), 8.57 (d, J = 5.1 Hz, 1H), 8.07 - 8.15 (m, 1H), 8.04 (d, J = 8.5 Hz, 1H), 7.34 - 7.49 (m, 4H), 7.00 - 7.23 (m, 5H), 6.92 (d, J = 5.1 Hz, 1H),-.3.31 (s, 3H) |
| 45 | 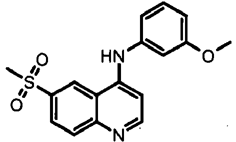 | N-[3-(methyloxy)phenyl]-6-(methylsulfonyl)-4-quinolinamine | 329 | ¹H NMR (DMSO-d6) δ 9.44 (br. s., 1H), 9.05 (br. s., 1H), 8.60 (d, J = 4.9 Hz, 1H), 8.09 - 8.14 (m, 1H), 8.03 - 8.08 (m, 1H), 7.35 (t, J = 8.1 Hz, 1H), 7.08 (d, J = 5.1 Hz, 1H), 6.98 (d, J = 7.6 Hz, 1H), 6.94 (br. s., 1H), 6.77 (d, J = 7.8 Hz, 1H), 3.78 (s, 3H), 3.31 (s, 3H) |
| 46 | 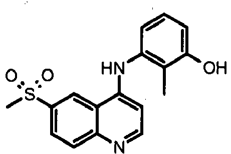 | 2-methyl-3-{[6-(methylsulfonyl)-4-quinolinyl]amino} phenol trifluoroacetate | 329 | ¹H NMR (DMSO-d6) δ 11.2 (s, 1H), 9.89 (br. s., 1H), 9.38 (s, 1H), 8.54 (d, J = 7.1 Hz, 1H), 8.46 (dd, J = 9.0, 1.5 Hz, 1H), 8.15 (d, J = 8.8 Hz, 1H), 7.23 (t, J = 7.9 Hz, 1H), 6.97 (d, J = 8.1 Hz, 1H), 6.82 (d, J = 7.8 Hz, 1H), 6.37 (d, J = 7.1 Hz, 1H), 3.39 (s, 3H), 2.01 (s, 3H) |
| 47 | 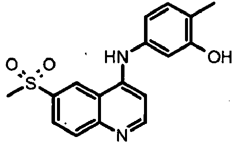 | 2-methyl-5-{[6-(methylsulfonyl)-4-quinolinyl]amino} phenol | 329 | ¹H NMR (DMSO-d6) δ 9.81 (s, 1H), 9.33 (s, 1H), 8.57 (d, J = 7.1 Hz, 1H), 8.41 (d, J = 9.3 Hz, 1H), 8.13 (d, J = 9.0 Hz, 1H), 7.26 (d, J = 8.1 Hz, 1H), 6.90 (d, J = 7.1 Hz, 1H), 6.84 (d, J = 1.7 Hz, 1H), 6.81 (dd, J = 7:8, 1.7 Hz, 1H), 3.37 (s, 3H), 2.19 (s, 3H) |
| 48 | 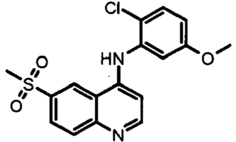 | N-[2-chloro-5-(methyloxy)phenyl]-6-(methylsulfonyl)-4-quinolinamine | 363, 365 | ¹H NMR (DMSO-d6) δ 9.47 (br. s., 1H), 9.09 (br. s., 1H), 8.55 (br. s., 1H), 7.99 - 8.17 (m, 2H), 7.53 (br. s., 1H), 6.89 - 7.13 (m, 2H), 6.30 (br. s., 1H), 3.79 (s, 3H), 3.32 (s, 3H) |
| 49 | 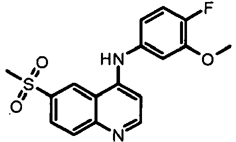 | N-[4-fluoro-3-(methyloxy)phenyl]-6-(methylsulfonyl)-4-quinolinamine | 347 | ¹H NMR (DMSO-d6) δ 9.44 (br. s., 1H), 9.05 (br. s., 1H), 8.57 (br. s., 1H), 8.08 - 8.16 (m, 1H), 8.05 (d, J = 8.5 Hz, 1H), 7.23 - 7.33 (m, 1H), 7.16 (d, J = 6.6 Hz, 1H), 6.96 (br. s., 2H), 3.85 (s, 3H), 3.31 (s, 3H) |
| 50 | 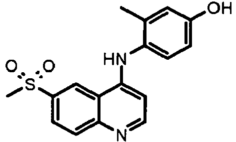 | 3-methyl-4-{[6-(methylsulfonyl)-4-quinolinyl]amino} phenol | 329 | ¹H NMR (DMSO-d6) δ 9.45 (br. s., 1H), 9.10 (d, J = 8.1 Hz, 2H), 8.42 (d, J = 5.4 Hz, 1H), 8.04 - 8.11 (m, 1H), 7.96 - 8.03 (m, 1H), 7.04 (d, J = 8.5 Hz, 1H), 6.78 (d, J = 2.2 Hz, 1H), 6.71 (dd, J = 8.3, 2.4 Hz, 1H), 6.08 (d, J = 5.4 Hz, 1H), 3.31 (s, 3H), 2.08 (s, 3H) |
| 51 | 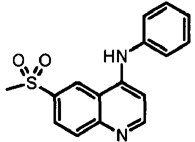 | 6-(methylsulfonyl)-N-phenyl-4-quinolinamine trifluoroacetate | 299 | ¹H NMR (500 MHz, DMSO-d₆) δ 3.38 (s, 3H), 6.89 (dd, J = 12.6, 7.2 Hz, 2H), 7.20 (t, J = 7.7 Hz, 1H), 7.44 - 7.54 (m, 2H), 7.55 - 7.67 (m, 1H), 8.17 (d, J = 8.8 Hz, 1H), 8.45 (dd, J = 9.0, 1.5 Hz, 1 H), 8.61 (d, J = 6.8 Hz, 1H), 9.37 (d, J = 1.2 Hz, 1H), 11.34 (br. s., 1H) |
| 52 | 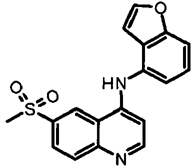 | N-1-benzofuran-4-yl-6-(methylsulfonyl)-4-quinolinamine | 339 | ¹H NMR (500 MHz, DMSO-d6) δ 3.38 (br. s., 3H), 6.74 (d, J = 7.0 Hz, 1H), 6.83 (d, J = 2.3 Hz, 1H), 7.41 (d, J = 7.8 Hz, 1H), 7.55 (t, J = 8.0 Hz, 1H), 7.71 (d, J = 8.3 Hz, 1H), 7.91 (d, J = 2.3 Hz, 1H), 8.15 (d, J = 9.0 Hz, 1H), 8.47 (d, J = 7.0 Hz, 1H), 8.51 (dd, J = 8.9, 1.6 Hz, 1H), 9.40 (d, J = 1.5 Hz, 1H) |
| 53 | 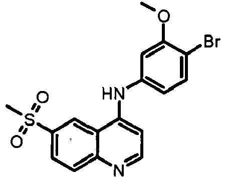 | N-[4-bromo-3-(methyloxy)phenyl]-6-(methylsulfonyl)-4-quinolinamine | 407, 409 | ¹H NMR (500 MHz, DMSO-d6) δ 3.31 (s, 3H) 3.86 (s, 3H) 6.95 (dd, J = 8.42, 2.08 Hz, 1H) 7.12 (d, J = 1.95 Hz, 1H) 7.16 (d, J = 5.13 Hz, 1H) 7.59 (d, J = 8.55 Hz, 1H) 8.05 - 8.16 (m, 2H) 8.63 (d, J = 5.13 Hz, 1H) 9.04 (s, 1H) 9.51 (s, 1H) |
| 54 | 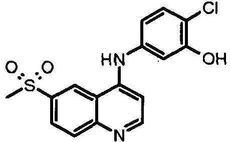 | 2-chloro-5-{[6-(methylsulfonyl)-4-quinolinyl]amino} phenol | 349, 351 | ¹H NMR (500 MHz, DMSO-d6) δ 3.36 (s, 3H) 6.93 (dd, J = 8.55, 2.20 Hz, 1H) 6.99 (d, J = 6.84 Hz, 1H) 7.05 (d, J = 2.20 Hz, 1H) 7.53 (d, J = 8.30 Hz, 1H) 8.15 (d, J = 8.79 Hz, 1H) 8.42 (d, J = 9.03 Hz, 1H) 8.62 (d, J = 6.84 Hz, 1H) 9.30 (s, 1H) 10.72 (s, 4H) 11.07 (br. s., 3H) |
| 55 | 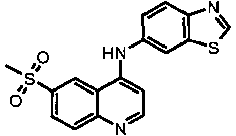 | N-1,3-benzothiazol-6-yl-6-(methylsulfonyl)-4-quinolinamine | 356 | ¹H NMR (400 MHz, DMSO-d6) δ 3.43 (s, 3H) 3.42 (m, 6H) 6.99 (d, J = 7.05 Hz, IH) 7.67 (dd, J =8.56, 2.01 Hz, IH) 8.23-8.33 (m, 2H) 8.37 (d, J = 2.01 Hz, 1H) 8.47 (dd, J = 8.94, 1.64 Hz, 1H) 8.62 (d, J = 7.05 Hz, 1H) 9.45 - 9.55 (m, 2H) 11.54 - 11.71 (m, 1H) 14.60 - 14.88 (m, 1H) |
| 56 | 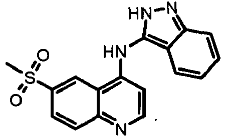 | N-2H-indazol-3-yl-6-(methylsulfonyl)-4-quinolinamine | 339 | ¹H NMR (500 MHz, DMSO-d6) δ 3.40 (s, 3H) 7.22 (t, J = 7.45 Hz, 1 H) 7.40 (d, J = 6.84 Hz, 1H) 7.48 (t, J = 7.69 Hz, 1H) 7.64 (d, J = 8.55 Hz, 1H) 7.82 (d, J = 8.30 Hz, 1H) 8.19 (d, J = 8.79 Hz, 1H) 8.43 (d, J = 8.30 Hz, 1H) 8.71 (d, J = 6.84 Hz, 1H) 9.52 (s, 1H) 11.38 (br. s., 1H) |
| 57 | 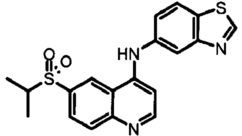 | N-1,3-benzothiazol-5-yl-6-[(1-methylethyl) sulfonyl]-4-quinolinamine | 384 | ¹H NMR (400 MHz, DMSO-d6) δ 1.23 (d, 6H) 3.55 (quin, J = 6.76 Hz, 1H) 7.08 (d, J = 5.31 Hz, 1H) 7.57 (dd, J = 8.59, 2.02 Hz, 1H) 8.01 - 8.12 (m, 3H) 8.24 (d, J = 8.59 Hz, 1H) 8.61 (d, J = 5.31 Hz, 1H) 9.07 (d, J = 1.26 Hz, 1H) 9.45 (s, 1H) 9.73 (s, 1H) |
| 58 | 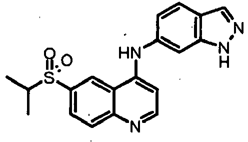 | N-1H-indazol-6-yl-6-[(1-methylethyl) sulfonyl]-4-quinolinamine | 367 | ¹H NMR (400 MHz, DMSO-d6) δ 1.23 (d, J = 6.82 Hz, 6H) 3.54 (quin, J = 6.82 Hz, 1H) 7.08 (d, J = 5.05 Hz, 1H) 7,14 - 7.23 (m, 1H) 7.49 (s, 1H) 7.82 (d, J = 8.59 Hz, 1H) 7.99 - 8.11 (m, 3H) 8.60 (d, J = 5.05 Hz, 1H) 9.06 (s, 1H) 9.71 (br. s., 1 H) 13.02 (s, 1H) |
| 59 | 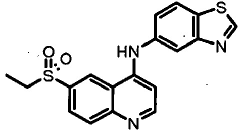 | N-1,3-benzothiazol-5-yl-6-(ethylsulfonyl)-4-quinolinamine | 370 | ¹H NMR (400 MHz, DMSO-d6) δ 1.19 (t, 3H) 3.50 (q, J = 7.33 Hz, 2H) 7.00 (d, J = 7.07 Hz, 1H) 7.63 (dd, J = 8.59, 2.02 Hz, 1H) 8.18 - 8.28 (m, 2H) 8.35 - 8.47 (m, 2H) 8.62 (d, J = 6.82 Hz, 1H) 9.41 (d, J = 1.52 Hz, 1H) 9.55 (s, 1H) 11.54 (br. s., 1H) 14.56 (br. s., 1H) |
| 60 | 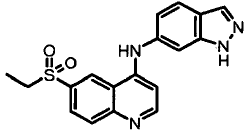 | 6-(ethylsulfonyl)-N-1H-indazol-6-yl-4-quinolinamine | 353 | ¹H NMR (400 MHz, DMSO-d6) δ 1.19 (t, J = 7.33 Hz, 3H) 3.49 (q, J = 7.33 Hz, 2H) 6.98 (d, J = 6.82 Hz, 1H) 7.22 (dd, J = 8.46, 1.64 Hz, 1H) 7.67 (s, 1H) 7.98 (d, J = 8.59 Hz, 1H) 8.15 - 8.23 (m, 2H) 8.40 (d, J = 9.09 Hz, 1H) 8.60 (d, J = 7.07 Hz, 1H) 9.37 (s, 1H) 11.43 (br. s., 1H) |
| 61 | 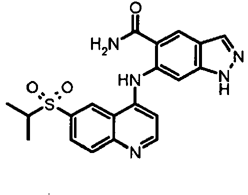 | 6-({6-[(1-methylethyl)sulfonyl]-4-quinolinyl}amino)-1H-indazole-5-carboxamide | 410 | ¹H NMR (400 MHz, DMSO-d6) δ 1.25 (d, J = 6.82 Hz, 6H) 3.60 (dt, J = 13.58, 6.73 Hz, 1H) 6.76 (d, J = 7.07 Hz, 1H) 7.49 (br. s., 1H) 7.74 (s, 1H) 8.11 (br. s., 1H) 8.21 (d, J = 8.84 Hz, 1H) 8.27 - 8.41 (m, 3H) 8.57 (d, J = 7.07 Hz, 1H) 9.26 (s, 1H) 11.83 (br. s., 1H) 13.51 (br. s., 1H) 14.42 (br. s., 1H) |
| 62 | 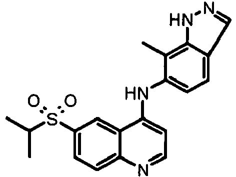 | 6-[(1-methylethyl) sulfonyl]-N-(7-methyl-1H-indazol-6-yl)-4-quinolinamine | 381 | ¹H NMR (400 MHz, DMSO-d6) δ 1.27 (d, J = 6.82 Hz, 6H) 2.43 (s, 3H) 3.56 - 3.68 (m, 1H) 6.42 (d, J = 7.07 Hz, 1 H) 7.12 (d, J = 8.59 Hz, 1H) 7.81 (d, J = 8.59 Hz, 1H) 8.13 - 8.24 (m, 2H) 8.38 (dd, J = 8.84, 1.52 Hz, 1H) 8.53 (d, J = 7.07 Hz, 1H) 9.40 (s, 1H) 11.44 (br. s., 1H) 13.49 (s, 1H) |
| 63 | 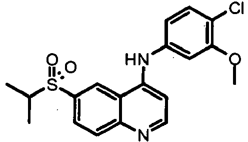 | N-[4-chloro-3-(methyloxy)phenyl]-6-[(1-methylethyl) sulfonyl]-4-quinolinamine | 391, 393 | ¹H NMR (500 MHz, DMSO-d6) δ 1.22 (d, J = 6.84 Hz, 6H) 3.49 - 3.57 (m, 1H) 3.87 (s, 3H) 6.97 - 7.04 (m, 1H) 7.10 - 7.20 (m, 2H) 7.46 (d, J = 8.30 Hz, 1H) 8.01 - 8.11 (m, 2H) 8.62 (d, J = 5.13 Hz, 1H) 9.00 (s, 1H) 9.55 (br. s., 1H) |
| 64 | 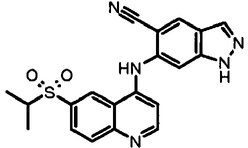 | 6-({6-[(1-methylethyl) sulfonyl]-4-quinolinyl} amino)-1H-indazole-5-carbonitrile | 392 | ¹H NMR (400 MHz, DMSO-d6) δ 1.26 (d, J = 6.57 Hz, 6H) 3.61 (m, 1H) 6.61 - 6.78 (m, 1H) 7.81 - 7.94 (m, 1H) 8.14 - 8.27 (m, 1H) 8.29 - 8.45 (m, 2H) 8.59 - 8.74 (m, 2H) 9.30 (s, 1H) 13.81 (br. s., 1H) |
| 65 | 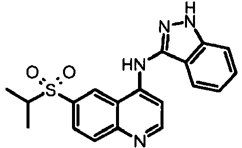 | N-1H-indazol-3-yl-6-[(1-methylethyl) sulfonyl]-4-quinolinamine | 367 | ¹H NMR (400 MHz, DMSO-d6) δ 1.27 (d, J = 6.57 Hz, 6H) 3.62 (dt, J= 13.58, 6.73 Hz, 1H) 7.23 (t, J = 7.45 Hz, 1H) 7.38 (d, J = 7.07 Hz, 1H) 7.50 (t, J = 7.33 Hz, 1H) 7.65 (d, J = 8.59 Hz, 1H) 7.82 (d, J = 8.34 Hz, 1H) 8.21 (d, J = 9.09 Hz, 1H) 8.38 (dd, J = 8.84, 1.52 Hz, 1H) 8.73 (d, J = 6.82 Hz, 1H) 9.50 (d, J = 1.26 Hz, 1H) 11.61 (br. s., 1H) 13.35 (s, 1H) |
| 66 | 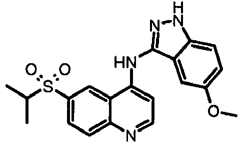 | 6-[(1-methylethyl) sulfonyl]-N-[5-(methyloxy)-1H-indazol-3-yl]-4-quinolinamine | 397 | ¹H NMR (400 MHz, DMSO-d6) δ 1.27 (d, J = 6.82 Hz, 6H) 3.62 (dt, J = 13.64, 6.82 Hz, 1H) 3.76 (s, 3H) 7.08 - 7.18 (m, 3H) 7.57 (d, J = 9.85 Hz, 1H) 8.21 (d, J = 8.84 Hz, 1H) 8.39 (dd, J = 8.97, 1.64 Hz, 1H) 8.69 (d, J = 6.82 Hz, 1H) 9.48 (s, 1H) 11.66 (br. s., 1H) 13.27 (s, 1H) |
| 67 | 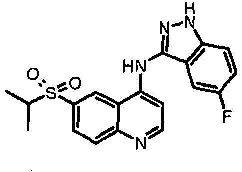 | N-(5-fluoro-1H-indazol-3-yl)-6-[(1-methylethyl)sulfonyl]-4-quinolinamine | 385 | ¹H NMR (400 MHz, DMSO-d6) 8 1.25 (d, 6H) 3.57 (quin, J = 6.82 Hz, 1H) 7.27 - 7.41 (m, 2H) 7.53 - 7.64 (m, 2H) 8.00 - 8.13 (m, 2H) 8.65 (d, J = 5.31 Hz, 1H) 9.22 (s, 1H) 9.89 (s, 1H) 12.98 (s, 1H) |
| 68 | 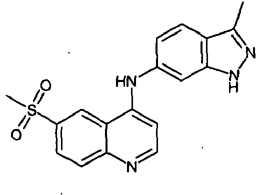 | N-(3-methyl-1H-indazol-6-yl)-6-(methylsulfonyl)-4-quinolinamine | 353 | ¹H NMR (500 MHz, DMSO-d6) δ 2.54 (s, 3H) 3.35-3.39 (m, 3H) 6.94 (d, J = 7.08 Hz, 1H) 7.17 (d, J= 8.55 Hz, 1H) 7.56 (s, 1H) 7.91 (d, J = 8.30 Hz, 1H) 8.17 (d, J = 9.03 Hz, 1H) 8.45 (d, J = 9.03 Hz, 1H) 8.59 (d, J = 7.08 Hz, 1H) 9.39 (s, 1H) 11.37 (br. s., 2H) 12.84 (br. s., 1H) |
| 69 | 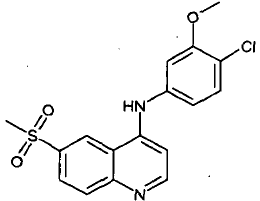 | N-[4-chloro-3-(methyloxy)phenyl]-6-(methylsulfonyl)-4-quinolinamine | 363, 365 | ¹H NMR (500 MHz, DMSO-d6) 8 3.37 (s, 3H) 3.89 (s, 3H) 7.01 - 7.12 (m, 2H) 7.27 (s, 1H) 7.61 (d, J = 8.30 Hz, 1H) 8.16 (d, J = 9.03 Hz, 1H) 8.41 (d, J = 9.03 Hz, 1H) 8.63 (d, J = 6.59 Hz, 1H) 9.30 (s, 1H) 11.02 (br. s., 1H) |
| 70 | 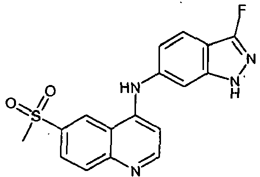 | N-(3-fluoro-1H-indazol-6-yl)-6-(methylsulfonyl)-4-quinolinamine | 357 | ¹H NMR (400 MHz, DMSO-d₆) δ 12.45 (s, 1H), 9.68 (s, 1H), 9.08 (d, J = 1.8 Hz, 1H), 8.65 (d, J = 5.3 Hz, 1H), 8.07 - 8.18 (m, 2H), 7.75 (d, J = 8.6 Hz, 1H), 7.40 (s, 1H), 7.24 (dd, J = 8.7, 1.6 Hz, 1H), 7.18 (d, J = 5.6 Hz, 1H), 3.33 (s, 3H). |
| 71 | 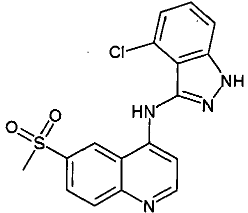 | N-(4-chloro-1H-indazol-3-yl)-6-(methylsulfonyl)-4-quinolinamine | 373 | ¹H NMR (400 MHz, DMSO-d₆) δ 13.41 (s, 1H), 9.72 (s, 1H), 9.18 (d, J = 1.8 Hz, 1H), 8.53 (d, J = 5.3 Hz, 1H), 8.05 - 8.17 (m, 2H), 7.58 (d, J = 8.3 Hz, 1H), 7.37 - 7.44 (m, 1H), 7.18 (d, J = 7.3 Hz, 1H), 6.49 (d, J = 5.3 Hz, 1H), 3.36 (s, 3H) |
| 72 | 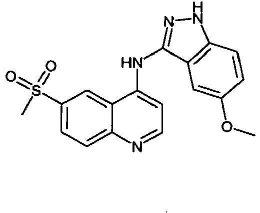 | N-[5-(methyloxy)-1H-indazol-3-yl]-6-(methylsulfonyl)-4-quinolinamine | 369 | ¹H NMR (400 MHz, DMSO-d₆) δ 12.74 (br. s., 1H), 9.84 (br. s., 1H), 9.27 (s, 1H), 8.61 (d, J = 5.3 Hz, 1H), 8.06 - 8.16 (m, 2H), 7.48 (d, J = 9.1 Hz, 1H), 7.20 (d, J = 5.6 Hz, 1 H), 7.14 (d, J = 2.3 Hz, 1H), 7.08 (dd, J = 9.1, 2.3 Hz, 1H), 3.76 (s, 3H), 3.36 (s, 3H) |
| 73 | 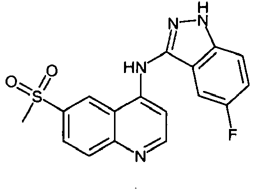 | N-(5-fluoro-1H-indazol-3-yl)-6-(methylsulfonyl)-4-quinolinamine | 357 | ¹H NMR (400 MHz, DMSO-d₆) δ 12.94 (br. s., 1H), 9.85 (br. s., 1H), 9.27 (s, 1H), 8.64 (br. s., 1H), 8.06 - 8.17 (m, 2H), 7.56 - 7.64 (m, 2H), 7.44 (d, J = 5.3 Hz, 1H), 7.33 (td, J = 9.1, 2.5 Hz, 1H), 3.36 (s, 3H) |
| 74 | 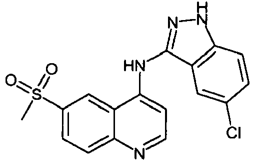 | N-(5-chloro-1H-indazol-3-yl)-6-(methylsulfonyl)-4-quinolinamine | 373 | ¹H NMR (400 MHz, DMSO-d₆) δ 13.00 (s, 1H), 9.87 (s, 1H), 9.29 (d, J = 1.3 Hz, 1H), 8.69 (d, J = 5.3 Hz, 1H), 8.08 - 8.20 (m, 2H), 8.04 (d, J = 1.3 Hz, 1H), 7.65 (d, J = 5.3 Hz, 1H), 7.59 (d, J = 8.8 Hz, 1H), 7.43 (dd, J = 8.8, 1.8 Hz, 1H), 3.37 (s, 3H) |
| 75 | 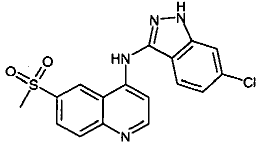 | N-(6-chloro-1H-indazol-3-yl)-6-(methylsulfonyl)-4-quinolinamine | 373 | ¹H NMR (400 MHz, DMSO-d₆) δ 12.94 (br. s., 1H), 9.95 (s, 1H), 9.29 (d, J = 1.3 Hz, 1H), 8.68 (d, J = 5.3 Hz, 1H), 8.08 - 8.18 (m, 2H), 7.93 (d, J = 8.6 Hz, 1H), 7.63 (d, J = 1.3 Hz, 1H), 7.59 (d, J = 5.6 Hz, 1H), 7.17 (dd, J = 8.7, 1.6 Hz, 1H), 3.36 (s, 3H) |
| 76 | 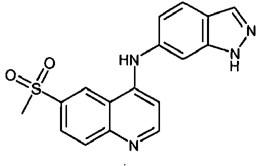 | N-1H-indazol-6-yl-6-(methylsulfonyl)-4-quinolinamine | 339 | ¹H NMR (400 MHz, DMSO-d₆) δ 13.00 (s, 1H), 9.63 (s, 1H), 9.10 (d, J = 1.8 Hz, 1H), 8.60 (d, J = 5.6 Hz, 1H), 8.03 - 8.18 (m, 3H), 7.82 (d, J = 8.6 Hz, 1H), 7.48 (s, 1H), 7.18 (dd, J = 8.6, 1.8 Hz, 1H), 7.09 (d, J = 5.3 Hz, 1H), 3.34 (s, 3H) |
| 77 | 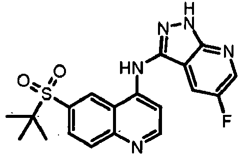 | 6-[(1,1-dimethylethyl) sulfonyl]-*N*-(5-fluoro-1*H*-pyrazolo[3,4-*b*]pyridin-3-yl)-4-quinolinamine | 400 | ¹H NMR (DMSO-d6) δ: 14.16 (s, 1H), 11.80 (br. s., 1H), 9.45 (s, 1H), 8.75 (d, J = 7.1 Hz, 1H), 8.72 - 8.74 (m, 1H), 8.31 - 8.42 (m, 2H), 8.23 (dd, J = 8.6, 2.5 Hz, 1H), 7.42 (d, J = 7.1 Hz, 1H), 1.35 (s, 9H) |
| 78 | 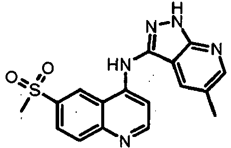 | N-(5-methyl-1*H-*pyrazolo[3,4-*b*]pyridin-3-yl)-6-(methylsulfonyl)-4-quinolinamine | 354 | ¹H NMR (METHANOL-d4) δ: 8.41 (d, J = 1.8 Hz, 1H), 7.80 (d, J = 5.8 Hz, 1H), 7.63 (d, J = 1.8 Hz, 1H), 7.42 (dd, J = 8.8, 2.0 Hz, 1H), 7.31 (d, J = 8.8 Hz, 2H), 6.81 (d, J = 5.6 Hz, 1H), 4.07 (s, 3H), 2.47 (s, 3H) |
| 79 | 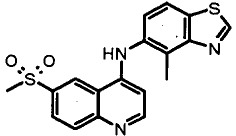 | *N*-(4-methyl-1,3-benzothiazol-5-yl)-6-(methylsulfonyl)-4-quinolinamine | 370 | ¹H NMR (DMSO-d6) δ: 9.61 (br. s., 1H), 9.46 (s, 1H), 9.20 (d, J = 1.5 Hz, 1H), 8.47 (d, J = 4.8 Hz, 1H), 8.10 - 8.19 (m, 2H), 8.03 - 8.09 (m, 1H), 7.44 (d, J = 8.5 Hz, 1H), 6.20 (d, J = 5.3 Hz, 1H), 3.36 (s, 3H), 2.58 (s, 3H) |
| 80 | 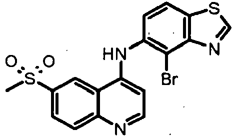 | *N*-(4-bromo-1,3-benzothiazol-5-yl)-6-(methylsulfonyl)-4-quinolinamine | 434 | ¹H NMR (METHANOL-d4) δ: 9.43 (s, 1H), 9.16 (s, 1H), 8.46 (br. s., 1H), 8.18 - 8.30 (m, 2H), 8.11 (d, J = 8.8 Hz, 1H), 7.62 (d, J = 8.6 Hz, 1 H), 6.41 (d, J = 5.3 Hz, 1H), 2.68 (s, 3H) |
| 81 | 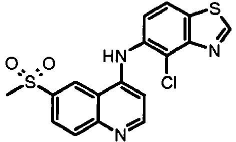 | *N*-(4-chloro-1,3-benzothiazol-5-yl)-6-(methylsulfonyl)-4-quinolinamine | 390 | ¹H NMR (DMSO-d6) δ: 11.76 (br. s., 1H), 9.65 (s, 1H), 9.56 (s, 1H), 8.63 (d, J = 6.8 Hz, 1H), 8.51 (d, J = 8.8 Hz, 1H), 8.42 (d, J = 8.6 Hz, 1H), 8.33 (d, J = 9.1 Hz, 1H), 7.73 (d, J = 8.6 Hz, 1H), 6.55 (d, J = 7.1 Hz, 1H), 3.46 (s, 3H) |
| 82 | 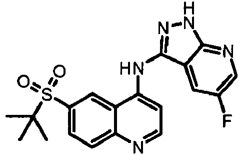 | 6-[(1,1-dimethylethyl) sulfonyl]-*N*-(5-fluoro-1*H*-pyrazolo[3,4-*b*]pyridin-3-yl)-4-quinolinamine | 400 | ¹H NMR (METHANOL-d4) δ: 9.26 (d, J = 1.8 Hz, 1H), 8.64 (d, J = 6.3 Hz, 1 H), 8.59 (t, J = 2.3 Hz, 1H), 8.30 (dd, J = 9.0, 1.9 Hz, 1 H), 8.16 (d, J = 8.8 Hz, 1H), 8.07 (dd, J = 8.2, 2.7 Hz, 1H), 7.63 (d, J = 6.3 Hz, 1H), 1.44 (s, 9H) |
| 83 | 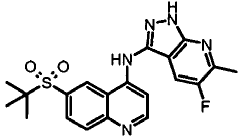 | 6-[(1,1-dimethylethyl) sulfonyl]-*N*-(5-fluoro-6-methyl-1*H-*pyrazolo[3,4-*b*] pyridin-3-yl)-4-quinolinamine | 414 | ¹H NMR (DMSO-d6) δ: 13.37 (s, 1H), 10.03 (s, 1H), 9.17 (d, J = 1.5 Hz, 1H), 8.68 (d, J = 5.3 Hz, 1H), 8.09 (t, J = 9.6 Hz, 2H), 7.98 - 8.05 (m, 1H), 7.50 (d, J = 5.6 Hz, 1H), 2.58 (d, J = 3.3 Hz, 3H), 1.33 (s, 9H) |
| 84 | 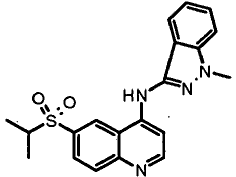 | 6-[(1-methylethyl) sulfonyl]-N-(1-methyl-1H-indazol-3-yl)-4-quinolinamine | 381 | ¹H NMR (DMSO-d6) δ: 14.97 (br. s., 1H), 11.72 (br. s., 1H), 9.53 (s, 1H), 8.74 (d, J = 7.1 Hz, 1H), 8.38 - 8.43 (m, 1H), 8.29 - 8.33 (m, 1H), 7.84 (d, J = 8.1 Hz, 1H), 7.79 (d, J = 8.6 Hz, 1H), 7.52 - 7.57 (m, 1H), 7.43 (d, J = 7.1 Hz, 1H), 7.26 (t, J = 7.6 Hz, 1H), 4.14 (s, 3H), 3.61 - 3.70 (m, 1H), 1.26 (d, J = 6.8 Hz, 6H) |
| 85 | 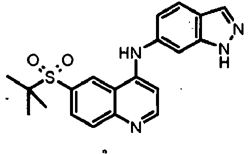 | 6-[(1,1-dimethylethyl) sulfonyl]-N-1H-indazol-6-yl-4-quinolinamine | 381 | ¹H NMR (DMSO-d6) δ: 13.02 (br. s., 1H), 9.73 (br. s., 1H), 9.04 (br. s., 1H), 8.60 (br. s., 1H), 7.97 - 8.10 (m, 4H), 7.82 (d, J = 8.3 Hz, 1H), 7.49 (m, 1H), 7.18 (d, J = 8.6 Hz, 1H), 7.06 (m, 1H), 1.32 (s, 9H) |
| 86 | 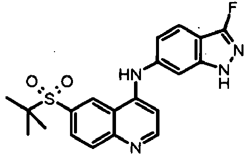 | 6-[(1,1-dimethylethyl) sulfonyl]-N-(3-fluoro-1H-indazol-6-yl)-4-quinolinamine | 399 | ¹H NMR (DMSO-d6) δ: 12.60 (br. s., 1H), 9.12 (s, 1H), 8.59 - 8.67 (m, 1H), 8.13 (s, 2H), 7.81 (d, J = 8.6 Hz, 1H), 7.47 - 7.53 (m, 1H), 7.26 (d, J = 8.8 Hz, 1H), 7.06 - 7.13 (m, 1H), 1.32 (s, 9H) |
| 87 | 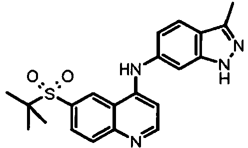 | 6-[(1,1-dimethylethyl) sulfonyl]-N-(3-methyl-1 H-indazol-6-yl)-4-quinolinamine | 395 | ¹H NMR (DMSO-d6) δ 12.58 (s, 1H), 9.69 (s, 1H), 9.04 (s, 1H), 8.59 (d, J = 5.3 Hz, 1H), 7.96 - 8.09 (m, 2H), 7.75 (d, J = 8.3 Hz, 1H), 7.40 (s, 1H), 7.14 (d, J = 8.6 Hz, 1H), 7.03 (d, J = 5.3 Hz, 1H), 2.50 (d, J = 1.8 Hz, 3H), 1.32 (s, 9H) |
| 88 | 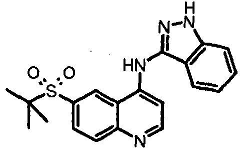 | 6-[(1,1-dimethylethyl) sulfonyl]-N-1H-indazol-3-yl-4-quinolinamine | 381 | ¹H NMR (DMSO-d6) δ: 12.87 (s, 1H), 10.03 (s, 1H), 9.22 (s, 1H), 8.62 (d, J = 5.3 Hz, 1H), 7.98 - 8.12 (m, 2H), 7.75 (d, J = 8.3 Hz, 1H), 7.56 (d, J = 8.3 Hz, 1H), 7.42 (t, J = 7.6 Hz, 1H), 7.28 (d, J = 5.6 Hz, 1H), 7.14 (t, J = 7.5 Hz, 1H), 1.34 (s, 9H) |
| 89 | 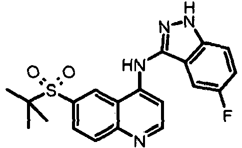 | 6-[(1,1-dimethylethyl) sulfonyl]-N-(5-fluoro-1H-indazol-3-yl)-4-quinolinamine | 399 | ¹H NMR (DMSO-d6) δ: 9.43 (s, 2H), 8.70 (d, J = 6.8 Hz, 1H), 8.34 (d, J = 9.0 Hz, 1H), 8.21 (d, J = 8.8 Hz, 1H), 7.70 (dd, J = 9.2, 4.0 Hz, 1H), 7.56 (dd, J = 9.0, 2.0 Hz, 1H), 7.39 (td, J = 9.0, 2.2 Hz, 1H), 7.23 (d, J = 6.8 Hz, 1H), 1.35 (s, 9H) |
| 90 | 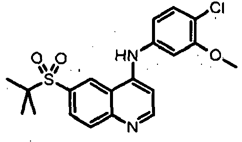 | N-[4-chloro-3-(methyloxy)phenyl]-6-[(1,1-dimethylethyl) sulfonyl]-4-quinolinamine | 405 | ¹H NMR (400 MHz, DMSO-d) δ ppm 1.31 (s, 9H), 3.87 (s, 3H), 7.00 (dd, J= 8.6 Hz, 2 Hz, 1H), 7.12 (d, J= 5.6 Hz, 1H), 7.16 (d, J= 2.1 Hz, 1H), 7.46 (d, J= 8.6 Hz, 1H), 7.99 (dd, J= 8.9 Hz, 1.8 Hz, 1H), 8.06 (d, J= 8.9 Hz, 1H), 8.62 (d, J= 5.3 Hz, 1H), 8.99 (s, 1H), 9.61 (s, 1H) |
| 91 | 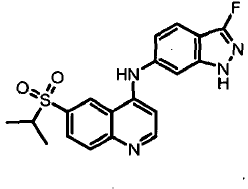 | N-(3-fluoro-1H-indazol-6-yl)-6-[(1-methylethyl)sulfonyl]-4-quinolinamine | 385 | ¹H NMR (400 MHz, DMSO-d6) δ ppm 12.46 (br. s., 1 H), 9.70 (s, 1 H), 9.03 (s, 1 H), 8.65 (d, J=5.3 Hz, 1 H), 8.07 (q, J=8.8 Hz, 2 H), 7.75 (d, J=8.8 Hz, 1 H), 7.41 (s, 1 H), 7.25 (d, J=8.8 Hz, 1 H), 7.17 (d, J=5.3 Hz, 1 H), 3.49 - 3.59 (m, 1 H), 1.23 (d, J=6.8 Hz, 6 H) |
| 92 | 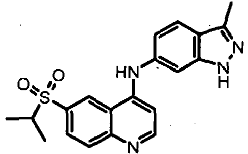 | 6-[(1-methylethyl) sulfonyl]-N-(3-methyl-1H-indazol-6-yl)-4-quinolinamine | 381 | ¹H NMR (400 MHz, DMSO-d6) δ ppm 12.57 (s, 1 H), 9.64 (s, 1 H), 9.06 (s, 1 H), 8.59 (d, J=5.3 Hz, 1 H), 7.98 - 8.14 (m, 2 H), 7.75 (d, J=8.6 Hz, 1 H), 7.40 (s, 1 H), 7.14 (d, J=8.3 Hz, 1 H), 7.06 (d, J=5.3 Hz, 1 H), 3.54 (dt, J=13.5, 6.6 Hz, 1 H), 3.33 (s, 3 H), 1.23 (d, J=6.8 Hz, 6 H) |
| 93 | 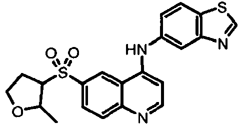 | N-1,3-benzothiazol-5-yl-6-[(2-methyltetrahydro-3-furanyl)sulfonyl]-4-quinolinamine (single diasteromer) | 426 | 1H NMR (400 MHz, DMSO-d6) δ ppm 9.72 (s, 1 H), 9.45 (s, 1 H), 9.12 (s, 1 H), 8.61 (d, J=5.6 Hz, 1 H), 8.24 (d, J=8.6 Hz, 1 H), 8.05 - 8.15 (m, 3 H), 7.58 (d, J=8.6 Hz, 1 H), 7.09 (d, J=5.6 Hz, 1 H), 4.15 - 4.28 (m, 2 H), 3.98 (td, J=8.3, 3.0 Hz, 1 H), 3.55 (q, J=8.1 Hz, 1 H), 2.17 - 2.36 (m, 1 H), 2.03 (td, J=8.0, 4.0 Hz, 1 H), 1.52 (d, J=5.3 Hz, 3 H) |
| 94 | 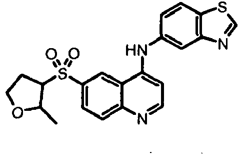 | N-1,3-benzothiazol-5-yl-6-[(2-methyltetrahydro-3-furanyl)sulfonyl]-4-quinolinamine (single diastereomer) | 426 | ¹H NMR (400 MHz, DMSO-d6) δ ppm 9.72 (s, 1 H), 9.46 (s, 1 H), 9.12 (s, 1 H), 8.62 (d, J=5.3 Hz, 1 H), 8.25 (d, J=8.6 Hz, 1 H), 8.09 (dd, J=6.6, 2.5 Hz, 3 H), 7.58 (dd, J=8.6, 2.0 Hz, 1 H), 7.10 (d, J=5.6 Hz, 1 H), 4.13 - 4.29 (m, 2 H), 3.98 (td, J=8.4, 3.4 Hz, 1 H), 3.55 (q, J=8.2 Hz, 1 H), 2.27 (dd, J=12.9, 6.3 Hz, 1 H), 2.03 (dt, J=7.8, 3.9 Hz, 1 H), 1.52 (d, 3 H) |
| 95 | 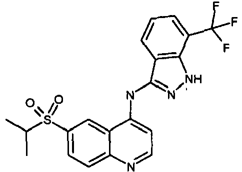 | 6-[(1-methylethyl)sulfonyl]-N-[7-(trifluoromethyl)-1H-indazol-3-yl]-4-quinolinamine | 435 | ¹H NMR (DMSO-d6) δ 13.39 (br. s., 1H), 10.07 (br. s., 1H), 9.25 (br. s., 1H), 8.69 (d, J = 5.3 Hz, 1H), 8.22 (d, J = 7.9 Hz, 1H), 8.12 (d, J = 8.7 Hz, 1H), 8.03 - 8.10 (m, J = 8.3 Hz, 1H), 7.83 (d, J = 6.8 Hz, 1H), 7.55 (d, J = 4.9 Hz, 1H), 7.32 (t, J = 7.6 Hz, 1H), 3.52 - 3.63 (m, 1H), 1.24 (d, J = 6.8 Hz, 6H) |
| 96 | 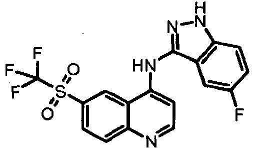 | N-(5-fluoro-1H-indazol-3-yl)-6-[(trifluoromethyl) sulfonyl]-4-quinolinamine | 411 | ¹H NMR (DMSO-d6) δ: 13.06 (br. s, 1H), 10.20 (s, 1H), 9.54 - 9.64 (m, 1H), 8.71 (d, J = 5.5 Hz, 1H), 8.17 - 8.27 (m, 2H), 7.53 - 7.65 (m, 2H), 7.41 (d, J = 5.5 Hz, 1H), 7.30 (t, J = 9.3 Hz, 1H) |
| 97 | 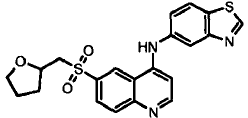 | N-(6-(((tetrahydrofuran-2-yl)methyl)sulfonyl) quinolin-4-yl)benzo[d]thiazol-5-amine | 426 | ¹H NMR (DMSO-d6) δ: 11.57 (br. s., 1H), 9.55 (s, 1H), 9.42 (s, 1H), 8.60 (d, J = 7.1 Hz, 1H), 8.36 - 8.48 (m, 2H), 8.27 (d, J = 1.8 Hz, 1 H), 8.19 (d, J = 9.1 Hz, 1H), 7.65 (dd, J = 8.3, 1.8 Hz, 1H), 7.00 (d, J = 7.1 Hz, 1H), 4.16 - 4.31 (m, 1H), 3.70 - 3.83 (m, 2H), 3.44 - 3.64 (m, 2H), 1.95 - 2.10 (m, 1H), 1.71 - 1.88 (m, 2H), 1.55 - 1.70 (m, 1H) |
| 98 | 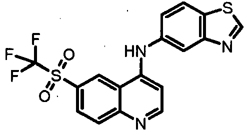 | 4-(1,3-benzothiazol-5-ylamino)-6-[(trifluoromethyl) sulfonyl]quinoline | 410 | ¹H NMR (METHANOL-d4) δ: 9,60 (d, J = 1.8 Hz, 1H), 9.43 (s, 1H), 8.50 - 8.59 (m, 2H), 8.36 (d, J = 8.6 H, 1H), 8.20 - 8.29 (m, 2H), 7.66 (dd, J = 10.6, 2.0 Hz, 1H), 7.08 (d, J = 7.3 Hz, 1H) |

### Example 99

### N-(6-(tert-butylsulfonyl)quinolin-4-yl)thiazolo[5,4-b]pyridin-6-amine

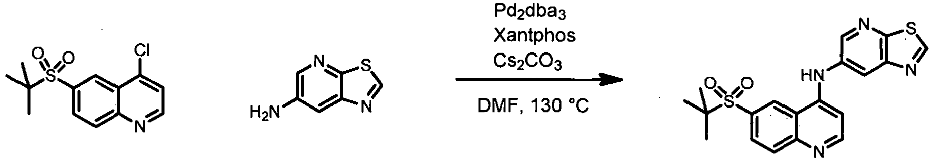

To a vial was added 4-chloro-6-[(1,1-dimethylethyl)sulfonyl]quinotine (500 mg, 1.76 mmol), [1,3]thiazolo[5,4-b]pyridin-6-amine (320 mg, 2.11 mmol), Pd₂(dba)₃ (161 mg, 0.18 mmol), Xantphos (102 mg, 0.18 mmol), and cesium carbonate (1.15 g, 3.52 mmol). The vial was evacuated and back-filled with nitrogen three times before DMF (18 mL) was added and the reaction was heated to 130°C for 10 min via microwave. It was then partitioned between 50 mL each of water and EtOAc. The organic layer was collected and the aqueous layer was backextracted with EtOAc (2 x 50 mL). The combined organics were dried over sodium sulfate, filtered, and concentrated. The crude was purified by flash chromatography. Concentration of desired fractions afforded the 362 mg (51 %) of the desired product. 1H NMR (DMSO-d6) Shift: 9.83 (s, 1H), 9.62 (s, 1H), 9.05 (d, J = 1.8 Hz, 1H), 8.79 (d, J = 2.5 Hz, 1H), 8.64 (d, J = 5.6 Hz, 1H), 8.51 (d, J = 2.3 Hz, 1H), 8.11 (d, J = 8.8 Hz, 1 H), 8.03 (dd, J = 8.8, 1.8 Hz, 1H), 7.09 (d, J = 5.3 Hz, 1H), 1.33 (s, 9H); MS (m/z) 399 (M+H⁺).

The following compound was prepared using a procedure analogous to that described above.

| Ex # | Structure | Name | MS (M+H)⁺ | NMR |
|---|---|---|---|---|
| 100 | 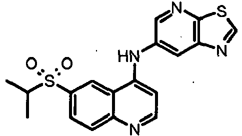 | 6-[(1-methylethyl)sulfonyl]-*N*-[1,3]thiazolo[5,4-*b*]pyridin-6-yl-4-quinolinamine | 385 | ¹H NMR (DMSO-d6) δ: 9.81 (s, 1H), 9.62 (s, 1H), 9.07 (s, 1H), 8.79 (d, J = 2.0 Hz, 1H), 8.65 (d, J = 5.3 Hz, 1H), 8.51 (d, J = 1.8 Hz, 1H), 8.05 - 8.16 (m, 2H), 7.11 (d, J = 5.3 Hz, 1H), 3.49 - 3.61 (m, 1H), 1.24 (d, 6H) |

### Example 101

### N-2,1,3-Benzoxadiazol-5-yl-6-(methylsulfonyl)-4-quinolinamine

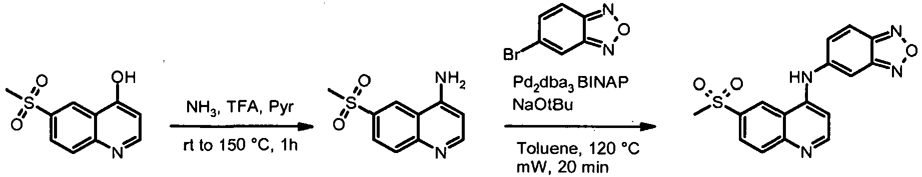

Step 1.6-(Methylsulfonyl)-4-quinolinyl trifluoromethanesulfonate: To a solution of 6-(methylsulfonyl)-4-quinolinol (500 mg, 2.24 mmol) in pyridine (7 mL) was added triflic anhydride (1.4 mL, 6.72 mmol) in a sealed tube. The reaction was stirred for 5 minutes and ammonia (44.8 mL, 22.4 mmol, 0.5M in dioxane) was added and heated at 150 °C for 1h. The solvent was removed *in vacuo* and the crude product 6-(methylsulfonyl)-4-quinolinyl trifluoromethanesulfonate was dissolved in MeOH and preabsorbed onto silica and purified by column chromatography (40g RediSep silica gel cartridge on Isco CombiFlash, MeOH in DCM, 2 to 20%). The fractions were pooled and the solvent removed *in vacuo.* The crude product was triturated with EtOAc, and the light yellow solid 6-(methylsulfonyl)-4-quinolinamine was filtered and dried in air. ¹H NMR (400 MHz, Methanol-d₄) δ 9.08 (d, *J* = 1.8 Hz, 1H), 8.35 - 8.47 (m, 2H), 8.03 (d, *J* = 9.1 Hz, 1H), 6.94 (d, *J* = 7.1 Hz, 1H), 3.27 (s, 3H); MS (m/z) 222.9 (M+H⁺).

Step 2. *N*-2,1,3-Benzoxadiazol-5-yl-6-(methylsulfonyl)-4-quinolinamine: 6-(Methylsulfonyl)-4-quinolinamine (50 mg, 0.225 mmol), 5-bromo-2,1,3-benzoxadiazole, Pd₂dba₃ (20.60 mg, 0.022 mmol), BINAP (14.01 mg, 0.022 mmol), and sodium tert-butoxide (43.2 mg, 0.450 mmol) were added to a vial which was purged with nitrogen. Toluene (2250 µl) was then added and the reactions were heated to 120 °C for 20 min via microwave. It was then concentrated, redissolved in DMSO, filtered, and purified by RP HPLC to afford the title compound as a solid (11 mg, 14 %). 1H NMR (400 MHz, DMSO-d6) δ 9.26 (s, 1H), 8.82 (d, *J* = 5.1 Hz, 1H), 8.39 (d, *J* = 8.5 Hz, 1H), 8.23 (m, 2H), 7.95 (br. s, 1H), 7.72 (d, *J* = 9.5 Hz, 1H), 7.40 - 7.46 (m, 1H), 3.38 (s, 3H); MS (m/z) 341.1 (M+H⁺).

### Example 102

### 3-{[4-(1,3-Benzothiazol-5-ylamino)-6-quinolinyl]sulfonyl}-3-methyl-1-butanol

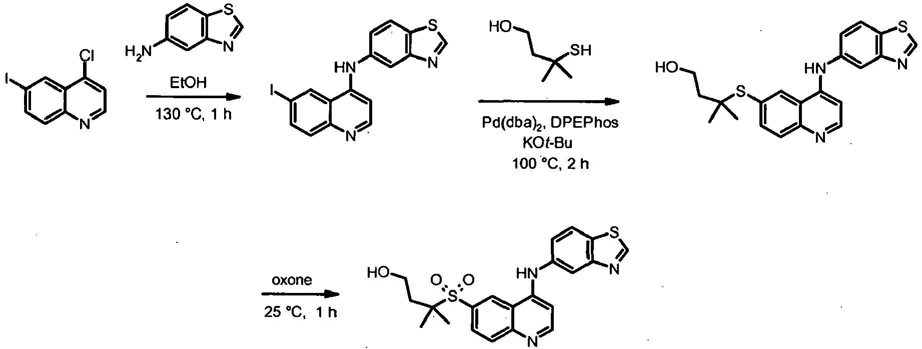

Step 1. N-1,3-Benzothiazol-5-yl-6-iodo-4-quinolinamine: A mixture of 4-chloro-6-iodoquinoline (3.5 g, 12 mmol) and 1,3-benzothiazol-5-amine (1.8 g, 12 mmol) was heated in EtOH (120 mL) at 130°C in a sealed tube for 1 h. The reaction mixture was cooled and diethyl ether (100 mL) was added, and N-1,3-benzothiazol-5-yl-6-iodo-4-quinolinamine was filtered and dried to a brown solid (4.88 g, 88 %). ¹H NMR (400 MHz, DMSO-*d*₆) δ 11.04-11.32 (m, 1H), 9.54 (s, 1H), 9.27 (d, *J* = 1.52 Hz, 1H), 8.53 (d, *J* = 7.07 Hz, 1H), 8.39 (d, *J* = 8.59 Hz, 1H), 8.33 (dd, *J* = 8.84, 1.77 Hz, 1H), 8.12-8.27 (m, 1H), 7.86 (d, *J* = 8.84 Hz, 1H), 7.60 (dd, *J* = 8.59, 2.02 Hz, 1H), 6.91 (d, *J* = 7.07 Hz, 1H); MS (m/z) 403.9 (M+H⁺).

Step 2. 3-{[4-(1,3-Benzothiazol-5-ylamino)-6-quinolinyl]thio}-3-methyl-1-butanol: A mixture of N-1,3-benzothiazol-5-yl-6-iodo-4-quinolinamine (400 mg, 0.99 mmol), 3-mercapto-3-methyl-1-butanol (119 mg, 0.99 mmol), potassium tert-butoxide (223 mg, 1.98 mmol), (oxydi-2,1-phenylene)bis-(diphenylphosphine) (53.4 mg, 0.10 mmol) and bis(dibenzylidineacetone)palladium (91 mg, 0.10 mmol) in 6.5 mL of DMF were heated at 100 °C in a sealed, nitrogen-purged vial for 2 h. The reaction was poured into EtOAc and washed 2x with saturated ammonium chloride and saturated sodium bicarbonate followed by brine. The organics were dried over MgSO₄ and the solvent was removed *in vacuo*. The crude product was purified by column chromatography (Isco CombiFlash, 40g silica gel cartridge, EtOAc to 20:80 mix of EtOAc:10% solution of ammonium hydroxide in IPA), and the solvent removed *in vacuo* to give 3-{[4-(1,3-benzothiazol-5-ylamino)-6-quinolinyl]thio}-3-methyl-1-butanol (200 mg, 76% yield) as a tan solid. ¹H NMR (400 MHz, CDCl₃) δ 9.08 (s, 1 H), 8.58 (d, *J* = 5.31 Hz, 1H), 8.28 (d, *J* = 1.77 Hz, 1H), 8.10 (d, *J* = 2.02 Hz, 1H), 8.00 (dd, *J* = 8.59, 2.27 Hz, 2H), 7.72 - 7.88 (m, 1H), 7.41 - 7.52 (m, 1H), 7.05 (d, *J* = 5.31 Hz, 1H), 4.01 (s, *J* = 6.82 Hz, 2H), 2.20 (s, 1H), 1.85 (t, *J* = 6.82 Hz, 2H), 1.33 (s, 6H); MS (m/z) 396.2 (M+H⁺).

Step 3. 3-{[4-(1,3-Benzothiazol-5-ylamino)-6-quinolinyl]thio}-3-methyl-1-butanol: To a solution of 3-{[4-(1,3-benzothiazol-5-ylamino)-6-quinolinyl]thio}-3-methyl-1-butanol (300 mg, 0.76 mmol) in MeOH (8 mL) was added oxone (933 mg, 1.52 mmol) and the reaction mixture was stirred for 1 h at 25 °C. The mixture was filtered and the solvent removed *in vacuo.* The crude product was dissolved in MeOH and preabsorbed on silica and purified by column chromatography (40g RediSep silica gel cartridge on Isco CombiFlash, EtOAc to 20:80 mix of EtOAc:10% solution of ammonium hydroxide in IPA). The fractions were pooled and the solvent was removed *in vacuo.* The resulting yellow solid was triturated with diethyl ether and filtered to give an off white solid of 3- {[4-(1,3-benzothiazol-5-ylamino)-6-quinolinyl]sulfonyl}-3-methyl-1-butanol (324 mg, 68% yield). ¹H NMR (400 MHz, Methanol-*d*4) δ 9.27 - 9.45 (m, 1H), 8.94 - 9.14 (m, 1H), 8.50 - 8.62 (m, 1H), 8.16 - 8.28 (m, 1H), 8.05 - 8.16 (m, 4H), 7.56 - 7.73 (m, 1H), 6.99 - 7.18 (m, 1H), 3.43 - 3.60 (t, *J =* 6.82 Hz, 2H), 2.00 - 2.19 (t, *J=* 6.82 Hz, 2H), 1.44 (s, 6H); MS (m/z) 428.2 (M+H⁺).

Alternatively, the sulfoxide may be formed by using only 0.4 equivalents of oxone. Tetrahydrofuran or ethylacetate may be used in place of MeOH as the cosolvent.

The following compounds were prepared using procedures analogous to those described above using the appropriate aniline and thiol:

| Ex # | Structure | Name | MS (M+H )⁺ | NMR |
|---|---|---|---|---|
| 103 | 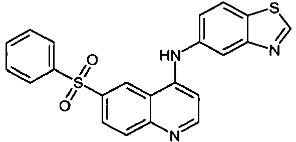 | N-1,3-benzothiazol-5-yl-6-(phenylsulfonyl)-4-quinolinamine | 418 | ¹H NMR (400 MHz, CDCl3) δ 9.11 (s, 1H), 8.94 (s, 1H), 8.63 (d, J = 5.31 Hz, 1H), 8.09 - 8.17 (m, 3H), 7.99 - 8.09 (m, 2H), 7.58 (s, 1H), 7.45 - 7.54 (m, 5H), 7.07 (d, J = 5.31 Hz, 1H) |
| 104 | 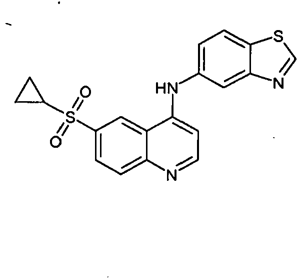 | N-1,3-benzothiazol-5-yl-6-(cyclopropyl sulfonyl)-4-quinolinamine | 382 | ¹H NMR (400 MHz, Methanol-d4) δ 9.43 (s, 1H), 9.31 (d, J = 1.52 Hz, 1H), 8.44 - 8.55 (m, 3H), 8.35 (d, J = 8.59 Hz, 1H), 8.24 (d, J = 1.77 Hz, 1H), 8.15 (d, J = 9.09 Hz, 1H), 7.66 (dd, J = 8.59, 2.02 Hz, 1H), 7.05 (d, J = 7.07 Hz, 1H), 2.87 (ddd, J = 7.89, 4.74, 3.03 Hz, 1H), 1.35 - 1.52 (m, 2H), 1.11 - 1.27 (m, 2H) |
| 105 | 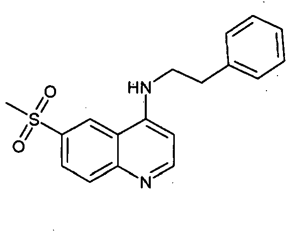 | 6-(methylsulfonyl)-N-(2-phenylethyl)-4-quinolinamine | 327 | ¹H NMR (400, MHz, Chloroform-d) δ 8.58 - 8.75 (m, 1H), 8.34 - 8.44 (m, 1H), 8.15 - 8.24 (m, 1H), 7.98 - 8.12 (m, 1H), 7.38 (d, J = 7.58 Hz, 2H), 7.30 (d, J = 2.02 Hz, 3H), 6.48 - 6.67 (m, 1H), 5.40 - 5.74 (m, 1H), 3.61 - 3.78 (m, 2H), 3.14 (s, 3H), 3.12 (d, J = 7.33 Hz, 2H) |
| 106 | 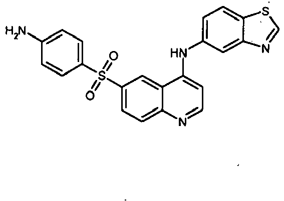 | 6-[(4-aminophenyl) sulfonyl]-N-1,3-benzothiazol-5-yl-4-quinolinamine tetrahydrochloride | 433 | ¹H NMR (400 MHz, Methanol-d4) δ 9.43 - 9.57 (m, 1H), 9.32 - 9.43 (m, 1H), 8.41 - 8.52 (m, 2H), 8.32 - 8.41 (m, 1H), 8.20 - 8.32 (m, 1H), 8.01 - 8.11 (m, 1H), 7.82 - 7.94 (m, 2H), 7.62 - 7.72 (m, 1H), 6.97 - 7.09 (m, 1H), 6.85 - 6.97 (m, 2H) |
| 107 | 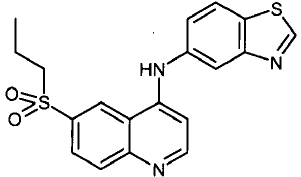 | 5- {[6-(propylsulfonyl)-4-quinolinyl] ammonio}-1,3-benzothiazol-3-ium bis(trifluoroacetate) | 384 | ¹H NMR (400 MHz, Methanol-d4) δ 9.43 (s, 1H), 9.33 (s, 1H), 8.50 (d, J = 7.07 Hz, 1H), 8.43 - 8.49 (m, 2H), 8.32 - 8.42 (m, 1H), 8.24 - 8.32 (m, 1H), 8.11 - 8.24 (m, 1H), 7.53 - 7.94 (m, 1H), 7.06 (d, J = 7.33 Hz, 1H), 3.36 - 3.51 (m, 2H), 1.63 - 1.89 (m, 2H), 1.08 (t, J = 7.45 Hz, 3H) |
| 108 | 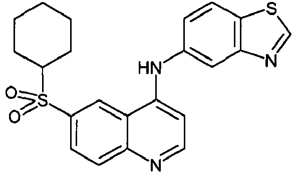 | N-1,3-benzothiazol-5-yl-6-(cyclohexylsulfonyl)-4-quinolinamine | 424 | ¹H NMR (400 MHz, Chloroform-d) δ 9.11 (s, 1H), 8.62 - 8.79 (m, 2H), 8.20 (d, J = 8.84 Hz, 1H), 8.16 (d, J = 2.02 Hz, 1H), 8.01 - 8.10 (m, 2H), 7.48 (dd, J = 8.46, 2.15 Hz, 1H), 7.40 (s, 1H), 7.12 (d, J = 5.31 Hz, 1H), 2.92 - 3.10 (m, 1H), 2.08 (d, J = 11.87 Hz, 2H), 1.85 (br. s., 2H), 1.45 (br. s., 2H), 1.02 - 1.41 (m, 3H), 0.68 - 1.02 (m, 1H) |
| 109 | 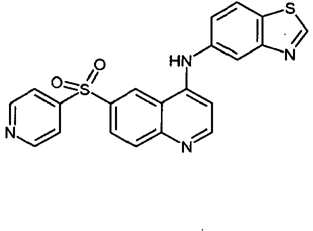 | N-1,3-benzothiazol-5-yl-6-(4-pyridinylsulfonyl)-4-quinolinamine | 419 | ¹H NMR (400 MHz, Chloroform-d) 8 9.12 (s, 1H), 8.81 - 8.92 (m, 3H), 8.68 (d, J = 5.56 Hz, 1H), 8.17 (d, J = 9.09 Hz, 1H), 8.13 (d, J = 2.02 Hz, 1H), 8.01 - 8.11 (m, 2H), 7.81 - 7.89 (m, 2H), 7.41 - 7.52 (m, 1H), 7.31 - 7.35 (m, 1H), 7.09 (d, J = 5.56 Hz, 1H) |
| 110 | 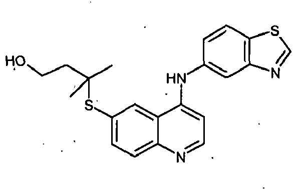 | 3-{[4-(1,3-benzothiazol-5-ylamino)-6-quinolinyl]thio}-3-methyl-1-butanol | 396 | ¹H NMR (400 MHz, Chloroform-d) δ 9.08 (s, 1H), 8.58 (d, J = 5.31 Hz, 1H), 8.28 (d, J = 1.77 Hz, 1H), 8.10 (d, J = 2.02 Hz, 1H), 8.00 (dd, J = 8.59, 2.27 Hz, 2H), 7.72 - 7.88 (m, 1H), 7.41 - 7.52 (m, 1H), 7.05 (d, J = 5.31 Hz, 1H), 4.01 (s, J = 6.82 Hz, 2H), 2.20 (s, 1H), 1.85 (t, J = 6.82 Hz, 2H), 1.33 (s, 6H) |
| 111 | 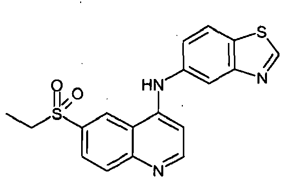 | N-1,3-benzothiazol-5-yl-6-(ethylsulfonyl)-4-quinolinamine | 370 | ¹H NMR (400 MHz, Chloroform-d) δ 9.12 (s, 2H), 8.65 - 8.76 (m, 2H), 8.19 - 8.28 (m, 1H), 8.14 - 8.19 (m, 1H), 8.09 - 8.14 (m, 1H), 8.03 - 8.09 (m, 1H), 7.41 - 7.53 (m, 1H), 7.08 - 7.15 (m, 1H), 3.16 - 3.35 (m, 2H), 1.36 (s, 3H) |
| 112 | 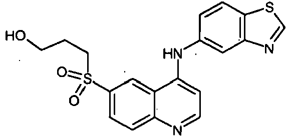 | 3-{[4-(1,3-benzothiazol-5-ylamino)-6-quinolinyl]sulfonyl}-1-propanol | 400 | ¹H NMR (400 MHz, Chloroform-d) δ 9.00 (d, J = 5.31 Hz, 1H), 8.47 (d, J = 5.31 Hz, 1H), 8.41 (d, J = 1.77 Hz, 1H), 8.30 (d, J = 8.84 Hz, 1H), 8.15 (dd, J = 8.84, 2.02 Hz, 1H), 8.08 - 8.12 (m, 1H), 7.30 - 7.37 (m, 4H), 7.18 - 7.26 (m, 1H), 3.12 - 3.21 (m, 4H), 2.95 (d, J = 7.33 Hz, 2H) |
| 113 | 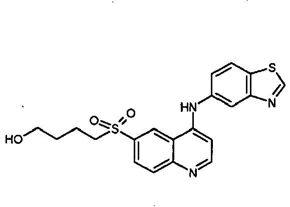 | 4-{[4-(1,3-benzothiazol-5-ylamino)-6-quinolinyl]sulfonyl}-1-butanol | 414 | ¹H NMR (400 MHz, Methanol-d4) δ 9.33 (s, 1H), 9.06 (d, J = 1.77 Hz, 1H), 8.49 - 8.61 (m, 1H), 8.17 (dd, J = 8.84, 2.27 Hz, 2H), 8.07 - 8.13 (m, 4H), 7.61 (dd, J = 8.59, 2.27 Hz, 1H), 7.11 (d, J = 5.81 Hz, 1H), 3.56 (t, J = 6.06 Hz, 2H), 3.35 - 3.44 (m, 2H), 1.79 - 1.89 (m, 2H), 1.60 - 1.71 (m, 2H) |
| 114 | 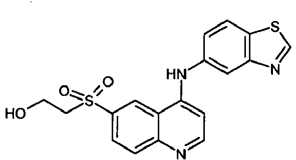 | 2-{[4-(1,3-benzothiazol-5-ylamino)-6-quinolinyl]sulfonyl} ethanol | 386 | ¹H NMR (400 MHz, Methanol-d4) δ 9.34 (s, 1H), 9.08 (d, J = 1.77 Hz, 2H), 8.55 (d, J = 5.56 Hz, 2H), 8.15 - 8.27 (m, 2H), 8.04 - 8.15 (m, 2H), 7.61 (dd, J = 8.59, 2.02 Hz, 1H), 7.10 (d, J = 5.81 Hz, 1H), 3.95 (t, J = 6.06 Hz, 2H), 3.55 (t, J = 6.06 H, 2H) |
| 115 | 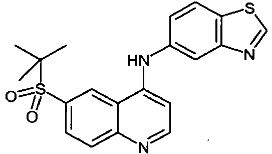 | N-1,3-benzothiazol-5-yl-6-[(1,1-dimethylethyl)sulfonyl] -4-quinolinamine | 398 | ¹H NMR (400 MHz, Chloroform-d) δ 9.10 (s, 1H), 8.70 (d, J = 5.56 Hz, 2H), 8.13 - 8.26 (m, 2H), 8.06 - 8.13 (m, 1H), 8.04 (d, J = 8.34 Hz, 1H), 7:33 - 7.58 (m, 2H), 7.08 (d, J = 5.31 Hz, 1H), 1.41 (s, 9H) |
| 116 | 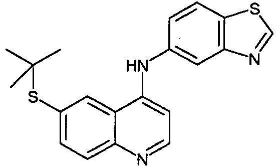 | N-1,3-benzothiazol-5-yl-6-[(1,1-dimethylethyl)thio]-4-quinolinamine | 366 | ¹H NMR (400 MHz, Chloroform-d) δ 9.18 (s, 1H), 8.59 (m, 1H), 8.10 (m, 1H), 8.21 (m, 1H), 7.94 (m, 2H), 7.79 (m, 1H), 7.42 (m, 1H), 7.13 (br. s, 1H), 7.04 (d, J = 5.31 Hz, 1H), 1.31 (s, 9H) |
| 117 | 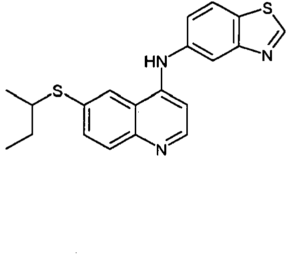 | N-1,3-benzothiazol-5-yl-6-[(1-methylpropyl)thio]-4-quinolinamine | 366 | ¹H NMR (400 MHz, Chloroform-d) δ 9.09 (s, 1H), 8.57 (d, J = 5.31 Hz, 1H), 8.08 - 8.14 (m, 1H), 7.96 - 8.07 (m, 3H), 7.74 (dd, J = 8.84, 2.02 Hz, 1H), 7.45 (dd, J = 8.59, 2.02 Hz, 1H), 7.06 (d, J = 5.31 Hz, 1H), 6.86 (br. s., 1H), 3.32 (sxt, J = 6.82 Hz, 1H), 1.67 - 1.80 (m, 2H), 1.34 (d, J = 6.57 Hz, 3H), 1.05 (t, J = 7.33 Hz, 3H) |
| 118 | 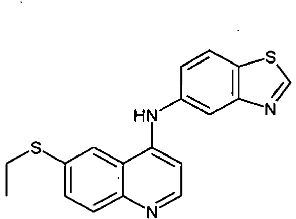 | N-1,3-benzothiazol-5-yl-6-(ethylthio)-4-quinolinamine | 338 | ¹H NMR (400 MHz, Chloroform-d) δ 9.08 (s, 1H), 8.56 (d, J = 5.05 Hz, 1H), 8.08 (d, J = 2.27 Hz, 1H), 8.02 (s, 1H), 7.99 (s, 1H), 7.94 (d, J = 1.77 Hz, 1H), 7.69 (dd, J = 8.84, 2.02 Hz, 1H), 7.45 (dd, J = 10.61,2.02 Hz, 1H), 7.05 (d, J = 5.31 Hz, 1H), 6.82 (br. s., 1H), 3.08(q,J=7.33Hz, 2H), 1.38 (t, J = 7.33 Hz, 3H) |
| 119 | 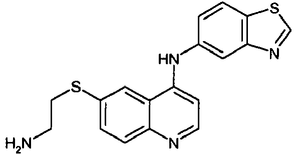 | 6-[(2-aminoethyl)thio]-N-1,3-benzothiazol-5-yl-4-quinolinamine | 353 | ¹H NMR (400 MHz, Methanol-d4) δ 9.30 - 9.35 (m, 1H), 8.41 (d, J = 5.56 Hz, 1H), 8.35 - 8.38 (m, 1H), 8.15 (d, J = 8.59 Hz, 1H), 8.04 - 8.09 (m, 1H), 7.87 (d, J = 8.84 Hz, 1H), 7.77 (dd, J = 8.84, 2.02 Hz, 1H), 7.58 (dd, J = 8.84, 2.27 Hz, 1H), 7.04 (d, J = 5.56 Hz, 1H), 3.26 (t, J = 6.82 Hz, 2H), 2.97 (t, J = 6.82 Hz, 2H) |
| 120 | 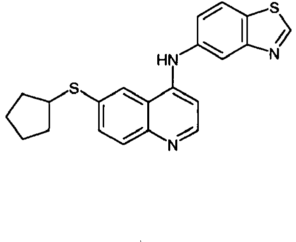 | N-1,3-benzothiazol-5- yl-6-(cyclopentylthio)-4-quinolmamine | 378 | ¹H NMR (400 MHz, CDCl3) δ 9.06 - 9.12 (m, 1H), 8.57 (d, J = 5.31 Hz, 1H), 8.08 (d, J = 2.02 Hz, 1H), 8.03 (d, J = 3.28 Hz, 1H), 8.00 (d, J = 3.54 Hz, 1 H), 7.96 (d, J = 2.02 Hz, 1 H), 7.74 (dd, J = 8.84, 2.02 Hz, 1H), 7.45 (dd, J = 8.59, 2.02 Hz, 1H), 7.06 (d, J = 5.31 Hz, 1H), 6.64 - 6.83 (m, 1H), 3.71 - 3.83 (m, 1H), 2.03 - 2.19 (m, 2H), 1.75 - 1.96 (m, 2H), 1.62 - 1.76 (m, 4H) |
| 121 | 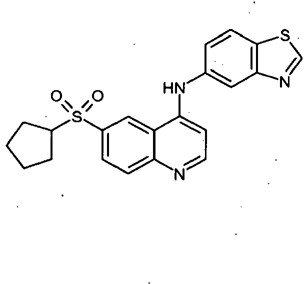 | N-1,3-benzothiazol-5- yl-6-(cyclopentyl sulfonyl)-4-quinolinamine | 410 | ¹H NMR (400 MHz, CDCl3) δ 9.10 (s, 1H), 8.79 (d, J = 1.77 Hz, 1H), 8.66 (d, J = 5.56 Hz, 1H), 8.19 (d, J = 8.84 Hz, 1H), 8.15 (d, J = 2.27 Hz, 1H), 8.07 (dd, J = 8.84, 1.77 Hz, 1H), 8.03 (d, J = 8.59 Hz, 1H), 7.48 (d, J = 8.59 Hz, 1H), 7.07 - 7.12 (m, 1H), 3.91 - 4.03 (m, 1H), 3.55 - 3.68 (m, 1H), 2.04 - 2.18 (m, 2H), 1.84 - 1.93 (m, 3H), 1.69 - 1.81 (m, 1H), 1.47 - 1.68 (m, 1H) |
| 122 | 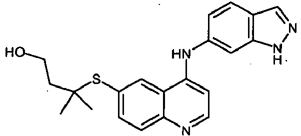 | 3-{[4-(1H-indazol-6- ylamino)-6-quinolinyl]thio}-3-methyl-1-butanol | 379 | ¹H NMR (400 MHz, Methanol-d4) δ 8.55 - 8.66 (m, 1H), 8.43 (d, J = 5.56 Hz, 1H), 8.02 - 8.10 (m, 1H), 7.80 - 7.88 (m, 5H), 7.49 - 7.55 (m, 1H), 7.24 (dd, J = 10.36, 1.77 Hz, 1H), 7.04 (d, J = 5.56 Hz, 1H), 3.86 (t, J = 7.33 Hz, 2H), 1.84 (t, J = 7.58 Hz, 2H), 1.35 (s, 6H), 1.16 (d, J = 6.06 Hz, 1H) |
| 123 | 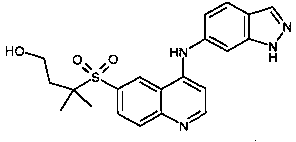 | 3-{[4-(1H-indazol-6-ylamino)-6-quinolinyl]sulfonyl}-3-methyl-1-butanol | 411 | ¹H NMR (400 MHz, Methanol-d4) δ 8.95 - 9.06 (m, 1H), 8.54 (d, J = 5.81 Hz, 1H), 8.02 - 8.17 (m, 3H), 7.82 - 7.95 (m, 1H), 7.48 - 7.70 (m, 1H), 7.25 (d,J = 10.36 Hz, 1H), 7.08 (d, J = 5.81 Hz, 1H), 5.43 - 5.50 (m, 1H), 3.76 (t, J = 7.07 Hz, 2H), 2.03 (t, J = 7.07 Hz, 2H), 1.44 (s, 6H) |
| 124 | 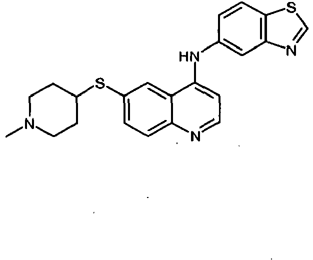 | N-1,3-benzothiazol-5-yl-6-[(1-methyl-4-piperidinyl)thio]-4-quinolinamine | 407 | ¹H NMR (Chloroform-d) δ 9.09 (s, 1H), 8.58 (d, J = 5.1 Hz, 1H), 8.09 (d, J = 2.3 Hz, 1H), 8.08 (d, J = 1.5 Hz, 1H), 8.02 (s, 1H), 8.00 (s, 1H), 7.75 (dd, J = 8.8, 1.8 Hz, 1H), 7.45 (d, J = 8.6 Hz, 1H), 7.06 (d, J = 5.3 Hz, 1H), 6.90 (s, 1H), 3.11 - 3.26 (m, 1H), 2.82 (br. s., 2H), 2.28 (s, 3H), 1.92 - 2.16 (m, 3H), 1.65 - 1.84 (m, 2H), 1.17 - 1.25 (m, 1H) |
| 125 | 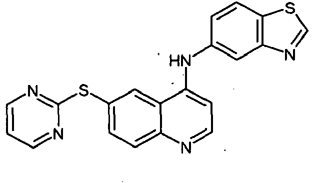 | N-1,3-benzothiazol-5-yl-6-(2-pyrimidinylthio)-4-quinolinamine | 388 | ¹H NMR (Chloroform-d) δ 9.08 (s, 1H), 8.62 (d, J = 5.3 Hz, 1H), 8.53 (d, J = 4.8 Hz, 2H), 8.37 (d, J = 1.8 Hz, 1H), 8.07 - 8.11 (m, 2H), 7.99 (d, J = 8.6 Hz, 1H), 7.91 (dd, J = 8.6, 1.8 Hz, 1H), 7.44 (dd, J = 8.6, 2.0 Hz, 1H), 7.07 (d, J = 5.3 Hz, 1H), 7.03 (t,J=4.8Hz,1H) |
| 126 | 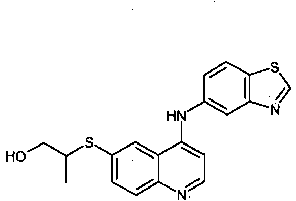 | 2-{[4-(1,3-benzothiazol-5-ylamino)-6-quinolinyl]thio}-1-propanol | 368 | ¹H NMR (Chloroform-d) δ 9.08 (s, 1H), 8.55 (d, J = 5.3 Hz, 1H), 8.15 (d, J = 1.8 Hz, 1H), 8.06 - 8.09 (m, 1H), 7.92 - 8.02 (m, 2H), 7.72 (dd, J = 10.6, 1.8 Hz, 1H), 7.44 (dd, J = 8.3, 2.0 Hz, 1H), 7.18 (br. s., 1H), 7.03 (d, J = 5.3 Hz, 1H), 3.65 (spt, J = 5.6 Hz, 2H), 3.40 - 3.52 (m, 1H), 1.37 (d, J = 7.1 Hz, 3H) |
| 127 | 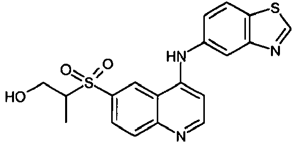 | 2-{[4-(1,3-benzothiazol-5-ylamino)-6-quinolinyl]sulfonyl}-1-propanol | 401 | ¹H NMR (Chloroform-d) δ 9.12 (s, 1H), 8.74 (d, J = 5.6 Hz, 1H), 8.64 - 8.69 (m, 1H), 8.24 (d, J = 8.8 Hz, 1H), 8.14-8.19 (m, 1H), 8.11 (dd, J = 8.8, 1.8 Hz, 1H), 8.05 (d, J = 8.6 Hz, 1H), 7.47 (dd, J = 8.6, 2.0 Hz, 1H), 7.21 (br. s., 1H), 7.12 (d, J = 5.3 Hz, 1H), 4.00 - 4.10 (m, 1H), 3.86 - 4.00 (m, 1H), 3.37 - 3.53 (m, 1H), 1.33 - 1.42 (m, J = 7.1 Hz, 3H) |
| 128 | 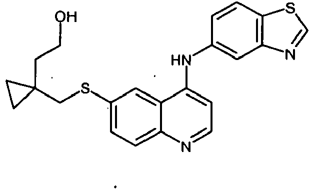 | 2-[1-({[4-(1,3-benzothiazol-5-ylamino)-6-quinolinyl]thio} methyl)cyclopropyl] ethanol | 408 | ¹H NMR (Chloroform-d) δ 9.05 (s, 1H), 8.47 (d, J = 5.3 Hz, 1H), 8.05 - 8.14 (m, 2H), 7.96 (d, J = 8.6 Hz, 1H), 7.89 (d, J = 8.8 Hz, 1H), 7.58 (d, J = 8.8 Hz, 2H), 7.48 (dd, J = 10.6, 2.0 Hz, 2H), 7.02 (d, J = 5.3 Hz, 1H), 3.89 (t, J = 6.3 Hz, 2H), 3.23 (s, 2H), 1.78 (t, J = 6.3 Hz, 2H), 0.51 - 0.58 (m, 2H), 0.43 - 0.49 (m, 2H) |
| 129 | 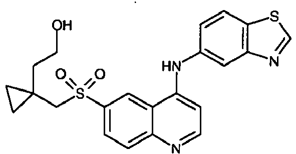 | 2-[1-({[4-(1,3-benzothiazol-5-ylamino)-6-quinolinyl]sulfonyl} methyl)cyclopropyl] ethanol | 440 | ¹H NMR (Methanol-d4) δ 9.33 - 9.38 (m, 1H), 9.08 (d, J = 2.0 Hz, 1H), 8.56 (d, J = 5.8 Hz, 1H), 8.19 - 8.25 (m, 1H), 8.15 - 8.18 (m, 1H), 8.06 - 8.15 (m, 2H), 7.62 (d, J = 8.3 Hz, 2H), 7.12 (d, J = 5.6 Hz, 1H), 3.77 (t, J = 6.6 Hz, 2H), 3.39 - 3.44 (m, 2H), -1.81 (t, J = 6.8 Hz, 2H), 1.30 - 1.36 . (m, 1H), 0.36 - 0.51 (m, 4H) |
| 130 | 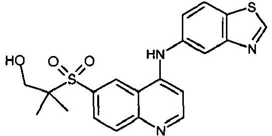 | 2-{[4-(1,3-benzothiazol-5-ylamino)-6-quinolinyl]sulfonyl}-2-methyl-1-propanol | 414 | ¹H NMR (DMSO-d6) δ 9.70 - 9.80 (m, 1H), 9.45 (s, 1H), 8.99 - 9.08 (m, 1H), 8.60 (d, J = 5.3 Hz, 1 H), 8.23 (d, J = 8.6 Hz, 1H), 8.10 (d, J = 2.0 Hz, 1H), 7.98 - 8.05 (m, 2H), 7.56 (dd, J = 10.9, 2.3 Hz, 1H), 7.05 (d, J = 5.3 Hz, 1H), 5.07 (t, J = 6.0 Hz, 1H), 3.60 (d, J = 6.0 Hz, 2H), 1.28 (s, 6H) |
| 131 | 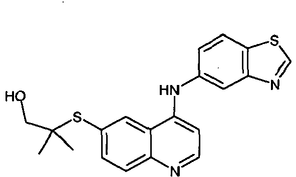 | 2-{[4-(1,3-benzothiazol-5-ylamino)-6-quinolinyl]thio}-2-methyl-1-propanol | 382 | ¹H NMR (Chloroform-d) δ 9.09 (s, 1H), 8.60 (d, J = 5.3 Hz, 1H), 8.20 - 8.25 (m, 1H), 8.09 (d, J = 2.0 Hz, 1H), 8.01 - 8.06 (m, 1H), 8.00 (d, J = 1.8 Hz, 1H), 7.78 (dd, J = 8.6, 1.8 Hz, 1H), 7.45 (dd, J = 10.4, 2.0 Hz, 1H), 7.04 (d, J = 5.3 Hz, 1H), 3.51 (s, 1H), 3.40 (s, 2H), 1.31 (s, 6H) |
| 132 | 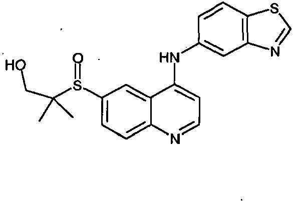 | 2-{[4-(1,3-benzothiazol-5-ylamino)-6-quinolinyl]sulfinyl}-2-methyl-1-propanol | 398 | ¹H NMR (Chloroform-d) δ 9.07 - 9.12 (m, 1H), 8.67 (d, J = 5.3 Hz, 1H), 8.48 - 8.55 (m, 1H), 8.12 - 8.19 (m, 2H), 8.03 (d, J = 8.6 Hz, 1H), 7.71 (dd, J = 8.6, 1.5 Hz, 1H), 7.48 (dd, J = 8.6, 2.0 Hz, 1H), 7.41 (br. s., 1H), 7.11 (d, J = 5.6 Hz, 1H), 3.75 - 3.99 (m, 2H), 1.38 (s, 6H) |
| 133 | 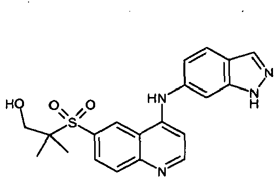 | 2-{[4-(1H-indazol-6-ylamino)-6-quinolinyl]sulfonyl}-2-methyl-1-propanol | 397 | ¹H NMR (Methanol-d4) δ 8.99 (d, J = 1.8 Hz, 1H), 8.53 (d, J = 5.6 Hz, 1H), 8.14 (dd, J = 8.8, 1.8 Hz, 1H), 8.02 - 8.11 (m, 2H), 7.88 (d, J = 8.6 Hz, 1H), 7.56 (br. s., 1H), 7.25 (dd, J = 8.6, 1.8 Hz, 1H), 7.08 (d, J = 5.6 Hz, 1H), 3.77 (s, 2H), 1.40 (s, 6H) |
| 134 | 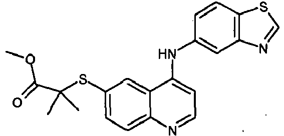 | methyl 2-{[4-(1,3-benzothiazol-5-ylamino)-6-quinolinyl]thio}-2-methylpropanoate | 410 | ¹H NMR (Chlorofonn-d) δ 9.08 (s, 1H), 8.59 (d, J = 5.3 Hz, 1H), 8.17 - 8.24 (m, 1H), 8.10 (d, J = 2.0 Hz, 1H), 8.02 - 8.07 (m, 1H), 7.99 (dd, J = 8.6, 1.8 Hz, 1H), 7.71 (dd, J = 8.8, 1.8 Hz, 1H), 7.45 (dd, J = 8.6, 2.3 Hz, 1H), 7.22 (br. s., 1H), 7.07 (d, J = 5.3 Hz, 1H), 3.68 (s, 3H), 1.55 (s, 6H) |
| 135 | 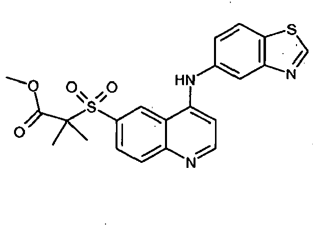 | methyl 2-{[4-(1,3-benzothiazol-5-ylamino)-6-quinolinyl]sulfonyl} -2-methylpropanoate | 442 | ¹H NMR (Chloroform-d) δ 9.12 (s, 1H), 8.73 (d, J = 5.6 Hz, 1H), 8.57 - 8.64 (m, 1H), 8.21 (d, J = 8.8 Hz, 1H), 8.14 - 8.17 (m, 1H), 7.99 - 8.10 (m, 2H), 7.47 (dd, J = 8.3, 2.0 Hz, 1H), 7.08 - 7.19 (m, 2H), 3.73 (s, 3H), 1.71 (s, 6H) |
| 136 | 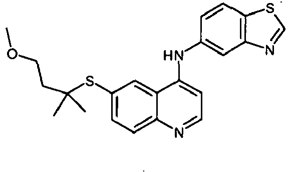 | N-1,3-benzothiazol-5-yl-6-{[1,1-dimethyl-3-(methyloxy)propyl] thio}-4-quinolinamine | 410 | ¹H NMR (Chloroform-d) δ 8.99 (s, 1H), 8.74 - 8.88 (m, 1H), 7.92 (d, J = 8.3 Hz, 1H), 7.76 (dd, J = 8.6, 2.3 Hz, 1H), 7.73 (d, J = 1.8 Hz, 1H), 7.23 (d, J = 8.8 Hz, 1H), 7.15 (d, J = 8.3 Hz, 1H), 6.95 (d, J = 8.1 Hz, 1H), 6.04 (d, J = 8.1 Hz, 1H), 3.98 (t, J = 7.1 Hz, 2H), 3.65 (s, 3H), 1.82 (t, J = 6.8 Hz, 2H), 1.33 (s, 6H) |
| 137 | 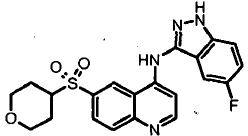 | N-(5-fluoro-1H-indazol-3-yl)-6-(tetrahydro-2H-pyran-4-ylsulfonyl)-4-quinolinamine | 427 | ¹H NMR (DMSO-d6) δ: 12.95 - 13.03 (m, 1H), 9.22 (s, 1H), 8.64 (br. s., 1H), 8.03 - 8.15 (m, 2H), 7.54 - 7.66 (m, 2H), 7.29 - 7.38 (m, 2H), 3.93 (dd, J = 11.2, 3.7 Hz, 2H), 3.64 (qd, J = 8.0, 3.7 Hz, 1H), 3.21 - 3.43 (m, 2H), 1.80 (d, J = 11.0 Hz, 2H), 1.65 (qd, J = 12.3, 4.6 Hz, 2H) |
| 138 | 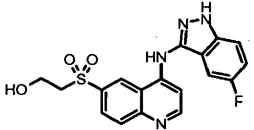 | 2-({4-[(5-fluoro-1H-indazol-3-yl)amino]-6-quinolinyl} sulfonyl) ethanol | 387 | ¹H NMR (DMSO-d6) δ: 12.94 (s, 1H), 9.83 (s, 1H), 9.23 (s, 1H), 8.64 (d, J = 5.1 Hz, 1H), 8.03 -8.14(m,2H),7.55-7.65 (m, 2H), 7.40 (d, J = 5.1 Hz, 1H), 7.32 (td, J = 9.0, 2.0 Hz, 1H), 4.90 (t, J = 5.4 Hz, 1H), 3.77 (q, J = 5.9 Hz, 2H), 3.60 (t, 2H) |
| 139 | 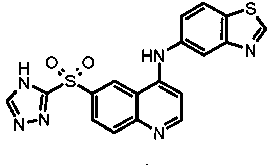 | N-1,3-benzothiazol-S-yl-6-(1H-1,2,4-triazol-3-ylsulfonyl)-4-quinolinamine | 409 | ¹H NMR (DMSO-d6) δ: 9.75 (s, 1H), 9.44 (s, 1H), 9.08 (s, 1H), 8.55 (d, J = 5.3 Hz, 1H), 8.21 (d, J = 8.6 Hz, 1H), 8.03 - 8.07 (m, 2H), 7.98 (s, 1H), 7.73 (s, 1H), 7.56 (d, J = 8.6 Hz, 1H), 7.05 (d, 1H) |
| 140 | 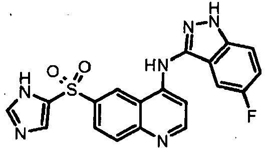 | N-(5-fluoro-1H-indazol-3-yl)-6-(1H-imidazol-4-ylsulfonyl)-4-quinolinamine | 409 | ¹H NMR (DMSO-d6) δ: 12.96 (s, 1H), 10.01 (s, 1H), 9.37 (s, 1H), 8.64 (d, J = 5.5 Hz, 1H), 8.07 (s, 2H), 7.55 - 7.69 (m, 2H), 7.42 (d, J = 5.5 Hz, 1H), 7.27 - 7.39 (m, 3H) |
| 141 | 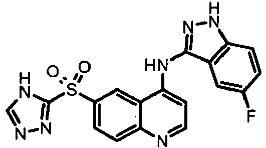 | N-(5-fluoro-1H-indazol-3-yl)-6-(1H-1,2,4-triazol-3-ylsulfonyl)-4-quinolinamine | 410 | ¹H NMR (DMSO-d6) δ: 12.93 (s, 1H), 9.98 (s, 1H), 9.36 (s, 1H), 8.63 (d, J = 5.8 Hz, 1H), 8.06 (s, 3H), 7.54 - 7.67 (m, 3H), 7.41 (d, J = 5.3 Hz, 1H), 7.32 (s, 1H) |
| 142 | 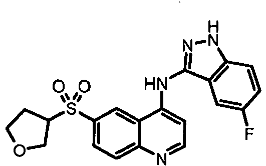 | N-(5-fluoro-1H-indazol-3-yl)-6-(tetrahydro-3-furanylsulfonyl)-4-quinolinamine* | 413 | ¹H NMR (400 MHz, DMSO-d6) δ ppm 12.98 (s, 1 H), 9.91 (s, 1 H), 9.27 (s, 1 H), 8.66 (d, J=5.3 Hz, 1 H), 8.11 (s, 2 H), 7.54 - 7.65 (m, 2 H), 7.26 - 7.42 (m, 2 H), 4.31 (dt, J=8.3, 4.4 Hz, 1 H), 4.16 (dd, J=10.1, 4.5 Hz, 1 H), 3.88 (dd, J=10.1, 8.1 Hz, 1 H), 3.76 - 3.85 (m, 1 H), 3.61 - 3.73 (m, 1 H), 2.28 (dt, J=12.8, 6.3 Hz, 1 H), 2.19 (dt, 1 H) |
| 143 | 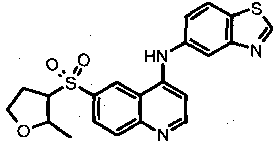 | N-1,3-benzothiazol-5-yl-6-[(2-methyltetrahydro-3-furanyl)sulfonyl]-4-quinolinamine (diastereomeric mixture) | 426 | ¹H NMR (CHLOROFORM-d) δ: 9.12 (s, 1H), 8.68 - 8.76 (m, 2H), 8.21 (d, J = 8.7 Hz, 1H), 8.08 - 8.17 (m, 2H), 8.06 (d, J = 8.3 Hz, 1H), 7.55 - 7.76 (m, 1H), 7.47 (dt, J = 8.7, 2.0 Hz, 1H), 7.12 (d, J = 5.3 Hz, 1H), 4.37 (m,, 1H), 3.86 (q, J = 8.1 Hz, 1H), 3.71 (m, 2H), 2.40 - 2.54 (m, 2H), 1.69 (d, J = 6.6 Hz, 3H) |
| 144 | 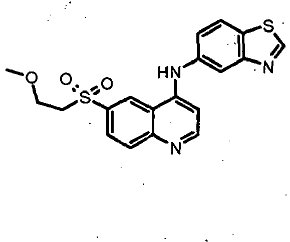 | N-1,3-benzothiazol-5-yl-6-{[2-(methyloxy)ethyl] sulfonyl}-4-quinolinamine | 400 | ¹H NMR (CHLOROFORM-d) δ: 9.12 (s, 1H), 8.76 - 8.85 (m, 1H), 8.67 (d, J = 5.6 Hz, 1H), 8.24 (d, J = 8.8 Hz, 1H), 8.15 (d, J = 2.0 Hz, 1H), 8.12 (dd, J = 8.8, 1.8 Hz, 1H), 8.06 (d, J = 8.4 Hz, 1H), 7.48 (dd, J = 8.4, 2.0 Hz, 1H), 7.10 (d, J = 5.6 Hz, 1H), 3.83 (t, J = 6.2 Hz, 2H), 3.53 (t, J = 6.2 Hz, 2H), 3.25 (s, 3H) |
| 145 | 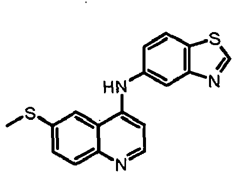 | N-1,3-benzothiazol-5-yl-6-(methylthio)-4-quinolinamine | 324 | ¹H NMR (DMSO-d6) δ: 11.02 (br. s., 1H), 9.53 (s, 1H), 8.44 - 8.53 (m, 2H), 8.39 (d, J = 8.3 Hz, 1H), 8.18 - 8.27 (m, 1H), 7.94 - 7.99 (m, 2H), 7.62 (d, J = 8.6 Hz, 1H), 6.85 (d, J = 6.8 Hz, 1H), 2.71 (s, 3H) |
| 146 | 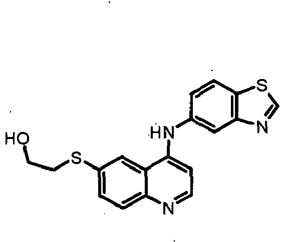 | 2-{[4-(1,3-benzothiazol-5-ylamino)-6-quinolinyl]thio}ethanol | 354 | ¹H NMR (DMSO-d6) δ: 9.43 (s, 1H), 9.16 (s, 1H), 8.42 (d, J = 5.3 Hz, 1H), 8.33 (s, 1H), 8.20 (d, J = 8.6 Hz, 1H), 7.99 - 8.05 (m, 1H), 7.81 (d, J = 8.7 Hz, 1H), 7.67 (dd, J = 10.6, 1.8 Hz, 1H), 7.53 (dd, J = 8.7, 1.8 Hz, 1H), 6.98 (d, J = 5.3 Hz, 1H), 4.97 (t, J = 5.6 Hz, 1H), 3,65 (td, J = 6.6, 5.8 Hz, 2H), 3.24 (t, J = 6.6 Hz, 2H) |
| 147 | 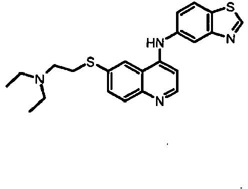 | N-1,3-benzothiazol-5-yl-6-{[2-(diethylamino)ethyl] thio}-4-quinolinamine | 409 | ¹H NMR (CHLOROFORM-d) δ: 9.09 (s, 1H), 8.55 (d, J = 5.3 Hz, 1 H), 8.10 (d, J = 2.0 Hz, 1H), 7.98 - 8.04 (m, 2H), 7.93 - 7.98 (m, 1H), 7.67 (dd, J = 8.8, 2.0 Hz, 1H), 7.46 (dd, J = 8.8, 2.0 Hz, 1H), 7.17 (br. s., 1H), 7.05 (d, J = 5.3 Hz, 1H), 3.10 - 3.25 (m, 2H), 2.75 - 2.84 (m, 2H), 2.62 (q, J = 7.1 Hz, 4H), 1.05 (t, J = 7.1 Hz, 6H) |
| 148 | 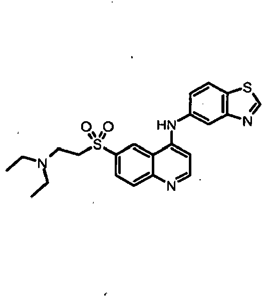 | N-1,3-benzothiazol-5-yl-6-{[2-(diethylamino)ethyl] sulfonyl}-4-quinolinamine | 441 | ¹H NMR (CHLOROFORM-d) δ: 9.12 (s, 1H), 8.58 - 8.77 (m, 2H), 8.19 - 8.25 (m, 1H), 8.15 - 8.17 (m, 1H), 8.12 (dd, J = 8.8, 1.8 Hz, 1H), 8.04 (d, J = 8.6 Hz, 1H), 7.48 (dd, J = 8.3, 2.0 Hz, 1H), 7.20 - 7.26 (m, 1H), 7.12 (d, J = 5.6 Hz, 1H), 3.40 (t, J = 7.3 Hz, 2H), 3.01 (t, J = 7.3 Hz, 2H), 2.48 (q, J = 7.3 Hz, 4H), 0.97 (t, J = 7.3 Hz, 6H) |
| 149 | 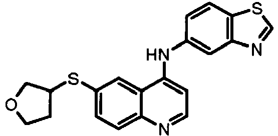 | N-1,3-benzothiazol-5-yl-6-(tetrahydro-3-furanylthio)-4-quinolinamine (racemic) | 380 | ¹H NMR (CHLOROFORM-d) δ: 9.09 (s, 1H), 8.54 (d, J = 5.4 Hz, 1H), 8.09 (s, 1H), 8.06 (d, J = 2.0 Hz, 1H), 8.01 - 8.04 (m, 1H), 7.99 - 8.01 (m, 1H), 7.71 (dd, J = 8.8, 2.0 Hz, 1H), 7.45 (dd, J = 8.6, 2.0 Hz, 1H), 7.03 (d, J = 5.4 Hz, 1H), 4.08 - 4.17 (m, 1H), 3.84 - 4.08 (m, 3H), 3.69 - 3.81 (m, 1H), 2.44 (br. s, 1H) 2.27 - 2.42 (m, 1H), 1.92 - 2.05 (m, 1H) |
| 150 | 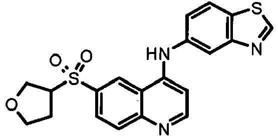 | N-1,3-benzothiazol-5-yl-6-(tetrahydro-3-furanylsulfonyl)-4-quinolinamine (racemic) | 412 | ¹H NMR (CHLOROFORM-d) δ: 9.11 (s, 1H), 8.73 (d, J = 5.3 Hz, 1H), 8.70 (d, J = 1.9 Hz, 1H), 8.24 (d, J = 8.7 Hz, 1H), 8.16 (d, J = 1.9 Hz, 1H), 8.12 (d, J = 1.9 Hz, 1H), 8.06 (d, J = 8.7 Hz, 1H), 7.47 (dd, J = 8.7, 1.9 Hz, 1H), 7.19 (s, 1H), 7.12 (d, J = 5.3 Hz, 1H), 4.29 (br. s., 1H), 3.91 - 4.06 (m, 3H), 3.79 - 3.90 (m, 1H), 2.43 - 2.55 (m, 1H), 2.18 - 2.32 (m, 1H) |
| 151 | 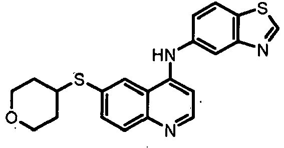 | N-1,3-benzothiazol-5-yl-6-(tetrahydro-2H-pyran4-ylthio)4-quinolinamine | 394 | ¹H NMR (CHLOROFORM-d) δ: 9.09 (s, 1H), 8.58 (d, J = 5.3 Hz, 1H), 8.06 - 8.17 (m, 2H), 7.93 - 8.06 (m, 2H), 7.76 (dd, J = 8.6, 1.9 Hz, 1H), 7.45 (dd, J = 8.6, 1.9 Hz, 1H), 7.06 (d, J = 5.3 Hz, 1H), 6.98 (br. s., 1H), 4.00 (dt, J = 11.6, 3.5 Hz, 2H), 3.44 (m, 3H), 1.95 (d, J = 13.1 Hz, 2H), 1.71-1.81 (m, 2H) |
| 152 | 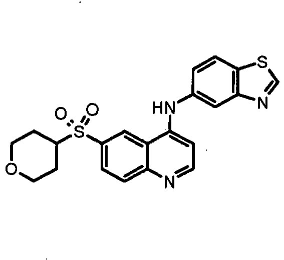 | N-1,3-benzothiazol-5-yl-6-(tetrahydro-2H-pyran-4-ylsulfonyl)-4-quinolinamine | 426 | ¹H NMR (CHLOROFORM-d) δ: 9.11 (s, 1H), 8.63 - 8.76 (m, 2H), 8.22 (d, J = 8.8 Hz, 1H), 8.15 (d, J = 2:0 Hz, 1H), 8.02 - 8.09 (m, 2H), 7.42 - 7.52 (m, 2H), 7.12 (d, J = 5.3 Hz, 1H), 4.06 (dd, J = 14.7, 3.0 Hz, 2H), 3.92 - 4.02 (m, 2H), 3.67 - 3.68 (m, 1H), 3.33 (td, J = 11.6, 2.5Hz, 2H), 1.79-1.95 (m, 2H) |
| 153 | 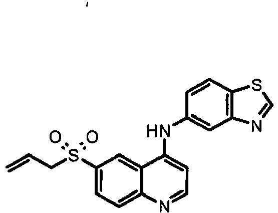 | N-1,3-benzothiazol-5-yl-6-(2-propen-1-ylsulfonyl)-4-quinolinamine | 382 | ¹H NMR (METHANOL-d4) δ: 9.33 (s, 1H), 9.02 (d, J = 1.8 Hz, 1H), 8.53 (d, J = 5.6 Hz, 1H), 8.03 - 8.22 (m, 4H), 7.60 (dd, J = 8.6, 1.8 Hz, 1H), 7.10 (d, J = 5.6 Hz, 1H), 5.75 - 5.97 (m, 1H), 5.35 (d, J = 10,1 Hz, 1H), 5.22 (d, J= 16.9 Hz, 1H), 4.12 (d,J=7.3 Hz, 2H) |
| 154 | 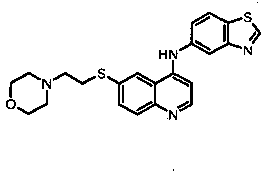 | N-1,3-benzothiazol-5-yl-6-{[2-(4-morpholinyl) ethyl]thio}-4-quinolinamine | 423 | ¹H NMR (CHLOROFORM-d) δ: 9.09 (s, 1H), 8.55 (d, J = 5.3 Hz, 1H), 8.09 (d, J = 1.9 Hz, 1H), 8.02 (d, J = 3.3 Hz, 1H), 8.00 (d, J = 3.3 Hz, 1H), 7.96 (d, J = 1.9 Hz, 1H), 7.70 (dd, J = 8.7, 2.0 Hz, 1H), 7.46 (dd, J = 8.7, 2.0 Hz, 1H), 7.06 (d, J = 5.3 Hz, 1H), 6.92 (s, 1H), 3.64 - 3.79 (m, 4H), 2.81 - 2.93 (m, 2H), 2.64 - 2.75 (m, 2H), 2.44 - 2.57 (m, 4H) |
| 155 | 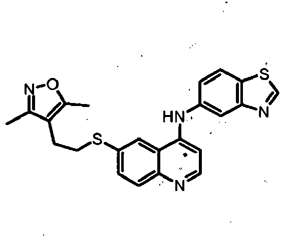 | N-1,3-benzothiazol-5-yl-6-{[2-(3,5-dimethyl-4-isoxazolyl) ethyl]thio}-4-quinolinamine | 433 | ¹H NMR (CHLOROFORM-d) δ: 9.07 (s, 1H), 8.55 (d, J = 5.3 Hz, 1H), 8.11 (d, J = 2.0 Hz, 1H), 8.01 (d, J = 2.0 Hz, 1H), 7.99 (d, J = 2.3 Hz, 1H), 7.80 (d, J = 1.8 Hz, 1H), 7.68 (dd, J = 8.7, 1.8 Hz, 1H), 7.48 (dd, J = 8.7, 2.3 Hz, 1H), 7.12 (br. s., 1H), 7.07 (d, J = 5.3 Hz, 1 H), 3.12 (t, J = 7.3 Hz, 2H), 2.69 (t, J = 7.3 Hz, 2H), 2.25 (s, 3H), 2.21 (s, 3H) |
| 156 | 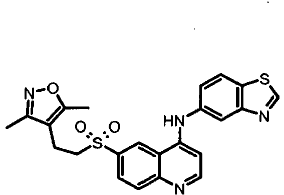 | N-1,3-benzothiazol-5-yl-6-{[2-(3,5-dimethyl-4-isoxazolyl)ethyl] sulfonyl}-4-quinolinamine | 465 | ¹H NMR (CHLOROFORM-d) δ: 9.09 (s, 1H), 8.70 (d, J = 5.3 Hz, 1H), 8.64 (d, J = 1.8 Hz, 1H), 8.20 (d, J = 8.7 Hz, 1H), 8.15 (d, J = 2.0 Hz, 1H), 8.08 (dd, J = 8.7, 2.0 Hz, 1H), 8.03 (d, J = 8.7 Hz, 1H), 7.67 (br. s., 1H), 7.52 (dd, J = 8.6, 2.3 Hz, 1H), 7.13 (d, J = 5.3 Hz, 1H), 3.32 (t, J = 7.6 Hz, 2H), 2.83 (t, J = 7.6 Hz, 2H), 2.20 (s, 3H), 2.14 (s, 3H) |
| 157 | 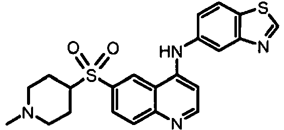 | N-(6-((1-methylpiperidin-4-yl)sulfonyl)quinolin-4-yl)benzo[d]thiazol-5-amine | 439 | ¹H NMR (METHANOL-d4) δ: 9.44 (s, 1H), 9.27 - 9.34 (m, 1H), 8.53 (d, J = 7.1 Hz, 2H), 8.44 (d, J = 10.1 Hz, 1H), 8.35 (d, J = 8.6 Hz, 1H), 8.24 (d, J = 1.8 Hz, 1H), 8.17 (d, J = 8.8 Hz, 1H), 7.64 (dd, J = 8.6, 2.0 Hz, 1H), 7.08 (d, J = 7.3 Hz, 1H), 3.60 - 3.74 (m, 3H), 2.97 - 3.11 (m, 2H), 2.88 (s, 3H), 2.28 - 2.41 (m, 2H), 2.01 - 2.15 (m, 2H) |
| 158 | 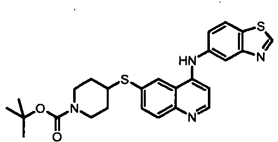 | 1,1-dimethylethyl 4-{[4-(1,3-benzothiazol-5-ylamino)-6-quinolinyl]thio}-1-piperidinecarboxylate | 493 | ¹H NMR (CHLOROFORM-d) δ: 9.08 (s, 1H), 8.58 (d, J = 5.1 Hz, 1H), 8.08 - 8.14 (m, 2H), 7.98 - 8.05 (m, 2H), 7.77 (dd, J = 8.6, 1.8 Hz, 1H), 7.46 (dd, J = 10.6, 2.0 Hz, 1H), 7.06 (d, J = 5.3 Hz, 1H), 6.85 (br. s., 1H), 3.90 - 4.09 (m, 2H), 3.49 - 3.60 (m, 2H), 3.24 - 3.39 (m, 1H), 2.94 - 3.00 (m, 2H), 1.92 - 2.02 (m, 2H), 1.39 - 1.51 (m, 9H) |
| 159 | 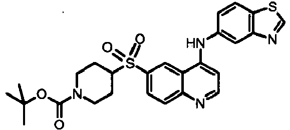 | 1,1-dimethylethyl 4-{[4-(1,3-benzothiazol-5-ylamino)-6-quinolinyl]sulfonyl}-1-piperidinecarboxylate | 525 | ¹H NMR (CHLOROFORM-d) δ: 9.12 (s, 1H), 8.67 - 8.76 (m, 1H), 8.60 - 8.66 (m, 1H), 8.22 (d, J = 8.8 Hz, 1H), 8.13 - 8.16 (m, 1H), 8.02 - 8.08 (m, 2H), 7.46 (dd, J = 8.6, 2.0 Hz, 1H), 7.12 (d, J = 5.3 Hz, 2H), 4.18 - 4.31 (m, 2H), 3.47 - 3.56 (m, 1H), 2.59 - 2.76 (m,2H), 1.98-2.04 (m, 1H), 1.57 - 1.74 (m, 3H), 1.39 - 1.47 (m, 9H) |
| 160 | 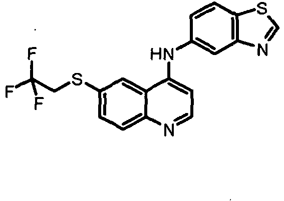 | N-1,3-benzothiazol-5-yl-6-[(2,2,2-trifluoroethyl)thio]-4-quinolinamine | 392 | ¹H NMR (CHLOROFORM-d) δ: 9.09 (s, 1H), 8.59 (d, J = 5.3 Hz, 1H), 8.21 (d, J = 1.8 Hz, 1H), 8.09 (d, J = 2.0 Hz, 1H), 7.98 - 8.07 (m, 2H), 7.79 (d, J = 2.0 Hz, 1H), 7.45 (dd, J = 8.6, 2.0 Hz, 1H), 7.05 (d, J = 5.3 Hz, 1H), 3.55 (q, J = 9.6 Hz, 2H), 1.87 (s, 1H) |
| 161 | 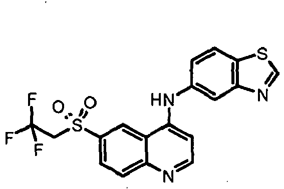 | N-1,3-benzothiazol-5-yl-6-[(2,2,2-trifluoroethyl)sulfonyl]-4-quinolinamine | 424 | ¹H NMR (CHLOROFORM-d) δ: 9.12 (s, 1H), 8.78 (d, J = 1.8 Hz, 1H), 8. 70 (d, J = 5.3 Hz, 1H), 8.25 (d, J = 9.1 Hz, 1H), 8.14 - 8.19 (m, 2H), 8.06 (d, J = 8.6 Hz, 1H), 8.03 - 8.08 (m, 1H), 7.48 (dd, J = 8.3, 2.0 Hz, 1H), 7.13 (d, J = 5.3 Hz, 1H), 4.06 (q, J = 9.1 Hz, 2H) |
| 162 | 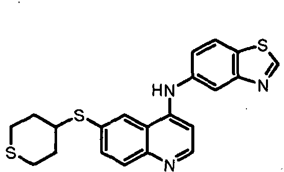 | N-1,3-benzothiazol-5-yl-6-(tetrahydro-2H-thiopyran-4-ylthio)-4-quinolinamine | 410 | ¹H NMR (CHLOROFORM-d) δ: 9.09 (s, 1H), 8.56 (d, J = 5.3 Hz, 1H), 8.06 - 8.16 (m, 2H), 7.95 - 8.05 (m, 2H), 7.74 (dd, J = 8.8, 1.8 Hz, 1H), 7.45 (dd, J = 8.6, 2.3 Hz, 1H), 7.14 - 7.22 (m, 1H), 7.05 (d, J = 5.3 Hz, 1H), 2.71 - 2.82 (m, 2H), 2.58 - 2.69 (m, 2H), 2.25 - 2.35 (m, 2H), 1.72 - 1.93 (m, 3H) |
| 163 | 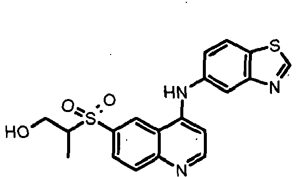 | 2-{[4-(1,3-benzothiazol-5-ylamino)-6-quinolinyl]sulfinyl} ethanol | 400 | ¹H NMR (CHLOROFORM-d) δ: 9.12 (s, 1H), 8.73 (d, J = 5.4 Hz, 1H), 8.64 - 8.70 (m, 1H), 8.24 (d, J = 8.8 Hz, 1H), 8.14 - 8.17 (m, 1H), 8.11 (dd, J = 8.8, 1.8 Hz, 1H), 8.05 (d, J = 8.6 Hz, 1H), 7.47 (dd, J = 8.6, 2.0 Hz, 1H), 7.21 (s, 1H), 7.12 (d, J = 5.4 Hz, 1H), 4.14 (q, J = 7.1 Hz, 1H), 3.94 (dd, J = 12.6, 3.5 Hz, 2H), 3.40 - 3.51 (m, 1H), 1.36 (d, J = 7.1 Hz, 3H) |
| 164 | 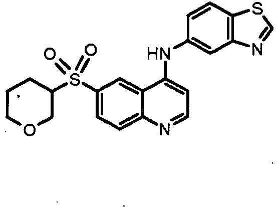 | N-(6-((tetrahydro-2H-pyran-3-yl)sulfonyl)quinolin-4-yl)benzo[d]thiazol-5-amine (each enantiomer (R) and (*S*) isolated) | 426 | ¹H NMR (CHLOROFORM-d) δ: 9.10 (s, 1H), 8.58 (d, J=4.8 Hz, 1H), 8.26 (d,J=8.8 Hz, 1H), 8.11-8.18 (m, 1H), 8.00 - 8.08 (m, 3H), 7.50 (d, J = 8.1 Hz, 1H), 7.04 (d, J = 5.3 Hz, 2H), 4.16 (d, J= 10.6 Hz, 1H), 3.85 (d, J = 11.1Hz, 1H), 3.53-3.63 (m, J=10.6, 10.6 Hz, 1H), 3.22 - 3.44 (m, 2H), 2.09-2.20 (m, 1H), 1.52-1.94 (m, 3H) |
| 165 | 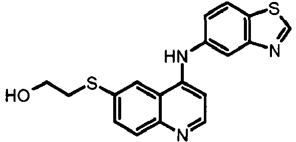 | 2-{[4-(1,3-benzothiazol-5-ylamino)-6-quinolinyl]thio} ethanol | 354 | ¹H NMR (DMSO-d6) δd: 9.43 (s, 1H), 9.16 (s, 1H), 8.42 (d, J = 5.3 Hz, 1H), 8.29 - 8.36 (m, 1H), 8.17 (d, J = 8.7 Hz, 1H), 7.99 - 8.05 (m, 1H), 7.81 (d, J = 8.7 Hz, 1H), 7.67 (d, J = 10.5 Hz, 1H), 7.53 (d, J = 10.5 Hz, 1H), 6.97 (d, J = 5.3 Hz, 1H), 5.00 (t, J = 5.6 Hz, 1H), 3.65 (m, 2H), 3.24 (t, J = 6.6 Hz, 2H) |
| 166 | 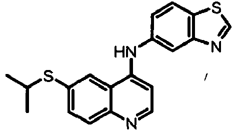 | *N*-1,3-benzothiazol-5-yl-6-[(1-methylethyl)thio]-4-quinolinamine | 352 | NA |

| | | | | |
|---|---|---|---|---|
| *The thiol coupling to N-(5-fluoro-1*H*-indazol-3-yl)-6-iodo-4-quinolinamine was done using conditions described in preparation 2. | | | | |

### Example 167

### 2-{[4-(1,3-benzothiazol-5-ylamino)-6-quinolinyl]thio}-2-methylpropanoic acid

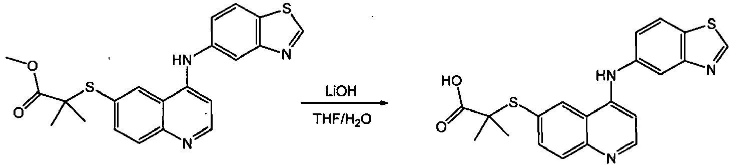

To a solution of methyl 2-{[4-(1,3-benzothiazol-5-ylamino)-6-quinolinyl]thio}-2-methylpropanoate (20 mg, 0.049 mmol) in 0.5 mL THF was added LiOH (2.34 mg, 0.098 mmol) in 0.2 mL water. After stirring for 1h at 65°C, the organic layer was concentrated to dryness to give 2-{[4-(1,3-benzothiazol-5-ylamino)-6-quinolinyl]thio}-2-methylpropanoic acid as a brown solid (19 mg, 98% yield). ¹H NMR (DMSO-*d₆*) δ: 9.28 - 9.40 (m, 1H), 9.15 - 9.27 (m, 1H), 8.51 - 8.55 (m, 1H), 8.42 - 8.51 (m, 2H), 7.71 - 7.77 (m, 1H), 7.60 - 7.69 (m, 2H), 7.55 - 7.60 (m, 1H), 7.50 (m, 1H), 1.29 (s, 6H). MS (m/z) 396.2 (M+H⁺).

### Example 168

### 2-{[4-(1,3-benzothiazol-5-ylamino)-6-quinolinyl]sulfonyl}-2-methylpropanoic acid

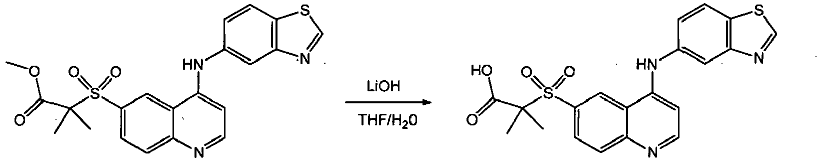

A mixture of methyl 2-{[4-(1,3-benzothiazol-5-ylamino)-6-quinolinyl]sulfonyl}-2-methylpropanoate (274 mg, 0.62 mmol) and LiOH (44.3 mg, 1.86 mmol) was heated with 6 mL THF and 1 mL water at 50°C for 5h. Water, MeOH and DMF were added (1mL each), and some inorganic solid was filtered out. The resulting filtrate was purified by reverse phase HPLC (8 to 60% 0.1%TFA/MeCN in water, 30 x 150 mm Sunfire 5 µm OBD C₁₈ column). The pure fractions were combined, and evaporated *in vacuo* to provide 2-{[4-(l,3-benzothiazol-5-ylamino)-6-quinolinyl]sulfonyl}-2-methylpropanoic acid (265 mg, 100% yield). ¹H NMR (METHANOL-*d4*) δ: 9.40 - 9.47 (m, 1H), 9.28 (d, *J=* 1.8 Hz, 1H), 8.50 (d, *J* = 7.1 Hz, 1H), 8.43 (dd, *J* = 8.8, 1.8 Hz, 1H), 8.34 (d, *J* = 8.3 Hz, 1H), 8.24 (d, *J=* 1.8 Hz, 1H), 8.10 (d, *J* = 8.8 Hz, 1H), 7.68 (dd, *J* = 10.6,2.0 Hz, 1H), 7.04 (d, *J* = 7.1 Hz, 1H), 1.71 (s, 6H). MS (m/z) 328.3 (M+H⁺).

### Example 169

### N-1,3-benzothiazol-5-yl-6-[(1,1-dioxidotetrahydro-2H-thiopyran-4-yl)sulfonyl]-4-quinolinamine

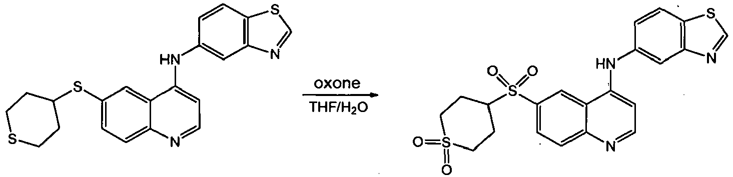

A mixture of N-1,3-benzothiazol-5-yl-6-(tetrahydro-2H-thiopyran-4-ylthio)-4-quinolinamine (21.5 mg, 0.052 mmol) and oxone (97 mg, 0.16 mmol) was stirred in 4.5 mL THF and 0.75 mL water for 4d. The reaction was concentrated to dryness, and the residue was dissolved in 4mL MeCN. The residue was purified via Gilson reverse phase HPLC (6% to 60% 0.1% TFA in MeCN in 0.1% TFA in water; 5µm 30x150 mm Waters Sunfire column). The pure fractions were collected and concentrated to dryness to provide N-1,3-benzothiazol-5-yl-6-[(1,1-dioxidotetrahydro-2H-thiopyran-4-yl)sulfonyl]-4-quinolinamine (9 mg, 36.2% yield). *¹*H NMR (DMSO-*d6*) δ: 9.79 (s, 1H), 9.33 - 9.57 (m, 1H), 8.95 - 9.21 (m, 1H), 8.54 - 8.71 (m, 1H), 8.28 (d, *J=* 8.6 Hz, 1H), 7.96 - 8.15 (m, 2H), 7.57 (d, *J* = 8.6 Hz, 1H), 7.14 (d, *J* = 4.5 Hz, 1H), 3.28 - 3.40 (m, 1H), 2.29 - 2.42 (m, 1H), 1.92 - 2.14 (m, 4H), 1.04 - 1.34 (m, 4H). MS (m/z) 474.1 (M+H⁺).

### Example 170

### N-1,3-benzothiazol-5-yl-6-(4-piperidinylsulfonyl)-4-quinolinamine

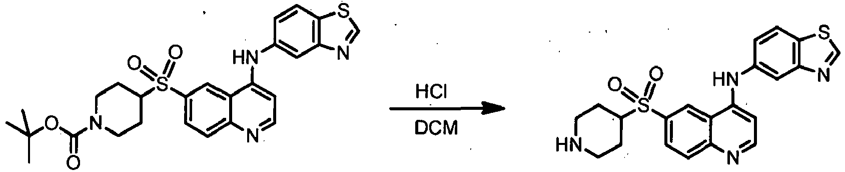

To a mixture of 1,1-dimethylethyl 4-{[4-(1,3-benzothiazol-5-ylamino)-6-quinolinyl]sulfonyl}-1-piperidinecarboxylate (105 mg, 0.200 mmol) in DCM was added HCl (0.15 mL, 4N in dioxane), and allowed the mixture to stir 1h. The reaction mixture was basified with sat'd sodium bicarbonate and concentrated to dryness. The resulting mixture was dissolved in 1mL MeOH/MeCN and purified via Gilson reverse phase HPLC (6% to 60% 0.1% TFA in MeCN in 0.1% TFA in water; 5µm 30x150 µm Waters Sunfire column). The pooled fractions were partitioned between EtOAc and sat'd sodium bicarbonate, the aqueous layer back extracted with EtOAc, and the combined organic layers washed with brine, then dried over MgSO₄. After evaporation *in vacuo,* N-1,3-benzothiazol-5-yl-6-(4-piperidinylsulfonyl)-4-quinolinamine (19.6 mg, 23% yield) was isolated as a white solid. ¹H NMR (CHLOROFORM-*d*) δ: 9.09 (s, 1H), 8.67 - 8.72 (m, 1H), 8.20 (d, *J* = 8.8 Hz, 1H), 8.14 (d, *J* = 1.8 Hz, 1H), 8.02 - 8.09 (m, 2H), 7.51 (dd, *J* = 8.6, 2.3 Hz, 1H), 7.43 (br. s., 1H), 7.11 (d, *J=* 5.3 Hz, 1H), 3.10 - 3.22 (m, 3H), 2.86 - 2.98 (m, 1H), 2.45 - 2.63 (m, 2H), 2.19 - 2.28 (m, 1H), 1.97 - 2.05 (m, 2H). MS (m/z) 425.1 (M+H⁺).

### Example 171

### 2-{[4-(1,3-benzothiazol-5-ylamino)-6-quinolinyl]sulfinyl}ethanol

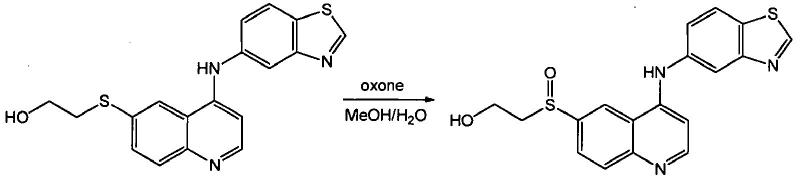

A solution of 2-{[4-(1,3-benzothiazol-5-ylamino)-6-quinolinyl]thio}ethanol (20 mg, 0.057 mmol) in 5.1 mL of MeOH was stirred before oxone (17.4 mg, 0.028 mmol) in 0.51mL water was added. The reaction mixture was stirred for 30 minutes before pouring into sat'd sodium bicarbonate and EtOAc. The aqueous layer was back extracted, the organic layers combined, washed with brine and dried over MgSO₄. After concentration *in vacuo*, the product was crystallized from 1:1 MeOH:MeCN, and filtered to give 2-{[4-(1,3-benzothiazol-5-ylamino)-6-quinolinyl]sulfinyl}ethanol (13.5 mg, 64.6% yield) as an orange solid. ¹H NMR (CHLOROFORM-*d*) δ: 9.11 (s, 1H), 8.67 (d, *J* = 5.3 Hz, 1H), 8.58 (d, *J* = 1.5 Hz, 1H), 8.13 - 8.18 (m, 2H), 8.02 (d, *J* = 8.6 Hz, 1H), 7.63 (dd, *J* = 8.8, 2.0 Hz, 1H), 7.49 (dd, *J* = 8.6, 2.0 Hz, 1H), 7.42 (br. s., 1H), 7.12 (d, *J* = 5.3 Hz, 1H), 4.16 - 4.31 (m, 1H), 3.44 (m, 2H), 3.22 (m, 2H). MS (m/z) 370.1 (M+H⁺).

The following compounds were prepared using procedures analogous to those described above. THF or EtOAc may be used instead of MeOH as a cosolvent.

| Ex # | Structure | Name | MS (M+H)⁺ | NMR |
|---|---|---|---|---|
| 172 | 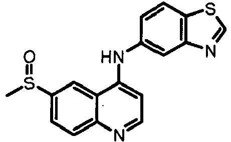 | N-1,3-benzothiazol-5-yl-6-(methylsulfinyl)-4-quinolinamine (each enantiomer isolated) | 340 | ¹H NMR (CHLOROFORM-d) δ: 9.10 (s, 1 H), 8.65 (d, J = 5.3 Hz, 1 H), 8.63 (d, J = 1.8 Hz, 1 H), 8.16 (s, 1 H), 8.16 (m, 1 H), 8.04 (d, J = 8.6 Hz, 1 H), 7.64 (dd, J = 8.6, 1.8 Hz, 1 H), 7.51 (br. s., 1 H), 7.47 (dd, J = 8.6, 2.0 Hz, 1 H), 7.12 (d, J = 5.3 Hz, 1 H), 3.51 (s, 3 H) |
| 173 | 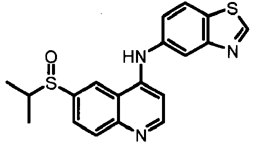 | N-1,3-benzothiazol-5-yl-6-[(1-methylethyl)sulfinyl]-4-quinolinamine | 368 | ¹H NMR (CHLOROFORM-d) δ: 9.10 (s, 1H), 8.64 (d, J = 5.3 Hz, 1H), 8.50 - 8.55 (m, 1H), 8.14 - 8.18 (m, 1H), 8.03 (d, J = 8.7 Hz, 2H), 7.64 (dd, J = 8.7, 1.8 Hz, 1H), 7.56 (s, 1H), 7.47 (dd, J = 8.3, 2.0 Hz, 1H), 7.11 (d, J = 5.3 Hz, 1H), 2.60 (spt, J = 7.1 Hz, 1H), 1.36 (d, J = 7.1 Hz, 6H) |

### Example 174

### 4-[(5-Hydroxy-2-methylphenyl)amino]-N-(phenylmethyl)-6-quinolinecarboxamide

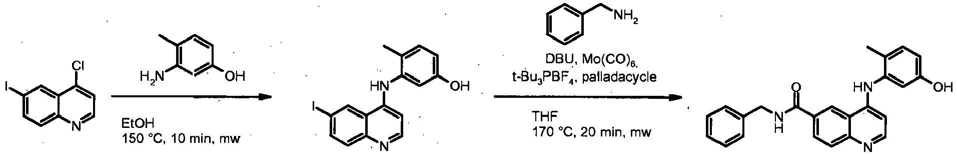

Step 1. 3-[(6-Iodo-4-quinolinyl)amino]-4-methylphenol: 4-Chloro-6-iodoquinoline (1.3 g, 4.5 mmol) and 3-amino-4-methylphenol (0.55 g, 4.5 mmol) in ethanol (6.9 mL) were sealed in a microwave vial and heated to 150 °C for 10 minutes. After cooling to room temperature, the solid precipitate was collected by filtration and washed with diethyl ether to give 3-[(6-iodo-4-quinolinyl)amino]-4-methylphenol. MS (m/z) 377.0 (M+H⁺).

Step 2. 4-[(5-Hydroxy-2-methylphenyl)amino]-*N*-(phenylmethyl)-6-quinolinecarboxamide: To a microwave vial containing 3-[(6-iodo-4-quinolinyl)amino]-4-methylphenol (0.53 g, 0.14 mmol), trans-di(µ-acetato)bis[0-(di-o-tolyl-phosphino)benzyl]dipalladium (II) (3 mg, 3 µmol), tri-t-butylphosphinonium tetrafluoroborate (2 mg, 7 µmol), and molybdenum hexacarbonyl (0.37 g, 0.14 mmol) was added THF (0.56 mL), 1,8-diazabicyclo[5.4.0]undec-7-ene (64 µL, 0.42 mmol), and benzylamine (46 µL, 0.42 mmol) The resulting mixture was sealed in a microwave vial and heated to 170 °C for 20 minutes. After cooling to room temperature, the mixture was filtered through a PTFE syringe filter and purified by reverse phase HPLC to give 4-[(5-hydroxy-2-methylphenyl)amino]-*N*-(phenylmethyl)-6-quinolinecarboxamide. 1 H NMR (400 MHz, DMSO-d6) δ 2.07 (s, 3H) 4.58 (d, *J=* 5.79 Hz, 2H) 6.32 (d, *J=* 7.05 Hz, 1H) 6.74 (d, *J* = 2.52 Hz, 1H) 6.84 (dd, *J* = 8.31, 2.52 Hz, 1H) 7.22 - 7.31 (m, 2H) 7.32 - 7.43 (m, 4H) 8.03 (d, *J* = 8.81 Hz, 1H) 8.42 (dd, *J* = 8.81, 1.51 Hz, 1H) 8.51 (d, *J* = 7.05 Hz, 1H) 9.24 (d, *J* = 1.26 Hz, 1H) 9.31 (t, *J* = 5.92 Hz, 1 H) 10.95 (s, 1H) 14.12 - 14.39 (m, 1H); MS (m/z) 384.2 (M+H⁺).

The following compounds were prepared using procedures analogous to those described above.

| Ex # | Structure | Name | MS (M+H) ⁺ | NMR |
|---|---|---|---|---|
| 175 | 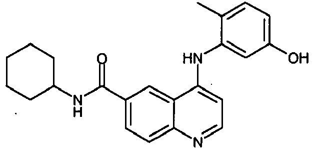 | N-cyclohexyl-4-[(5-hydroxy-2-methylphenyl)amino]-6-quinolinecarboxamide | 376 | NA |
| 176 | 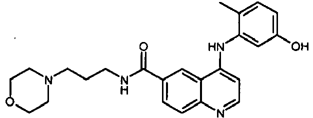 | 4-[(5-hydroxy-2-methylphenyl)amino]-N-[3-(4-morpholinyl)propyl]-6-quinolinecarboxamide | 421 | NA |
| 177 | 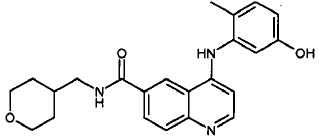 | 4-[(5-hydroxy-2-methylphenyl)amino]-N-(tetrahydro-2H-pyran-4-ylmethyl)-6-quinolinecarboxamide | 392 | NA |
| 178 | 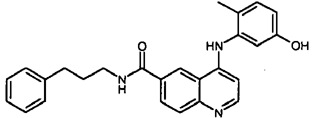 | 4-[(5-hydroxy-2-methylphenyl)amino]-N-(3-phenylpropyl)-6-quinolinecarboxamide | 412 | NA |
| 179 | 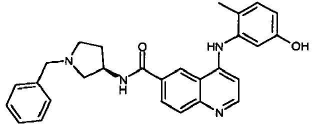 | 4-[(5-hydroxy-2-methylphenyl)amino]-N-[(3R)-1-(phenylmethyl)-3-pyrrolidinyl]-6-quinolinecarboxamide | 453 | NA |
| 180 | 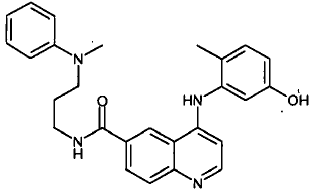 | 4-[(5-hydroxy-2-methylphenyl)amino]-N-{3-[methyl(phenyl) amino]propyl}-6-quinolinecarboxamide | 441 | NA |
| 181 | 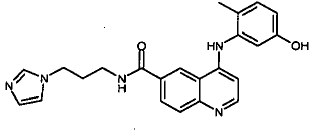 | 4-[(5-hydroxy-2-methylphenyl)amino]-N-[3-(1H-imidazol-1-yl)propyl]-6-quinolinecarboxamide | 402 | NA |
| 182 | 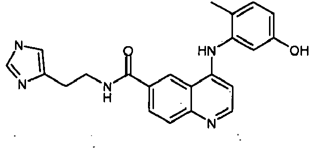 | 4-[(5-hydroxy-2-methylphenyl)amino]-N-[2-(1H-imidazol-4-yl)ethyl]-6-quinolinecarboxamide | 388 | NA |
| 183 | 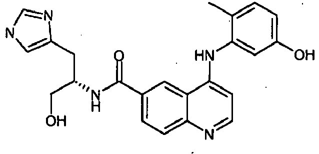 | N-[(1S)-2-hydroxy-1-(1H-imidazol-4-ylmethyl)ethyl]-4-[(5-hydroxy-2-methylphenyl)amino]-6-quinolinecarboxamide | 418 | NA |
| 184 | 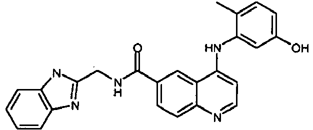 | N-(1H-benzimidazol-2-ylmethyl)-4-[(5-hydroxy-2-methylphenyl)amino]-6-quinolinecarboxamide | 424 | NA |
| 185 | 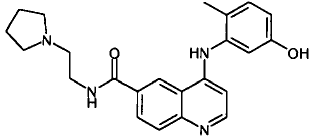 | 4-[(5-hydroxy-2-methylphenyl)amino]-N-[2-(1-pyrrolidinyl)ethyl]-6-quinolinecarboxamide | 391 | NA |
| 186 | 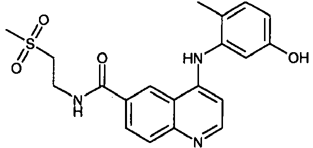 | 4-[(5-hydroxy-2-methylphenyl)amino]-N-[2-(methylsulfonyl) ethyl]-6-quinolinecarboxamide | 400 | NA |
| 187 | 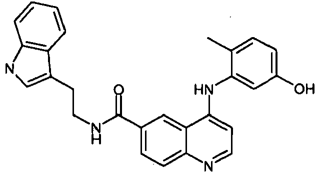 | 4-[(5-hydroxy-2-methylphenyl)amino]-N-[2-(1H-indol-3-yl)ethyl]-6-quinolinecarboxamide | 437 | NA |
| 188 | 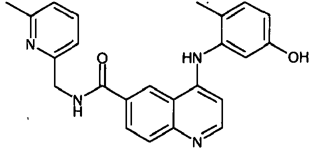 | 4-[(5-hydroxy-2-methylphenyl)amino]-N-[(6-methyl-2-pyridinyl)methyl]-6-quinolinecarboxamide | 399 | NA |
| 189 | 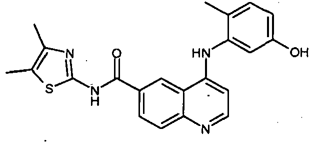 | N-(4,5-dimethyl-1,3-thiazol-2-yl)-4-[(5-hydroxy-2-methylphenyl)amino]-6-quinolinecarboxamide | 405 | NA |
| 190 | 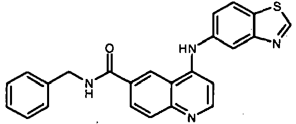 | 4-(1,3-benzothiazol-5-ylamino)-N-(phenylmethyl)-6-quinolinecarboxamide | 411 | ¹H NMR (400 MHz, DMSO-d6) δ 4.59 (d, J = 5.77 Hz, 2H) 6.95 (d, J = 7.03 Hz, 1H) 7.19 - 7.49 (m, 4H) 7.63 (dd, J = 8.66, 1.88 Hz, 1H) 8.07 (d, J = 8.78 Hz, 1H) 8.23 (d, J = 1.76 Hz, 1H) 8.36 - 8.51 (m, 2H) 8.58 (d, J = 7.03 Hz, 1H) 9.29 (d, J = 1.25 Hz, 1H) 9.37 (t, J = 6.02 Hz, 1H) 9.55 (s, 1H) 11.29 (s, 1H) 14.37 (br. s., 1H) |

### Example 191

### N-1,3-Benzothiazol-5-yl-6-(4-morpholinylsulfonyl)-4-quinolinamine

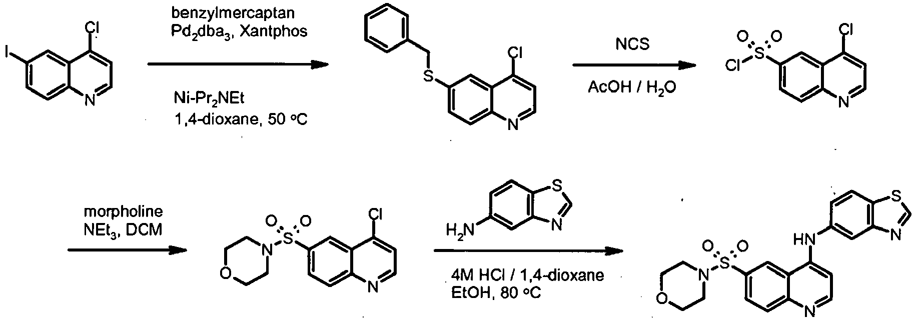

Step 1.4-Chloro-6-[(phenylmethyl)thio]quinoline: A 250 ml round bottom flask was charged with 4-chloro-6-iodoquinoline (5000 mg, 17.27 mmol), Xantphos (1999 mg, 3.45 mmol), Pd₂(dba)₃ (1.58 g, 1.73 mmol) and 1,4-Dioxane (100 mL). The brown reaction was sparged with argon for 10 min, then Hunig's base (6.0 mL, 35 mmol) and finally benzyl mercaptan (2.4 mL, 21 mmol) were added. The reaction was heated at 50 °C for 2 h. The solvent was then removed in vacuo and the residue was purified by Biotage column (0 - 50 % EtOAc/hexanes) to afford the title compound as a brown solid (3.60 g). MS (m/z) 285.9, 287.9 (M+H⁺).

Step 2. 4-Chloro-6-quinolinesulfonyl chloride: To a solution of 4-chloro-6-[(phenylmethyl)thio]quinoline (3.8 g, 13 mmol) in acetic acid (90 mL) and water (10.00 mL) was added NCS (5.3 g, 40 mmol) at room temperature. The reaction mixture was stirred at this temperature overnight. Solvent was then removed and the residue was azeotroped twice with toluene to provide 7.2 g of crude material containing the title compound. The light brown solid was moved to next step without purification. MS (m/z) 261.8, 263.8 (M+H⁺).

Step 3. 4-Chloro-6-(4-morpholinylsulfonyl)quinoline: To a solution of morpholine (1330 mg, 15.26 mmol) in DCM (60 mL), was added TEA (3.19 mL, 22.89 mmol) and 4-chloro-6-quinolinesulfonyl chloride (2.0 g, 7.6 mmol). The reaction mixture was stirred at room temperature for 30 min. Solvent was then removed in vacuo and the crude material was purified by biotage column (0 to 3 % MeOH / DCM) to provide 1.70 g light yellow solid. MS (m/z) 313 (M+H⁺).

The synthesis of diverse sulfonamides employed either DCM or THF as solvent, and was successful with or without the addition of DMAP.

Step 4. N-1,3-benzothiazol-5-yl-6-(4-morpholinylsulfonyl)-4-quinolinamine: To a solution of 4-chloro-6-(4-morpholinylsulfonyl)quinoline (1.3 g, 4.16 mmol) in ethanol (30 mL) was added 1,3-benzothiazol-5-amine (0.75 g, 5.0 mmol) and 4 M HCl in dioxane (0.50 ml, 2.0 mmol). The reaction mixture was heated at 80 °C. The bright yellow solid was precipitated out from ethanol. After 2 hours the reaction was allowed to cool to room temperature. The solution was filtered and the solid was suspended in DCM (200 ml). 1 N NaOH (20 ml) was added and the solution was stirred for 10 minutes during which time the solid dissolved. The two layers were then separated and the organic layer was washed with NaOH again, dried over Na₂SO₄, and concentrated in vacuo. The residue was triturated with hot ethanol to provide the title compound as an off white solid (1.2 g). ¹H NMR(DMSO-d₆) δ 9.71 (s, 1H), 9.45 (s, 1H), 8.94 (d, *J* = 1.8 Hz, 1H), 8.61 (d, *J* = 5.3 Hz, 1H), 8.24 (d, *J* = 8.6 Hz, 1H), 8.04 - 8.12 (m, 2H), 7.94 (dd, *J* = 8.8, 1.8 Hz, 1 H), 7.57 (dd, *J* = 8.6, 2.0 Hz, 1H), 7.07 (d, *J* = 5.3 Hz, 1 H), 3.59 - 3.72 (m, 4H), 2.93 - 3.04 (m, 4H). MS (m/z) 427.1 (M+H⁺).

Alternatively, the incorporation of diverse amines and anilines into quinoline sulfonamides (step 4) can be conducted via four general sets of conditions:

### A: EtOH elevated temperature.

A reaction vessel containing a 4-chloro-6-quinolinesulfonamide (1 eq.), ethanol (0.05 M), and amine/aniline (1 eq.) was sealed and heated to 160 °C. After completion, as determined by LCMS, the reaction was cooled to room temperature, then diluted with Et₂O to generate a precipitate. The solid was isolated by filtration.

### B: EtOH + HCl at elevated temperature.

To a solution of a 4-chloro-6-quinolinesulfonamide (1eq.) in ethanol (0.02 M) was added amine/aniline (1.2 eq.) and 4M HCl in dioxane (0.2 eq.). The reaction was heated to 80 °C. The resulting product was basified with carbonate bases and purified by silica gel chromatography.

### C: NMP + HCl at elevated temperature.

A 10 mL microwave vial was charged with a 4-chloro-6-quinolinesulfonamide (1 eq.), amine/aniline (2 eq.), N-methyl-2-pyrrolidone (NMP) (0.06 M), and 4M HCl in dioxane (0.3 eq.). The vessel was sealed and heated to 80 °C. After 1 hr the reaction was cooled to room temperature, filtered and purified directly via reverse phase HPLC.

### D: Pd-Catalyzed.

To a solution of a 4-chloro-6-quinolinesulfonamide (1 eq.), amine/aniline (1 eq.), Cs₂CO₃ (2.4 eq.) and Xantphos (0.2 eq.) in 1,4-Dioxane (0.1 M) in a sealed tube bubbled with N₂ was added Pd₂(dba)₃ (0.2 eq.). The reaction mixture was heated to 120 °C for 1 hr. The mixture was then purified by silica gel chromatography.

### E:

A reaction vial was charged with 4-chloro-6-quinolinesulfonamide (1.0 eq.), amine/aniline (1.1 eq.), ethanol (0.06 M), and finally 4M HCl in Dioxane (0.3 eq.). The vessel was sealed and heated to 60 °C in a heating block. After 4 hrs the reaction was cooled to room temperature. Et₂O was added to further precipitate products. The precipitate was isolated by filtration.

### Example 192

### 4-(1,3-Benzothiazol-5-ylamino)-N-(3-methyl-3-oxetanyl)-6-quinolinesulfonamide

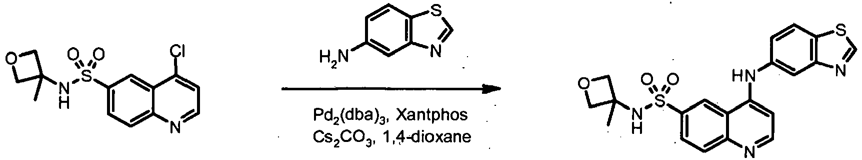

To a solution of 4-chloro-N-(3-methyl-3-oxetanyl)-6-quinolinesulfonamide (30 mg, 0.096 mmol), 1,3-benzothiazol-5-amine (14.41 mg, 0.096 mmol), Cs₂CO₃ (75 mg, 0.230 mmol) and Xantphos (11.10 mg, 0.019 mmol) in 1,4-Dioxane (1 mL) in a sealed tube bubbled with N₂ was added Pd₂(dba)₃ (17.57 mg, 0.019 mmol). The reaction mixture was heated to 120°C for 1h. The mixture was then loaded directly onto a Biotage column and purified (0 - 5 % MeOH/DCM) to provide 15.0 mg of 4-(1,3-benzothiazol-5-ylamino)-N-(3-methyl-3-oxetanyl)-6-quinolinesulfonamide. MS (m/z) 427.1 (M+H⁺) ¹H NMR (DMSO-d₆) d: 9.69 (s, 1H), 9.44 (s, 1H), 8.98 (d, J = 1.3 Hz, 1H), 8.58 (d, J = 5.6 Hz, 1H), 8.50 (s, 1H), 8.22 (d, J = 8.6 Hz, 1H), 7.98 - 8.12 (m, 3H), 7.57 (dd, J = 8.6, 2.0 Hz, 1H), 7.08 (d, J = 5.6 Hz, 1H), 4.61 (d, J = 6.1 Hz, 2H), 4.15 (d, J = 6.3 Hz, 2H), 1.44 (s, 3H).

The following compounds were prepared using procedures analogous to those described above (Methods A, B, C, or D).

| Ex # | Structure | Name | MS (M+H)⁺ | NMR | Method |
|---|---|---|---|---|---|
| 193 | 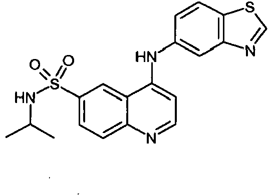 | 4-(1,3-benzothiazol-5-ylamino)-N-(1-methylethyl)-6-quinoline sulfonamide | 399 | ¹H NMR (DMSO-d6) δ 11.43 (dq, 1H), 9.54 (s, 1H), 9.28 (br. s., 1H), 8.58 (d, J = 7.1 Hz, 1H), 8.40 (d, J = 8.6 Hz, 1H), 8.32 (d, J = 9.1 Hz, 1H), 8.24 (d, J = 1.8 Hz, 1H), 8.20 (d, J = 8.8 Hz, 1H), 7.98 (d, J = 7.3 Hz, 1H), 7.63 (dd, J = 8.5, 1.9 Hz, 1H), 6.98 (d, J = 7.1 Hz, 1H), 3.37 - 3.46 (m, 1H), 1.01 (d, J = 6.6 Hz, 6H) | A |
| 194 | 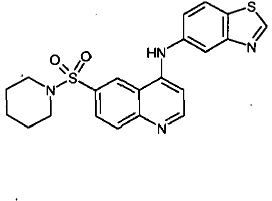 | N-1,3-benzothiazol-5-yl-6-(1-piperidinyl sulfonyl)-4-quinolinamine | 425 | ¹H NMR (DMSO-d6) δ 9.66 (s, 1H), 9.44 (s, 0H), 8.91 (s, 1H), 8.59 (d, J = 5.4 Hz, 1H), 8.22 (d, J = 8.5 Hz, 1H), 8.04 - 8.08 (m, 2H), 7.93 (dd, J = 9.0, 1.2 Hz, 1H), 7.56 (dd, J = 8.6, 1.5 Hz, 1H), 7.06 (d, J = 5.4 Hz, 1H), 2.96 - 3.02 (m, 4H), 1.52 - 1.60 (m, 4H), 1.33 - 1.40 (m, 2H) | C |
| 195 | 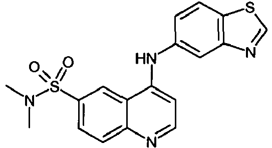 | 4-(1,3-benzothiazol-5-ylamino)-N,N-dimethyl-6-quinoline sulfonamide | 385 | ¹H NMR (DMSO-d6) δ 9.71 (br. s., 1H), 9.44 (s, 1H), 8.94 (s, 1H), 8.56 (br. s., 1H), 8.22 (d, J = 8.6 Hz, 1H), 8.05 (br. s., 2H), 7.94 (d, J = 8.3 Hz, 1H), 7.55 (d, J = 8.1 Hz, 1H), 7.03 (br. s., 1H), 2.70 (s, 6H) | C |
| 196 | 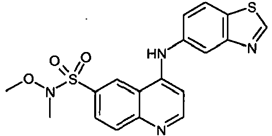 | 4-(1,3-benzothiazol-5-ylamino)-N-methyl-N-(methyloxy)-6-quinoline sulfonamide | 401 | ¹H NMR (DMSO-d6) δ 9.81 (br. s., 1H), 9.44 (s, 1H), 9.03 (s, 1H), 8.54 (br. s., 1H), 8.21 (d, J = 8.6 Hz, 1H), 7.88 - 8.13 (m, 3H), 7.53 (d, J = 7.1 Hz, 1H), 7.00 (br. s., 1H), 3.77 (s, 3H), 2.83 (s, 3H) | C |
| 197 | 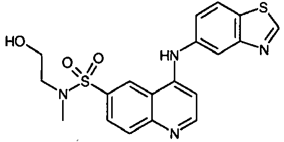 | 4-(1,3-benzothiazol-5-ylamino)-N-(2-hydroxyethyl)-N-methyl-6-quinoline sulfonamide | 415 | ¹H NMR (DMSO-d6) δ 9.69 (s, 1H), 9.45 (s, 1H), 8.96 (s, 1H), 8.59 (d, J = 3.8 Hz, 1H), 8.23 (d, J = 8.3 Hz, 1H), 8.01 - 8.14 (m, 2H), 7.97 (d, J = 8.1 Hz, 1H), 7.5 (d, J = 7.8 Hz, 1H), 7.07 (d, J = 5.3 Hz, 1H), 4.77 - 4.90 (m, 1H), 3.50 - 3.61 (m, 2H), 3.08 - 3.14 (m, 2H), 2.81 (s, 3H) | C |
| 198 | 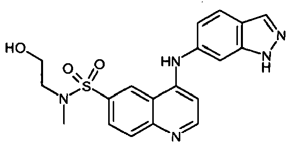 | N-(2-hydroxyethyl)-4-(1H-indazol-6-ylamino)-N-methyl-6-quinoline sulfonamide | 398 | ¹H NMR (DMSO-d6) δ 13.39 (br. s., 1H), 10.07 (br. s., 1H), 9.25 (br. s., 1H), 8.69 (d, J = 5.3 Hz, 1H), 8.22 (d, J = 7.9 Hz, 1H), 8.12 (d, J = 8.7 Hz; 1H), 8.03 - 8.10 (m, J = 83 Hz, 1H), 7.83 (d, J = 6.8 Hz, 1H), 7.55 (d, J = 4.9 Hz, 1H), 7.32 (t, J = 7.6 Hz, 1H), 3.52 - 3.63 (m, 1H), 1.24 (d, J = 6.8 Hz, 6H) | C |
| 199 | 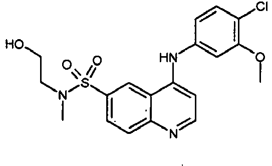 | 4-{[4-chloro-3-(methyloxy)phen yl]ammo}-N-(2-hydroxyethyl)-N-methyl-6-quinoline sulfonamide | 422 | ¹H NMR (DMSO-d6) δ 9.55 (br. s., 1H), 8.88 (br. s., 1H), 8.60 (br. s., 1H), 7.85 - 8.18 (m, 2H), 7.44 (br. s., 1H), 6.88 - 7.27 (m, 3H), 3.87 (s, 3H), 3.54 (t, J = 6.1 Hz, 2H), 3.05 (br. s., 1H), 3.00 - 3.14 (m, 2H), 2.79 (s, 3H) | C |
| 200 | 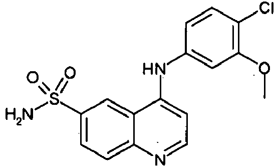 | 4-{[4-chloro-3-(methyloxy)phen yl]amino}-6-quinoline sulfonamide | 364 | ¹H NMR (DMSO-d6) δ 9.52 (br. s., 1 H), 8.90 (s, 1H), 8.58 (d, J = 4.4 Hz, 1H), 8.08 (d, J = 8.8 Hz, 1H), 8.03 (d, J = 8.8 Hz, 1H), 7.37 - 7.49 (m, 3H), 7.08 - 7.19 (m, 2H), 6.98 (d, J = 8.1 Hz, 1H), 3.86 (s, 3H) | C |
| 201 | 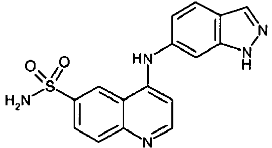 | 4-(1H-indazol-6-ylamino)-6-quinoline sulfonamide | 340 | ¹H NMR (DMSO-d6) δ 12.96 (br. s., 1H), 9.56 (s, 1H), 8.96 (d, J = 1.8 Hz, 1H), 8.56 (d, J = 5.3 Hz, 1H), 7.98 - 8.14 (m, 3H), 7.80 (d, J = 8.8 Hz, 1H), 7.45 (d, J = 6.8 Hz, 3H), 7.17 (dd, J = 8.7, 1.6 Hz, 1H), 7.08 (d, 1H) | C |
| 202 | 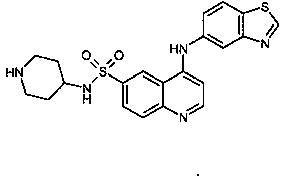 | 4-(1,3-benzothiazol-5-ylamino)-N-4-piperidinyl-6-quinoline sulfonamide | 440 | ¹H NMR (400 MHz, DMSO-d₆) δ 1.50 - 1.89 (m, 4H) 2.92 (d, J = 10.36 Hz, 2H) 3.17 (t, J = 6.44 Hz, 2H) 3.41 - 3.76 (m, 2H) 6.98 (d, J = 7.07 Hz, 1H) 7.65 (dd, J = 8.59, 2.02 Hz, 1H) 8.20 - 8.45 (m, 4H) 8.58 (d, J = 7.07 Hz, 1H) 8.81 (br. s., 1H) 8.95 (br. s., 1H) 9.45 (d, J = 1.52 Hz, 1H) 9.55 (s, 1H) 11.76 (s, 1H) 15.11 (br. s., 1H) | C |
| 203 | 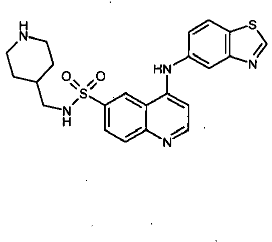 | 4-(1,3-benzothiazol-5-ylamino)-N-(4-piperidinylmethyl )-6-quinoline sulfonamide | 454 | ¹H NMR (400 MHz, DMSO-d₆) 1.13 - 1.39 (m, 4H) 1.65 - 1.86 (m, 2H) 2.71 - 2.91 (m, 3H) 3.08 (dd, J = 7.33, 4.80 Hz, 1H) 3.23 (br. s., 2H) 6.98 (d, J = 7.07 Hz, 1H) 7.64 (dd, J = 8.59, 1.77 Hz, 1H) 8.16 (t, J = 6.19 Hz, 1H) 8.23 - 8.30 (m, 1H) 8.33 - 8.36 (m, 1H) 8.41 (d, J = 8.59 Hz, 1H) 8.59 (d, J = 6.82 Hz, 1H) 8.78 (br. s., 1H) 9.37 (s, 1H) 9.55 (s, 1H) 11.60 (s, 1H) 14.83 (br. s., 1H) | C |
| 204 | 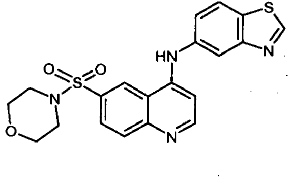 | N-1,3-benzothiazol-5-yl-6-(4-morpholinyl sulfonyl)-4-quinolinamine hydrochloride | 427 | ¹H NMR (400 MHz, DMSO-d₆) δ 2.99 - 3.06 (m, 4H) 3.63 - 3.72 (m, 4H) 6.97 (d, J = 7.07 Hz, 1H) 7.64 (dd, J = 8.46, 1.89 Hz, 1H) 8.22 - 8.32 (m, 3H) 8.42 (d, J = 8.34 Hz, 1H) 8.61 (d, J = 7.07 Hz, 1H) 9.27 (s, 1H) 9.55 (s, 1H) 11.61 (br. s., 1H) | B |
| 205 | 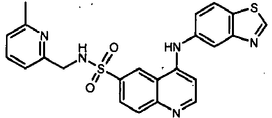 | 4-(1,3-benzothiazol-5-ylamino)-N-[(6-methyl-2-pyridinyl)methyl] -6-quinoline sulfonamide trifluoro acetate | 462 | ¹H NMR (500 MHz, DMSO-d₆) δ 2.32 (s, 3H) 4.22 (d, J = 6.10 Hz, 2H) 6.96 (d, J = 6.84 Hz, 1H) 7.09 (d, J = 7.57 Hz, 1H) 7.23 (d, J = 7.57 Hz, 1H) 7.57 - 7.71 (m, 2H) 8.12 (d, J = 9.03 Hz, 1H) 8.23 (d, J = 1.46 Hz, 1H) 8.28 - 8.34 (m, 1H) 8.41 (d, J = 8.55 Hz, 1H) 8.54 - 8.67 (m, 2H) 9.18 (s, 1H) 9.54 (s, 1H) 11.46 (br. s., 1H) | C |
| 206 | 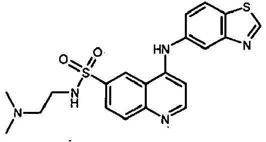 | 4-(1,3-benzothiazol-5-ylamino)-N-[2-(dimethylamino)e thyl]-6-quinoline sulfonamide trifluoroacetate | 428 | ¹H NMR (400 MHz, Methanol-d₄) δ 2.68 (s, 3H) 2.99 (s, 6H) 3.35 - 3.38 (m, 4H) 7.19 (dd, J = 8.59, 2.02 Hz, 2H) 7.67 (d, J = 2.02 Hz, 2H) 7.97 (d, J = 8.59 Hz, 2H) 8.11 - 8.56 (m, 2H) 9.25 (s, 2H) | C |
| 207 | 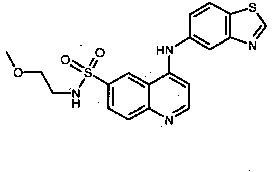 | 4-(1,3-benzothiazol-5-ylamino)-N-[2-(methyloxy)ethyl] -6-quinoline sulfonamide trifluoroacetate | 415 | ¹H NMR (500 MHz, DMSO-d₆) δ 3.04 (q, 2H) 3.16 (s, 3H) 3.35 (t, J = 5.62 Hz, 2H) 6.96 (d, J = 7.08 Hz, 1H) 7.62 (dd, J = 8.55, 1.71 Hz, 1H) 8.08 (t, J = 5.86 Hz, 1H) 8.16 (d, J = 8.79 Hz, 1H) 8.23 (d, J = 1.46 Hz, 1H) 8.30 - 8.36 (m, 1H) 8.40 (d, J = 8.54 Hz, 1H) 8.58 (d, J = 7.08 Hz, 1H) 9.25 (s, 1H) 9.54 (s, 1H) 11.46 (br. s., 1H) | C |
| 208 | 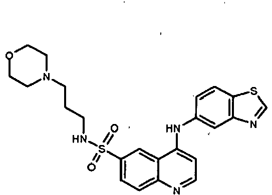 | 4-(1,3-benzothiazol-5-ylamino)-N-[3-(4-morpholinyl)prop yl]-6-quinoline sulfonamide trifluoroacetate | 484 | ¹H NMR (500 MHz, DMSO-d₆) δ 1.67 - 1.98 (m, 2H) 2.93 (d, J = 6.10 Hz, 4H) 3.04 - 3.20 (m, 4H) 6.94 - 7.05 (m, 2H) 7.41 (br. s., 1H) 7.61 (dd, J = 8.55, 1.46 Hz, 1H) 7.87 (d, J = 8.55 Hz, 1H) 8.10 - 8.16 (m, 1 H) 8.17 - 8.27 (m, 2H) 8.28 - 8.36 (m, 1H) 8.41 (d, J = 8.55 Hz, 1H) 8.60 (d, J = 7.08 Hz, 1H) 9.26 (d, J = 13.67 Hz, 2H) 9.54 (s, 1H) 11.49 (br. s., 1H) | C |
| 209 | 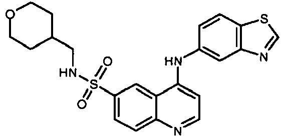 | 4-(1,3-benzothiazol-5-ylamino)-N-(tetrahydro-2H-pyran-4-ylmethyl)-6-quinoline sulfonamide trifluoroacetate | 455 | ¹H NMR (500 MHz, DMSO-d₆) δ 1.00 - 1.19 (m, 2H) 1.51 - 1.69 (m, 3H) 2.74 (t, J = 6.35 Hz, 2H) 3.22 (t, J = 10.99 Hz, 2H) 3.81 (dd, J = 11.11, 2.08 Hz, 2H) 6.97 (d, J = 7.08 Hz, 1H) 7.62 (dd, J = 8.55, 1.71 Hz, 1H) 8.01 (t, J = 5.86 Hz, 1H) 8.18 (d, J = 8.79 Hz, 1H) 8.24 (d, J = 1.46 Hz, 1H) 8.32 (dd, J = 9.03, 1.46 Hz, 1H) 8.40 (d, J = 8.54 Hz, 1H) 8.58 (d, J = 7.08 Hz, 1H) 9.26 (s, 1H) 9.54 (s, 1H) 11.48 (br. s., 1H) | C |
| 210 | 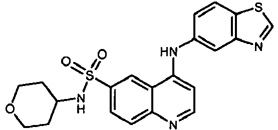 | 4-(1,3-benzothiazol-5-ylamino)-N-(tetrahydro-2H-pyran-4-yl)-6-quinoline sulfonamide trifluoroacetate | 441 | ¹H NMR (500 MHz, DMSO-d₆) δ 1.32 - 1.71 (m, 2H) 2.07 (s, 1H) 3.08 - 3.40 (m, 3H) 3.56 - 3.67 (m, 1H) 3.75 - 3.79 (m, 1H) 3.77 - 3.87 (m, 2H) 6.87 - 7.03 (m, 3H) 7.37 (s, 2H) 7.62 (dd, J = 8.42, 1.59 Hz, 1H) 7.84 (d, J = 8.55 Hz, 2H) 8.11 - 8.28 (m, 1H) 8.28 - 8.48 (m, 1H) 8.58 (d, J = 7.08 Hz, 1H) 9.23 (s, 1H) 11.48 (br. s., 1H) | C |
| 211 | 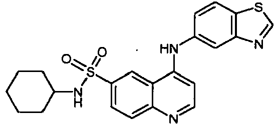 | 4-(1,3-benzothiazol-5-ylamino)-N-cyclohexyl-6-quinoline sulfonamide | 439 | ¹H NMR (400 MHz, DMSO-d₆) δ 3.14 - 3.19 (m, 9H) 3.43 (s, 1H) 7.09 (d, J = 5.52 Hz, 1H) 7.57 (dd, J = 8.66, 1.88 Hz, 1H) 7.79 (br. s., 1H) 8.02 - 8.09 (m, 2H) 8.22 (d, J = 8.53 Hz, 1H) 8.58 (d, J = 5.52 Hz, 1H) 8.92 - 9.00 (m, 1H) 9.44 (s, 1H) 9.51 (s, 1H) 9.65 (s, 1H) | C |
| 212 | 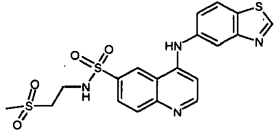 | 4-(1,3-benzothiazol-5-ylamino)-N-[2-(methylsulfonyl)e thyl]-6-quinoline sulfonamide | 463 | ¹H NMR (400 MHz, DMSO-d₆) δ 3.03 (s, 3H) 3.29-3.33(m, 2H) 3.39 (s, 2H) 7.08 (d, J = 5.52 Hz, 1H) 7.57 (dd, J = 8.66, 1.88 Hz, 1H) 7.99 - 8.11 (m, 5H) 8.23 (d, J = 8.78 Hz, 1H) 8.59 (d, J = 5.52 Hz, 1H) 9.00 (d, J = 1.25 Hz, 1H) 9.45 (s, 1H) 9.68 (s, 1H) | C |
| 213 | 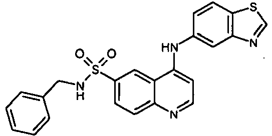 | 4-(1,3-benzothiazol-5-ylamino)-N-(phenylmethyl)-6-quinoline sulfonamide | 447 | ¹H NMR (400 MHz, DMSO-d₆) δ 4.06 (s, 2H) 7.08 (d, J = 5.56 Hz, 1H) 7.13 - 7.36 (m, 5H) 7.57 (dd, J = 8.59, 2.02 Hz, 1H) 7.96 - 8.12 (m, 3H) 8.16 - 8.38 (m, 2H) 8.58 (d, J = 5.31 Hz, 1H) 8.96 (s, 1H) 9.44 (s, 1H) 9.65 (s, 1H) | C |
| 214 | 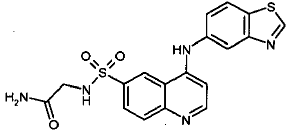 | N∼2∼-{[4-(1,3-benzothiazol-5-ylamino)-6-quinolinyl] sulfonyl} glycinamide | 414 | ¹H NMR (500 MHz, DMSO-d₆) δ 3.45 (s, 2H) 6.99 - 7.17 (m, 2H) 7.26 (br. s., 1H) 7.56 (dd, J = 8.55, 1.71 Hz, 1H) 7.99 - 8.12 (m, 3H) 8.21 (d, J = 8.55 Hz, 1H) 8.57 (d, J = 5.13 Hz, 1H) 8.96 (s, 1H) 9.40 (m, 1H) 9.43 (s, 1H) 9.61 (s, 1H) | C |
| 215 | 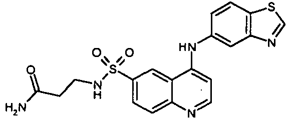 | N~3∼-[4-(1,3-benzothiazol-5-ylamino)-6-quinolinyl] sulfonyl}-beta-alaninamide | 428 | ¹H NMR (500 MHz, DMSO-d₆) δ 2.25 (t, 2H) 3.00 (q, J = 7.08 Hz, 2H) 4.08 (q, J = 5.21 Hz, 1H) 6.83 (br. s., 1H) 7.07 (d, J = 5.37 Hz, 1H) 7.32 (br. s., 1H) 7.56 (d, J = 7.57 Hz, 1H) 7.70 (br. s., 1H) 7.95 - 8.11 (m, 3H) 8.21 (d, J = 8.79 Hz, 1H) 8.57 (d, J = 5.37 Hz, 1H) 8.96 (s, 1H) 9.43 (s, 1H) 9.64 (br. s., 1H) | C |
| 216 | 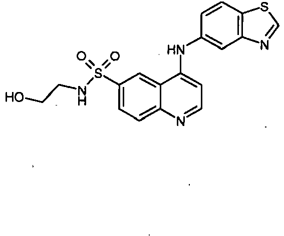 | 4-(1,3-benzothiazol-5-ylamino)-N-(2-hydroxyethyl)-6-quinoline sulfonamide | 401 | ¹H NMR (500 MHz, DMSO-d₆) δ 2.87 (q, J = 6.10 Hz, 2H) 3.14 - 3.20 (m, 2H) 3.17 (d, J = 5.13 Hz, 2H) 3.17 (m, 2H) 3.15 - 3.19 (m, 2H) 4.08 (q, J = 5.29 Hz, 1H) 4.68 (t, J = 5.62 Hz, 1H) 7.07 (d, J = 4.64 Hz, 1H) 7.56 (d, J = 8.30 Hz, 1H) 7.68 (t, J = 5.98 Hz, 1H) 7.95 - 8.11 (m, 2H) 8.21 (d, J = 8.54 Hz, 1H) 8.57 (d, J = 4.88 Hz, 1H) 8.95 (s, 1H) 9.43 (s, 1H) 9.65 (br. s., 1H) | C |
| 217 | 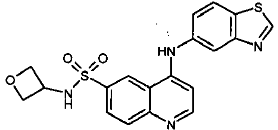 | 4-(1,3-benzothiazol-5-ylamino)-N-3-oxetanyl-6-quinoline sulfonamide | 413 | ¹H NMR (400 MHz, DMSO-d₆) δ 3.17 (d, 1H) 4.25 - 4.31 (m, 2H) 4.43 - 4.58 (m, 3H) 7.07 (d, J = 5.31 Hz, 1H) 7.57 (dd, J = 8.59, 2.02 Hz, 1H) 7.95 - 8.10 (m, 3H) 8.23 (d, J = 8.59 Hz, 1H) 8.58 (d, J = 5.31 Hz, 1H) 8.69 (d, J = 7.33 Hz, 1H) 8.95 (d, J = 1.77 Hz, 1H) 9.45 (s, 1H) 9.68 (s, 1H) | D |
| 218 | 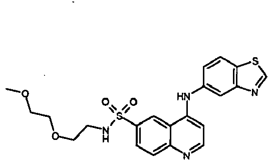 | 4-(1,3-benzothiazol-5-ylamino)-N-(2-{[2-(methyloxy)ethyl] oxy}ethyl)-6-quinoline sulfonamide | 459 | ¹H NMR (500 MHz, DMSO-d₆) δ 2.98 (q, J = 5.70 Hz, 2H) 3.18 (s, 3H) 3.25-3.35(m, 2H) 3.37 - 3.44 (m, 4H) 7.07 (d, J = 5.37 Hz, 1H) 7.55 (dd, J = 8.67, 1.83 Hz, 1H) 7.79 (t, J = 5.86 Hz, 1H) 7.98 - 8.11 (m, 3H) 8.21 (d, J = 8.54 Hz, 1H) 8.57 (d, J = 5.37 Hz, 1H) 8.95 (s, 1H) 9.43 (s, 1H) 9.62 (s, 1H) | C |
| 219 | 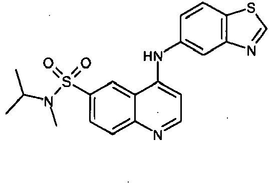 | 4-(1,3-benzothiazol-5-ylamino)-N-methyl-N-(1-methylethyl)-6-quinoline sulfonamide | 413 | ¹H NMR (500 MHz, DMSO-d₆) δ 0.93 (d, 6H) 2.73 (s, 3H) 4.21 (dt, J = 13.43, 6.71 Hz, 1H) 7.06 (br. s., 1H) 7.57 (d, J = 8.06 Hz, 1H) 7.94 - 8.11 (m, 3H) 8.23 (d, J = 8.55 Hz, 1H) 8.57 (d, J = 4.64 Hz, 1H) 8.98 (s, 1H) 9.44 (s, 1H) 9.69 (br. s., 1H) | C |
| 220 | 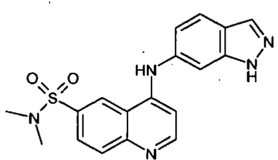 | 4-(1H-indazol-6-ylamino)-N,N-dimethyl-6-quinoline sulfonamide | 368 | ¹H NMR (400 MHz, DMSO-d₆) δ 2.70 (s, 6H) 7.07 (d, J = 5.52 Hz, 1H) 7.19 (dd, J = 8.66, 1.63 Hz, 1H) 7.49 (s, 1H) 7.82 (d, J = 8.53 Hz, 1H) 7.95 (dd, J = 8.91, 1.88 Hz, 1H) 8.05 (s, 1H) 8.08 (s, 1H) 8.59 (d, J = 5.27 Hz, 1H) 8.93 (d, J = 1.76 Hz, 1H) 9.63 (s, 1H) | C |
| 221 | 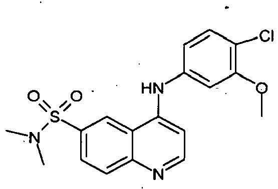 | 4-{[4-chloro-3-(methyloxy) phenyl]amino}-N,N-dimethyl-6-quinoline sulfonamide | 392 | ¹H NMR (400 MHz, DMSO-d₆) δ 2.69 (s, 6H) 3.33 (s, 2H) 7.01 (dd, J = 8.53, 2.26 Hz, 1H) 7.12 (d, J = 5.27 Hz, 1H) 7.16 (d, J = 2.01 Hz, 1H) 7.47 (d, J = 8.53 Hz, 1H) 7.95 (dd, J = 8.91, 1.88 Hz, 1H) 8.07 (d, J = 8.78 Hz, 1H) 8.61 (d, J = 5.27 Hz, 1H) 8.89 (d, J = 1.76 Hz, 1H) 9.55 (s, 1H) | C |
| 222 | 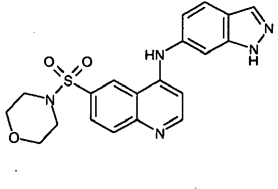 | N-1H-indazol-6-yl-6-(4-morpholinyl sulfonyl)-4-quinolinamine | 410 | ¹H NMR (400 MHz, DMSO-d₆) δ 2.91 - 3.01 (m, 4H) 3.62 - 3.71 (m, 4H) 7.07 (d, J = 5.27 Hz, 1H) 7.19 (dd, J = 8.53, 1.76 Hz, 1H) 7.49 (s, 1H) 7.82 (d, J = 8.53 Hz, 1H) 7.94 (dd,J = 8.91, 1.88 Hz, 1H) 8.04 - 8.13 (m, 2H) 8.60 (d, J = 5.52 Hz, 1H) 8.93 (d, J = 1.76 Hz, 1H) 9.64 (s, 1H) | C |
| 223 | 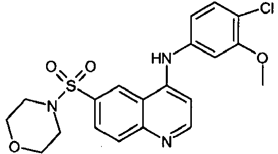 | N-[4-chloro-3-(methyloxy) phenyl]-6-(4-morpholinyl sulfonyl)-4-quinolinamine | 434 | ¹H NMR (400 MHz, DMSO-d₆) δ 2.88 - 3.03 (m, 4H) 3.60 - 3.70 (m, 4H) 3.88 (s, 3H) 6.94 - 7.05 (m, 2H) 7.10-7.19 (m, 2H) 7.47 (d, J = 8.53 Hz, 1H) 7.94 (dd, J = 8.78, 1.76 Hz, 1H) 8.09 (d, J = 9.03 Hz, 1H) 8.62 (d, J = 5.27 Hz, 1H) 8.88 (d, J = 1.51 Hz, 1H) 9.56 (s, 1H) | C |
| 224 | 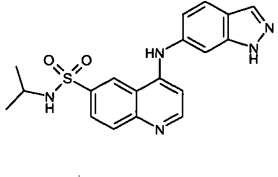 | 4-(1H-indazol-6-ylamino)-N-(1-methylethyl)-6-quinoline sulfonamide | 382 | ¹H NMR (400 MHz, DMSO-d₆) δ 0.97 (d, J = 6.53 Hz, 6H) 3.40-3.50 (m, 1H) 7.09 (d, J = 5.52 Hz, 1H) 7.19 (dd, J = 8.66, 1.63 Hz, 1H) 7.47 (s, 1H) 7.72 (br. s., 1H) 7.80 (d, J = 8.53 Hz, 1H) 8.01 - 8.11 (m, 3H) 8.57 (d, J = 5.27 Hz, 1H) 8.96 (s, 1H) 9.59 (s, 1H) 12.98 (br. s., 1H) | C |
| 225 | 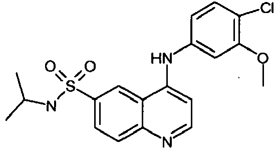 | 4-{[4-chloro-3-(methyloxy) phenyl]amino}-N-(1-methylethyl)-6-quinoline sulfonamide | 406 | ¹H NMR (400 MHz, DMSO-d₆) δ 0.96 (d, J = 6.53 Hz, 6H) 3.30-3.40 (m, 1H) 3.87 (s, 3H) 7.00 (dd, J = 8.41, 1.88 Hz, 1H) 7.10 - 7.21 (m, 2H) 7.44 (d, J = 8.53 Hz, 1H) 7.73 (br. s., 1H) 7.98 - 8.10 (m, 2H) 8.59 (d, J = 5.27 Hz, 1H) 8.90 (s, 1H) 9.51 (br. s., 1H) | C |
| 226 | 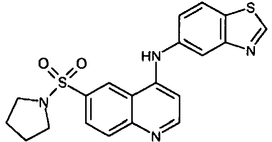 | N-1,3-benzothiazol-5-yl-6-(1-pyrrolidinyl sulfonyl)-4-quinolinamine | 411 | ¹H NMR (500 MHz, DMSO-d₆) δ 1.66 (br. s., 4H) 3.17-3.25(m, 4H) 7.05 (br. s., 1H) 7.57 (d, J = 8.55 Hz, 1H) 8.06 (br. s., 3H) 8.25 (br. s., 1H) 8.59 (br. s., 1H) 9.00 (br. s., 1H) 9.45 (s, 1H) 9.86 (br. s., 1H) | C |
| 227 | 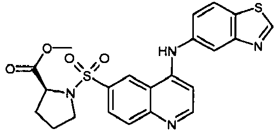 | methyl 1-{[4-(1,3-benzothiazol-5-ylamino)-6-quinolinyl] sulfonyl}-L-prolinate | 469 | ¹H NMR (500 MHz, DMSO-d₆) δ 1.63 (dd, J = 6.96,5.01 Hz, 1H) 1.77 - 2.03 (m, 4H) 3.40 - 3.50 (m, 1H) 3.66 (s, 3H) 4.37 - 4.48 (m, 1H) 7.05 (br. s., 1H) 7.57 (d, J = 8.30 Hz, 1H) 8.00 - 8.12 (m, 3H) 8.24 (d, J = 8.54 Hz, 1H) 8.59 (br. s., 1H) 9.00 (s, 1H) 9.45 (s, 1H) 9.78 (br. s., 1H) | C |
| 228 | 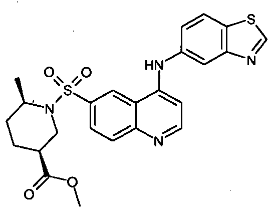 | methyl (3S,6R)-1-{[4-(1,3-benzothiazol-5-ylamino)-6-quinolinyl] sulfonyl}-6-methyl-3-piperidine carboxylate | 497 | ¹H NMR (500 MHz, DMSO-d₆) δ 1.04 (d, J = 6.84 Hz, 3H) 1.39 - 1.82 (m, 3H) 2.61 - 2.77 (m, 1H) 3.08 (t, J = 12.70 Hz, 2H) 3.61 (s, 3H) 3.94 - 4.06 (m, 1H) 4.25 (br. s., 1H) 7.03 (d, J = 5.13 Hz, 1H) 7.57 (d, J = 8.55 Hz, 1H) 8.02 - 8.14 (m, 3H) 8.26 (d, J = 8.54 Hz, 1H) 8.57 (d, J = 5.37 Hz, 1H) 9.08 (s, 1H) 9.46 (s, 1H) 10.05 (br. s., 1H) | C |
| 229 | 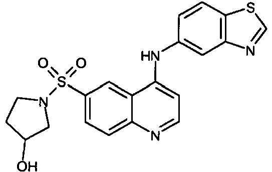 | 1-{[4-(1,3-benzothiazol-5-ylamino)-6-quinolinyl] sulfonyl}-3-pyrrolidinol | 427 | ¹H NMR (500 MHz, DMSO-d₆) δ 1.58 - 1.81 (m, 2H) 2.07 (s, 1H) 3.13 (d, J = 10.50 Hz, 1H) 3.35-3.42 (m, 2H) 4.16 (d, J = 2.20 Hz, 1H) 4.87 (d, J = 3.42 Hz, 1H) 7.06 (d, J = 4.39 Hz, 1H) 7.57 (d, J = 8.55 Hz, 1H) 7.97 - 8.12 (m, 3H) 8.23 (d, J = 8.55 Hz, 1H) 8.58 (d, J = 4.39 Hz, 1H) 8.97 (s, 1H) 9.44 (s, 1H) 9.72 (br. s., 1H) | C |
| 230 | 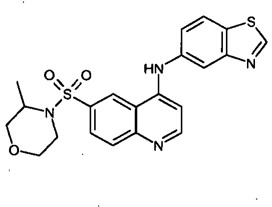 | N-1,3-benzothiazol-5-yl-6-[(3-methyl-4-morpholinyl) sulfonyl]-4-quinolinamine | 441 | ¹H NMR (500 MHz, DMSO-d₆) δ 1.10 (s, 3H) 3.20-3.40 (m, 2H) 3.54 (d, J = 10.99 Hz, 3H) 3.76 (br. s., 1H) 3.95 (br. s., 1H) 7.04 (br. s., 1H) 7.57 (d, J = 8.55 Hz, 1H) 8.09 (d, J = 11.23 Hz, 3H) 8.26 (d, J = 8.55 Hz, 1H) 8.58 (d, J = 5.37 Hz, 1H) 9.06 (s, 1H) 9.46 (s, 1H) 10.00 (br. s., 1H) | C |
| 231 | 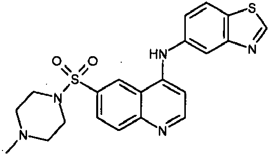 | N-1,3-benzothiazol-5-yl-6-[(4-methyl-1-piperazinyl) sulfonyl]-4-quinolinamine | 440 | ¹H NMR (500 MHz, DMSO-d₆) δ 1.23 (br. s., 1H) 2.76 (br. s., 3H) 3.15-3.30 (m, 2H) 3.85 (br. s., 3H) 7.01 (d, J = 6.84 Hz, 1H) 7.42 - 7.53 (m, 1H) 7.57 - 7.69 (m, 1H) 7.95 (d, J = 7.08 Hz, 1H) 8.16 - 8.31 (m, 3H) 8.39 (d, J = 8.54 Hz, 1H) 8.63 (d, J = 6.84 Hz, 1H) 9.24 (br. s., 1H) 9.53 (s, 1H) | C |
| 232 | 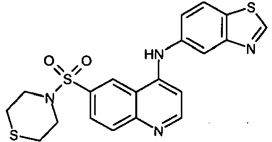 | N-1,3-benzothiazol-5-yl-6-(4-thiomorpholinyl sulfonyl)-4-quinolinamine | 443 | ¹H NMR (500 MHz, DMSO-d₆) δ 2.69 (d, J = 3.91 Hz, 4H) 2.84 - 3.00 (m, 2H) 3.44 - 3.58 (m, 2H) 7.18 (d, J = 5.13 Hz, 1H) 7.57 (d, J = 8.55 Hz, 1H) 7.90 - 8.02 (m, 1H) 8.02 - 8.20 (m, 1H) 8.21 - 8.36 (m, 2H) 8.59 (d, J = 4.64 Hz, 1H) 8.85 (d, J = 5.13 Hz, 1H) 8.96 (br. s., 1H) 9.45 (s, 1H) | C |
| 233 | 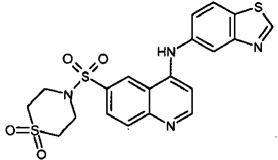 | N-1,3-benzothiazol-5-yl-6-[(1,1-dioxido-4-thiomorpholinyl) sulfonyl]-4-quinolinamine | 475 | ¹H NMR (500 MHz, DMSO-d₆) δ 2.40-2.55(m, 4H) 3.58 (br. s, 4H) 7.03 (d, J = 5.62 Hz, 1H) 7.59 (dd, J = 8.55, 1.71 Hz, 1H) 8.12 (br. s., 3H) 8.29 (d, J = 8.55 Hz, 1H) 8.60 (d, J = 5.86 Hz, 1H) 9.09 (s, 1H) 9.48 (s, 1H) 10.24 (br. s., 1H) | C |
| 234 | 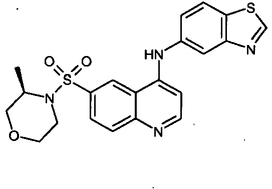 | N-1,3-benzothiazol-5-yl-6-{[(3R)-3-methyl-4-morpholinyl] sulfonyl}-4-quinolinamine | 441 | ¹H NMR (500 MHz, DMSO-d₆) δ 1.13 (d, J = 6.84 Hz, 3H) 3.40 (dd, 3H) 3.56 (d, J = 10.99 Hz, 2H) 3.80 (s, 1H) 3.97 (br. s., 1H) 6.98 (d, J = 6.84 Hz, 1H) 7.62 (dd, J = 8.42, 1.83 Hz, 1H) 8.15 (d, J = 8.79 Hz, 1H) 8.15 J = 8.79 1H) 8.23 (d, J = 1.22 Hz, 1H) 8.29 - 8.44 (m, 2H) 8.59 (d, J = 7.08 Hz, 1H) 9.27 (s, 1H) 9.53 (s, 1H) 11.30 (br. s., 1H) | C |
| 235 | 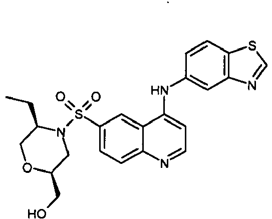 | ((2S,5R)-4-{[4-(1,3-benzothiazol-5-ylamino)-6-quinolinyl] sulfonyl}-5-ethyl-2-morpholinyl) methanol | 485 | ¹H NMR (500 MHz, DMSO-d₆) δ 0.86 (t, J = 7.32 Hz, 3H) 1.23 (br. s., 1H) 1.53 - 1.69 (m, 2H) 2.92 - 3.10 (m, 2H) 3.25-3.32(m, 1H) 3.60 - 3.83 (m, 3H) 4.74 (br. s., 1 H) 7.01 (d, J = 6.10 Hz, 1H) 7.59 (dd, J = 8.55, 1.71 Hz, 1H) 8.07 - 8.24 (m; 3H) 8.32 (d, J = 8.55 Hz, 1H) 8.58 (d, J = 6.10 Hz, 1H) 9.18 (s, 1H) 9.49 (s, 1H) 10.57 (br. s., 1H) | C |
| 236 | 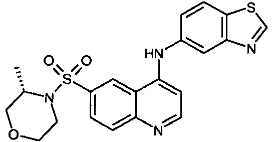 | N-1,3-benzothiazol-5-yl-6-{[(3S)-3-methyl-4-morpholinyl] sulfonyl}-4-quinolinamine | 441 | ¹H NMR (400 MHz, DMSO-d₆) δ 1.10 (d, 3H) 3.22-3.30 (m, 2H) 3.38 (dd, J = 11.49, 2.91 Hz, 1H) 3.54 (d, J = 11.37 Hz, 2H) 3.72-3.82 (m, 1H) 3.94 (d, J = 6.57 Hz, 1H) 7.07 (d, J = 5.31 Hz, 1H) 7.53 - 7.62 (m, 1H) 7.95 - 8.11 (m, 3H) 8.23 (d, J = 8.59 Hz, 1H) 8.59 (d, J = 5.30 Hz, 1H) 9.01 (s, 1H) 9.45 (s, 1H) 9.69 (s, 1H) | C |
| 237 | 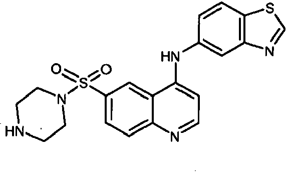 | N-1,3-benzothiazol-5-yl-6-(1-piperazinyl sulfonyl)-4-quinolinamine hydrochloride | 426 | ¹H NMR (400 MHz, DMSO-d₆) δ 3.33 (d, J = 5.05 Hz, 3H) 3.92 (br. s., 4H) 3.92 (m, 4H) 6.99 (d, J = 7.07 Hz, 1H) 7.65 (dd, J = 8.59, 2.02 Hz, 1H) 8.27 (d, J = 1.77 Hz, 1H) 8.29 - 8.38 (m, 1H) 8.43 (d, J = 8.59 Hz, 1H) 8.63 (d, J = 7.07 Hz, 1H) 9.20 (br. s., 1H) 9.36 (d, J = 1.26 Hz, 1H) 9.56 (s, 1H) 11.76 (br. s., 1H) | C |
| 238 | 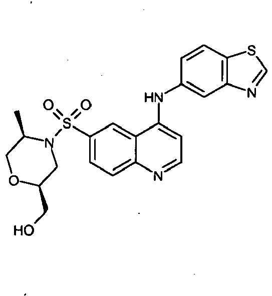 | ((2S,5R)-4-{[4-(1,3-benzothiazol-5-ylamino)-6-quinolinyl] sulfonyl}-5-methyl-2-morpholinyl) methanol | 471 | ¹H NMR (500 MHz, DMSO-d₆) δ 1.07 (d, J = 6.84 Hz, 3H) 2.95 (dd, J = 12.82, 11.35 Hz, 1H) 3.24 (d, 1H) 3.36 - 3.53 (m, 4H) 3.56 - 3.75 (m, 2H) 4.02 (d, J = 7.08 Hz, 1H) 4.70 - 4.85 (m, 1H) 7.03 (d, J = 5.62 Hz, 1H) 7.58 (d, J = 8.30 Hz, 1H) 8.10 (d, J = 14.65 Hz, 3H) 8.28 (d, J = 8.55 Hz, 1H) 8.31 - 8.42 (m, 1H) 8.58 (d, J = 5.61 Hz, 1H) 9.10 (s, 1H) 9.47 (s, 1H) 10.19 (br. s., 1H) | C |
| 239 | 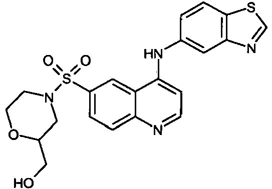 | (4-{[4-(1,3-benzothiazol-5-ylamino)-6-quinolinyl] sulfonyl}-2-morpholinyl) methanol | 457 | ¹H NMR (400 MHz, DMSO-d₆) δ 2.20 (t, J = 10.92 Hz, 1H) 2.36 - 2.46 (m, 1H) 2.53 - 2.63 (m, 1H) 3.17 (s, 1H) 3.46 - 3.64 (m, 3H) 3.69 (d, J = 11.04 Hz, 1H) 3.92 (dd, J = 11.42, 2.38 Hz, 1H) 4.85 (br. s., 1H) 7.00 (d, J = 6.78 Hz, 1H) 7.63 (dd, J = 8.53, 2.01 . Hz, 1H) 8.11 - 8.26 (m, 3H) 8.39 (d, J = 8.53 Hz, 1H) 8.61 (d, J = 6.78 Hz, 1H) 9.20 (s, 1H) 9.54 (s, 1H) 11.25 (br. s., 1H) | C |
| 240 | 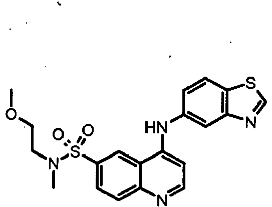 | 4-(1,3-benzothiazol-5-ylamino)-N-methyl-N-[2-(methyloxy)ethyl] -6-quinoline sulfonamide | 429 | ¹H NMR (DMSO-d₆) δ: 9.68 (s, 1H), 9.45 (s, 1H), 8.97 (d, J = 1.5 Hz, 1H), 8.59 (d, J = 5.3 Hz, 1H), 8.24 (d, J = 8.6 Hz, 1H), 8.02 - 8.10 (m, 2H), 7.98 (dd, J = 8.8, 2.0 Hz, 1H), 7.57 (dd, J = 8.6, 2.0 Hz, 1H), 7.06 (d, J = 5.6 Hz, 1H), 3.49 (t, J = 5.6 Hz, 2H), 3.20 - 3.27 (m, 5H), 2.80 (s, 3H) | B |
| 241 | 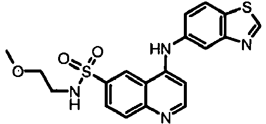 | 4-(1,3-benzothiazol-5-ylamino)-N-[2-(methyloxy)ethyl] -6-quinoline sulfonamide | 429 | ¹H NMR (DMSO-d⁶) δ: 9.65 (s, 1H), 9.44 (s, 1H), 8.95 (d, J = 1.3 Hz, 1H), 8.58 (d, J = 5.6 Hz, 1H), 8.22 (d, J = 8.6 Hz, 1H), 7.98 - 8.11 (m, 3H), 7.85 (t, J = 5.8 Hz, 1H), 7.56 (dd, J = 8.6, 2.0 Hz, 1H), 7.08 (d, J = 5.3 Hz, 1H), 3.26 - 3.39 (m, 2H), 3.16 (s, 3H), 2.98 (q, 2H) | B |
| 242 | 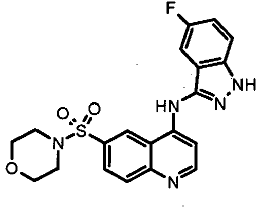 | N-(5-fluoro-1H-indazol-3-yl)-6-(4-morpholinyl sulfonyl)-4-quinolinamine | 428 | ¹H NMR(DMSO-d₆) δ: 15.23 (br. s., 1H), 13.60 (s, 1H), 11.69 (br. s., 1H), 9.36 (s, 1H), 8.71 (d, J = 7.1 Hz, 1H), 8.36 (d, J = 9.1 Hz, 1 H), 8.30 (dd, J = 8.8, 1.8 Hz, 1H), 7.71 (dd, J = 9.2, 4.2 Hz, 1H), 7.62 (dd, J = 9.0, 2.4 Hz, 1H), 7.40 (td, J = 9.1, 2.5 Hz, 1H), 7.25 (d, J = 7.1 Hz, 1H), 3.63 - 3.74 (m, 4H), 2.98 - 3.09 (m, 4H) | E |
| 243 | 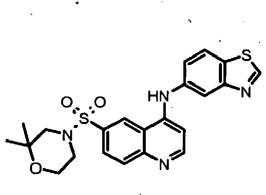 | N-1,3-benzothiazol-5-yl-6-[(2,2-dimethyl-4-morpholinyl) sulfonyl]-4-quinolinamine | 455 | ¹H NMR (400 MHz, DMSO-d₆) δ 1.16 - 1.24 (m, 6 H), 2.80 (s, 2 H), 2.91 - 3.01 (m, 2 H), 3.69 - 3.77 (m, 2 H), 7.03 (d, *J* = 6.27 Hz, 1 H), 7.60 (dd, *J* = 8.53, 2.01 Hz, 1 H), 8.04 - 8.23 (m, 3 H), 8.33 (d, *J* = 8.53 Hz, 1 H), 8.61 (d, *J* = 6.27 Hz, 1 H), 9.07 (s, 1 H), 9.51 (s, 1 H) | B |
| 244 | 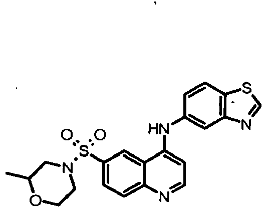 | N-1,3-benzothiazol-5-yl-6-[(2-methyl-4-morpholinyl) sulfonyl]-4-quinolinamine | 441 | ¹H NMR (400 MHz, DMSO-d₆) δ 1.07 (d, 3 H), 1.96 - 2.18 (m, 1 H), 2.27 - 2.46 (m, 1 H), 3.46 - 3.70 (m, 4 H), 3.87 (dd, *J* = 11.29, 2.26 Hz, 1 H), 7.03 (d, *J* = 6.02 Hz, 1 H), 7.60 (dd, *J* = 8.53, 2.01 Hz, 1 H), 8.07-8.19(m,3H),8.33 (d, *J* = 8.78 Hz, 1 H), 8.61 (d, *J* = 6.02 Hz, 1 H), 9.08 (s, 1 H), 9.50 (s, 1 H), 10.58 (br. s., 1 H) | B |
| 245 | 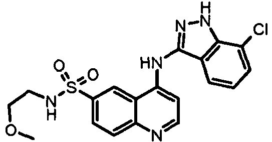 | 4-[(7-chloro-1H-indazol-3-yl)amino]-N-[2-(methyloxy)ethyl] -6-quinoline sulfonamide | 432 | ¹H NMR (400 MHz, DMSO-d₆) δ 2.99 (t, *J* = 5.81 Hz , 2 H), 3.15 (s, 3 H), 3.32 (t, *J* = 5.81 Hz, 2 H), 7.01 (br. s., 1 H), 7.38 (d, J = 6.57 Hz, 2 H), 7.71 (br. s., 1 H), 7.95 (s, 2 H), 8.46 (br. s., 1 H), 9.16 (br. s., 1 H) | B |

### Example 246

### N-1,3-Benzothiazol-5-yl-N-[6-(methylsulfonyl)-4-quinolinyl]acetamide

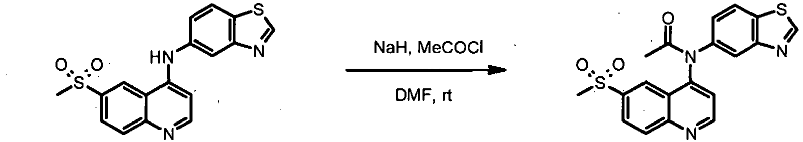

To a solution of N-1,3-benzothiazol-5-yl-6-(methylsulfonyl)-4-quinolinamine (60 mg, 0.15 mmol) in DMF (1 mL) was added sodium hydride (15 mg, 0.38 mmol). After 10 min, acetyl chloride (27 µL, 0.38 mmol) in DMF (1 mL) was added and the reaction was stirred at room temperature overnight. Water was added (0.5 mL), the reaction was passed through a syringe filter, and the solution was purified by RP HPLC to afford the title compound (47 mg, 77 %). ¹H NMR (400 MHz, DMSO-d₆) δ 9.48 (br. s., 1H), 9.16 (br. s., 1H), 8.69 (d, *J* = 2.0 Hz, 1H), 8.15 - 8.54 (m, 5H), 7.55 - 7.89 (m, 1H), 3.39 (s, 3H), 2.19 (br. s., 3H); MS (m/z) 398 (M+H⁺).

The following compound was prepared using procedures analogous to that described above

| Example | Structure | Name | MS (M+H)⁺ | NMR |
|---|---|---|---|---|
| 247 | 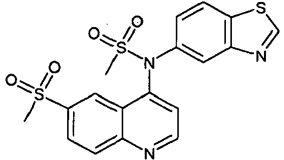 | N-1,3-benzothiazol-5-yl-N-[6-(methylsulfonyl)-4-quinolinyl]methane sulfonamide | 434 | ¹H NMR (400 MHz, DMSO-d6) δ 9.49 (s, 1H), 9.28 (d, J = 4.8 Hz, 1H), 8.87 (d, J = 2.0 Hz, 1H), 8.15 - 8.45 (m, 5H), 7.75 (dd, J = 8.7, 2.1 Hz, 1H), 3.53 (s, 3H), 3.35 (s, 3H) |

### Example 248

### N-1,3-Benzoxazol-5-yl-6-(methylsulfonyl)-4-quinolinamine

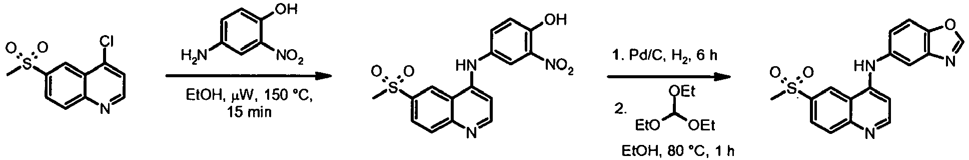

Step 1. 4-{[6-(Methylsulfonyl)-4-quinolinyl]amino}-2-nitrophenol: 4-Chloroquinoline (2.0 g, 8.3 mmol), 4-amino-2-nitrophenol (1.3 g, 8.3 mmol), and ethanol (17 ml) were added to a vial which was capped and heated to 150 °C for 15 minutes via microwave. The reaction mixture was poured into 300 mL of ether and the suspension was filtered. The cake was rinsed with ether and air dried to provide the desired product (2.91 g, 87 %). ¹H NMR (400 MHz, DMSO-d₆) δ 11.60 (br. s., 1H), 11.53 (br. s., 1H), 9.46 (d,J= 1.8 Hz, 1H), 8.63 (d, *J* = 7.1 Hz, 1H), 8.46 (dd,*J* = 8.8, 1.8 Hz, 1H), 8.29 (d, *J* = 9.1 Hz, 1H), 8.05 (d, *J* = 2.8 Hz, 1H), 7.69 (dd, *J* = 8.8, 2.5 Hz, 1H), 7.40 (d, *J* = 8.8 Hz, 1H), 6.92 (d, *J* = 7.1 Hz, 1H), 3.43 (s, 3H); MS (m/z) 360.1 (M+H⁺).

Step 2. *N*-1,3-Benzoxazol-5-yl-6-(methylsulfonyl)-4-quinolinamine: To a flask was added 4-{[6-(methylsulfonyl)-4-quinolinyl]amino}-2-nitrophenol (2.9 g, 8.1 mmol), Pd/C (0.86 g, 0.81 mmol), and ethanol (81 ml). The flask was placed on a Parr shaker at 40 psi for 4 hours. It was not complete and was placed back on the shaker at 50 psi for an additional 2 hours. The reaction mixture was filtered through celite eluting with MeOH. The mother liquor was concentrated to the aminophenol (2.7 g, 91 %). MS (m/z) 330.1 (M+H⁺). The aminophenol (2.7 g, 7.4 mmol) was taken up in ethanol (73 ml) before triethyl orthoformate (1.2 ml, 7.4 mmol) was added and the reaction was heated to 80 °C. After 1h the reaction mixture was cooled to room temperature and concentrated. The residue was dissolved in DCM and concentrated onto silica gel. The dry load was purified by flash chromatography (0 -> 10 % 2M NH₃/MeOH in DCM). Desired fractions were combined and concentrated to a red-brown solid which was dried in a vacuum oven at 40 °C overnight (970 mg, 38 %). ¹H NMR (400 MHz, DMSO-d₆) δ 9.63 (s, 1H), 9.11 (d, *J* = 1.8 Hz, 1H), 8.81 (s, 1H), 8.56 (d,J= 5.6 Hz, 1H), 8.13 (dd, *J* = 8.8,2.0 Hz, 1H), 8.06 (d, *J* = 8.8 Hz, 1H), 7.87 (d, *J* = 8.6 Hz, 1H), 7.79 (d, *J* = 1.8 Hz, 1H), 7.47 (dd, *J* = 8.8, 2.0 Hz, 1H), 6.90 (d, *J* = 5.3 Hz, 1H), 3.34 (s, 3H); MS (m/z) 340.1 (M+H⁺).

The following compounds were prepared using procedures analogous to those described above.

| Example | Structure | Name | MS (M+H)⁺ | NMR |
|---|---|---|---|---|
| 249 | 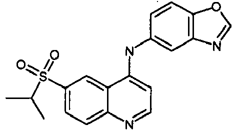 | N-1,3-benzoxazol-5-yl-6-[(1-methylethyl)sulfonyl]-4-quinolinamine | 368 | ¹H NMR (400 MHz, DMSO-d₆) δ 9.66 (s, 1H), 9.07 (s, 1H), 8.81 (s, 1H), 8.57 (d, J = 5.3 Hz, 1H), 8.00 - 8.14 (m, 2H), 7.87 (d, J = 8.6 Hz, 1H), 7.81 (d, J = 2.0 Hz, 1H), 7.48 (dd, J = 8.6, 2.0 Hz, 1H), 6.90 (d, J = 5.6 Hz, 1H), 3.54 (spt, 1H), 1.23 (d, J = 6.8 Hz, 6H) |
| 250 | 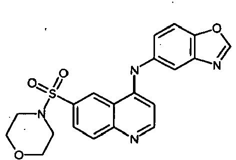 | N-1,3-benzoxazol-5-yl-6-(4-morpholinylsulfonyl)-4-quinolinamine | 411 | ¹H NMR (400 MHz, DMSO-d₆) δ 9.64 (s, 1H), 8.94 (d, J = 1.5 Hz, 1H), 8.81 (s, 1H), 8.56 (d, J = 5.6 Hz, 1H), 8.07 (d, J = 8.8 Hz, 1H), 7.93 (dd, J = 8.8, 1.8 Hz, 1H), 7.87 (d, J = 8.6 Hz, 1H), 7.81 (d, J = 1.8 Hz, 1H), 7.48 (dd, J = 8.6, 2.0 Hz, 1H), 6.89 (d, J = 5.6 Hz, 1H), 3.62 - 3.71 (m, 4H), 2.92 - 3.01 (m, 4H) |

### Example 251

N-[4-chloro-3-(methyloxy)phenyl]-6-(tetrahydro-2H-pyran-4-ylsulfonyl)-4-quinolinamine

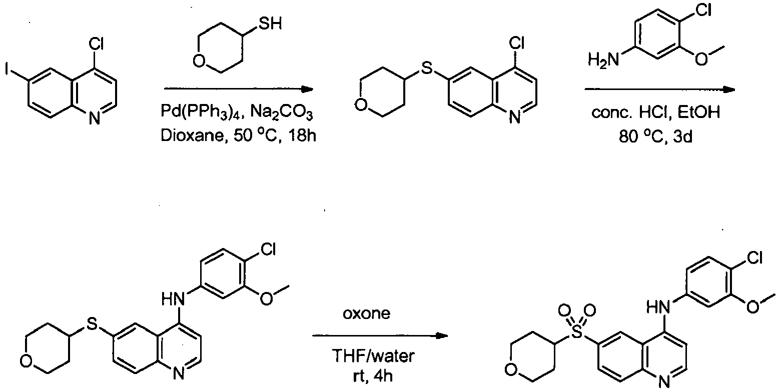

Step 1: 4-chloro-6-(tetrahydro-2H-pyran-4-ylthio)quinoline: 4-chloro-6-iodoquinoline (1.5 g, 5.18 mmol), sodium carbonate (2.317 g, 12.95 mmol), 1,4-dioxane (51.8 ml) and tetrakis (0.299 g, 0.259 mmol) were added to microwave vial and purged with nitrogen for 10 min. Tetrahydro-2H-pyran-4-thiol (0.643 g, 5.44 mmol) was added and the reaction was heated at 70 °C for 48h. The reaction was partitioned between ethyl acetate and a solution of aqueous sodium thiosulfate/sodium bicarbonate (5:1, 2M). The aqueous layer was extracted with ethyl acetate (1x) and the combined organic extracts were dried over magnesium sulfate, filtered and dry-loaded onto silica. The crude product was purified via column chromatography (ISCO-Rf,120g column, 0-15% methanol/DCM) to afford 4-chloro-6-(tetrahydro-2H-pyran-4-ylthio)quinoline (1.25 g, 3.89 mmol, 75 % yield). ¹H NMR (400 MHz, DMSO-d₆) δ ppm 8.81 (d, *J*=4.8 Hz, 1 H), 8.03 - 8.11 (m, 2 H), 7.89 (dd, *J*=8.8, 2.0 Hz, 1 H), 7.79 (d, *J*=4.8 Hz, 1 H), 3.70 - 3.92 (m, 3 H), 3.46 (td, *J*=11.2, 2.4 Hz, 2 H), 1.85 - 1.99 (m, 2 H), 1.48 - 1.66 (m, 2 H). MS (m/z) 280 (M+H)+.

Step 2: N-[4-chloro-3-(methyloxy)phenyl]-6-(tetrahydro-2H-pyran-4-ylthio)-4-quinolinamine: A mixture of 4-chloro-6-(tetrahydro-2H-pyran-4-ylthio)quinoline (1.4 g, 5.00 mmol), 4-chloro-3-(methyloxy)aniline (0.789 g, 5.00 mmol) and ethanol (16.68 ml) was treated with concentrated HCl (1 drop) and at 80 °C for 3d. The reaction was cooled to rt, poured into diethylether (300 mL) and filtered to provide crude product (1g). The material was partitioned between ethyl acetate and sat. sodium bicarbonate. The aqueous layer was extracted with ethyl acetate (1x) and the combined organic extracts were washed with brine (1x), dried over magnesium sulfate, dry-loaded onto silica gel and purified via column chromatography (ISCO-Rf, 40g column, 0-10% methanol/DCM) to afford N-[4-chloro-3-(methyloxy)phenyl]-6-(tetrahydro-2H-pyran-4-ylthio)-4-quinolinamine (700 mg, 1.746 mmol, 34.9 % yield) as a brown oil. ¹H NMR (400 MHz, DMSO-*d*₆) δ ppm 9.07 (s, 1 H), 8.48 (d, *J*=5.3 Hz, 1 H), 8.41 (d, *J*=1.8 Hz, 1 H), 7.85 (d, *J*=8.8 Hz, 1 H), 7.70 - 7.79 (m, 1 H), 7.43 (d, *J*=8.6 Hz, 1 H), 7.12 (d, *J*=2.3 Hz, 1 H), 7.06 (d, *J*=5.3 Hz, 1 H), 6.96 (dd, *J*=8.5, 2.4 Hz, 1 H), 3.81 - 3.90 (m, 5 H), 3.63 - 3.67 (m, 1 H), 3.40 (td, *J*=11.2, 2.4 Hz, 2 H), 1.84 - 1.94 (m, 2 H), 1.48 - 1.62 (m, 2 H). MS (m/z) 401(M+H)+.

Step 3: N-[4-chloro-3-(methyloxy)phenyl]-6-(tetrahydro-2H-pyran-4-ylsulfonyl)-4-quinolinamine: A mixture of N-[4-chloro-3-(methyloxy)phenyl]-6-(tetrahydro-2H-pyran-4-ylthio)-4-quinolinamine (150 mg, 0.374 mmol), oxone (253 mg, 0.412 mmol), and tetrahydrofuran (THF) (3741 µl) was stirred at room temperature. After 2h the reaction was 3:1 SM/sulfoxide. Water (1 mL) was added and the reaction was stirred 2h and partitioned between ethyl acetate and saturated aqueous sodium bicarbonate. The aqueous layer was extracted with ethyl acetate (1x) and the combined organic extracts were washed with brine, dried over magnesium sulfate, filtered, dry-loaded onto silica gel and purified via column chromatography (ISCO-Rf, 12g, 0-10% methanol/DCM) to afford N-[4-chloro-3-(methyloxy)phenyl]-6-(tetrahydro-2H-pyran-4-ylsulfonyl)-4-quinolinamine (90 mg, 0.206 mmol, 55.0 % yield). 1H NMR (400 MHz, DMSO-d6) δ ppm 9.59 (s, 1 H), 9.00 (d, J=1.5 Hz, 1 H), 8.63 (d, J=5.3 Hz, 1 H), 7.96 - 8.14 (m, 2 H), 7.46 (d, J=8.6 Hz, 1 H), 7.09 - 7.22 (m, 2 H), 7.01 (dd, J=8.3, 2.3 Hz, 1 H), 3.89 - 3.96 (m, 2 H), 3.88 (s, 3 H), 3.57 - 3.68 (m, 1 H), 3.25 - 3.31 (m, 2 H), 1.75 (br. s., 2 H), 1.63 (dd, J=12.6, 4.8 Hz, 2 H). MS (m/z) 433 (M+H)+.

### Example 252

### N-[4-chloro-3-(methyloxy)phenyl]-6-(tetrahydro-2H-pyran-4-ylsulfinyl)-4-quinolinamine

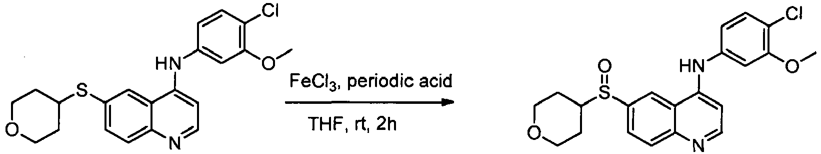

A mixture of N-[4-chloro-3-(methyloxy)phenyl]-6-(tetrahydro-2H-pyran-4-ylthio)-4-quinolinamine (190 mg, 0.474 mmol), iron(III) chloride (2.306 mg, 0.014 mmol), and tetrahydrofuran (THF) (1185 µl) was stirred at room temperature for 5 min and periodic acid (119 mg, 0.521 mmol) was added. After 2h, the reaction was ∼75% complete (over-oxidation was beginning to occur). The reaction was partitioned between DCM and 2M 5:1 Na2S2O3/NaHCO3. The aqueous layer was extracted with DCM (1x) and the combined organic extracts were washed with brine, dried over magnesium sulfate, dry-loaded onto silica gel and purified via column chromatography (ISCO-Rf, 12g, 0-10% MeOH/DCM) to afford N-[4-chloro-3-(methyloxy)phenyl]-6-(tetrahydro-2H-pyran-4-ylsulfinyl)-4-quinolinamine (118 mg, 0.283 mmol, 59.7 % yield). 1H NMR (400 MHz, DMSO-d6) δ ppm 9.35 (s, 1 H), 8.64 (d, J=1.8 Hz, 1 H), 8.58 (d, J=5.3 Hz, 1 H), 8.05 (d, J=8.8 Hz, 1 H), 7.89 (dd, J=8.8, 1.8 Hz, 1 H), 7.44 (d, J=8.3 Hz, 1 H), 7.10 - 7.20 (m, 2 H), 7.00 (dd, J=8.6, 2.3 Hz, 1 H), 3.87 (m, 5 H), 3.28 (m, 2 H), 3.09 - 3.20 (m, 1 H), 1.50 - 1.83 (m, 4 H). MS (m/z) 417 (M+H)+.

### Pharmaceutical Compositions

### Example A

Tablets are prepared using conventional methods and are formulated as follows:

| Ingredient | Amount per tablet |
|---|---|
| Compound of Example 1 | 5mg |
| Microcrystalline cellulose | 100mg |
| Lactose | 100mg |
| Sodium starch glycollate | 30mg |
| Magnesium stearate | 2mg |
| Total | 237mg |

### Example B

Capsules are prepared using conventional methods and are formulated as follows:

| Ingredient | Amount per tablet |
|---|---|
| Compound of Example 3 | 15mg |
| Dried starch | 178mg |
| Magnesium stearate | 2mg |
| Total | 195mg |

### Biological Assay:

A fluorescent polarization based binding assay was developed to quantitate interaction of novel test compounds at the ATP binding pocket of RIPK2, by competition with a fluorescently labeled ATP competitive ligand. Full length FLAG His tagged RIPK2 was purified from a Baculovirus expression system and was used at a final assay concentration of twice the KDapparent. A fluorescent labeled ligand (5-({[2-({[3-({4-[(5-hydroxy-2-methylphenyl)amino]-2-pyrimidinyl}amino)phenyl]carbonyl}amino)ethyl] amino}carbonyl)-2-(6-hydroxy-3-oxo-3H-xanthen-9-yl)benzoic acid, prepared as described below) was used at a final assay concentration of 5nM. Both the enzyme and ligand were prepared in solutions in 50mM HEPES pH7.5, 150mM NaCl, 10mM MgCl2, 1mM DTT, and 1mM CHAPS. Test compounds were prepared in 100% DMSO and 100nL was dispensed to individual wells of a multiwell plate. Next, 5ul RIPK2 was added to the test compounds at twice the final assay concentration, and incubated at room temperature for 10 minutes. Following the incubation, 5ul of the fluorescent labeled ligand solution, was added to each reaction, at twice the final assay concentration, and incubated at room temperature for at least 10 minutes. Finally, samples were read on an instrument capable of measuring fluorescent polarization. Test compound inhibition was expressed as percent (%) inhibition of internal assay controls.

For concentration response experiments, normalized data were fit and pIC₅₀s determined using conventional techniques. For example, the following four parameter logistic equation may be used: y = A + ((B-C))/(1+(10^{x})/(10^{C})^{D}), where: y is the % activity (% inhibition) at a specified compound concentration; A is the minimum % activity; B is the maximum % activity; C = log₁₀(IC₅₀); D= Hill slope; x = log₁₀ (compound concentration [M]); and pIC₅₀ = (-C).

The pIC₅₀s are averaged to determine a mean value, for a minimum of 2 experiments. As determined using the above method, each of the compounds of Examples 1-252 exhibited a pIC₅₀ greater than 6.0. For instance, the compound of Example 12 inhibited RIP2 kinase in the above method with a mean pIC₅₀ of 6.4 and the compounds of Examples 75 and 106 each inhibited RIP2 kinase in the above method with a mean pIC₅₀ of 6.9.

### FLAG His tagged RIPK2 Preparation:

Full-length human RIPK2 (receptor-interacting serine-threonine kinase 2) cDNA was purchased from Invitrogen (Carlsbad, California, USA, Clone ID:IOH6368, RIPK2-pENTR 221). Gateway® LR cloning was used to site-specifically recombine RIPK2 downstream to an N-terminal FLAG-6His contained within the destination vector pDEST8-FLAG-His6 according to the protocol described by Invitrogen. Transfection into *Spodoptera frugiperda*(*Sf9*) insect cells was performed using Cellfectin® (Invitrogen), according to the manufacturer's protocol.

Sf9 cells were grown in Excell 420 (SAFC Biosciences, Lenexa, Kansas, US; Andover, Hampshire UK) growth media at 27°C, 80 rpm in shake flask until of a sufficient volume to inoculate a bioreactor. The cells were grown in a 50 litre working volume bioreactor (Applikon, Foster City, California, US; Schiedam, Netherlands) at 27°C, 30% dissolved oxygen and an agitation rate of 60-140 rpm until the required volume was achieved with a cell concentration of approximately 3.7xe6 cells/ml. The insect cells were infected with Baculovirus at a multiplicity of infection (MOI) of 12.7. The cultivation was continued for a 43 hour expression phase. The infected cells were removed from the growth media by centrifugation at 2500 g using a Viafuge (Carr) continuous centrifuge at a flow rate of 80 litres/hour. The cell pellet was immediately frozen and subsequently supplied for purification.

9.83 x 10¹⁰ Insect cells were re-suspended in 1.4 L lysis buffer (50mM Tris (pH 8.0), 150mM NaCl, 0.5mM NaF, 0.1% Triton X-100, 1mL/litre Protease Inhibitor Cocktail Set III (available from EMD Group; CalBiochem/Merck Biosciences, Gibbstown, New Jersey, US; Damstadt, Germany) and processed by dounce homogenization on ice. The suspension was then clarified by centrifugation at 47,900g for 2 hours, at 4°C. The lysate was decanted from the insoluble pellet and loaded at a linear flow rate of 16 cm/h onto a 55 mL FLAG-M2 affinity column (2.6 x 10.4 cm) that had been pre-equilibrated with 10 column volumes buffer A (50mM Tris (pH 8.0), 150mM NaCl, 0.5mM NaF, 1mL/litre Protease Inhibitor Cocktail Set III). The column was then washed with 15 column volumes buffer A, and eluted with 6 column volumes buffer B (buffer A + 150µg/mL 3X FLAG peptide) at a linear flow rate of 57 cm/h. Fractions identified by SDS-PAGE as containing protein of interest were dialyzed to remove the 3X FLAG peptide from the preparation against 5 L of Buffer A (not containing the Protease Inhibitor Cocktail) overnight, using 10 kDa MWCO SnakeSkin Pleated Dialysis Tubing. The purification process yielded 11.3 mg of total protein, with the RIPK2 present at 40% purity by gel densitometry scanning, and identity confirmed by peptide mass fingerprinting. The main contaminating proteins in the preparation were identified as lower molecular weight degraded species of RIPK2.

### Fluorescent Ligand Preparation:

### 2-Methyl-5-(2-propen-1-yloxy)aniline:

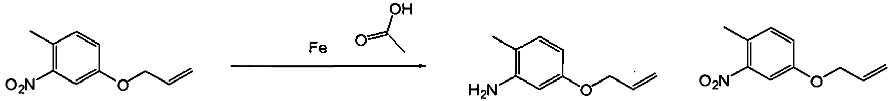

1-Methyl-2-nitro-4-(2-propen-1-yloxy)benzene (25.2 g, 130 mmol) was dissolved in ethanol (280 ml), water (28 ml), and acetic acid (5.6 ml, 98 mmol). Iron (29.1 g, 522 mmol) was added in six portions. The reaction was stirred for 72 hours, and then additional acetic acid (5.6 ml, 98 mmol) and 4 eq. of iron were added. The mixture was filtered through celite rinsing with EtOH and water and the filtrates were concentrated to remove EtOH. Diethylether (300 mL) was added along with 100 mL of 2 N HCl. The layers were separated and the ether layer was extracted with 2x100 mL of 2 N HCl. The acidic aqueous layer was slowly made pH 9 with NaOH pellets, and then dichloromethane (DCM, 300 mL) was added. The resulting emulsion was filtered using a Buchner funnel. The layers were separated and the aqueous layer extracted with DCM (2 X 100 mL). The combined extracts were dried over MgSO₄), filtered, and concentrated to a dark red oil (15.2 g). The crude material was purified via flash chromatography using a 120 g silica cartridge eluting with 5-15% EtOAc/hexanes for 30 min then 15-30% EtOAc/hexanes for 10 min. to give the titled compound as a red oil. MS (m/z) 1H NMR (400 MHz, CHLOROFORM-d) δ ppm 2.23 (s, 3 H) 4.51 (dt, J=5.29, 1.51 Hz, 2 H) 5.29 (dd, J=10.45, 1.38 Hz, 1 H) 5.38 - 5.46 (m, 1 H) 5.99 - 6.12 (m, 1 H) 6.01 - 6.10 (m, 1 H) 6.46 (dd, J=8.31, 2.52 Hz, 1 H) 6.56 (d, J=2.52 Hz, 1 H) 7.01 (d, J=8.56 Hz, 1 H); 164 (M+H+).

### 2-Chloro-N-[2-methyl-5-(2-propen-1-yloxy)phenyl]-4-pyrimidinamine:

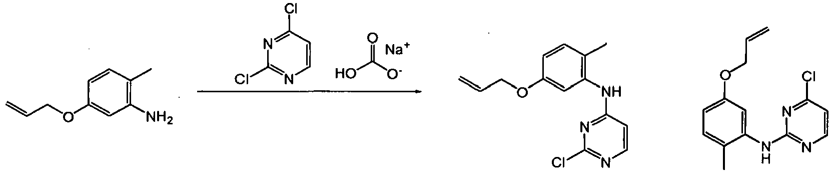

2-Methyl-5-(2-propen-1-yloxy)aniline (11.8 g, 72.3 mmol) was dissolved in tert-butanol (103 ml) and 2,4-dichloropyrimidine (10.77 g, 72.3 mmol) was added followed by sodium bicarbonate (18.22 g, 217 mmol). The reaction was heated at 80°C for 17 hrs then additional 1,4-dichloropyrimidine (5.38 g, 36.6 mmol) was added and the reaction was stirred for 6 days. Additional 2,4-dichloropyrimidine (2.69 g, 17.8 mmol) was added and the reaction stirred for 2 days. The reaction was cooled to room temp diluting with EtOAc (200 mL) and water (200 mL). The layers were separated and the aqueous layer extracted with EtOAc (2 X 100 mL). The combined extracts were washed with brine (100 mL), dried over Na₂SO₄, filtered, and concentrated. The crude material was purified via flash chromatography using a 330 g silica cartridge eluting with 1-20% EtOAc/hexanes for 30 min then 20% EtOAc/hexanes for 50 min to give the titled compound (15.1 g). ¹H NMR (400 MHz, CHLOROFORM-*d*) δ ppm 2.20 (s, 3 H) 4.54 (d, *J*=5.29 Hz, 2 H) 5.32 (dd, *J*=10.45, 1.38 Hz, 1 H) 5.42 (dd, *J*=17.37, 1.51 Hz, 1 H) 5.99 - 6.12 (m, 1 H) 6.35 (d, *J*=5.79 Hz, 1 H) 6.83 (dd, *J*=8.44, 2.64 Hz, 1 H) 6.89 (d, *J*=2.52 Hz, 6 H) 7.14 (br. s., 6 H) 7.21 (d, *J*=8.56 Hz, 7 H) 8.10 (d, *J*=5.79 Hz, 6 H); MS (m/z) 276 (M+H+).

### 3-[(4-{[2-Methyl-5-(2-propen-1-yloxy)phenyl]amino}-2-pyrimidinyl)amino]benzoic acid:

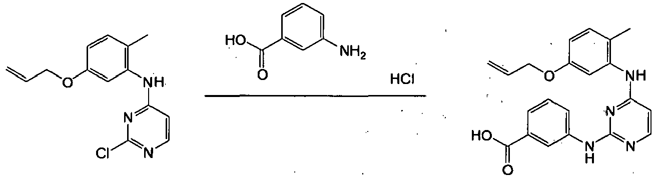

2-Chloro-N-[2-methyl-5-(2-propen-1-yloxy)phenyl]-4-pyrimidinamine (8 g, 29.0 mmol), 3-aminobenzoic acid (3.98 g, 29.0 mmol), and HCl (14.51 ml, 29.0 mmol) were dissolved in acetone (58.0 ml) and water (58.0 ml). The reaction was heated to 60°C for 48 hrs. The reaction was cooled to room temperature passing air over it and a solid crashed out. Water (150 mL) was added and the solid was filtered washing with 3 X 50 mL water. The solid was dried in the vacuum funnel overnight affording the desired compound (10.9 g). ¹H NMR (400 MHz, METHANOL-*d*₄) ppm 2.21 (s, 3 H) 4.47 (d, *J*=5.04 Hz, 2 H) 5.24 (dd, *J*=10.58, 1.51 Hz, 1 H) 5.37 (dd, *J*=17.25, 1.64 Hz, 1 H) 5.97 - 6.09 (m, 4 H) 6.29 - 6.39 (m, 1 H) 6.89 (dd, *J*=8.44, 2.64 Hz, 4 H) 6.96 (d, *J*=2.77 Hz, 1 H) 7.04 (none, 0 H) 7.23 (d, *J*=8.56 Hz, 1 H) 7.34 - 7.41 (m, 1 H) 7.75 - 7.79 (m, 1 H) 7.81 (s, 1 H) 7.85 (d, *J*=7.30 Hz, 3 H) 7.98 - 8.09 (m, 3 H); MS (m/z) 377 (M+H+).

### 1,1-Dimethylethyl {2-[({3-[(4-{[2-methyl-5-(2-propen-1-yloxy)phenyl]amino}-2-pyrimidinyl)amino]phenyl} carbonyl)amino]ethyl}carbamate:

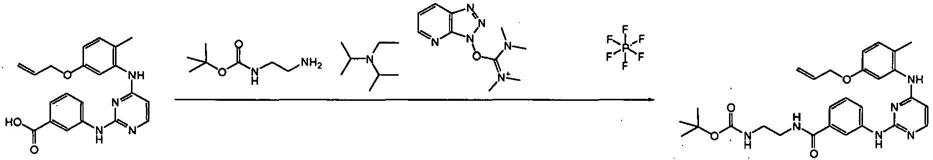

A solution of 3-[(4-{[2-methyl-5-(2-propen-1-yloxy)phenyl]amino}-2-pyrimidinyl)amino]benzoic acid (6.83 g, 18.15 mmol) and DIEA (9.51 ml, 54.4 mmol) in N,N-Dimethylformamide (DMF) (51.8 ml). was treated with N-(2-aminoethyl) carbamic acid tert-butyl ester (3.20 g, 19.96 mmol) and HATU (8.28 g, 21.77 mmol). EtOAc/Et₂O (400 mL, 1:1) was added and the layers separated. The organic layer was washed with water (3 X 300 mL) and brine (100 mL), dried over Na₂SO₄, filtered, and concentrated to give the titled compound (8.3 g). ¹H NMR (400 MHz, DMSO-*d*₆) δ ppm 1.38 (s, 9 H) 2.15 (s, 3 H) 3.09 (q, *J*=6.19 Hz, 2 H) 3.27 (q, *J*=6.19 Hz, 2 H) 4.51 (d, *J*=5.27 Hz, 2 H) 5.24 (dd, *J*=10.54, 1.51 Hz, 1 H) 5.37 (dd, *J*=17.32, 1.76 Hz, 1 H) 6.02 (m, *J=*17.29, 10.51, 5.18, 5.18 Hz, 1 H) 6.13 (d, *J*=5.77 Hz, 1 H) 6.73 (dd, *J*=8.41, 2.63 Hz, 1 H) 6.90 (t, *J*=5.65 Hz, 1 H) 7.09 (d, *J*=2.51 Hz, 1 H) 7.15 (d, *J*=8.28 Hz, 1 H) 7.17 - 7.22 (m, 1 H) 7.28 (d, *J*=7.78 Hz, 1 H) 7.94 - 7.99 (m, 2 H) 7.99 - 8.05 (m, 2 H) 8.26 (t, *J*=5.65 Hz, 1 H) 8.66 (s, 1 H) 9.17 (s, 1 H); MS (m/z) 519 (M+H+).

### 1,1-Dimethylethyl[2-({[3-({4-[(5-hydroxy-2-methylphenyl)amino]-2-pyrimidinyl}amino)phenyl]carbonyl} amino)ethyl]carbamate:

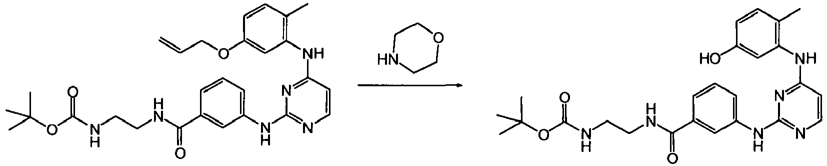

1,1-Dimethylethyl {2-[({3-[(4-{[2-methyl-5-(2-propen-1-yloxy)phenyl]amino}-2-pyrimidinyl)amino]phenyl}carbonyl)amino]ethyl}carbamate (5.5 g, 10.61 mmol) and morpholine (1.016 ml, 11.67 mmol) were dissolved in N,N-dimethylformamide (DMF) (42.4 ml) The atmosphere was exchanged for nitrogen and then it was treated with Tetrakis (1.226 g, 1.061 mmol). The reaction was heated to 80 °C for 3 hrs. The reaction was diluted with EtOAc (250 mL) and washed with water (3 X 200 mL) then brine (100 mL). The organic layer was dried over Na2SO4, filtered, and concentrated to about 50 mL and let stand overnight. A solid formed and to the suspension was added 50 mL ether. The solid was filtered washing with ether to give the desired product as an orange solid (4.75 g). ¹H NMR (400 MHz, METHANOL-*d*₄) δ ppm 1.42 (s, 9 H) 2.17 (s, 3 H) 3.29 (t, *J*=6.04 Hz, 2 H) 3.46 (t, *J*=6.17 Hz, 2 H) 6.04 (d, *J*=6.04 Hz, 1 H) 6.65 (dd, *J*=8.31, 2.52 Hz, 1 H) 6.87 (d, *J*=2.52 Hz, 1 H) 7.09 (d, *J*=8.31 Hz, 1 H) 7.27 - 7.33 (m, 1 H) 7.35 - 7.41 (m, 1 H) 7.53 - 7.61 (m, 1 H) 7.62 - 7.70 (m, 2 H) 7.75 (d, *J*=8.06 Hz, 1 H) 7.91 (d, *J*=6.04 Hz, 1 H) 8.11 (s, 1 H); MS (m/z) 479 (M+H+).

### N-(2-Aminoethyl)-3-({4-[(5-hydroxy-2-methylphenyl)amino]-2-pyrimidinyl}amino)benzamide:

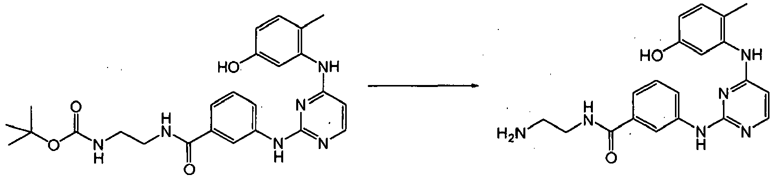

1,1-Dimethylethyl [2-({[3-({4-[(5-hydroxy-2-methylphenyl)amino]-2-pyrimidinyl}amino)phenyl]carbonyl}amino)ethyl]carbamate (4.75 g, 8.93 mmol) (contaminated with tetrakis or related entities) was dissolved in dichloromethane (DCM) (28.6 ml) and trifluoroacetic acid (TFA) (7.15 ml). The reaction concentrated to give the desired product as the TFA salt containing the same impurities going into the reaction (6.5 g) MS (m/z) 379 (M+H+).

### 5-({[2-({[3-({4-[(5-Hydroxy-2-methylphenyl)amino]-2-pyrimidinyl}amino)phenyl]carbonyl}amino)ethyl] amino}carbonyl)-2-(6-hydroxy-3-oxo-3H-xanthen-9-yl)benzoic acid:

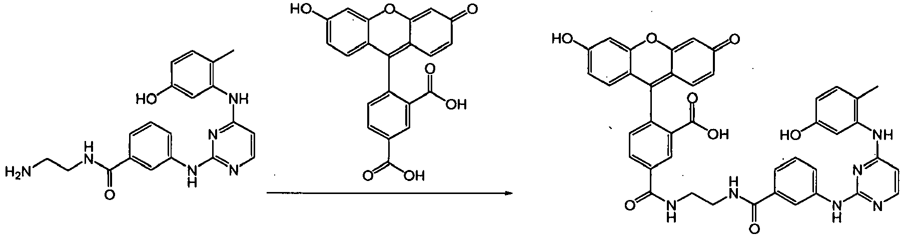

To a suspension of N-(2-aminoethyl)-3-({4-[(5-hydroxy-2-methylphenyl)amino]-2-pyrimidinyl}amino)benzamide(1 g, 1.319 mmol) in N,N-dimethylformamide (DMF) (13.19 ml) was added 5-FAM (5-carboxyfluorescein single isomer) (0.397 g, 1.055 mmol), triethylamine (0.919 ml, 6.60 mmol), EDC (0.506 g, 2.64 mmol), and HOBT (0.202 g, 1.319 mmol). The reaction was stirred overnight then the pH was adjusted to 3 with 2 N HCl. The solution was extracted with EtOAc (100 mL) and the organic layer washed with water (1 X 50 mL), dried over Na₂SO₄, filtered, and concentrated to give the titled compound. MS (m/z) 737 (M+H+).

### Biological in vivo Assay

The efficacy of the RIP2 inhibitors of this invention may also be *evaluated in vivo* in rodents. Intraperitoneal (*i.p.*) or intravenous (*i.v.*) administration of L18-MDP in mice has been shown to induce an inflammatory response through activation of the NOD2 signaling pathway (Rosenweig, H. L., et al. 2008. Journal of Leukocyte Biology 84:529-536). The level of the inflammatory response in the L18-MDP treated mice/rats is monitored using conventional techniques by measuring increases in cytokine levels (IL8, TNFα, IL6 and IL-1β) in serum and/or peritoneal lavage fluid and by measuring neutrophil influx into the peritoneal space (when L18-MDP is dosed *i.p*.). Inhibition of the L18-MDP induced inflammatory response in treated rodents may be shown by orally pre-dosing with selected compounds of this invention, then measuring and comparing cytokine levels (IL8, TNFα, IL6 and IL-1β) in serum and/or peritoneal lavage fluid and neutrophil influx into the peritoneal space (when L18-MDP is dosed *i.p*.) using conventional techniques.

## Claims

1. A compound according to Formula (I) 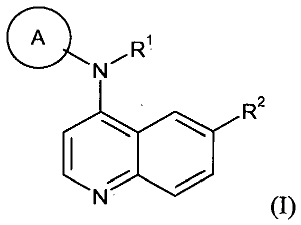 wherein:
R¹ is H, -SO₂(C₁-C₄alkyl), -CO(C₁-C₄alkyl), (C₁-C₄alkyl) or phenyl(C₁-C₄alkyl)-;
R² is -SR^{a}, -SOR^{a}, -SO₂R^{a}, -SO₂NH₂, -SO₂NR^{b}R^{c} or -CONR^{b}R^{c}, wherein
R^{a} is (C₁-C₆)alkyl, halo(C₁-C₄)alkyl, (C₂-C₆)alkenyl, (C₃-C₇)cycloalkyl, 4-7 membered heterocycloalkyl, aryl, or heteroaryl, wherein:
said (C₁-C₆)alkyl is optionally substituted by one or two groups each independently selected from cyano, hydroxyl, (C₁-C₆)alkoxy, (C₁-C₆)alkoxy(C₂-C₆)alkoxy, -CO₂H, -CO₂(C₁-C₄)alkyl, -SO₂(C₁-C₄ alkyl), -CONH₂, -CONH(C₁-C₄ alkyl), -CON(C₁-C₄ alkyl)(C₁-C₄ alkyl), -SO₂NH₂, -SO₂NH(C₁-C₄ alkyl), -SO₂N(C₁-C₄ alkyl)(C₁-C₄ alkyl), amino, (C₁-C₄ alkyl)amino-, (C₁-C₄ alkyl)(C₁-C₄ alkyl)amino-, C₃-C₇cycloalkyl, phenyl, 5-6 membered heteroaryl, 9-10 membered heteroaryl, 4-7 membered heterocycloalkyl and (phenyl)(C₁-C₄ alkyl)amino-, wherein said C₃-C₇cycloalkyl, phenyl, (phenyl)(C₁-C₄ alkyl)amino-, 5-6 membered heteroaryl, 9-10 membered heteroaryl or 4-7 membered heterocycloalkyl is optionally substituted by 1-3 groups each independently selected from halogen, -CF₃, (C₁-C₄)alkyl, hydroxy(C₁-C₄)alkyl and (C₁-C₄)alkoxy,
said (C₃-C₇)cycloalkyl or 4-7 membered heterocycloalkyl is optionally substituted by 1-3 groups each independently selected from halogen, -CF₃, hydroxyl, amino, (C₁-C₄)alkyl, phenyl(C₁-C₄)alkyl-, (C₁-C₄)alkoxycarbonyl-, hydroxy(C₁-C₄)alkyl-, oxo and (C₁-C₄)alkoxy, and
said aryl or heteroaryl is optionally substituted by 1-3 groups each independently selected from halogen, -CF₃, hydroxyl, amino, (C₁-C₄)alkyl, phenyl(C₁-C₄)alkyl-, hydroxy(C₁-C₄)alkyl- and (C₁-C₄)alkoxy;
R^{b} is (C₁-C₆)alkyl, (C₃-C₇)cycloalkyl, 4-7 membered heterocycloalkyl, aryl, or heteroaryl, wherein:
said (C₁-C₆)alkyl is optionally substituted by one or two groups each independently selected from cyano, hydroxyl, (C₁-C₆)alkoxy, (C₁-C₆)alkoxy(C₂-C₆)alkoxy, -CO₂H, -CO₂(C₁-C₄)alkyl, -SO₂(C₁-C₄ alkyl), -CONH₂, -CONH(C₁-C₄ alkyl), -CON(C₁-C₄ alkyl)(C₁-C₄ alkyl), -SO₂NH₂, -SO₂NH(C₁-C₄ alkyl), -SO₂N(C₁-C₄ alkyl)(C₁-C₄ alkyl), amino, (C₁-C₄ alkyl)amino-, (C₁-C₄ alkyl)(C₁-C₄ alkyl)amino-, C₃-C₇cycloalkyl, phenyl, 5-6 membered heteroaryl, 9-10 membered heteroaryl, 4-7 membered heterocycloalkyl and (phenyl)(C₁-C₄ alkyl)amino-, wherein said C₃-C₇cycloalkyl, phenyl, (phenyl)(C₁-C₄ alkyl)amino-, 5-6 membered heteroaryl, 9-10 membered heteroaryl or 4-7 membered heterocycloalkyl is optionally substituted by 1-3 groups each independently selected from halogen, -CF₃, (C₁-C₄)alkyl, hydroxy(C₁-C₄)alkyl and (C₁-C₄)alkoxy,
said (C₃-C₇)cycloalkyl or 4-7 membered heterocycloalkyl is optionally substituted by 1-3 groups each independently selected from halogen, -CF₃, hydroxyl, amino, (C₁-C₄)alkyl, phenyl(C₁-C₄)alkyl-, (C₁-C₄)alkoxycarbonyl-, hydroxy(C₁-C₄)alkyl-, oxo and (C₁-C₄)alkoxy, and
said aryl or heteroaryl is optionally substituted by 1-3 groups each independently selected from halogen, -CF₃, hydroxyl, amino, (C₁-C₄)alkyl, phenyl(C₁-C₄)alkyl-, hydroxy(C₁-C₄)alkyl- and (C₁-C₄)alkoxy;
R^{c} is H, (C₁-C₄)alkoxy or (C₁-C₆)alkyl;
or R^{b} and R^{c} taken together with the nitrogen atom to which they are attached form a 3-7 membered heterocycloalkyl or 5-6 membered heteroaryl group, optionally containing one or two additional ring heteroatoms each independently selected from nitrogen, oxygen and sulfur, wherein said 3-7 membered heterocycloalkyl or 5-6 membered heteroaryl is optionally substituted by 1-3 groups each independently selected from (C₁-C₄)alkyl, halo(C₁-C₄)alkyl, phenyl(C₁-C₄)alkyl-, hydroxyl, hydroxy(C₁-C₄)alkyl-, C₁-C₄ alkoxy, C₁-C₄ haloalkoxy, amino, (C₁-C₄ alkyl)amino-, (C₁-C₄ alkyl)(C₁-C₄ alkyl)amino-, -CO₂H, -CO₂(C₁-C₄)alkyl, -CO(C₁-C₄)alkyl, -CO(4-7 membered heterocycloalkyl), -CONH₂, -CONH(C₁-C₄alkyl), -CON(C₁-C₄alkyl)(C₁-C₄alkyl), -SO₂(C₁-C₄)alkyl, -SO₂(4-7 membered heterocycloalkyl), -SO₂NH₂, -SO₂NH(C₁-C₄alkyl) and -SO₂N(C₁-C₄alkyl)(C₁-C₄alkyl);
A is phenyl or aryl(C₁-C₄)alkyl-, substituted by R³, R⁴ and R⁵, wherein:
R³ is H, hydroxyl, halogen, -CF₃, hydroxy(C₁-C₄)alkyl, (C₁-C₄)alkyl or (C₁-C₄)alkoxy;
R⁴ is H, halogen, cyano, (C₁-C₄)alkyl, halo(C₁-C₄)alkyl, (C₁-C₄)alkoxy, phenoxy, phenyl(C₁-C₄)alkoxy, hydroxyl, hydroxy(C₁-C₄)alkyl-, or aminocarbonyl, wherein the phenyl moiety of said phenoxy or phenyl(C₁-C₄)alkoxy- is optionally substituted by 1-3 substituents each independently selected from halogen, -CF₃, (C₁-C₄)alkyl and (C₁-C₄)alkoxy; and
R⁵ is H, hydroxyl, halogen, -CF₃, hydroxy(C₁-C₄)alkyl, (C₁-C₄)alkyl or (C₁-C₄)alkoxy; or
A is phenyl or pyridinyl, substituted by R⁶, R⁷ and R⁸, wherein:
R⁶ and R⁷ are located on adjacent atoms and taken together with the atoms to which they are attached form a 5-membered heterocyclic group containing 1, 2 or 3 heteroatoms each independently selected from N, O and S, which 5-membered heterocyclic group is substituted by R⁹;
wherein one of R⁸ or R⁹ is H, halogen, cyano, (C₁-C₄)alkyl, halo(C₁-C₄)alkyl, (C₁-C₄)alkoxy, phenoxy, phenyl(C₁-C₄)alkoxy, hydroxyl, hydroxy(C₁-C₄)alkyl-, or aminocarbonyl, where the phenyl moiety of said phenoxy or phenyl(C₁-C₄)alkoxy is optionally substituted by 1-3 substituents each independently selected from halogen, -CF₃, (C₁-C₄)alkyl and (C₁-C₄)alkoxy; and
the other of R⁸ or R⁹ is H, hydroxyl, halogen, -CF₃, hydroxy(C₁-C₄)alkyl, (C₁-C₄)alkyl or (C₁-C₄)alkoxy; or
A is pyrazolyl, substituted by R¹⁰ and R¹¹, wherein:
R¹⁰ and R¹¹ are located on adjacent carbon atoms and taken together with the atoms to which they are attached form a 6 membered carbocyclic ring or heterocyclic ring substituted by R¹² and R¹³;
wherein R¹² is H, halogen, cyano, (C₁-C₄)alkyl, halo(C₁-C₄)alkyl, (C₁-C₄)alkoxy, phenoxy, phenyl(C₁-C₄)alkoxy, hydroxyl, hydroxy(C₁-C₄)alkyl-, or aminocarbonyl, wherein the phenyl moiety of said phenoxy or phenyl(C₁-C₄)alkoxy is optionally substituted by 1-3 substituents each independently selected from halogen, -CF₃, (C₁-C₄)alkyl and (C₁-C₄)alkoxy; and
R¹³ is H, hydroxyl, halogen, -CF₃, hydroxy(C₁-C₄)alkyl, (C₁-C₄)alkyl or (C₁-C₄)alkoxy;
provided that the compound is not:
2-fluoro-5[[6-(methylsulfonyl)-4-quinolinyl]amino-1,4-benzenediol;
N-(cyclopropylmethyl)-4-[(5-hydroxy-2-methylphenyl)amino]-6-quinolinesulfonamide;
4-methyl-3-[[6-(methylsulfonyl)-4-quinolinyl]amino]-phenol;
4-chloro-2-fluoro-5-[[6-(methylsulfonyl)-4-quinolinyl]amino]-phenol; or
4-[(5-hydroxy-2-methylphenyl)amino]-N-(1-methylethyl)-6-quinolinecarboxamide;
or a pharmaceutically acceptable salt thereof.

2. The compound or pharmaceutically acceptable salt according to claim 1, wherein:
R¹ is H, -SO₂(C₁-C₄alkyl), -CO(C₁-C₄alkyl), (C₁-C₄alkyl) or phenyl(C₁-C₄alkyl)-;
R² is -SR^{a}, -SOR^{a}, -SO₂R^{a}, -SO₂NH₂, -SO₂NR^{b}R^{c} or -CONR^{b}R^{c}, wherein
R^{a} or R^{b} is (C₁-C₆)alkyl, (C₃-C₇)cycloalkyl, 4-7 membered heterocycloalkyl, aryl, or heteroaryl, wherein:
said (C₁-C₆)alkyl is optionally substituted by one or two groups each independently selected from cyano, hydroxyl, (C₁-C₆)alkoxy, (C₁-C₆)alkoxy(C₂-C₆)alkoxy, -CO₂H, -CO₂(C₁-C₄)alkyl, -SO₂(C₁-C₄ alkyl), -CONH₂, -CONH(C₁-C₄ alkyl), -CON(C₁-C₄ alkyl)(C₁-C₄ alkyl), -SO₂NH₂, -SO₂NH(C₁-C₄ alkyl), -SO₂N(C₁-C₄ alkyl)(C₁-C₄ alkyl), amino, (C₁-C₄ alkyl)amino-, (C₁-C₄ alkyl)(C₁-C₄ alkyl)amino-, C₃-C₇cycloalkyl, phenyl, 5-6 membered heteroaryl, 9-10 membered heteroaryl, 4-7 membered heterocycloalkyl and (phenyl)(C₁-C₄ alkyl)amino-, wherein said C₃-C₇cycloalkyl, phenyl, (phenyl)(C₁-C₄ alkyl)amino-, 5-6 membered heteroaryl, 9-10 membered heteroaryl or 4-7 membered heterocycloalkyl is optionally substituted by 1-3 groups each independently selected from halogen, -CF₃, (C₁-C₄)alkyl, hydroxy(C₁-C₄)alkyl and (C₁-C₄)alkoxy,
said (C₃-C₇)cycloalkyl or 4-7 membered heterocycloalkyl is optionally substituted by 1-3 groups each independently selected from halogen, -CF₃, hydroxyl, amino, (C₁-C₄)alkyl, phenyl(C₁-C₄)alkyl-, hydroxy(C₁-C₄)alkyl-, oxo and (C₁-C₄)alkoxy, and
said aryl or heteroaryl is optionally substituted by 1-3 groups each independently selected from halogen, -CF₃, hydroxyl, amino, (C₁-C₄)alkyl, phenyl(C₁-C₄)alkyl-, hydroxy(C₁-C₄)alkyl- and (C₁-C₄)alkoxy;
R^{c} is H, (C₁-C₄)alkoxy or (C₁-C₆)alkyl;
or R^{b} and R^{c} taken together with the nitrogen atom to which they are attached form a 5-7 membered heterocycloalkyl or 5-6 membered heteroaryl group, optionally containing one additional ring heteroatom selected from nitrogen, oxygen and sulfur, wherein said 5-7 membered heterocycloalkyl or 5-6 membered heteroaryl is optionally substituted by 1-3 groups each independently selected from (C₁-C₄)alkyl, halo(C₁-C₄)alkyl, phenyl(C₁-C₄)alkyl-, hydroxy(C₁-C₄)alkyl-, C₁-C₄ alkoxy, C₁-C₄ haloalkoxy, amino, (C₁-C₄ alkyl)amino-, (C₁-C₄ alkyl)(C₁-C₄ alkyl)amino-, -CO₂H, -CO₂(C₁-C₄)alkyl, -CO(C₁-C₄)alkyl, -CO(4-7 membered heterocycloalkyl), -CONH₂, -CONH(C₁-C₄alkyl), -CON(C₁-C₄alkyl)(C₁-C₄alkyl), -SO₂(C₁-C₄)alkyl, -SO₂(4-7 membered heterocycloalkyl), -SO₂NH₂, -SO₂NH(C₁-C₄alkyl) and -SO₂N(C₁-C₄alkyl)(C₁-C₄alkyl);
A is phenyl or aryl(C₁-C₄)alkyl-, substituted by R³, R⁴ and R⁵, wherein:
R³ is H, hydroxyl, halogen, -CF₃, hydroxy(C₁-C₄)alkyl, (C₁-C₄)alkyl or (C₁-C₄)alkoxy;
R⁴ is H, halogen, cyano, (C₁-C₄)alkyl, halo(C₁-C₄)alkyl, (C₁-C₄)alkoxy, phenoxy, phenyl(C₁-C₄)alkoxy, hydroxyl, hydroxy(C₁-C₄)alkyl-, or aminocarbonyl, wherein the phenyl moiety of said phenoxy or phenyl(C₁-C₄)alkoxy- is optionally substituted by 1-3 substituents each independently selected from halogen, -CF₃, (C₁-C₄)alkyl and (C₁-C₄)alkoxy; and
R⁵ is H, hydroxyl, halogen, -CF₃, hydroxy(C₁-C₄)alkyl, (C₁-C₄)alkyl or (C₁-C₄)alkoxy; or
A is phenyl substituted by R⁶, R⁷ and R⁸, wherein:
R⁶ and R⁷ are located on adjacent atoms and taken together with the atoms to which they are attached form a 5-membered heterocyclic group containing 1,2 or 3 heteroatoms each independently selected from N, O and S, which 5-membered heterocyclic group is substituted by R⁹;
wherein one of R⁸ or R⁹ is H, halogen, cyano, (C₁-C₄)alkyl, halo(C₁-C₄)alkyl, (C₁-C₄)alkoxy, phenoxy, phenyl(C₁-C₄)alkoxy, hydroxyl, hydroxy(C₁-C₄)alkyl-, or aminocarbonyl, where the phenyl moiety of said phenoxy or phenyl(C₁-C₄)alkoxy is optionally substituted by 1-3 substituents each independently selected from halogen, -CF₃, (C₁-C₄)alkyl and (C₁-C₄)alkoxy; and
the other of R⁸ or R⁹ is H, hydroxyl, halogen, -CF₃, hydroxy(C₁-C₄)alkyl, (C₁-C₄)alkyl or (C₁-C₄)alkoxy; or
A is pyrazolyl, substituted by R¹⁰ and R¹¹, wherein:
R¹⁰ and R¹¹ are located on adjacent carbon atoms and taken together with the atoms to which they are attached form a 6 membered carbocyclic ring or heterocyclic ring substituted by R¹² and R¹³;
wherein R¹² is H, halogen, cyano, (C₁-C₄)alkyl, halo(C₁-C₄)alkyl, (C₁-C₄)alkoxy, phenoxy, phenyl(C₁-C₄)alkoxy, hydroxyl, hydroxy(C₁-C₄)alkyl-, or aminocarbonyl, wherein the phenyl moiety of said phenoxy or phenyl(C₁-C₄)alkoxy is optionally substituted by 1-3 substituents each independently selected from halogen, -CF₃, (C₁-C₄)alkyl arid (C₁-C₄)alkoxy; and
R¹³ is H, hydroxyl, halogen, -CF₃, hydroxy(C₁-C₄)alkyl, (C₁-C₄)alkyl or (C₁-C₄)alkoxy.

3. The compound or pharmaceutically acceptable salt according to claim 1 or claim 2, wherein R¹ is H.

4. The compound or pharmaceutically acceptable salt according to any one of claims 1-3, wherein R² is or -SO₂R^{a}.

5. The compound or pharmaceutically acceptable salt according to any one of claims 1-4, wherein R^{a} is (C₁-C₆)alkyl and said (C₁-C₆)alkyl is optionally substituted by 1 or 2 substituents each independently selected from hydroxyl; (C₁-C₂)alkoxy; (C₁-C₂)alkoxy(C₂-C₃)alkoxy-; amino; (C₁-C₃ alkyl)amino-; (C₁-C₃ alkyl)(C₁-C₂ alkyl)amino-; (phenyl)(C₁-C₂ alkyl)amino-; -CO₂(C₁-C₂)alkyl; -CONH₂; -SO₂(C₁-C₂)alkyl; and a C₃-C₆cycloalkyl, optionally substituted by (C₁-C₄)alkyl or hydroxy(C₁-C₄)alkyl; 4-6-membered heterocycloalkyl, optionally substituted by (C₁-C₄)alkyl; 5-6-membered heteroaryl, optionally substituted by (C₁-C₄)alkyl; phenyl; or 9-10-membered heteroaryl.

6. The compound or pharmaceutically acceptable salt according to any one of claims 1-4, wherein R^{a} is substituted 4-6-membered heterocycloalkyl, 5-6-membered heteroaryl or phenyl,
said 4-6-membered heterocycloalkyl is substituted by (C₁-C₄)alkyl, (C₁-C₄)alkoxycarbonyl-, or benzyl,
said 5-6-membered heteroaryl is substituted by (C₁-C₄)alkyl or hydroxy(C₁-C₄)alkyl, and
said phenyl is substituted by amino.

7. The compound or pharmaceutically acceptable salt according to any one of claims 1-4, wherein R^{a} is -CH₃, -CF₃, -CH₂CH₃, -CH₂CF₃, -CH₂CH₂CH₃, -CH₂CH=CH₂, -CH(CH₃)₂, -CH(CH₃)CH₂CH₃, -C(CH₃)₃, -CH₂CH₂OH, -CH₂CH₂OCH₃, -CH₂CH₂OCH₂CH₂OCH₃, -CH₂CH₂CH₂OH, -CH₂CH₂CH₂CH₂OH, -CH(CH₃)CH₂OH, -C(CH₃)₂CH₂OH, -C(CH₃)₂CO₂H, -C(CH₃)₂CH₂CH₂OH, -C(CH₃)₂CH₂CH₂OCH₃, -CH₂CH₂NH₂, -CH₂CH₂N(CH₃)₂, -CH₂CH₂N(CH₂CH₃)₂, -CH₂CH₂SO₂CH₃, -CH₂CONH₂, -CH₂CH₂CONH₂, -C(CH₃)₂CO₂CH₃, [1-(2-hydroxyethyl)cyclopropyl]methyl-, cyclopropyl, cyclopentyl, cyclohexyl, oxetan-3-yl, 3-methyl-oxetan-3-yl, tetrahydro-2*H-*pyran-4-yl, tetrahydro-2*H-*pyran-3-yl, -CH₂-tetrahydro-2*H-*pyran-4-yl, tetrahydrofuran-3-yl, 2-methyltetrahydrofuran-3-yl, -CH₂- tetrahydrofuran-2-yl, 1*H*-imidazol-4-yl, 1*H-*1,2,4-triazol-3-yl, phenyl, benzyl, -CH₂CH₂CH₂-phenyl, 4-amino-phenyl-, pyridin-4-yl, -CH₂-(6-methyl-pyridin-2-yl), piperidin-4-yl, 1-methyl-piperidin-4-yl-, 1-((CH₃)₃C-O-CO-piperidin-4-yl-, -CH₂-piperidin-4-yl, -CH₂CH₂-morpholin-4-yl, -CH₂CH₂CH₂-morpholin-4-yl, pyrimidin-2-yl, -CH₂CH₂-indol-3-yl, 4,5-dimethyl-thiazol-2-yl, (3R)-1-benzyl-pyrrolidin-3-yl-, -CH₂CH₂-pyrrolidin-1-yl, -CH₂-benzimidazol-2-yl, -CH₂CH₂CH₂-imidazol-1-yl, -CH₂CH₂-imidazol-4-yl, -CH₂CH₂-(3,5-dimethyl-isoxazol-4-yl), (2*S*)-1-hydroxy-3-(1*H*-imidazol-4-yl)prop-2-yl, 3-[methyl(phenyl)amino]prop-1-yl tetrahydro-2*H*-thiopyran-4-yl, or 1,1-dioxidotetrahydro-2*H*-thiopyran-4-yl.

8. The compound or pharmaceutically acceptable salt according to any one of claims 1-7, wherein A is phenyl or phenyl(C₁-C₄)alkyl-, wherein any phenyl is substituted by R³, R⁴ and R⁵, wherein:
R³ is H, hydroxyl, halogen, hydroxy(C₁-C₄)alkyl, (C₁-C₄)alkyl or (C₁-C₄)alkoxy;
R⁴ is H, halogen, cyano, (C₁-C₄)alkyl, halo(C₁-C₄)alkyl, (C₁-C₄)alkoxy, phenoxy, phenyl(C₁-C₄)alkoxy, hydroxyl, hydroxy(C₁-C₄)alkyl-, or aminocarbonyl, wherein the phenyl moiety of said phenoxy or phenyl(C₁-C₄)alkoxy- is optionally substituted by 1-3 substituents each independently selected from halogen, -CF₃, (C₁-C₄)alkyl and (C₁-C₄)alkoxy; and
R⁵ is H, hydroxyl, halogen; hydroxy(C₁-C₄)alkyl, (C₁-C₄)alkyl or (C₁-C₄)alkoxy.

9. The compound or pharmaceutically acceptable salt according to any one of claims 1-7, wherein
A is phenyl, 2-hydroxy-5-chloro-phenyl, 2-hydroxy-5-fluoro-phenyl, 2-hydroxy-4-fluoro-phenyl, 3-hydroxy-4-fluoro-phenyl, 3-hydroxy-4-chloro-phenyl, 2-methyl-4-hydroxy-phenyl, 2-fluoro-4-hydroxy-phenyl, 2-fluoro-5-hydroxy-phenyl, 3-fluoro-4-hydroxy-phenyl, 2-methyl-6-hydroxy-phenyl, 3-methyl-5-hydroxy-phenyl, 2-chloro-5-hydroxy-phenyl, 2-chloro-4-hydroxy-phenyl, 3-chloro-4-hydroxy-phenyl, 4-hydroxy-phenyl, 2-fluoro-phenyl, 4-fluoro-phenyl, 3-chloro-phenyl, 4-chloro-phenyl, 2,5-difluoro-phenyl, 2,6-difluoro-phenyl, 3,5-difluoro-phenyl, 3,4,5-trifluoro-phenyl, 2,4-dichloro-phenyl, 3,4,5-trichloro-phenyl, 2,4-dimethyl-phenyl, 2-methyl-5-fluoro-phenyl, 2-chloro-3-hydroxy-4-methyl-phenyl, 2-methyl-phenyl, 3-methoxy-phenyl, 2-methyl-3-hydroxy-phenyl, 2-methyl-5-hydroxy-phenyl, 3-methoxy-4-methyl-phenyl, 2,4-dimethyl-5-hydroxy-phenyl, 2,4-dimethyl-5-methoxy-phenyl, 3-hydroxy-4-methyl-phenyl, 2-chloro-5-methoxy-phenyl, 3-methoxy-4-fluoro-phenyl, 3-methoxy-4-chloro-phenyl, 3-methoxy-4-bromo-phenyl, 3-hydroxymethyl-phenyl, 2-methyl-5-hydroxymethyl-phenyl, 4-phenoxy-phenyl or 4-[(2-chlorophenyl)methoxy]phenyl.

10. The compound or pharmaceutically acceptable salt according to any one of claims 1-7, wherein A is an optionally substituted 1*H*-pyrazolo[3,4-6]pyridinyl or [1,3]thiazolo[5,4-*b*]pyridinyl, optionally substituted by one or two substituents each independently selected from halogen, (C₁-C₄)alkyl, halo(C₁-C₄)alkyl, and (C₁-C₄)alkoxy.

11. The compound or pharmaceutically acceptable salt according to any one of claims 1-8, wherein A is an optionally substituted indol-6-yl, indazol-3-yl, indazol-6-yl, 1*H*-1,2,3-benzotriazol-4-yl, 1*H*-1,2,3-benzotriazol-5-yl, 1,2,3-benzothiadiazol-5-yl, 2,1,3-benzoxadiazol-5-yl, 1,3-benzodioxol-4-yl, 1,3-benzodioxol-4-yl, 1,3-benzoxathiol-2-on-5-yl, benzofuran-4-yl, benzothiazol-4-yl, benzothiazol-5-yl, benzothiazol-6-yl, benzoxazol-5-yl, or 1,2-benzoisoxazol-6-yl group, wherein said group is optionally substituted by hydroxyl, chloro, fluoro, bromo, -CF₃, cyano, hydroxymethyl-, methyl, methoxy or aminocarbonyl.

12. The compound or pharmaceutically acceptable salt according to any one of claims 1-7, wherein A is indol-6-yl, indazol-3-yl, indazol-6-yl, 5-methoxy-indazol-3-yl, 5-fluoro-indazol-3-yl, 4-chloro-indazol-3-yl, 5-chloro-indazol-3-yl, 6-chloro-indazol-3-yl, 7-trifluoromethyl-indazol-3-yl, 7-chloro-indazol-3-yl, 1-methyl-indazol-3-yl, 5-cyano-indazol-6-yl, 7-methyl-indazol-6-yl, 5-aminocarbonyl-indazol-6-yl, 3-fluoro-indazol-6-yl, 3-methyl-indazol-6-yl, 1*H*-1,2,3-benzotriazol-4-yl, 1*H*-1,2,3-benzotriazol-5-yl, 4-methyl-1*H-*1,2,3-benzotriazol-6-yl, 5-methyl-1*H-*1,2,3-benzotriazol-6-yl, 5-fluoro-1*H*-1,2,3-benzotriazol-6-yl, 1,2,3-benzothiadiazol-5-yl, 2,1,3-benzoxadiazol-5-yl, 1,3-benzodioxol-4-yl, 5-chloro-1,3-benzodioxol-4-yl, 2-oxo-1,3-benzoxathiol-5-yl, benzofuran-4-yl, benzothiazol-5-yl, benzothiazol-6-yl, 6-methyl-benzothiazol-5-yl, 6-fluorobenzothiazol-5-yl, 4-methyl-benzothiazol-5-yl, 4-fluoro-benzothiazol-5-yl, 4-chloro-benzothiazol-5-yl, 4-bromo-benzothiazol-5-yl, benzoxazol-5-yl, 1,2-benzoisoxazol-6-yl, 2,1-benzisothiazol-6-yl, 5-fluoro-1*H*-pyrazolo[3,4-b]pyridine-3-yl, 5-methyl-1*H*-pyrazolo[3,4-*b*]pyridine-3-yl, 5-fluoro-6-methyl-1*H*-pyrazolo[3,4-*b*]pyridine-3-yl, or [1,3]thiazolo[5,4-*b*]pyridin-6-yl.

13. A compound according to claim 1 which is:
*N*-1,3-benzothiazol-5-yl-6-(methylsulfonyl)-4-quinolinamine,
*N*-(2-methylphenyl)-6-(methylsulfonyl)-4-quinolinamine,
4-chloro-2-{[6-(methylsulfonyl)-4-quinolinyl]amino} phenol,
4-fluoro-2-{[6-(methylsulfonyl)-4-quinolinyl]amino} phenol,
N-1*H*-1,2,3-benzotriazol-5-yl-6-(methylsulfonyl)-4-quinolinamine,
3-fluoro-4-{[6-(methylsulfonyl)-4-quinolinyl]amino} phenol,
2-chloro-4-{[6-(methylsulfonyl)-4-quinolinyl]amino} phenol,
3-methyl-2-{[6-(methylsulfonyl)-4-quinolinyl]amino} phenol,
4-chloro-3-{[6-(methylsulfonyl)-4-quinolinyl]amino} phenol,
3-chloro-4-{[6-(methylsulfonyl)-4-quinolinyl]amino} phenol,
4-{[6-(methylsulfonyl)-4-quinolinyl] amino}phenol,
2-fluoro-4-{[6-(methylsulfonyl)-4-quinolinyl]amino} phenol, N-1*H*-indol-6-yl-6-(methylsulfonyl)-4-quinolinamine,
5-fluoro-2-{[6-(methylsulfonyl)-4-quinolinyl]amino} phenol
2-chloro-6-methyl-3-{[6-(methylsulfonyl)-4-quinolinyl]amino} phenol,
6-(methylsulfonyl)-*N*-phenyl-4-quinolinamine,
*N*-(2-fluorophenyl)-6-(methylsulfonyl)-4-quinolinamine,
*N*-(2,5-difluorophenyl)-6-(methylsulfonyl)-4-quinolinamine,
*N*-(2,6-difluorophenyl)-6-(methylsulfonyl)-4-quinolinamine,
*N*-(2,4-dichlorophenyl)-6-(methylsulfonyl)-4-quinolinamine,
*N*-(2,4-diimethylphenyl)-6-(methylsulfonyl)-4-quinolinamine,
*N*-(3,5-difluorophenyl)-6-(methylsulfonyl)-4-quinolinamine,
*N*-(5-fluoro-2-methylphenyl)-6-(methylsulfonyl)-4-quinolinamine,
*N*-(3-chlorophenyl)-6-(methylsulfonyl)-4-quinolinamine,
*N*-(4-fluorophenyl)-6-(methylsulfonyl)-4-quinolinamine,
*N*-(4-chlorophenyl)-6-(methylsulfonyl)-4-quinolinamine,
(4-methyl-3-{[6-(methylsulfonyl)-4-quinolinyl]amino}phenyl)methanol,
*N*-[4-methyl-3-(methyloxy)phenyl]-6-(methylsulfonyl)-4-quinolinamine,
(3-{[6-(methylsulfonyl)-4-quinolinyl]amino} phenyl)methanol,
*N*-1,3-benzodioxol-4-yl-6-(methylsulfonyl)-4-quinolinamine,
*N*-(4-{[(2-chlorophenyl)methyl]oxy}phenyl)-6-(methylsulfonyl)-4-quinolinamine,
2-fluoro-5-{[6-(methylsulfonyl)-4-quinolinyl]amimo} phenol,
4-fluoro-3- {[6-(methylsulfonyl)-4-quinolinyl]amino} phenol,
2,4-dimethyl-5-{[6-(methylsulfonyl)-4-quinolinyl]amino} phenol,
3-methyl-5-{[6-(methylsulfonyl)-4-quinolinyl]amino} phenol,
*N*-1*H*-1,2,3-benzotriazol-4-yl-6-(methylsulfonyl)-4-quinolinamine,
5-{[6-(methylsulfonyl)-4-quinolinyl]amino}-1,3-benzoxathiol-2-one,
*N*-(4-methyl-1*H*-1,2,3-benzotriazol-6-yl)-6-(methylsulfonyl)-4-quinolinamine,
*N*-(5-fluoro-1*H*-1,2,3-benzotriazol-6-yl)-6-(methylsulfonyl)-4-quinolinamine
*N*-(5-methyl-1*H*-1,2,3-benzotriazol-6-yl)-6-(methylsulfonyl)-4-quinolinamine,
*N*-1,2,3-benzothiadiazol-5-yl-6-(methylsulfonyl)-4-quinolinamine,
*N*-1,2-benzisoxazol-6-yl-6-(methylsulfonyl)-4-quinolinamine,
*N*-[2,4-dimethyl-5-(methyloxy)phenyl]-6-(methylsulfonyl)-4-quinolinamine,
6-(methylsulfonyl)-*N*-[4-(phenyloxy)phenyl]-4-quinolinamine,
*N*-[3-(methyloxy)phenyl]-6-(methylsulfonyl)-4-quinolinamine,
2-methyl-3-{[6-(methylsulfonyl)-4-quinolinyl]amino} phenol,
2-methyl-5-{[6-(methylsulfonyl)-4-quinolinyl]amino} phenol,
*N*-[2-chloro-5-(methyloxy)phenyl]-6-(methylsulfonyl)-4-quinolinamine,
*N*-[4-fluoro-3-(methyloxy)phenyl]-6-(methylsulfonyl)-4-quinolinamine,
3-methyl-4-{[6-(methylsulfonyl)-4-quinolinyl]amino} phenol,
6-(methylsulfonyl)-*N*-phenyl-4-quinolinamine,
*N*-1-benzofuran-4-yl-6-(methylsulfonyl)-4-quinolinamine,
*N*-[4-bromo-3-(methyloxy)phenyl]-6-(methylsulfonyl)-4-quinolinamine,
2-chloro-5-{[6-(methylsulfonyl)-4-quinolinyl]amino}phenol,
*N*-1,3-benzothiazol-6-yl-6-(methylsulfonyl)-4-quinolinamine,
*N*-2*H*-indazol-3-yl-6-(methylsulfonyl)-4-quinolinamine,
*N*-1,3-benzothiazol-5-yl-6-[(1-methylethyl)sulfonyl]-4-quinolinamine, *N*-1*H*-indazol-6-yl-6-[(1-methylethyl)sulfonyl]-4-quinolinamine,
*N*-1,3-benzothiazol-5-yl-6-(ethylsulfonyl)-4-quinolinamine,
6-(ethylsulfonyl)-*N*-1*H*-indazol-6-yl-4-quinolinamine,
6-({6-[(1-methylethyl)sulfonyl]-4-quinolinyl}amino)-1*H*-indazole-5-carboxamide,
6-[(1-methylethyl)sulfonyl]-*N*-(7-methyl-1*H*-indazol-6-yl)-4-quinolinamine,
*N*-[4-chloro-3-(methyloxy)phenyl]-6-[(1-methylethyl) sulfonyl]-4-quinolinamine,
6-({6-[(1-methylethyl)sulfonyl]-4-quinolinyl}amino)-1*H*-indazole-5-carbonitrile,
*N*-1*H*-indazol-3-yl-6-[(1-methylethyl) sulfonyl]-4-quinolinamine,
6-[(1-methylethyl)sulfonyl]-*N*-[5-(methyloxy)-1*H*-indazol-3-yl]-4-quinolinamine,
*N*-(5-fluoro-1*H*-indazol-3-yl)-6-[(1-methylethyl)sulfonyl]-4-quinolinamine,
*N*-(3-methyl-1*H*-indazol-6-yl)-6-(methylsulfonyl)-4-quinolinamine,
*N*-[4-chloro-3-(methyloxy)phenyl]-6-(methylsulfonyl)-4-quinolinamine,
*N*-(3-fluoro-1*H*-indazol-6-yl)-6-(methylsulfonyl)-4-quinolinamine,
*N*-(4-chloro-1*H*-indazol-3-yl)-6-(methylsulfonyl)-4-quinolinamine,
*N*-[5-(methyloxy)-1*H*-indazol-3-yl]-6-(methylsulfonyl)-4-quinolinamine,
*N*-(5-fluoro-1*H*-indazol-3-yl)-6-(methylsulfonyl)-4-quinolinamine,
*N*-(5-chloro-1*H*-indazol-3-yl)-6-(methylsulfonyl)-4-quinolinamine,
*N*-(6-chioro-1*H*-indazol-3-yl)-6-(methylsulfonyl)-4-quinolinamine,
*N*-1H-indazol-6-yl-6-(methylsulfonyl)-4-quinolinaimine,
6-[(1,1-dimethylethyl)sulfonyl]-*N*-(5-fluoro-1*H*-pyrazolo[3,4-*b*]pyridin-3-yl)-4-quinolinamine,
*N*-(5-methyl-1*H*-pyrazolo[3,4-*b*]pyridin-3-yl)-6-(methylsulfonyl)-4-quinolinamine,
*N*-(4-methyl-1,3-benzothiazol-5-yl)-6-(methylsulfonyl)-4-quinolinamine,
*N*-(4-bromo-1,3-benzothiazol-5-yl)-6-(methylsulfonyl)-4-quinolinamine,
*N*-(4-chloro-1,3-benzothiazol-5-yl)-6-(methylsulfonyl)-4-quinolinamine,
6-[(1,1-dimethylethyl)sulfonyl]-*N*-(5-fluoro-1*N*-pyrazolo[3,4-6]pyridin-3-yl)-4-quinolinamine,
6-[(1,1-dimethylethyl)sulfonyl]-*N*-(5-fluoro-6-methyl-1*H*-pyrazolo[3,4-*b*]pyridin-3-yl)-4-quinolinamine,
6-[(1-methylethyl)sulfonyl]-*N*-(1-methyl-1*H*-indazol-3-yl)-4-quinolinamine,
6-[(1,1-dimethylethyl)sulfonyl]-*N*-1*H*-indazol-6-yl-4-quinolinamine,
6-[(1,1-dimethylethyl)sulfonyl]-*N*-(3-fluoro-1*H*-indazol-6-yl)-4-quinolinamine,
6-[(1,1-dimethylethyl)sulfonyl]-*N*-(3-methyl-1*H*-indazol-6-yl)-4-quinolinamine,
6-[(1,1-dimethylethyl)sulfonyl]-*N*-1H-indazol-3-yl-4-quinolinamine,
6-[(1,1-dimethylethyl)sulfonyl]-*N*-(5-fluoro-1*H*-indazol-3-yl)-4-quinolinamine,
*N*-[4-chloro-3-(methyloxy)phenyl]-6-[(1,1-dimethylethyl)sulfonyl]-4-quinolinamine,
*N*-(3-fluoro-1*H*-indazol-6-yl)-6-[(1-methylethyl)sulfonyl]-4-quinolinamine,
6-[(1-methylethyl)sulfonyl]-*N*-(3-methyl-*N*-indazol-6-yl)-4-quinolinamine,
6-[(1-methylethyl)sulfonyl]-*N*-[7-(trifluoromethyl)-1*H*-indazol-3-yl]-4-quinolinamine,
*N*-(5-fluoro-1*H*-indazol-3-yl)-6-[(trifluoromethyl)sulfonyl]-4-quinolinamine,
*N*-(6-(((tetrahydrofuran-2-yl)methyl)sulfonyl)quinolin-4-yl)benzo[d]thiazol-5-amine,
4-(1,3-benzothiazol-5-ylamino)-6-[(trifluoromethyl)sulfonyl]quinoline,
*N*-(6-(tert-butylsulfonyl)quinolin-4-yl)thiazolo[5,4-b]pyridin-6-amine,
6-[(1-methylethyl)sulfonyl]-*N*-[1,3]thiazolo[5,4-*b*]pyridin-6-yl-4-quinolinamine,
*N*-2,1,3-benzoxadiazol-5-yl-6-(methylsulfonyl)-4-quinolinamine,
3-{[4-(1,3-benzothiazol-5-ylamino)-6-quinolinyl]sulfonyl}-3-methyl-1-butanol,
*N*-1,3-benzothiazol-5-yl-6-(phenylsulfonyl)-4-quinolinamine,
*N*-1,3-benzothiazol-5-yl-6-(cyclopropyl sulfonyl)-4-quinolinamine,
6-(methylsulfonyl)-*N*-(2-phenylethyl)-4-quinolinamine,
6-[(4-aminophenyl)sulfonyl]-*N*-1,3-benzothiazol-5-yl-4-quinolinamine,
*N*³-methylidene-4-(methylthio)-*N*¹-[6-(propylsulfonyl)-4-quinolinyl]-1,3-benzenediamine,
*N*-1,3-benzothiazol-5-yl-6-(cyclohexylsulfonyl)-4-quinolinamine,
*N*-1,3-benzothiazol-5-yl-6-(4-pyridinylsulfonyl)-4-quinolinamine,
3-{[4-(1,3-benzothiazot-5-ylamino)-6-quinolinyl]thio}-3-methyl-1-butanol,
*N*-1,3-benzothiazol-5-yl-6-(ethylsulfonyl)-4-quinolinamine,
3-{[4-(1,3-benzothiazol-5-ylamino)-6-quinolinyl]sulfonyl}-1-propanol,
4-{[4-(1,3-benzothiazol-5-ylamino)-6-quinolinyl]sulfonyl}-1-butanol,
2-{[4-(1,3-benzothiazol-5-ylamino)-6-quinolinyl]sulfonyl}ethanol,
*N*-1,3-benzothiazol-5-yl-6-[(1,1-dimethylethyl)sulfonyl]-4-quinolinamine,
*N*-1,3-benzotliiazol-5-yl-6-[(1,1-dimethylethyl)thio]-4-quinolinamine,
*N*-1,3-benzothiazol-5-yl-6-[(1-methylpropyl)thio]-4-quinolinamine,
*N*-1,3-benzothiazol-5-yl-6-(ethylthio)-4-quinolinamine,
6-[(2-aminoethyl)thio]-*N*-1,3-benzothiazol-5-yl-4-quinolinamine,
*N*-1,3-benzothiazol-5-yl-6-(cyclopentylthio)-4-quinolinamine,
*N*-1,3-benzothiazol-5-yl-6-(cyclopentyl sulfonyl)-4-quinolinamine,
3-{[4-(1*H*-indazol-6-ylamino)-6-quinolinyl]thio}-3-methyl-1-butanol,
3-{[4-(1*H*-indazol-6-ylamino)-6-quinolinyl]sulfonyl}-3-methyl-1-butanol,
*N*-1,3-benzothiazol-5-yl-6-[(1-methyl-4-piperidinyl)thio]-4-quinolinamine,
*N*-1,3-benzothiazol-5-yl-6-(2-pyrimidinylthio)-4-quinolinamine,
2-{[4-(1,3-benzothiazol-5-ylamino)-6-quinolinyl]thio}-1-propanol,
2-{[4-(1,3-benzothiazol-5-ylamino)-6-quinolinyl]sulfonyl}-1-propanol,
2-[1-({[4-(1,3-benzothiazol-5-ylamino)-6-quinolinyl]thio} methyl)cyclopropyl]ethanol,
2-[1-({[4-(1,3-benzothiazol-5-ylamino)-6-quinolinyl]sulfonyl}methyl)cyclopropyl]ethanol,
2-{[4-(1,3-benzothiazol-5-ylamino)-6-quinolinyl]sulfonyl}-2-methyl-1-propanol,
2-{[4-(1,3-benzothiazol-5-ylamino)-6-quinolinyl]thio}-2-methyl-1-propanol,
2-{[4-(1,3-benzothiazol-5-ylamino)-6-quinolinyl]sulfinyl}-2-methyl-1-propanol
2-{[4-(1*H*-indazol-6-ylamino)-6-quinolinyl]sulfonyl}-2-methyl-1-propanol,
methyl 2-{[4-(1,3-benzothiazol-5-ylamino)-6-quinolinyl]thio}-2-methylpropanoate,
methyl 2-{[4-(1,3-benzothiazol-5-ylamino)-6-quinolinyl]sulfonyl}-2-methylpropanoate,
*N*-1,3-benzothiazol-5-yl-6-{[1,1-dimethyl-3-(methyloxy)propyl]thio}-4-quinolinamine,
*N*-(5-fluoro-1*H*-indazol-3-yl)-6-(tetrahydro-2*H*-pyran-4-ylsulfonyl)-4-quinolinamine,
2-({4-[(5-fluoro-1*H*-indazol-3-yl)amino]-6-quinolinyl}sulfonyl)ethanol,
*N*-1,3-benzothiazol-5-yl-6-(1*H*-1,2,4-triazol-3-ylsulfonyl)-4-quinolinamine,
*N*-(5-fluoro-1*H*-indazol-3-yl)-6-(1*H*-imidazol-4-ylsulfonyl)-4-quinolinamine,
*N*-(5-fluoro-1*H*-indazol-3-yl)-6-(1*H*-1,2,4-triazol-3-ylsulfonyl)-4-quinolinamine,
*N*-(5-fluoro-1*H*-indazol-3-yl)-6-(tetrahydro-3-furanylsulfonyl)-4-quinolinamine,
*N*-1,3-benzothiazol-5-yl-6-[(2-methyltetrahydro-3-furanyl)sulfonyl]-4-quinolinamine,
*N*-1,3-benzothiazol-5-yl-6-{[2-(methyloxy)ethyl]sulfonyl}-4-quinolinamine,
*N*-1,3-benzothiazol-5-yl-6-(methylthio)-4-quinolinamine,
2-{[4-(1,3-benzothiazol-5-ylamino)-6-quinolinyl]thio} ethanol,
*N*-1,3-benzothiazol-5-yl-6-{[2-(diethylamino)ethyl]thio}-4-quinolinamine,
*N*-1,3-benzothiazol-5-yl-6-{[2-(diethylamino)ethyl]sulfonyl}-4-quinolinamine,
*N*-1,3-benzothiazol-5-yl-6-(tetrahydro-3-furanylthio)-4-quinolinamine,
*N*-1,3-benzothiazol-5-yl-6-(tetrahydro-3-furanylsulfonyl)-4-quinolinamine,
*N*-1,3-benzothiazol-5-yl-6-(tetrahydro-2*H*-pyran-4-ylthio)-4-quinolinamine,
*N-*1,3-benzothiazol-5-yl-6-(tetrahydro-2*H*-pyran-4-ylsulfonyl)-4-quinolinamine,
*N*-1,3-benzothiazol-5-yl-6-(2-propen-1-ylsulfonyl)-4-quinolinamine,
*N*-1,3-benzothiazol-5-yl-6-{[2-(4-morpholinyl)ethyl]thio}-4-quinolinamine,
*N*-1,3-benzothiazol-5-yl-6-{[2-(3,5-dimethyl-4-isoxazolyl)ethyl]thio}-4-quinolinamine,
*N*-1,3-benzothiazol-5-yl-6-{[2-(3,5-dimethyl-4-isoxazolyl)ethyl]sulfonyl}-4-quinolinamine,
*N*-(6-((1-methylpiperidin-4-yl)sulfonyl)quinolin-4-yl)benzo[d]thiazol-5-amine,
1,1-dimethylethyl4-{[4-(1,3-benzothiazol-5-ylamino)-6-quinolinyl]thio}-1-piperidinecarboxylate,
1,1-dimethylethyl 4-{[4-(1,3-benzothiazol-5-ylamino)-6-quinolinyl]sulfonyl}-1-piperidinecarboxylate,
*N*-1,3-benzothiazol-5-yl-6-[(2,2,2-trifluoroethyl)thio]-4-quinolinamine,
*N*-1,3-benzothiazol-5-yl-6-[(2,2,2-trifluoroethyl)sulfonyl]-4-quinolinamine,
*N*-1,3-benzothiazol-5-yl-6-(tetrahydro-2*H*-thiopyran-4-ylthio)-4-quinolinamine,
2-{[4-(1,3-benzothiazol-5-ylamino)-6-quinolinyl]sulfinyl}ethanol,
*N*-(6-(((3,5)-tetrahydro-2H-pyran-3-yl)sulfonyl)quinolin-4-yl)benzo[d]thiazol-5-amine,
*N*-(6-(((3R)-tetrahydro-2H-pyran-3-yl)sulfonyl)quinolin-4-yl)benzo[d]thiazol-5-amine,
2-{[4-(1,3-benzothiazol-5-ylamino)-6-quinolinyl]thio}ethanol,
*N-*1,3-benzothiazol-5-yl-6-[(1-methylethyl)thio]-4-quinolinamine,
2-{[4-(1,3-benzothiazol-5-ylamino)-6-quinolinyl]thio}-2-methylpropanoic acid,
2-{[4-(1,3-benzothiazol-5-ylamino)-6-quinolinyl]sulfonyl}-2-methylpropanoic acid,
*N*-1,3-benzothiazol-5-yl-6-[(1,1-dioxidotetrahydro-2*H*-thiopyran-4-yl)sulfonyl]-4-quinolinamine,
*N*-1,3-benzothiazol-5-yl-6-(4-piperidinylsulfonyl)-4-quinolinamine,
2-{[4-(1,3-benzothiazol-5-ylamino)-6-quinolinyl]sulfinyl}ethanol,
*N*-1,3-benzothiazol-5-yl-6-((*R*)methylsulfinyl)-4-quinolinamine,
*N*-1,3-benzothiazol-5-yl-6-((*S*)methylsulfinyl)-4-quinolinamine,
*N*-1,3-benzothiazol-5-yl-6-[(1-methylethyl)sulfinyl]-4-quinolinamine,
4-[(5-hydroxy-2-methylphenyl)amino]-*N*-(phenylmethyl)-6-quinolinecarboxamide,
*N*-cyclohexyl-4-[(5-hydroxy-2-methylphenyl)amino]-6-quinolinecarboxamide,
4-[(5-hydroxy-2-methylphenyl)amino]-*N*-[3-(4-morpholinyl)propyl]-6-quinolinecarboxamide,
4-[(5-hydroxy-2-methylphenyl)amino]-*N*-(tetrahydro-2H-pyran-4-ylmethyl)-6-quinolinecarboxamide,
4-[(5-hydroxy-2-methylphenyl)amino]-*N*-(3-phenylpropyl)-6-quinolinecarboxamide,
4-[(5-hydroxy-2-methylphenyl)amino]-*N*-[(3*R*)-1-(phenylmethyl)-3-pyrrolidinyl]-6-quinolinecarboxamide,
4-[(5-hydroxy-2-methylphenyl)amino]-*N*-{3-[methyl(phenyl)amino]propyl}-6-quinolinecarboxamide,
4-[(5-hydroxy-2-methylphenyl)amino]-*N*-[3-(1*H*-imidazol-1-yl)propyl]-6-quinolinecarboxamide,
4-[(5-hydroxy-2-methylphenyl)amino]-*N*-[2-(1*H*-imidazol-4-yl)ethyl]-6-quinolinecarboxamide,
*N*-[(1S)-2-hydroxy-1-(1H-imidazol-4-ylmethyl)ethyl]-4-[(5-hydroxy-2-methylphenyl)amino]-6-quinolinecarboxamide,
*N-*(1H-benzimidazol-2-ylmethyl)-4-[(5-hydroxy-2-methylphenyl)amino]-6-quinolinecarboxamide,
4-[(5-hydroxy-2-methylphenyl)amino]-*N*-[2-(1-pyrrolidinyl)ethyl]-6-quinolinecarboxamide,
4-[(5-hydroxy-2-methylphenyl)amino]-*N*-[2-(methylsulfonyl)ethyl]-6-quinolinecarboxamide,
4-[(5-hydroxy-2-methylphenyl)amino]-*N*-[2-(1*H*-indol-3-yl)ethyl]-6-quinolinecarboxamide,
4-[(5-hydroxy-2-methylphenyl)amino]-*N*-[(6-methyl-2-pyridinyl)methyl]-6-quinolinecarboxamide,
*N*-(4,5-dimethyl-1,3-thiazol-2-yl)-4-[(5-hydroxy-2-methylphenyl)amino]-6-quinolinecarboxamide,
4-(1,3-benzothiazol-5-ylamino)-*N*-(phenylmethyl)-6-quinolinecarboxamide,
*N*-1,3-benzothiazol-5-yl-6-(4-morpholinylsulfonyl)-4-quinolinamine,
4-(1,3-benzothiazol-3-ylamino)-*N*-(3-methyl-3-oxetanyl)-6-quinolinesulfonamide,
4-(1,3-benzothiazol-5-ylamino)-*N*-(1-methylethyl)-6-quinoline sulfonamide,
*N*-1,3-benzothiazol-5-yl-6-(1-piperidinylsulfonyl)-4-quinolinamine,
4-(1,3-benzothiazol-5-ylamino)-*N,N*-dimethyl-6-quinolinesulfonamide,
4-(1,3-benzothiazol-5-ylamino)-*N*-methyl-*N*-(methyloxy)-6-quinolinesulfonamide,
4-(1,3-benzothiazol-5-ylamino)-*N*-(2-hydroxyethyl)-*N*-methyl-6-quinolinesulfonamide,
*N*-(2-hydroxyethyl)-4-(1H-indazol-6-ylamino)-*N*-methyl-6-quinolinesulfonamide,
4-{[4-chloro-3-(methyloxy)phenyl]amino}-*N*-(2-hydroxyethyl)-*N*-methyl-6-quinolinesulfonamide,
4-{[4-chloro-3-(methyloxy)phenyl]amino}-6-quinolinesulfonamide,
4-(1*H*-indazol-6-ylamino)-6-quinolinesulfonamide,
4-(1,3-benzothiazol-5-ylamino)-*N*-4-piperidinyl-6-quinolinesulfonamide,
4-(1,3-benzothiazol-5-ylamino)-*N*-(4-piperidinylmethyl)-6-quinolinesulfonamide,
*N*-1,3-benzothiazol-5-yl-6-(4-morpholinylsulfonyl)-4-quinolinamine,
4-(1,3-benzothiazol-5-ylamino)-*N*-[(6-methyl-2-pyridinyl)methyl]-6-quinolinesulfonamide,
4-(1,3-benzothiazol-5-ylamino)-*N*-[2-(dimethylamino)ethyl]-6-quinolinesulfonamide,
4-(1,3-benzothiazol-5-ylamino)-*N*-[2-(methyloxy)ethyl]-6-quinolinesulfonamide,
4-(1,3-benzothiazol-5-ylamino)-*N*-[3-(4-morpholinyl)propyl]-6-quinolinesulfonamide,
4-(1,3-benzothiazol-5-ylamino)-*N*-(tetrahydro-2*H*-pyran-4-ylmethyl)-6-quinolinesulfonamide,
4-(1,3-benzothiazol-5-ylamino)-*N*-(tetrahydro-2*H*-pyran-4-yl)-6-quinolinesulfonamide,
4-(1,3-benzothiazol-5-ylamino)-*N*-cyclohexyl-6-quinolinesulfonamide,
4-(1,3-benzothiazol-5-ylamino)-*N*-[2-(methylsulfonyl)ethyl]-6-quinolinesulfonamide,
4-(1,3-benzothiazol-5-ylamino)-*N*-(phenylmethyl)-6-quinolinesulfonamide,
*N*∼2∼-{[4-(1,3-benzothiazol-5-ylamino)-6-quinolinyl]sulfonyl}glycinamide,
*N*∼3∼-{[4-(1,3-benzothiazol-5-ylamino)-6-quinolinyl]sulfonyl}-beta-alaninamide,
4-(1,3-benzothiazol-5-ylamino)-*N*-(2-hydroxyethyl)-6-quinolinesulfonamide,
4-(1,3-benzothiazol-5-ylamino)-*N*-3-oxetanyl-6-quinolinesulfonamide,
4-(1,3-benzothiazol-5-ylamino)-*N-*(2-{[2-(methyloxy)ethyl]oxy}ethyl)-6-quinolinesulfonamide,
4-(1,3-benzothiazol-5-ylamino)-*N*-methyl-N(1-methylethyl)-6-quinolinesulfonamide,
4-(1*H*-indazol-6-ylamino)-N,N-dimethyl-6-quinolinesulfonamide,
4-{[4-chloro-3-(methyloxy)phenyl]amino}-*N,N*-dimethyl-6-quinolinesulfonamide,
*N*-1*H*-indazol-6-yl-6-(4-morpholinylsulfonyl)-4-quinolinamine,
*N*-[4-chloro-3-(methyloxy)phenyl]-6-(4-morpholinylsulfonyl)-4-quinolinamine,
4-(1*H*-indazol-6-ylamino)-*N*-(1-methylethyl)-6-quinolinesulfonamide,
4-{[4-chloro-3-(methytoxy)phenyl]amino}-*N*-(1-methylethyl)-6-quinolinesulfonamide,
*N*-1,3-benzothiazol-5-yl-6-(1-pyrrolidinylsulfonyl)-4-quinolinamine,
methyl 1-{[4-(1,3-benzothiazol-5-ylamino)-6-quinolinyl]sulfonyl}-L-prolinate,
methyl (3*S*,6*R*)-1-{[4-(1,3-benzothiazol-5-ylamino)-6-quinolinyl]sulfonyl}-6-methyl-3-piperidinecarboxylate,
1-{[4-(1,3-benzothiazol-5-ylamino)-6-quinolinyl]sulfonyl}-3-pyrrolidinol,
*N*-1,3-benzothiazol-5-yl-6-[(3-methyl-4-morpholinyl)sulfonyl]-4-quinolinamine,
*N*-1,3-benzothiazol-5-yl-6-[(4-methyl-1-piperazinyl)sulfonyl]-4-quinolinamine,
*N*-1,3-benzothiazol-5-yl-6-(4-thiomorpholinylsulfonyl)-4-quinolinamine,
*N*-1,3-benzothiazol-5-yl-6-[(1,1-dioxido-4-thiomorpholinyl)sulfonyl]-4-quinolinamine,
*N*-1,3-benzothiazol-5-yl-6-{[(3*R*)-3-methyl-4-morpholinyl]sulfonyl}-4-quinolinamine,
((2*S*,5*R*)-4-{[4-(1,3-benzothiazol-5-ylamino)-6-quinolinyl]sulfonyl}-5-ethyl-2-morpholinyl)methanol,
*N*-1,3-benzothiazol-5-yl-6-{[(3*S*)-3-methyl-4-morpholinyl]sulfonyl}-4-quinolinamine,
*N*-1,3-benzothiazol-5-yl-6-(1-piperazinylsulfonyl)-4-quinolinamine,
((2*S*,S*R*)-4-{[4-(1,3-benzothiazol-5-ylamino)-6-quinolinyl]sulfonyl}-5-methyl-2-morpholinyl)methanol,
(4-{[4-(1,3-benzothiazol-5-ylamino)-6-quinolinyl]sulfonyl}-2-morpholinyl)methanol,
4-(1,3-benzothiazol-5-ylamino)-*N*-methyl-*N*-[2-(methyloxy)ethyl]-6-quinolinesulfonamide,
4-(1,3-benzothiazol-5-ylamino)-*N*-[2-(methyloxy)ethyl]-6-quinolinesulfonamide,
*N*-(5-fluoro-1*H*-indazol-3-yl)-6-(4-morpholinylsulfonyl)-4-quinolinamine,
*N*-1,3-benzothiazol-5-yl-6-[(2,2-dimethyl-4-morpholinyl)sulfonyl]-4-quinolinamine,
*N*-1,3-benzothiazol-5-yl-6-[(2-methyl-4-morpholinyl)sulfonyl]-4-quinolinamine,
4-[(7-chloro-1*H*-indazol-3-yl)amino]-*N*-[2-(methyloxy)ethyl]-6-quinolinesulfonamide,
*N*-1,3-benzothiazol-5-yl-N-[6-(methylsulfonyl)-4-quinolinyl]acetamide,
*N*-1,3-benzothiazol-5-yl-N-[6-(methylsulfonyl)-4-quinolinyl]methanesulfonamide,
*N*-1,3-benzoxazol-5-yl-6-(methylsulfonyl)-4-quinolinamine,
*N*-1,3-benzoxazol-5-yl-6-[(1-methylethyl)sulfonyl]-4-quinolinamine,
*N*-1,3-benzoxazol-5-yl-6-(4-morpholinylsulfonyl)-4-quinolinamine,
*N*-[4-chloro-3-(methyloxy)phenyl]-6-(tetrahydro-2*H*-pyran-4-ylsulfonyl)-4-quinolinamine,
or
*N*-[4-chloro-3-(methyloxy)phenyl]-6-(tetrahydro-2*H*-pyran-4-ylsulfinyl)-4-quinolinamine, or a pharmaceutically acceptable salt thereof.

14. A pharmaceutical composition comprising the compound or pharmaceutically acceptable salt according to any one of claims 1-13 and one or more pharmaceutically acceptable excipients.

15. A compound or pharmaceutically acceptable salt according to any one of claims 1-14, for use in therapy.

## Patentansprüche

1. Verbindung gemäss Formel (I): 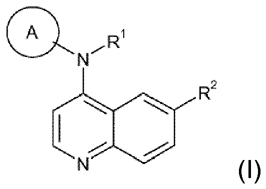 worin:
R¹ H, -SO₂(C₁₋₄-Alkyl), -CO(C₁₋₄-Alkyl), (C₁₋₄-Alkyl) oder Phenyl(C₁₋₄-alkyl)- ist;
R² -SR^{a}, -SOR^{a}, -SO₂R^{a}, -SO₂NH₂, -SO₂NR^{b}R^{c} oder -CONR^{b}R^{c} ist, worin
R^{a} (C₁₋₆)-Alkyl, Halogen-(C₁₋₄)-alkyl, (C₂₋₆)-Alkenyl, (C₃₋₇)-Cycloalkyl, 4- bis 7-gliedriges Heterocycloalkyl, Aryl oder Heteroaryl ist, worin
das (C₁₋₆)-Alkyl gegebenenfalls mit einer oder zwei Gruppe(n) substituiert ist, die jeweils unabhängig ausgewählt ist/sind aus Cyano, Hydroxyl, (C₁₋₆)-Alkoxy, (C₁₋₆)-Alkoxy-(C₂₋₆)-alkoxy, -CO₂H, -CO₂(C₁₋₄)-Alkyl, -SO₂(C₁₋₄-Alkyl), -CONH₂, -CONH(C₁₋₄-Alkyl), -CON(C₁₋₄-Alkyl)(C₁₋₄-alkyl), -SO₂NH₂, -SO₂NH(C₁₋₄-Alkyl), -SO₂N(C₁₋₄-Alkyl)(C₁₋₄-alkyl), Amino, (C₁₋₄-Alkyl)-amino-, (C₁₋₄-Alkyl)(C₁₋₄-alkyl)-amino-, C₃₋₇-Cycloalkyl, Phenyl, 5- bis 6-gliedrigem Heteroaryl, 9- bis 10-gliedrigem Heteroaryl, 4- bis 7-gliedrigem Heterocycloalkyl und (Phenyl)(C₁₋₄-alkyl)-amino-, wobei
das C₃₋₇-Cycloalkyl, Phenyl, (Phenyl)(C₁₋₄-alkyl)-amino-, 5- bis 6-gliedrige Heteroaryl, 9- bis 10-gliedrige Heteroaryl oder 4- bis 7-gliedrige Heterocycloalkyl gegebenenfalls mit 1 bis 3 Gruppe(n) substituiert ist, die jeweils unabhängig ausgewählt ist/sind aus Halogen, -CF₃, (C₁₋₄)-Alkyl, Hydroxy-(C₁₋₄)-alkyl und (C₁₋₄)-Alkoxy,
das (C₃₋₇)-Cycloalkyl oder 4- bis 7-gliedrige Heterocycloalkyl gegebenenfalls mit 1 bis 3 Gruppe(n) substituiert ist, die jeweils unabhängig ausgewählt ist/sind aus Halogen, -CF₃, Hydroxyl, Amino, (C₁₋₄)-Alkyl, Phenyl-(C₁₋₄)-alkyl-, (C₁₋₄)-Alkoxycarbonyl-, Hydroxy-(C₁₋₄)-alkyl-, Oxo und (C₁₋₄)-Alkoxy und
das Aryl oder Heteroaryl gegebenenfalls mit 1 bis 3 Gruppe(n) substituiert ist, die jeweils unabhängig ausgewählt ist/sind aus Halogen, -CF₃, Hydroxyl, Amino, (C₁₋₄)-Alkyl, Phenyl-(C₁₋₄)-alkyl-, Hydroxy-(C₁₋₄)-alkyl- und (C₁₋₄)-Alkoxy;
R^{b} (C₁₋₆)-Alkyl, (C₃₋₇)-Cycloalkyl, 4- bis 7-gliedriges Heterocycloalkyl, Aryl oder Heteroaryl ist, worin
das (C₁₋₆)-Alkyl gegebenenfalls mit einer oder zwei Gruppe(n) substituiert ist, die jeweils unabhängig ausgewählt ist/sind aus Cyano, Hydroxyl, (C₁₋₆)-Alkoxy, (C₁₋₆)-Alkoxy-(C₂₋₆)-alkoxy, -CO₂H, -CO₂(C₁₋₄)-Alkyl, -SO₂(C₁₋₄-Alkyl), -CONH₂, -CONH(C₁₋₄-Alkyl), -CON(C₁₋₄-Alkyl)(C₁₋₄-alkyl), -SO₂NH₂, -SO₂NH(C₁₋₄-Alkyl), -SO₂N(C₁₋₄-Alkyl)(C₁₋₄-alkyl), Amino, (C₁₋₄-Alkyl)-amino-, (C₁₋₄-Alkyl)(C₁₋₄-alkyl)-amino-, C₃₋₇-Cycycloalkyl, Phenyl, 5- bis 6-gliedrigem Heteroaryl, 9- bis 10-gliedrigem Heteroaryl, 4- bis 7-gliedrigem Heterocycloalkyl und (Phenyl)(C₁₋₄-alkyl)-amino-, worin
das C₃₋₇-Cycycloalkyl, Phenyl, (Phenyl)(C₁₋₄-alkyl)amino-, 5- bis 6-gliedrige Heteroaryl, 9- bis 10-gliedrige Heteroaryl oder 4- bis 7-gliedrige Heterocycloalkyl gegebenenfalls mit 1 bis 3 Gruppe(n) substituiert ist, die jeweils unabhängig ausgewählt ist/sind aus Halogen, -CF₃, (C₁₋₄)-Alkyl, Hydroxy-(C₁₋₄)-alkyl und (C₁₋₄)-Alkoxy,
das (C₃₋₇)-Cycloalkyl oder 4- bis 7-gliedrige Heterocycloalkyl gegebenenfalls mit 1 bis 3 Gruppe(n) substituiert ist, die jeweils unabhängig ausgewählt ist/sind aus Halogen, -CF₃, Hydroxyl, Amino, (C₁₋₄)-Alkyl, Phenyl-(C₁₋₄)-alkyl-, (C₁₋₄)-Alkoxycarbonyl-, Hydroxy-(C₁₋₄)-alkyl-, Oxo und (C₁₋₄)-Alkoxy und
das Aryl oder Heteroaryl gegebenenfalls mit 1 bis 3 Gruppe(n) substituiert ist, die jeweils unabhängig ausgewählt ist/sind aus Halogen, -CF₃, Hydroxyl, Amino, (C₁₋₄)-Alkyl, Phenyl-(C₁₋₄)-alkyl-, Hydroxy-(C₁₋₄)-alkyl- und (C₁₋₄)-Alkoxy;
R^{c} H, (C₁₋₄)-Alkoxy oder (C₁₋₆)-Alkyl ist;
oder R^{b} und R^{c} zusammen mit dem Stickstoffatom, an das sie gebunden sind, eine 3- bis 7-gliedrige Heterocycloalkyl- oder 5- bis 6-gliedrige Heteroarylgruppe bilden, die gegebenenfalls ein oder zwei zusätzliche(s) Ringheteroatom(e) enthält, die jeweils unabhängig aus Stickstoff, Sauerstoff und Schwefel ausgewählt sind, worin das 3- bis 7-gliedrige Heterocycloalkyl oder 5- bis 6-gliedrige Heteroaryl gegebenenfalls mit 1 bis 3 Gruppe(n) substituiert ist, die jeweils unabhängig ausgewählt ist/sind aus (C₁₋₄)-Alkyl, Halogen-(C₁₋₄)-alkyl, Phenyl-(C₁₋₄)-alkyl-, Hydroxyl, Hydroxy-(C₁₋₄)-alkyl-, C₁₋₄-Alkoxy, C₁₋₄-Halogenalkoxy, Amino, (C₁₋₄-Alkyl)-amino-, (C₁₋₄-Alkyl)(C₁₋₄-alkyl)-amino-, -CO₂H, -CO₂(C₁₋₄)-Alkyl, -CO(C₁₋₄)-Alkyl, -CO(4- bis 7-gliedrigem Heterocycloalkyl), -CONH₂, -CONH(C₁₋₄-Alkyl), -CON(C₁₋₄-Alkyl)(C₁₋₄-alkyl), -SO₂(C₁₋₄)-Alkyl, -SO₂(4- bis 7-gliedrigem Heterocycloalkyl), -SO₂NH₂, -SO₂NH(C₁₋₄-Alkyl) und -SO₂N(C₁₋₄-Alkyl)(C₁₋₄-alkyl) ;
A Phenyl oder Aryl-(C₁₋₄)-alkyl-, substituiert mit R³, R⁴ und R⁵, ist, worin
R³ H, Hydroxyl, Halogen, -CF₃, Hydroxy-(C₁₋₄)-alkyl, (C₁₋₄)-Alkyl oder (C₁₋₄)-Alkoxy ist;
R⁴ H, Halogen, Cyano, (C₁₋₄)-Alkyl, Halogen-(C₁₋₄)-alkyl, (C₁₋₄)-Alkoxy, Phenoxy, Phenyl-(C₁₋₄)-alkoxy, Hydroxyl, Hydroxy-(C₁₋₄)-alkyl- oder Aminocarbonyl ist, wobei der Phenylrest von Phenoxy oder Phenyl-(C₁₋₄)-alkoxy- gegebenenfalls mit 1 bis 3 Substituenten substituiert ist, der/die jeweils unabhängig ausgewählt ist/sind aus Halogen, -CF₃, (C₁₋₄)-Alkyl und (C₁₋₄)-Alkoxy; und
R⁵ H, Hydroxyl, Halogen, -CF₃, Hydroxy-(C₁₋₄)-alkyl, (C₁₋₄)-Alkyl oder (C₁₋₄)-Alkoxy ist; oder
A Phenyl oder Pyridinyl, substituiert mit R⁶, R⁷ und R⁸, ist, worin
R⁶ und R⁷ an benachbarten Atomen angeordnet sind und zusammen mit den Atomen, an die sie gebunden sind, eine 5-gliedrige heterocyclische Gruppe bilden, die 1, 2 oder 3 Heteroatom(e) enthält, das/die jeweils unabhängig ausgewählt ist/sind aus N, O und S, wobei die 5-gliedrige heterocyclische Gruppe mit R⁹ substituiert ist;
worin eines von R⁸ oder R⁹ H, Halogen, Cyano, (C₁₋₄)-Alkyl, Halogen-(C₁₋₄)-alkyl, (C₁₋₄)-Alkoxy, Phenoxy, Phenyl-(C₁₋₄)-alkoxy, Hydroxyl, Hydroxy-(C₁₋₄)-alkyl- oder Aminocarbonyl ist, wobei der Phenylrest von Phenoxy oder Phenyl-(C₁₋₄)-alkoxy gegebenenfalls mit 1 bis 3 Substituenten substituiert ist, der/die jeweils unabhängig ausgewählt ist/sind aus Halogen, -CF₃, (C₁₋₄)-Alkyl und (C₁₋₄)-Alkoxy; und
das andere von R⁸ oder R⁹ H, Hydroxyl, Halogen, -CF₃, Hydroxy-(C₁₋₄)-alkyl, (C₁₋₄)-Alkyl oder (C₁₋₄)-Alkoxy ist; oder
A Pyrazolyl, substituiert mit R¹⁰ und R¹¹, ist, worin
R¹⁰ und R¹¹ an benachbarten Kohlenstoffatomen angeordnet sind und zusammen mit den Atomen, an die sie gebunden sind, einen 6-gliedrigen carbocyclischen Ring oder heterocyclischen Ring bilden, der mit R¹² und R¹³ substituiert ist;
worin R¹² H, Halogen, Cyano, (C₁₋₄)-Alkyl, Halogen-(C₁₋₄)-alkyl, (C₁₋₄)-Alkoxy, Phenoxy, Phenyl-(C₁₋₄)-alkoxy, Hydroxyl, Hydroxy-(C₁₋₄)-alkyl- oder Aminocarbonyl ist, wobei der Phenylrest von Phenoxy oder Phenyl-(C₁₋₄)-alkoxy gegebenenfalls mit 1 bis 3 Substituenten substituiert ist, der/die jeweils unabhängig ausgewählt ist/sind aus Halogen, -CF₃, (C₁₋₄)-Alkyl und (C₁₋₄)-Alkoxy; und
R¹³ H, Hydroxyl, Halogen, -CF₃, Hydroxy-(C₁₋₄)-alkyl, (C₁₋₄)-Alkyl oder (C₁₋₄)-Alkoxy ist;
vorausgesetzt, dass die Verbindung nicht ist:
2-Fluor-5[[6-(methylsulfonyl)-4-chinolinyl]amino-1,4-benzoldiol;
N-(Cyclopropylmethyl)-4-[(5-hydroxy-2-methylphenyl)-amino]-6-chinolinsulfonamid;
4-Methyl-3-[[6-(methylsulfonyl)-4-chinolinyl]amino]-phenol;
4-Chlor-2-fluor-5-[[6-(methylsulfonyl)-4-chinolinyl]-amino]-phenol; oder
4-[(5-Hydroxy-2-methylphenyl)amino]-N-(1-methylethyl)-6-chinolincarboxamid;
oder ein pharmazeutisch annehmbares Salz davon.

2. Verbindung oder ein pharmazeutisch annehmbares Salz gemäss Anspruch 1, worin:
R¹ H, -SO₂(C₁₋₄-Alkyl), -CO(C₁₋₄-Alkyl), (C₁₋₄-Alkyl) oder Phenyl-(C₁₋₄-alkyl)- ist;
R² -SR^{a}, -SOR^{a}, -SO₂R^{a}, -SO₂NH₂, -SO₂NR^{b}R^{c} oder -CONR^{b}R^{c} ist, worin
R^{a} oder R^{b} (C₁₋₆)-Alkyl, (C₃₋₇)-Cycloalkyl, 4- bis 7-gliedriges Heterocycloalkyl, Aryl oder Heteroaryl ist, worin
das (C₁₋₆)-Alkyl gegebenenfalls mit einer oder zwei Gruppe(n) substituiert ist, die jeweils unabhängig ausgewählt ist/sind aus Cyano, Hydroxyl, (C₁₋₆)-Alkoxy, (C₁₋₆)-Alkoxy-(C₂₋₆)-alkoxy, -CO₂H, -CO₂(C₁₋₄)-Alkyl, -SO₂(C₁₋₄-Alkyl), -CONH₂, -CONH(C₁₋₄-Alkyl), -CON(C₁₋₄-Alkyl)(C₁₋₄-alkyl), -SO₂NH₂, -SO₂NH(C₁₋₄-Alkyl), -SO₂N(C₁₋₄-Alkyl)(C₁₋₄-alkyl), Amino, (C₁₋₄-Alkyl)-amino-, (C₁₋₄-Alkyl)(C₁₋₄-alkyl)-amino-, C₃₋₇-Cycloalkyl, Phenyl, 5- bis 6-gliedrigem Heteroaryl, 9- bis 10-gliedrigem Heteroaryl, 4- bis 7-gliedrigem Heterocycloalkyl und (Phenyl)(C₁₋₄-alkyl)-amino-, worin
das C₃₋₇-Cycloalkyl, Phenyl, (Phenyl)(C₁₋₄-alkyl)-amino-, 5- bis 6-gliedrige Heteroaryl, 9- bis 10-gliedrige Heteroaryl oder 4- bis 7-gliedrige Heterocycloalkyl gegebenenfalls mit 1 bis 3 Gruppe(n) substituiert ist, die jeweils unabhängig ausgewählt ist/sind aus Halogen, -CF₃, (C₁₋₄)-Alkyl, Hydroxy-(C₁₋₄)-alkyl und (C₁₋₄)-Alkoxy,
das (C₃₋₇)-Cycloalkyl oder 4- bis 7-gliedrige Heterocycloalkyl gegebenenfalls mit 1 bis 3 Gruppe(n) substituiert ist, die jeweils unabhängig ausgewählt ist/sind aus Halogen, -CF₃, Hydroxyl, Amino, (C₁₋₄)-Alkyl, Phenyl-(C₁₋₄)-alkyl-, Hydroxy-(C₁₋₄)-alkyl-, OXO und (C₁₋₄)-Alkoxy und
das Aryl oder Heteroaryl gegebenenfalls mit 1 bis 3 Gruppe(n) substituiert ist, die jeweils unabhängig ausgewählt ist/sind aus Halogen, -CF₃, Hydroxyl, Amino, (C₁₋₄)-Alkyl, Phenyl-(C₁₋₄)-alkyl-, Hydroxy-(C₁₋₄)-alkyl- und (C₁₋₄)-Alkoxy;
R^{c} H, (C₁₋₄)-Alkoxy oder (C₁₋₆)-Alkyl ist;
oder R^{b} und R^{c} zusammen mit dem Stickstoffatom, an das sie gebunden sind, eine 5- bis 7-gliedrige Heterocycloalkyl- oder 5- bis 6-gliedrige Heteroarylgruppe bilden, die gegebenenfalls ein zusätzliches Ringheteroatom enthält, das aus Stickstoff, Sauerstoff und Schwefel ausgewählt ist, wobei das 5- bis 7-gliedrige Heterocycloalkyl oder 5-bis 6-gliedrige Heteroaryl gegebenenfalls mit 1 bis 3 Gruppe(n) substituiert ist, die jeweils unabhängig ausgewählt ist/sind aus (C₁₋₄)-Alkyl, Halogen-(C₁₋₄)-alkyl, Phenyl-(C₁₋₄)-alkyl-, Hydroxy-(C₁₋₄)-alkyl-, C₁₋₄-Alkoxy, C₁₋₄-Halogenalkoxy, Amino, (C₁₋₄-Alkyl)-amino-, (C₁₋₄-Alkyl)(C₁₋₄-alkyl)-amino-, -CO₂H, -CO₂(C₁₋₄)-Alkyl, -CO(C₁₋₄)-Alkyl, -CO(4- bis 7-gliedrigem Heterocycloalkyl), -CONH₂, -CONH(C₁₋₄-Alkyl), -CON(C₁₋₄-Alkyl)(C₁₋₄-alkyl), -SO₂(C₁₋₄)-Alkyl, -SO₂(4- bis 7-gliedrigem Heterocycloalkyl), -SO₂NH₂, -SO₂NH(C₁₋₄-Alkyl) und -SO₂N(C₁₋₄-Alkyl)(C₁₋₄-alkyl);
A Phenyl oder Aryl-(C₁₋₄)-alkyl-, substituiert mit R³, R⁴ und R⁵, ist, worin
R³ H, Hydroxyl, Halogen, -CF₃, Hydroxy-(C₁₋₄)-alkyl, (C₁₋₄)-Alkyl oder (C₁₋₄)-Alkoxy ist;
R⁴ H, Halogen, Cyano, (C₁₋₄)-Alkyl, Halogen-(C₁₋₄)-alkyl, (C₁₋₄)-Alkoxy, Phenoxy, Phenyl-(C₁₋₄)-alkoxy, Hydroxyl, Hydroxy-(C₁₋₄)-alkyl- oder Aminocarbonyl ist, wobei der Phenylrest von Phenoxy oder Phenyl-(C₁₋₄)-alkoxy- gegebenenfalls mit 1 bis 3 Substituenten substituiert ist, der/die jeweils unabhängig ausgewählt ist/sind aus Halogen, -CF₃, (C₁₋₄)-Alkyl und (C₁₋₄)-Alkoxy; und
R⁵ H, Hydroxyl, Halogen, -CF₃, Hydroxy-(C₁₋₄)-alkyl, (C₁₋₄)-Alkyl oder (C₁₋₄)-Alkoxy ist; oder
A Phenyl, substituiert mit R⁶, R⁷ und R⁸, ist, worin
R⁶ und R⁷ an benachbarten Atomen angeordnet sind und zusammen mit den Atomen, an die sie gebunden sind, eine 5-gliedrige heterocyclische Gruppe bilden, die 1, 2 oder 3 Heteroatom(e) enthält, das/die jeweils unabhängig aus N, O und S ausgewählt ist/sind, wobei die 5-gliedrige heterocyclische Gruppe mit R⁹ substituiert ist;
worin eines von R⁸ oder R⁹ H, Halogen, Cyano, (C₁₋₄)-Alkyl, Halogen-(C₁₋₄)-alkyl, (C₁₋₄)-Alkoxy, Phenoxy, Phenyl-(C₁₋₄)-alkoxy, Hydroxyl, Hydroxy-(C₁₋₄)-alkyl- oder Aminocarbonyl ist, wobei der Phenylrest von Phenoxy oder Phenyl-(C₁₋₄)-alkoxy gegebenenfalls mit 1 bis 3 Substituenten substituiert ist, der/die jeweils unabhängig ausgewählt ist/sind aus Halogen, -CF₃, (C₁₋₄)-Alkyl und (C₁₋₄)-Alkoxy; und
das andere von R⁸ oder R⁹ H, Hydroxyl, Halogen, -CF₃, Hydroxy-(C₁₋₄)-alkyl, (C₁₋₄)-Alkyl oder (C₁₋₄)-Alkoxy ist; oder
A Pyrazolyl, substituiert mit R¹⁰ und R¹¹, ist, worin
R¹⁰ und R¹¹ an benachbarten Kohlenstoffatomen angeordnet sind und zusammen mit den Atomen, an die sie gebunden sind, einen 6-gliedrigen carbocyclischen Ring oder heterocyclischen Ring bilden, der mit R¹² und R¹³ substituiert ist;
worin R¹² H, Halogen, Cyano, (C₁₋₄)-Alkyl, Halogen-(C₁₋₄)-alkyl, (C₁₋₄)-Alkoxy, Phenoxy, Phenyl-(C₁₋₄)-alkoxy, Hydroxyl, Hydroxy-(C₁₋₄)-alkyl- oder Aminocarbonyl ist, wobei der Phenylrest von Phenoxy oder Phenyl-(C₁₋₄)-alkoxy gegebenenfalls mit 1 bis 3 Substituenten substituiert ist, der/die jeweils unabhängig ausgewählt ist/sind aus Halogen, -CF₃, (C₁₋₄)-Alkyl und (C₁₋₄)-Alkoxy; und
R¹³ H, Hydroxyl, Halogen, -CF₃, Hydroxy-(C₁₋₄)-alkyl, (C₁₋₄)-Alkyl oder (C₁₋₄)-Alkoxy ist.

3. Verbindung oder ein pharmazeutisch annehmbares Salz gemäss Anspruch 1 oder Anspruch 2, worin R¹ H ist.

4. Verbindung oder ein pharmazeutisch annehmbares Salz gemäss irgendeinem der Ansprüche 1 bis 3, worin R² -SO₂R^{a} ist.

5. Verbindung oder ein pharmazeutisch annehmbares Salz gemäss irgendeinem der Ansprüche 1 bis 4, worin R^{a} (C₁₋₆)-Alkyl ist und das (C₁₋₆)-Alkyl gegebenenfalls mit 1 oder 2 Substituenten substituiert ist, der/die jeweils unabhängig ausgewählt ist/sind aus Hydroxyl, (C₁₋₂)-Alkoxy, (C₁₋₂)-Alkoxy-(C₂₋₃)-Alkoxy-, Amino, (C₁₋₃-Alkyl)-amino-, (C₁₋₃-Alkyl)(C₁₋₂-alkyl)amino-, (Phenyl)(C₁₋₂-alkyl)-amino-, -CO₂(C₁₋₂)-Alkyl, -CONH₂, -SO₂(C₁₋₂)-Alkyl und einem C₃₋₆-Cycloalkyl, gegebenenfalls substituiert mit (C₁₋₄)-Alkyl oder Hydroxy-(C₁₋₄)-alkyl; 4- bis 6-gliedrigem Heterocycloalkyl, gegebenenfalls substituiert mit (C₁₋₄)-Alkyl; 5- bis 6-gliedrigem Heteroaryl, gegebenenfalls substituiert mit (C₁₋₄)-Alkyl; Phenyl oder 9- bis 10-gliedrigem Heteroaryl.

6. Verbindung oder ein pharmazeutisch annehmbares Salz gemäss irgendeinem der Ansprüche 1 bis 4, worin R^{a} substituiertes 4- bis 6-gliedriges Heterocycloalkyl, 5- bis 6-gliedriges Heteroaryl oder Phenyl ist,
das 4- bis 6-gliedrige Heterocycloalkyl mit (C₁₋₄)-Alkyl, (C₁₋₄)-Alkoxycarbonyl- oder Benzyl substituiert ist,
das 5- bis 6-gliedrige Heteroaryl mit (C₁₋₄)-Alkyl oder Hydroxy-(C₁₋₄)-alkyl substituiert ist und
das Phenyl mit Amino substituiert ist.

7. Verbindung oder ein pharmazeutisch annehmbares Salz gemäss irgendeinem der Ansprüche 1 bis 4, worin R^{a} -CH₃, -CF₃, -CH₂CH₃, -CH₂CF₃, -CH₂CH₂CH₃, -CH₂CH=CH₂, -CH(CH₃)₂, -CH(CH₃)CH₂CH₃, -C(CH₃)₃, -CH₂CH₂OH, -CH₂CH₂OCH₃, -CH₂CH₂OCH₂CH₂OCH₃, -CH₂CH₂CH₂OH, -CH₂CH₂CH₂CH₂OH, -CH(CH₃)CH₂OH, -C(CH₃)₂CH₂OH, -C(CH₃)₂CO₂H, -C(CH₃)₂CH₂CH₂OH, -C(CH₃)₂CH₂CH₂OCH₃, -CH₂CH₂NH₂, -CH₂CH₂N(CH₃)₂, -CH₂CH₂N(CH₂CH₃)₂, -CH₂CH₂SO₂CH₃, -CH₂CONH₂, -CH₂CH₂CONH₂, -C(CH₃)₂CO₂CH₃, [1- (2-Hydroxyethyl)cyclopropyl]methyl-, Cyclopropyl, Cyclopentyl, Cyclohexyl, Oxetan-3-yl, 3-Methyl-oxetan-3-yl, Tetrahydro-2H-pyran-4-yl, Tetrahydro-2H-pyran-3-yl, -CH₂-Tetrahydro-2H-pyran-4-yl, Tetrahydrofuran-3-yl, 2-Methyl-tetrahydrofuran-3-yl, -CH₂-Tetrahydrofuran-2-yl, 1H-Imidazol-4-yl, 1H-1,2,4-Triazol-3-yl, Phenyl, Benzyl, -CH₂CH₂CH₂-Phenyl, 4-Amino-phenyl-, Pyridin-4-yl, -CH₂-(6-Methyl-pyridin-2-yl), Piperidin-4-yl, 1-Methyl-piperidin-4-yl-, 1-((CH₃)₃C-O-CO-Piperidin-4-yl-, -CH₂-Piperidin-4-yl, -CH₂CH₂-Morpholin-4-yl, -CH₂CH₂CH₂-Morpholin-4-yl, Pyrimidin-2-yl, -CH₂CH₂-Indol-3-yl, 4,5-Dimethylthiazol-2-yl, (3R)-1-Benzyl-pyrrolidin-3-yl-, -CH₂CH₂-Pyrrolidin-1-yl, -CH₂-Benzimidazol-2-yl, -CH₂CH₂CH₂-Imidazol-1-yl, -CH₂CH₂-Imidazol-4-yl, -CH₂CH₂-(3,5-Dimethyl-isoxazol-4-yl), (2S)-1-Hydroxy-3-(1H-imidazol-4-yl)prop-2-yl, 3-[Methyl(phenyl)amino]prop-1-yltetrahydro-2H-thiopyran-4-yl oder 1,1-Dioxidtetrahydro-2H-thiopyran-4-yl.

8. Verbindung oder ein pharmazeutisch annehmbares Salz gemäss irgendeinem der Ansprüche 1 bis 7, worin:
A Phenyl oder Phenyl-(C₁₋₄)-alkyl- ist, worin jedes Phenyl mit R³, R⁴ und R⁵ substituiert ist, worin R³ H, Hydroxyl, Halogen, Hydroxy-(C₁₋₄)-alkyl, (C₁₋₄)-Alkyl oder (C₁₋₄)-Alkoxy ist;
R⁴ H, Halogen, Cyano, (C₁₋₄)-Alkyl, Halogen-(C₁₋₄)-alkyl, (C₁₋₄)-Alkoxy, Phenoxy, Phenyl-(C₁₋₄)-alkoxy, Hydroxyl, Hydroxy-(C₁₋₄)-alkyl- oder Aminocarbonyl ist, wobei der Phenylrest von Phenoxy oder Phenyl-(C₁₋₄)-alkoxy- gegebenenfalls mit 1 bis 3 Substituenten substituiert ist, der/die jeweils unabhängig ausgewählt ist/sind aus Halogen, -CF₃, (C₁₋₄)-Alkyl und (C₁₋₄)-Alkoxy; und
R⁵ H, Hydroxyl, Halogen, Hydroxy-(C₁₋₄)-alkyl, (C₁₋₄)-Alkyl oder (C₁₋₄)-Alkoxy ist.

9. Verbindung oder ein pharmazeutisch annehmbares Salz gemäss irgendeinem der Ansprüche 1 bis 7, worin:
A Phenyl, 2-Hydroxy-5-chlor-phenyl, 2-Hydroxy-5-fluor-phenyl, 2-Hydroxy-4-fluor-phenyl, 3-Hydroxy-4-fluor-phenyl, 3-Hydroxy-4-chlor-phenyl, 2-Methyl-4-hydroxy-phenyl, 2-Fluor-4-hydroxy-phenyl, 2-Fluor-5-hydroxy-phenyl, 3-Fluor-4-hydroxy-phenyl, 2-Methyl-6-hydroxy-phenyl, 3-Methyl-5-hydroxy-phenyl, 2-Chlor-5-hydroxy-phenyl, 2-Chlor-4-hydroxy-phenyl, 3-Chlor-4-hydroxy-phenyl, 4-Hydroxy-phenyl, 2-Fluor-phenyl, 4-Fluor-phenyl, 3-Chlor-phenyl, 4-Chlor-phenyl, 2,5-Difluor-phenyl, 2,6-Difluor-phenyl, 3,5-Difluor-phenyl, 3,4,5-Trifluor-phenyl, 2,4-Dichlor-phenyl, 3,4,5-Trichlor-phenyl, 2,4-Dimethyl-phenyl, 2-Methyl-5-fluor-phenyl, 2-Chlor-3-hydroxy-4-methyl-phenyl, 2-Methyl-phenyl, 3-Methoxy-phenyl, 2-Methyl-3-hydroxy-phenyl, 2-Methyl-5-hydroxy-phenyl, 3-Methoxy-4-methyl-phenyl, 2,4-Dimethyl-5-hydroxy-phenyl, 2,4-Dimethyl-5-methoxy-phenyl, 3-Hydroxy-4-methyl-phenyl, 2-Chlor-5-methoxy-phenyl, 3-Methoxy-4-fluor-phenyl, 3-Methoxy-4-chlor-phenyl, 3-Methoxy-4-brom-phenyl, 3-Hydroxymethyl-phenyl, 2-Methyl-5-hydroxymethyl-phenyl, 4-Phenoxy-phenyl oder 4-[(2-Chlorphenyl)methoxy]phenyl ist.

10. Verbindung oder ein pharmazeutisch annehmbares Salz gemäss irgendeinem der Ansprüche 1 bis 7, worin A gegebenenfalls substituiertes 1H-Pyrazolo[3,4-b]-pyridinyl oder [1,3]Thiazolo[5,4-b]pyridinyl ist, das gegebenenfalls mit einem oder zwei Substituenten substituiert ist, der/die jeweils unabhängig ausgewählt ist/sind aus Halogen, (C₁₋₄)-Alkyl, Halogen-(C₁₋₄)-alkyl und (C₁₋₄)-Alkoxy.

11. Verbindung oder ein pharmazeutisch annehmbares Salz gemäss irgendeinem der Ansprüche 1 bis 8, worin A eine gegebenenfalls substituierte Indol-6-yl-, Indazol-3-yl-, Indazol-6-yl-, 1H-1,2,3-Benzotriazol-4-yl-, 1H-1,2,3-Benzotriazol-5-yl-, 1,2,3-Benzothiadiazol-5-yl-, 2,1,3-Benzoxadiazol-5-yl-, 1,3-Benzodioxol-4-yl-, 1,3-Benzodioxol-4-yl-, 1,3-Benzoxathiol-2-on-5-yl-, Benzofuran-4-yl-, Benzothiazol-4-yl-, Benzothiazol-5-yl-, Benzothiazol-6-yl-, Benzoxazol-5-yl- oder 1,2-Benzoisoxazol-6-yl-Gruppe ist, wobei die Gruppe gegebenenfalls mit Hydroxyl, Chlor, Fluor, Brom, -CF₃, Cyano, Hydroxymethyl-, Methyl, Methoxy oder Aminocarbonyl substituiert ist.

12. Verbindung oder ein pharmazeutisch annehmbares Salz gemäss irgendeinem der Ansprüche 1 bis 7, worin A Indol-6-yl, Indazol-3-yl, Indazol-6-yl, 5-Methoxy-indazol-3-yl, 5-Fluor-indazol-3-yl, 4-Chlor-indazol-3-yl, 5-Chlor-indazol-3-yl, 6-Chlor-indazol-3-yl, 7-Trifluormethyl-indazol-3-yl, 7-Chlor-indazol-3-yl, 1-Methyl-indazol-3-yl, 5-Cyano-indazol-6-yl, 7-Methyl-indazol-6-yl, 5-Aminocarbonyl-indazol-6-yl, 3-Fluor-indazol-6-yl, 3-Methyl-indazol-6-yl, 1H-1,2,3-Benzotriazol-4-yl, 1H-1,2,3-Benzotriazol-5-yl, 4-Methyl-1H-1,2,3-benzotriazol-6-yl, 5-Methyl-1H-1,2,3-benzotriazol-6-yl, 5-fluor-1H-1,2,3-benzotriazol-6-yl, 1,2,3-Benzothiadiazol-5-yl, 2,1,3-Benzoxadiazol-5-yl, 1,3-Benzodioxol-4-yl, 5-Chlor-1,3-benzodioxol-4-yl, 2-Oxo-1,3-benzoxathiol-5-yl, Benzofuran-4-yl, Benzothiazol-5-yl, Benzothiazol-6-yl, 6-Methyl-benzothiazol-5-yl, 6-Fluorbenzothiazol-5-yl, 4-Methyl-benzothiazol-5-yl, 4-Fluor-benzothiazol-5-yl, 4-Chlor-benzothiazol-5-yl, 4-Brom-benzothiazol-5-yl, Benzoxazol-5-yl, 1,2-Benzoisoxazol-6-yl, 2,1-Benzisothiazol-6-yl, 5-Fluor-1H-pyrazolo[3,4-b]pyridin-3-yl, 5-Methyl-1H-pyrazolo[3,4-b]pyridin-3-yl, 5-Fluor-6-methyl-1H-pyrazolo[3,4-b]pyridin-3-yl oder [1,3]Thiazolo[5,4-b]pyridin-6-yl ist.

13. Verbindung gemäss Anspruch 1, die
N-1,3-Benzothiazol-5-yl-6-(methylsulfonyl)-4-chinolin-amin,
N-(2-Methylphenyl)-6-(methylsulfonyl)-4-chinolinamin,
4-Chlor-2-{[6-(methylsulfonyl)-4-chinolinyl]amino}-phenol,
4-Fluor-2-{[6-(methylsulfonyl)-4-chinolinyl]amino}-phenol,
N-1H-1,2,3-Benzotriazol-5-yl-6-(methylsulfonyl)-4-chinolinamin,
3-Fluor-4-{[6-(methylsulfonyl)-4-chinolinyl]amino}-phenol,
2-Chlor-4-{[6-(methylsulfonyl)-4-chinolinyl]amino}-phenol,
3-Methyl-2-{[6-(methylsulfonyl)-4-chinolinyl]amino}-phenol,
4-Chlor-3-{[6-(methylsulfonyl)-4-chinolinyl]amino}-phenol,
3-Chlor-4-{[6-(methylsulfonyl)-4-chinolinyl]amino}-phenol,
4-{[6-(Methylsulfonyl)-4-chinolinyl]amino}phenol,
2-Fluor-4-{[6-(methylsulfonyl)-4-chinolinyl]amino}-phenol,
N-1H-Indol-6-yl-6-(methylsulfonyl)-4-chinolinamin,
5-Fluor-2-{[6-(methylsulfonyl)-4-chinolinyl]amino}-phenol
2-Chlor-6-methyl-3-{[6-(methylsulfonyl)-4-chinolinyl]-amino}phenol,
6-(Methylsulfonyl)-N-phenyl-4-chinolinamin,
N-(2-Fluorphenyl)-6-(methylsulfonyl)-4-chinolinamin,
N-(2,5-Difluorphenyl)-6-(methylsulfonyl)-4-chinolin-amin,
N-(2,6-Difluorphenyl)-6-(methylsulfonyl)-4-chinolin-amin,
N-(2,4-Dichlorphenyl)-6-(methylsulfonyl)-4-chinolin-amin,
N-(2,4-Dimethylphenyl)-6-(methylsulfonyl)-4-chinolin-amin,
N-(3,5-Difluorphenyl)-6-(methylsulfonyl)-4-chinolin-amin,
N-(5-Fluor-2-methylphenyl)-6-(methylsulfonyl)-4-chinolinamin,
N-(3-Chlorphenyl)-6-(methylsulfonyl)-4-chinolinamin,
N-(4-Fluorphenyl)-6-(methylsulfonyl)-4-chinolinamin,
N-(4-Chlorphenyl)-6-(methylsulfonyl)-4-chinolinamin,
(4-Methyl-3-{[6-(methylsulfonyl)-4-chinolinyl]amino}-phenyl)methanol,
N-[4-Methyl-3-(methyloxy)phenyl]-6-(methylsulfonyl)-4-chinolinamin,
(3-{[6-(Methylsulfonyl)-4-chinolinyl]amino}phenyl)-methanol,
N-1,3-Benzodioxol-4-yl-6-(methylsulfonyl)-4-chinolin-amin,
N-(4-{[(2-Chlorphenyl)methyl]oxy}phenyl)-6-(methyl-sulfonyl)-4-chinolinamin,
2-Fluor-5-{[6-(methylsulfonyl)-4-chinolinyl]amino}-phenol,
4-Fluor-3-{[6-(methylsulfonyl)-4-chinolinyl]amino}-phenol,
2,4-Dimethyl-5-{[6-(methylsulfonyl)-4-chinolinyl]-amino}phenol,
3-Methyl-5-{[6-(methylsulfonyl)-4-chinolinyl]amino}-phenol,
N-1H-1,2,3-Benzotriazol-4-yl-6-(methylsulfonyl)-4-chinolinamin,
5-{[6-(Methylsulfonyl)-4-chinolinyl]amino}-1,3-benzoxathiol-2-on,
N-(4-Methyl-1H-1,2,3-benzotriazol-6-yl)-6-(methyl-sulfonyl)-4-chinolinamin,
N-(5-Fluor-1H-1,2,3-benzotriazol-6-yl)-6-(methyl-sulfonyl)-4-chinolinamin,
N-(5-Methyl-1H-1,2,3-benzotriazol-6-yl)-6-(methyl-sulfonyl)-4-chinolinamin,
N-1,2,3-Benzothiadiazol-5-yl-6-(methylsulfonyl)-4-chinolinamin,
N-1,2-Benzisoxazol-6-yl-6-(methylsulfonyl)-4-chinolin-amin,
N-[2,4-Dimethyl-5-(methyloxy)phenyl]-6-(methyl-sulfonyl)-4-chinolinamin,
6-(Methylsulfonyl)-N-[4-(phenyloxy)phenyl]-4-chinolin-amin,
N-[3-(Methyloxy)phenyl]-6-(methylsulfonyl)-4-chinolin-amin,
2-Methyl-3-{[6-(methylsulfonyl)-4-chinolinyl]amino}-phenol,
2-Methyl-5-{[6-(methylsulfonyl)-4-chinolinyl]amino}-phenol,
N-[2-Chlor-5-(methyloxy)phenyl]-6-(methylsulfonyl)-4-chinolinamin,
N-[4-Fluor-3-(methyloxy)phenyl]-6-(methylsulfonyl)-4-chinolinamin,
3-Methyl-4-{[6-(methylsulfonyl)-4-chinolinyl]amino}-phenol,
6-(Methylsulfonyl)-N-phenyl-4-chinolinamin,
N-1-Benzofuran-4-yl-6-(methylsulfonyl)-4-chinolinamin,
N-[4-Brom-3-(methyloxy)phenyl]-6-(methylsulfonyl)-4-chinolinamin,
2-Chlor-5-{[6-(methylsulfonyl)-4-chinolinyl]amino}-phenol,
N-1,3-Benzothiazol-6-yl-6-(methylsulfonyl)-4-chinolin-amin,
N-2H-Indazol-3-yl-6-(methylsulfonyl)-4-chinolinamin,
N-1,3-Benzothiazol-5-yl-6-[(1-methylethyl)sulfonyl]-4-chinolinamin,
N-1H-Indazol-6-yl-6-[(1-methylethyl)sulfonyl]-4-chinolinamin,
N-1,3-Benzothiazol-5-yl-6-(ethylsulfonyl)-4-chinolin-amin,
6-(Ethylsulfonyl)-N-1H-indazol-6-yl-4-chinolinamin,
6-({6-[(1-Methylethyl)sulfonyl]-4-chinolinyl}amino)-1H-indazol-5-carboxamid,
6-[(1-Methylethyl)sulfonyl]-N-(7-methyl-1H-indazol-6-yl)-4-chinolinamin,
N-[4-Chlor-3-(methyloxy)phenyl]-6-[(1-methylethyl)-sulfonyl]-4-chinolinamin,
6-({6-[(1-Methylethyl)sulfonyl]-4-chinolinyl}amino)-1H-indazol-5-carbonitril,
N-1H-Indazol-3-yl-6-[(1-methylethyl)sulfonyl]-4-chinolinamin,
6-[(1-Methylethyl)sulfonyl]-N-[5-(methyloxy)-1H-indazol-3-yl]-4-chinolinamin,
N-(5-Fluor-1H-indazol-3-yl)-6-[(1-methylethyl)-sulfonyl]-4-chinolinamin,
N-(3-Methyl-1H-indazol-6-yl)-6-(methylsulfonyl)-4-chinolinamin,
N-[4-Chlor-3-(methyloxy)phenyl]-6-(methylsulfonyl)-4-chinolinamin,
N-(3-Fluor-1H-indazol-6-yl)-6-(methylsulfonyl)-4-chinolinamin,
N-(4-Chlor-1H-indazol-3-yl)-6-(methylsulfonyl)-4-chinolinamin,
N-[5-(Methyloxy)-1H-indazol-3-yl]-6-(methylsulfonyl)-4-chinolinamin,
N-(5-Fluor-1H-indazol-3-yl)-6-(methylsulfonyl)-4-chinolinamin,
N-(5-Chlor-1H-indazol-3-yl)-6-(methylsulfonyl)-4-chinolinamin,
N-(6-Chlor-1H-indazol-3-yl)-6-(methylsulfonyl)-4-chinolinamin,
N-1H-Indazol-6-yl-6-(methylsulfonyl)-4-chinolinamin,
6-[(1,1-Dimethylethyl)sulfonyl]-N-(5-fluor-1H-pyrazolo[3,4-b]pyridin-3-yl)-4-chinolinamin,
N-(5-Methyl-1H-pyrazolo[3,4-b]pyridin-3-yl)-6-(methylsulfonyl)-4-chinolinamin,
N-(4-Methyl-1,3-benzothiazol-5-yl)-6-(methylsulfonyl)-4-chinolinamin,
N-(4-Brom-1,3-benzothiazol-5-yl)-6-(methylsulfonyl)-4-chinolinamin,
N-(4-Chlor-1,3-benzothiazol-5-yl)-6-(methylsulfonyl)-4-chinolinamin,
6-[(1,1-Dimethylethyl)sulfonyl]-N-(5-fluor-1H-pyrazolo[3,4-b]pyridin-3-yl)-4-chinolinamin,
6-[(1,1-Dimethylethyl)sulfonyl]-N-(5-fluor-6-methyl-1H-pyrazolo[3,4-b]pyridin-3-yl)-4-chinolinamin,
6-[(1-Methylethyl)sulfonyl]-N-(1-methyl-1H-indazol-3-yl)-4-chinolinamin,
6-[(1,1-Dimethylethyl)sulfonyl]-N-1H-indazol-6-yl-4-chinolinamin,
6-[(1,1-Dimethylethyl)sulfonyl]-N-(3-fluor-1H-indazol-6-yl)-4-chinolinamin,
6-[(1,1-Dimethylethyl)sulfonyl]-N-(3-methyl-1H-indazol-6-yl)-4-chinolinamin,
6-[(1,1-Dimethylethyl)sulfonyl]-N-1H-indazol-3-yl-4-chinolinamin,
6-[(1,1-Dimethylethyl)sulfonyl]-N-(5-fluor-1H-indazol-3-yl)-4-chinolinamin,
N-[4-Chlor-3-(methyloxy)phenyl]-6-[(1,1-dimethyl-ethyl)sulfonyl]-4-chinolinamin,
N-(3-Fluor-1H-indazol-6-yl)-6-[(1-methylethyl)-sulfonyl]-4-chinolinamin,
6-[(1-Methylethyl)sulfonyl]-N-(3-methyl-1H-indazol-6-yl)-4-chinolinamin,
6-[(1-Methylethyl)sulfonyl]-N-[7-(trifluormethyl)-1H-indazol-3-yl]-4-chinolinamin,
N-(5-Fluor-1H-indazol-3-yl)-6-[(trifluormethyl)-sulfonyl]-4-chinolinamin,
N-(6-(((Tetrahydrofuran-2-yl)methyl)sulfonyl)chinolin-4-yl)benzo[d]thiazol-5-amin,
4-(1,3-Benzothiazol-5-ylamino)-6-[(trifluormethyl)-sulfonyl]chinolin,
N-(6-(tert-Butylsulfonyl)chinolin-4-yl)thiazolo-[5,4-b]pyridin-6-amin,
6-[(1-Methylethyl)sulfonyl]-N-[1,3]thiazolo[5,4-b]-pyridin-6-yl-4-chinolinamin,
N-2,1,3-Benzoxadiazol-5-yl-6-(methylsulfonyl)-4-chinolinamin,
3-{[4-(1,3-Benzothiazol-5-ylamino)-6-chinolinyl]-sulfonyl}-3-methyl-1-butanol,
N-1,3-Benzothiazol-5-yl-6-(phenylsulfonyl)-4-chinolin-amin,
N-1,3-Benzothiazol-5-yl-6-(cyclopropylsulfonyl)-4-chinolinamin,
6-(Methylsulfonyl)-N-(2-phenylethyl)-4-chinolinamin,
6-[(4-Aminophenyl)sulfonyl]-N-1,3-benzothiazol-5-yl-4-chinolinamin,
N³-Methyliden-4-(methylthio)-N¹-[6-(propylsulfonyl)-4-chinolinyl]-1,3-benzoldiamin,
N-1,3-Benzothiazol-5-yl-6-(cyclohexylsulfonyl)-4-chinolinamin,
N-1,3-Benzothiazol-5-yl-6-(4-pyridinylsulfonyl)-4-chinolinamin,
3-{[4-(1,3-Benzothiazol-5-ylamino)-6-chinolinyl]thio}-3-methyl-1-butanol,
N-1,3-Benzothiazol-5-yl-6-(ethylsulfonyl)-4-chinolin-amin,
3-{[4-(1,3-Benzothiazol-5-ylamino)-6-chinolinyl]-sulfonyl}-1-propanol,
4-{[4-(1,3-Benzothiazol-5-ylamino)-6-chinolinyl]-sulfonyl}-1-butanol,
2-{[4-(1,3-Benzothiazol-5-ylamino)-6-chinolinyl]-sulfonyl}ethanol,
N-1,3-Benzothiazol-5-yl-6-[(1,1-dimethylethyl)-sulfonyl]-4-chinolinamin,
N-1,3-Benzothiazol-5-yl-6-[(1,1-dimethylethyl)thio]-4-chinolinamin,
N-1,3-Benzothiazol-5-yl-6-[(1-methylpropyl)thio]-4-chinolinamin,
N-1,3-Benzothiazol-5-yl-6-(ethylthio)-4-chinolinamin,
6-[(2-Aminoethyl)thio]-N-1,3-benzothiazol-5-yl-4-chinolinamin,
N-1,3-Benzothiazol-5-yl-6-(Cyclopentylthio)-4-chinolinamin,
N-1,3-Benzothiazol-5-yl-6-(cyclopentylsulfonyl)-4-chinolinamin,
3-{[4-(1H-Indazol-6-ylamino)-6-chinolinyl]thio}-3-methyl-1-butanol,
3-{[4-(1H-Indazol-6-ylamino)-6-chinolinyl]sulfonyl}-3-methyl-1-butanol,
N-1,3-Benzothiazol-5-yl-6-[(1-methyl-4-piperidinyl)-thio]-4-chinolinamin,
N-1,3-Benzothiazol-5-yl-6-(2-pyrimidinylthio)-4-chinolinamin,
2-{[4-(1,3-Benzothiazol-5-ylamino)-6-chinolinyl]thio}-1-propanol,
2-{[4-(1,3-Benzothiazol-5-ylamino)-6-chinolinyl]-sulfonyl}-1-propanol,
2-[1-({[4-(1,3-Benzothiazol-5-ylamino)-6-chinolinyl]-thio}methyl)cyclopropyl]ethanol,
2-[1-({[4-(1,3-Benzothiazol-5-ylamino)-6-chinolinyl]-sulfonyl}methyl)cyclopropyl]ethanol,
2-{[4-(1,3-Benzothiazol-5-ylamino)-6-chinolinyl]-sulfonyl}-2-methyl-1-propanol,
2-{[4-(1,3-Benzothiazol-5-ylamino)-6-chinolinyl]thio}-2-methyl-1-propanol,
2-{[4-(1,3-Benzothiazol-5-ylamino)-6-chinolinyl]-sulfinyl}-2-methyl-1-propanol,
2-{[4-(1H-Indazol-6-ylamino)-6-chinolinyl]sulfonyl}-2-methyl-1-propanol,
Methyl-2-{[4-(1,3-benzothiazol-5-ylamino)-6-chinolinyl]thio}-2-methylpropanoat,
Methyl-2-{[4-(1,3-benzothiazol-5-ylamino)-6-chinolinyl]sulfonyl}-2-methylpropanoat,
N-1,3-Benzothiazol-5-yl-6-{[1,1-dimethyl-3-(methyloxy)propyl]thio}-4-chinolinamin,
N-(5-Fluor-1H-indazol-3-yl)-6-(tetrahydro-2H-pyran-4-ylsulfonyl)-4-chinolinamin,
2-({4-[(5-Fluor-1H-indazol-3-yl)amino]-6-chinolinyl}-sulfonyl)ethanol,
N-1,3-Benzothiazol-5-yl-6-(1H-1,2,4-triazol-3-ylsulfonyl)-4-chinolinamin,
N-(5-Fluor-1H-indazol-3-yl)-6-(1H-imidazol-4-ylsulfonyl)-4-chinolinamin,
N-(5-Fluor-1H-indazol-3-yl)-6-(1H-1,2,4-triazol-3-ylsulfonyl)-4-chinolinamin,
N-(5-Fluor-1H-indazol-3-yl)-6-(tetrahydro-3-furanyl-sulfonyl)-4-chinolinamin,
N-1,3-Benzothiazol-5-yl-6-[(2-methyltetrahydro-3-furanyl)sulfonyl]-4-chinolinamin,
N-1,3-Benzothiazol-5-yl-6-{[2-(methyloxy)ethyl]-sulfonyl}-4-chinolinamin,
N-1,3-Benzothiazol-5-yl-6-(methylthio)-4-chinolinamin,
2-{[4-(1,3-Benzothiazol-5-ylamino)-6-chinolinyl]thio}-ethanol,
N-1,3-Benzothiazol-5-yl-6-{[2-(diethylamino)ethyl]-thio}-4-chinolinamin,
N-1,3-Benzothiazol-5-yl-6-{[2-(diethylamino)ethyl]-sulfonyl}-4-chinolinamin,
N-1,3-Benzothiazol-5-yl-6-(tetrahydro-3-furanylthio)-4-chinolinamin,
N-1,3-Benzothiazol-5-yl-6-(tetrahydro-3-furanyl-sulfonyl)-4-chinolinamin,
N-1,3-Benzothiazol-5-yl-6-(tetrahydro-2H-pyran-4-ylthio)-4-chinolinamin,
N-1,3-Benzothiazol-5-yl-6-(tetrahydro-2H-pyran-4-ylsulfonyl)-4-chinolinamin,
N-1,3-Benzothiazol-5-yl-6-(2-propen-1-ylsulfonyl)-4-chinolinamin,
N-1,3-Benzothiazol-5-yl-6-{[2-(4-morpholinyl)ethyl]-thio}-4-chinolinamin,
N-1,3-Benzothiazol-5-yl-6-{[2-(3,5-dimethyl-4-isoxazolyl)ethyl]thio}-4-chinolinamin,
N-1,3-Benzothiazol-5-yl-6-{[2-(3,5-dimethyl-4-isoxazolyl)ethyl]sulfonyl}-4-chinolinamin,
N-(6-((1-Methylpiperidin-4-yl)sulfonyl)chinolin-4-yl)benzo[d]thiazol-5-amin,
1,1-Dimethylethyl-4-{[4-(1,3-benzothiazol-5-ylamino)-6-chinolinyl]thio}-1-piperidincarboxylat,
1,1-Dimethylethyl-4-{[4-(1,3-benzothiazol-5-ylamino)-6-chinolinyl]sulfonyl}-1-piperidincarboxylat,
N-1,3-Benzothiazol-5-yl-6-[(2,2,2-trifluorethyl)thio]-4-chinolinamin,
N-1,3-Benzothiazol-5-yl-6-[(2,2,2-trifluorethyl)-sulfonyl]-4-chinolinamin,
N-1,3-Benzothiazol-5-yl-6-(tetrahydro-2H-thiopyran-4-ylthio)-4-chinolinamin,
2-{[4-(1,3-Benzothiazol-5-ylamino)-6-chinolinyl]-sulfinyl}ethanol,
N-(6-(((3S)-Tetrahydro-2H-pyran-3-yl)sulfonyl)-chinolin-4-yl)benzo[d]thiazol-5-amin,
N-(6-(((3R)-Tetrahydro-2H-pyran-3-yl)sulfonyl)-chinolin-4-yl)benzo[d]thiazol-5-amin,
2-{[4-(1,3-Benzothiazol-5-ylamino)-6-chinolinyl]thio}-ethanol,
N-1,3-Benzothiazol-5-yl-6-[(1-methylethyl)thio]-4-chinolinamin,
2-{[4-(1,3-Benzothiazol-5-ylamino)-6-chinolinyl]thio}-2-methylpropansäure,
2-{[4-(1,3-Benzothiazol-5-ylamino)-6-chinolinyl]-sulfonyl}-2-methylpropansäure,
N-1,3-Benzothiazol-5-yl-6-[(1,1-dioxidtetrahydro-2H-thiopyran-4-yl)sulfonyl]-4-chinolinamin,
N-1,3-Benzothiazol-5-yl-6-(4-piperidinylsulfonyl)-4-chinolinamin,
2-{[4-(1,3-Benzothiazol-5-ylamino)-6-chinolinyl]-sulfinyl}ethanol,
N-1,3-Benzothiazol-5-yl-6-((R)methylsulfinyl)-4-chinolinamin,
N-1,3-Benzothiazol-5-yl-6-((S)methylsulfinyl)-4-chinolinamin,
N-1,3-Benzothiazol-5-yl-6-[(1-methylethyl)sulfinyl]-4-chinolinamin,
4-[(5-Hydroxy-2-methylphenyl)amino]-N-(phenylmethyl)-6-chinolincarboxamid,
N-Cyclohexyl-4-[(5-hydroxy-2-methylphenyl)amino]-6-chinolincarboxamid,
4-[(5-Hydroxy-2-methylphenyl)amino]-N-[3-(4-morpholinyl)propyl]-6-chinolincarboxamid,
4-[(5-Hydroxy-2-methylphenyl)amino]-N-(tetrahydro-2H-pyran-4-ylmethyl)-6-chinolincarboxamid,
4-[(5-Hydroxy-2-methylphenyl)amino]-N-(3-phenyl-propyl)-6-chinolincarboxamid,
4-[(5-Hydroxy-2-methylphenyl)amino]-N-[(3R)-1-(phenyl-methyl)-3-pyrrolidinyl]-6-chinolincarboxamid,
4-[(5-Hydroxy-2-methylphenyl)amino]-N-(3-[methyl-(phenyl)amino]propyl}-6-chinolincarboxamid,
4-[(5-Hydroxy-2-methylphenyl)amino]-N-[3-(1H-imidazol-1-yl)propyl]-6-chinolincarboxamid,
4-[(5-Hydroxy-2-methylphenyl)amino]-N-[2-(1H-imidazol-4-yl)ethyl]-6-chinolincarboxamid,
N-[(1S)-2-Hydroxy-1-(1H-imidazol-4-ylmethyl)ethyl]-4-[(5-hydroxy-2-methylphenyl)amino]-6-chinolincarboxamid,
N-(1H-Benzimidazol-2-ylmethyl)-4-[(5-hydroxy-2-methylphenyl)amino]-6-chinolincarboxamid,
4-[(5-Hydroxy-2-methylphenyl)amino]-N-[2-(1-pyrrolidinyl)ethyl]-6-chinolincarboxamid,
4-[(5-Hydroxy-2-methylphenyl)amino]-N-[2-(methylsulfonyl)ethyl]-6-chinolincarboxamid,
4-[(5-Hydroxy-2-methylphenyl)amino]-N-[2-(1H-indol-3-yl)ethyl]-6-chinolincarboxamid,
4-[(5-Hydroxy-2-methylphenyl)amino]-N-[(6-methyl-2-pyridinyl)methyl]-6-chinolincarboxamid,
N-(4,5-Dimethyl-1,3-thiazol-2-yl)-4-[(5-hydroxy-2-methylphenyl)amino]-6-chinolincarboxamid,
4-(1,3-Benzothiazol-5-ylamino)-N-(phenylmethyl)-6-chinolincarboxamid,
N-1,3-Benzothiazol-5-yl-6-(4-morpholinylsulfonyl)-4-chinolinamin,
4-(1,3-Benzothiazol-5-ylamino)-N-(3-methyl-3-oxetanyl)-6-chinolinsulfonamid,
4-(1,3-Benzothiazol-5-ylamino)-N-(1-methylethyl)-6-chinolinsulfonamid,
N-1,3-Benzothiazol-5-yl-6-(1-piperidinylsulfonyl)-4-chinolinamin,
4-(1,3-Benzothiazol-5-ylamino)-N,N-dimethyl-6-chinolinsulfonamid,
4-(1,3-Benzothiazol-5-ylamino)-N-methyl-N-(methyloxy)-6-chinolinsulfonamid,
4-(1,3-Benzothiazol-5-ylamino)-N-(2-hydroxyethyl)-N-methyl-6-chinolinsulfonamid,
N-(2-Hydroxyethyl)-4-(1H-indazol-6-ylamino)-N-methyl-6-chinolinsulfonamid,
4-{[4-Chlor-3-(methyloxy)phenyl]amino}-N-(2-hydroxyethyl)-N-methyl-6-chinolinsulfonamid,
4-{[4-Chlor-3-(methyloxy)phenyl]amino}-6-chinolinsulfonamid,
4-(1H-Indazol-6-ylamino)-6-chinolinsulfonamid,
4-(1,3-Benzothiazol-5-ylamino)-N-4-piperidinyl-6-chinolinsulfonamid,
4-(1,3-Benzothiazol-5-ylamino)-N-(4-piperidinylmethyl)-6-chinolinsulfonamid,
N-1,3-Benzothiazol-5-yl-6-(4-morpholinylsulfonyl)-4-chinolinamin,
4-(1,3-Benzothiazol-5-ylamino)-N-[(6-methyl-2-pyridinyl)methyl]-6-chinolinsulfonamid,
4-(1,3-Benzothiazol-5-ylamino)-N-[2-(dimethylamino)-ethyl]-6-chinolinsulfonamid,
4-(1,3-Benzothiazol-5-ylamino)-N-[2-(methyloxy)ethyl]-6-chinolinsulfonamid,
4-(1,3-Benzothiazol-5-ylamino)-N-[3-(4-morpholinyl)-propyl]-6-chinolinsulfonamid,
4-(1,3-Benzothiazol-5-ylamino)-N-(tetrahydro-2H-pyran-4-ylmethyl)-6-chinolinsulfonamid,
4-(1,3-Benzothiazol-5-ylamino)-N-(tetrahydro-2H-pyran-4-yl)-6-chinolinsulfonamid,
4-(1,3-Benzothiazol-5-ylamino)-N-cyclohexyl-6-chinolinsulfonamid,
4-(1,3-Benzothiazol-5-ylamino)-N-[2-(methylsulfonyl)-ethyl]-6-chinolinsulfonamid,
4-(1,3-Benzothiazol-5-ylamino)-N-(phenylmethyl)-6-chinolinsulfonamid,
N∼2∼-{[4-(1,3-Benzothiazol-5-ylamino)-6-chinolinyl]-sulfonyl}glycinamid,
N∼3∼-{[4-(1,3-Benzothiazol-5-ylamino)-6-chinolinyl]-sulfonyl}-beta-alaninamid,
4-(1,3-Benzothiazol-5-ylamino)-N-(2-hydroxyethyl)-6-chinolinsulfonamid,
4-(1,3-Benzothiazol-5-ylamino)-N-3-oxetanyl-6-chinolinsulfonamid,
4-(1,3-Benzothiazol-5-ylamino)-N-(2-{[2-(methyloxy)-ethyl]oxy}ethyl)-6-chinolinsulfonamid,
4-(1,3-Benzothiazol-5-ylamino)-N-methyl-N-(1-methylethyl)-6-chinolinsulfonamid,
4-(1H-Indazol-6-ylamino)-N,N-dimethyl-6-chinolinsulfonamid,
4-{[4-Chlor-3-(methyloxy)phenyl]amino}-N,N-dimethyl-6-chinolinsulfonamid,
N-1H-Indazol-6-yl-6-(4-morpholinylsulfonyl)-4-chinolinamin,
N-[4-Chlor-3-(methyloxy)phenyl]-6-(4-morpholinylsulfonyl)-4-chinolinamin,
4-(1H-Indazol-6-ylamino)-N-(1-methylethyl)-6-chinolinsulfonamid,
4-{[4-Chlor-3-(methyloxy)phenyl]amino}-N-(1-methylethyl)-6-chinolinsulfonamid,
N-1,3-Benzothiazol-5-yl-6-(1-pyrrolidinylsulfonyl)-4-chinolinamin,
Methyl-1-{[4-(1,3-benzothiazol-5-ylamino)-6-chinolinyl]sulfonyl}-L-prolinat,
Methyl-(3S,6R)-1-{[4-(1,3-benzothiazol-5-ylamino)-6-chinolinyl]sulfonyl}-6-methyl-3-piperidincarboxylat,
1-{[4-(1,3-Benzothiazol-5-ylamino)-6-chinolinyl]-sulfonyl}-3-pyrrolidinol,
N-1,3-Benzothiazol-5-yl-6-[(3-methyl-4-morpholinyl)-sulfonyl]-4-chinolinamin,
N-1,3-Benzothiazol-5-yl-6-[(4-methyl-1-piperazinyl)-sulfonyl]-4-chinolinamin,
N-1,3-Benzothiazol-5-yl-6-(4-thiomorpholinylsulfonyl)-4-chinolinamin,
N-1,3-Benzothiazol-5-yl-6-[(1,1-dioxid-4-thiomorpholinyl)sulfonyl]-4-chinolinamin,
N-1,3-Benzothiazol-5-yl-6-{[(3R)-3-methyl-4-morpholinyl]sulfonyl}-4-chinolinamin,
((2S,5R)-4-{[4-(1,3-Benzothiazol-5-ylamino)-6-chinolinyl]sulfonyl}-5-ethyl-2-morpholinyl)methanol,
N-1,3-Benzothiazol-5-yl-6-{[(3S)-3-methyl-4-morpholinyl]sulfonyl}-4-chinolinamin,
N-1,3-Benzothiazol-5-yl-6-(1-piperazinylsulfonyl)-4-chinolinamin,
((2S,5R)-4-{[4-(1,3-Benzothiazol-5-ylamino)-6-chinolinyl]sulfonyl}-5-methyl-2-morpholinyl)methanol,
(4-{[4-(1,3-Benzothiazol-5-ylamino)-6-chinolinyl]-sulfonyl}-2-morpholinyl)methanol,
4-(1,3-Benzothiazol-5-ylamino)-N-methyl-N-[2-(methyloxy)ethyl]-6-chinolinsulfonamid,
4-(1,3-Benzothiazol-5-ylamino)-N-[2-(methyloxy)ethyl]-6-chinolinsulfonamid,
N-(5-Fluor-1H-indazol-3-yl)-6-(4-morpholinylsulfonyl)-4-chinolinamin,
N-1,3-Benzothiazol-5-yl-6-[(2,2-dimethyl-4-morpholinyl)sulfonyl]-4-chinolinamin,
N-1,3-Benzothiazol-5-yl-6-[(2-methyl-4-morpholinyl)-sulfonyl]-4-chinolinamin,
4-[(7-Chlor-1H-indazol-3-yl)amino]-N-[2-(methyloxy)-ethyl]-6-chinolinsulfonamid,
N-1,3-Benzothiazol-5-yl-N-[6-(methylsulfonyl)-4-chinolinyl]acetamid,
N-1,3-Benzothiazol-5-yl-N-[6-(methylsulfonyl)-4-chinolinyl]methansulfonamid,
N-1,3-Benzoxazol-5-yl-6-(methylsulfonyl)-4-chinolinamin,
N-1,3-Benzoxazol-5-yl-6-[(1-methylethyl)sulfonyl]-4-chinolinamin,
N-1,3-Benzoxazol-5-yl-6-(4-morpholinylsulfonyl)-4-chinolinamin,
N-[4-Chlor-3-(methyloxy)phenyl]-6-(tetrahydro-2H-pyran-4-ylsulfonyl)-4-chinolinamin oder
N-[4-Chlor-3-(methyloxy)phenyl]-6-(tetrahydro-2H-pyran-4-ylsulfinyl)-4-chinolinamin
ist, oder ein pharmazeutisch annehmbares Salz davon.

14. Pharmazeutische Zusammensetzung, die eine Verbindung oder ein pharmazeutisch annehmbares Salz gemäss irgendeinem der Ansprüche 1 bis 13 und einen oder mehrere pharmazeutisch annehmbare(n) Hilfsstoff(e) umfasst.

15. Verbindung oder ein pharmazeutisch annehmbares Salz gemäss irgendeinem der Ansprüche 1 bis 14 zur Verwendung in der Therapie.

## Revendications

1. Composé répondant à la formule (I) 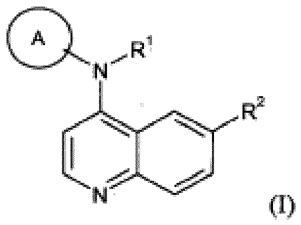 dans laquelle :
R¹ est H, un groupe -SO₂(alkyle en C₁ à C₄), -CO(alkyle en C₁ à C₄), alkyle en C₁ à C₄ ou phénylalkyle en C₁ à C₄ ;
R² est -SR^{a}, -SOR^{a}, -SO₂R^{a}, -SO₂NH2_{,} -SO₂NR^{b}R^{c} ou -CONR^{b}R^{c}, où
R^{a} est un groupe alkyle en C₁ à C₆, halogénoalkyle en C₁ à C₄, alcényle en C₂ à C₆, cycloalkyle en C₃ à C₇, hétérocycloalkyle de 4 à 7 éléments, aryle ou hétéroaryle, où :
ledit groupe alkyle en C₁ à C₆ est facultativement substitué par un ou deux groupes, chacun choisi indépendamment parmi les groupes cyano, hydroxyle, alcoxy en C₁ à C₆, (alcoxy en C₁ à C₆) alcoxy en C₂ à C₆, -CO₂H, -CO₂(alkyle en C₁ à C₄), -SO₂ (alkyle en C₁ à C₄), -CONH₂, -CONH(alkyle en C₁ à C₄), -CON(alkyle en C₁ à C₄) (alkyle en C₁ à C₄), -SO₂NH₂, -SO₂NH(alkyle en C₁ à C₄), -SO₂N (alkyle en C₁ à C₄) (alkyle en C₁ à C₄), amino, (alkyle en C₁ à C₄) amino, (alkyle en C₁ à C₄) (alkyle en C₁ à C₄) amino, cycloalkyle en C₃ à C₇, phényle, hétéroaryle de 5 à 6 éléments, hétéroaryle de 9 à 10 éléments, hétérocycloalkyle de 4 à 7 éléments et phényl(alkyle en C₁ à C₄) amino, où ledit groupe cycloalkyle en C₃ à C₇, phényle, phényl (alkyle en C₁ à C₄) amino, hétéroaryle de 5 à 6 éléments, hétéroaryle de 9 à 10 éléments ou hétérocycloalkyle de 4 à 7 éléments est facultativement substitué par 1 à 3 groupes, chacun choisi indépendamment parmi un atome d'halogène, les groupes -CF₃, alkyle en C₁ à C₄, hydroxyalkyle en C₁ à C₄ et alcoxy en C₁ à C₄,
ledit groupe cycloalkyle en C₃ à C₇ ou hétérocycloalkyle de 4 à 7 éléments est facultativement substitué par 1 à 3 groupes, chacun choisi indépendamment parmi un atome d'halogène, les groupes -CF₃, hydroxyle, amino, alkyle en C₁ à C₄, phénylalkyle en C₁ à C₄, (alcoxy en C₁ à C₄) carbonyle, hydroxyalkyle en C₁ à C₄, oxo et alcoxy en C₁ à C₄, et
ledit groupe aryle ou hétéroaryle est facultativement substitué par 1 à 3 groupes, chacun choisi indépendamment parmi un atome d'halogène, les groupes -CF₃, hydroxyle, amino, alkyle en C₁ à C₄, phénylalkyle en C₁ à C₄, hydroxyalkyle en C₁ à C₄ et alcoxy en C₁ à C₄ ;
R^{b} est un groupe alkyle en C₁ à C₆, cycloalkyle en C₃ à C₇, hétérocycloalkyle de 4 à 7 éléments, aryle ou hétéroaryle, où :
ledit groupe alkyle en C₁ à C₆ est facultativement substitué par un ou deux groupes, chacun choisi indépendamment parmi les groupes cyano, hydroxyle, alcoxy en C₁ à C₆, (alcoxy en C₁ à C₆) alcoxy en C₂ à C₆, -CO₂H, -CO₂(alkyle en C₁ à C₄), -SO₂ (alkyle en C₁ à C₄), -CONH₂, -CONH(alkyle en C₁ à C₄), -CON(alkyle en C₁ à C₄) (alkyle en C₁ à C₄), -SO₂NH₂, -SO₂NH (alkyle en C₁ à C₄), -SO₂N (alkyle en C₁ à C₄) (alkyle en C₁ à C₄), amino, (alkyle en C₁ à C₄) amino, (alkyle en C₁ à C₄) (alkyle en C₁ à C₄) amino, cycloalkyle en C₃ à C₇, phényle, hétéroaryle de 5 à 6 éléments, hétéroaryle de 9 à 10 éléments, hétérocycloalkyle de 4 à 7 éléments et phényl(alkyle en C₁ à C₄) amino, où ledit cycloalkyle en C₃ à C₇, phényle, phényl (alkyle en C₁ à C₄) amino, hétéroaryle de 5 à 6 éléments, hétéroaryle de 9 à 10 éléments ou hétérocycloalkyle de 4 à 7 éléments est facultativement substitué par 1 à 3 groupes, chacun choisi indépendamment parmi un atome d'halogène, les groupes -CF₃, alkyle en C₁ à C₄, hydroxyalkyle en C₁ à C₄ et alcoxy en C₁ à C₄,
ledit groupe cycloalkyle en C₃ à C₇ ou hétérocycloalkyle de 4 à 7 éléments est facultativement substitué par 1 à 3 groupes, chacun choisi indépendamment parmi un atome d'halogène, les groupes -CF₃, hydroxyle, amino, alkyle en C₁ à C₄, phénylalkyle en C₁ à C₄, (alcoxy en C₁ à C₄) carbonyle, hydroxyalkyle en C₁ à C₄, oxo et alcoxy en C₁ à C₄, et
ledit groupe aryle ou hétéroaryle étant facultativement substitué par 1 à 3 groupes, chacun choisi indépendamment parmi un atome d'halogène, les groupes -CF₃, hydroxyle, amino, alkyle en C₁ à C₄, phénylalkyle en C₁ à C₄, hydroxyalkyle en C₁ à C₄ et alcoxy en C₁ à C₄ ;
R^{c} est H, un groupe alcoxy en C₁ à C₄ ou alkyle en C₁ à C₆ ;
ou R^{b} et R^{c} ensemble avec l'atome d'azote auquel ils sont attachés forment un groupe hétérocycloalkyle de 3 à 7 éléments ou hétéroaryle de 5 à 6 éléments, contenant facultativement un ou deux hétéroatomes sur le cycle supplémentaires, chacun choisi indépendamment parmi un atome d'azote, un atome d'oxygène et un atome de soufre, où ledit groupe hétérocycloalkyle de 3 à 7 éléments ou hétéroaryle de 5 à 6 éléments est facultativement substitué par 1 à 3 groupes, chacun choisi indépendamment parmi les groupes alkyle en C₁ à C₄, halogénoalkyle en C₁ à C₄, phénylalkyle en C₁ à C₄, hydroxyle, hydroxyalkyle en C₁ à C₄, alcoxy en C₁ à C₄, halogénoalcoxy en C₁ à C₄, amino, (alkyle en C₁ à C₄) amino, (alkyle en C₁ à C₄) (alkyle en C₁ à C₄) amino, -CO₂H, -CO₂ (alkyle en C₁ à C₄), -CO (alkyle en C₁ à C₄), -CO (hétérocycloalkyle de 4 à 7 éléments), -CONH₂, -CONH(alkyle en C₁ à C₄), -CON(alkyle en C₁ à C₄) (alkyle en C₁ à C₄), -SO₂(alkyle en C₁ à C₄), -SO₂ (hétérocycloalkyle de 4 à 7 éléments), -SO₂NH₂, -SO₂NH(alkyle en C₁ à C₄) et -SO₂N(alkyle en C₁ à C₄)(alkyle en C₁ à C₄) ;
A est un groupe phényle ou arylalkyle en C₁ à C₄, substitué par R³, R⁴ et R⁵, où :
R³ est H, un atome d'halogène, un groupe hydroxyle, -CF₃, hydroxyalkyle en C₁ à C₄, alkyle en C₁ à C₄ ou alcoxy en C₁ à C₄ ;
R⁴ est H, un atome d'halogène, un groupe cyano, alkyle en C₁ à C₄, halogénoalkyle en C₁ à C₄, alcoxy en C₁ à C₄, phénoxy, phénylalcoxy en C₁ à C₄, hydroxyle, hydroxyalkyle en C₁ à C₄ ou aminocarbonyle, où le fragment phényle dudit groupe phénoxy ou phénylalcoxy en C₁ à C₄ est facultativement substitué par 1 à 3 substituants, chacun choisi indépendamment parmi un atome d'halogène, les groupes -CF₃, alkyle en C₁ à C₄ et alcoxy en C₁ à C₄ ; et
R⁵ est H, un atome d'halogène, un groupe hydroxyle, -CF₃, hydroxyalkyle en C₁ à C₄, alkyle en C₁ à C₄ ou alcoxy en C₁ à C₄ ; ou
A est un groupe phényle ou pyridinyle substitué par R⁶, R⁷ et R⁸, où :
R⁶ et R⁷ sont situés sur des atomes adjacents et ensemble avec les atomes auxquels ils sont attachés forment un groupe hétérocyclique de 5 éléments contenant 1, 2 ou 3 hétéroatomes, chacun choisi indépendamment parmi N, O et S, lequel groupe hétérocyclique de 5 éléments est substitué par R⁹ ;
un de R⁸ ou R⁹ est H, un atome d'halogène, un groupe cyano, alkyle en C₁ à C₄, halogénoalkyle en C₁ à C₄, alcoxy en C₁ à C₄, phénoxy, phénylalcoxy en C₁ à C₄, hydroxyle, hydroxyalkyle en C₁ à C₄ ou aminocarbonyle, où le fragment phényle dudit groupe phénoxy ou phénylalcoxy en C₁ à C₄ est facultativement substitué par 1 à 3 substituants, chacun choisi indépendamment parmi un atome d'halogène, un groupe -CF₃, alkyle en C₁ à C₄ et alcoxy en C₁ à C₄ ; et
l'autre de R⁸ ou R⁹ est H, un atome d'halogène, un groupe hydroxyle, -CF₃, hydroxyalkyle en C₁ à C₄, alkyle en C₁ à C₄ ou alcoxy en C₁ à C₄ ; ou
A est un groupe pyrazolyle substitué par R¹⁰ et R¹¹, où :
R¹⁰ et R¹¹ sont situés sur des atomes de carbone adjacents et ensemble avec les atomes auxquels ils sont attachés forment un cycle carbocyclique ou un cycle hétérocyclique de 6 éléments substitué par R¹² et R¹³ ;
où R¹² est H, un atome d'halogène, un groupe cyano, alkyle en C₁ à C₄, halogénoalkyle en C₁ à C₄, alcoxy en C₁ à C₄, phénoxy, phénylalcoxy en C₁ à C₄, hydroxyle, hydroxyalkyle en C₁ à C₄ ou aminocarbonyle, où le fragment phényle dudit phénoxy ou phénylalcoxy en C₁ à C₄ est facultativement substitué par 1 à 3 substituants, chacun choisi indépendamment parmi un atome d'halogène, les groupes -CF₃, alkyle en C₁ à C₄ et alcoxy en C₁ à C₄ ; et
R¹³ est H, un atome d'halogène, un groupe hydroxyle, -CF₃, hydroxyalkyle en C₁ à C₄, alkyle en C₁ à C₄ ou alcoxy en C₁ à C₄ ;
à condition que le composé ne soit pas :
2-fluoro-5[[6-(méthylsulfonyl)-4-quinoléinyl]amino-1,4-benzènediol ;
N-(cyclopropylméthyl)-4-[(5-hydroxy-2-méthylphényl)amino]-6-quinoléinesulfonamide ;
4-méthyl-3-[[6-(méthylsulfonyl)-4-quinoléinyl]amino]-phénol ;
4-chloro-2-fluoro-5-[[6-(méthylsulfonyl)-4-quinoléinyl]amino]phénol ; ou
4-[(5-hydroxy-2-méthylphényl)amino]-N-(1-méthyléthyl)-6-quinoléinecarboxamide ;
ou un sel pharmaceutiquement acceptable de ceux-ci.

2. Composé ou sel pharmaceutiquement acceptable selon la revendication 1, dans lequel :
R¹ est H, un groupe -SO₂ (alkyle en C₁ à C₄), -CO(alkyle en C₁ à C₄), alkyle en C₁ à C₄ ou phénylalkyle en C₁ à C₄ ;
R² est -SR^{a}, -SOR^{a}, -SO₂R^{a}, -SO₂NH₂, -SO₂NR^{b}R^{c} ou -CONR^{b}R^{c}, où
R^{a} ou R^{b} est un groupe alkyle en C₁ à C₆, cycloalkyle en C₃ à C₇, hétérocycloalkyle de 4 à 7 éléments, aryle ou hétéroaryle, où :
ledit groupe alkyle en C₁ à C₆ est facultativement substitué par un ou deux groupes, chacun choisi indépendamment parmi les groupes cyano, hydroxyle, alcoxy en C₁ à C₆, (alcoxy en C₁ à C₆) alcoxy en C₂ à C₆, -CO₂H, -CO₂ (alkyle en C₁ à C₄), -SO₂ (alkyle en C₁ à C₄) , -CONH₂, -CONH(alkyle en C₁ à C₄), -CON(alkyle en C₁ à C₄) (alkyle en C₁ à C₄), -SO₂NH₂, -SO₂NH(alkyle en C₁ à C₄), -SO₂N (alkyle en C₁ à C₄) (alkyle en C₁ à C₄), amino, (alkyle en C₁ à C₄)amino, (alkyle en C₁ à C₄) (alkyle en C₁ à C₄) amino, cycloalkyle en C₃ à C₇, phényle, hétéroaryle de 5 à 6 éléments, hétéroaryle de 9 à 10 éléments, hétérocycloalkyle de 4 à 7 éléments et phényl(alkyle en C₁ à C₄) amino, où ledit groupe cycloalkyle en C₃ à C₇, phényle, phényl(alkyle en C₁ à C₄) amino, hétéroaryle de 5 à 6 éléments, hétéroaryle de 9 à 10 éléments ou hétérocycloalkyle de 4 à 7 éléments est facultativement substitué par 1 à 3 groupes, chacun choisi indépendamment parmi un atome d'halogène, les groupes -CF₃, alkyle en C₁ à C₄, hydroxyalkyle en C₁ à C₄ et alcoxy en C₁ à C₄,
ledit groupe cycloalkyle en C₃ à C₇ ou hétérocycloalkyle de 4 à 7 éléments étant facultativement substitué par 1 à 3 groupes, chacun choisi indépendamment parmi un atome d'halogène, les groupes -CF₃, hydroxyle, amino, alkyle en C₁ à C₄, phénylalkyle en C₁ à C₄, hydroxyalkyle en C₁ à C₄, oxo et alcoxy en C₁ à C₄, et
ledit groupe aryle ou hétéroaryle étant facultativement substitué par 1 à 3 groupes, chacun choisi indépendamment parmi un atome d'halogène, les groupes -CF₃, hydroxyle, amino, alkyle en C₁ à C₄, phénylalkyle en C₁ à C₄, hydroxyalkyle en C₁ à C₄ et alcoxy en C₁ à C₄ ;
R^{c} est H, alcoxy en C₁ à C₄ ou alkyle en C₁ à C₆ ;
ou R^{b} et R^{c} ensemble avec l'atome d'azote auquel ils sont attachés forment un groupe hétérocycloalkyle de 5 à 7 éléments ou hétéroaryle de 5 à 6 éléments, contenant facultativement un hétéroatome sur le cycle supplémentaire, chacun choisi indépendamment parmi un atome d'azote, un atome d'oxygène et un atome de soufre, ledit hétérocycloalkyle de 5 à 7 éléments ou hétéroaryle de 5 à 6 éléments étant facultativement substitué par 1 à 3 groupes, chacun choisi indépendamment parmi les groupes alkyle en C₁ à C₄, halogénoalkyle en C₁ à C₄, phénylalkyle en C₁ à C₄, hydroxyalkyle en C₁ à C₄, alcoxy en C₁ à C₄, halogénoalcoxy en C₁ à C₄, amino, (alkyle en C₁ à C₄) amino, (alkyle en C₁ à C₄) (alkyle en C₁ à C₄) amino, -CO₂H, -CO₂(alkyle en C₁ à C₄), -CO(alkyle en C₁ à C₄), -CO(hétérocycloalkyle de 4 à 7 éléments), -CONH₂, -CONH(alkyle en C₁ à C₄), -CON (alkyle en C₁ à C₄) (alkyle en C₁ à C₄), -SO₂(alkyle en C₁ à C₄), -SO₂(hétérocycloalkyle de 4 à 7 éléments), -SO₂NH₂, -SO₂NH(alkyle en C₁ à C₄) et -SO₂N(alkyle en C₁ à C₄) (alkyle en C₁ à C₄) ;
A est un groupe phényle ou arylalkyle en C₁ à C₄ substitué par R³, R⁴ et R⁵, où :
R³ est H, un atome d'halogène, un groupe hydroxyle, -CF₃, hydroxyalkyle en C₁ à C₄, alkyle en C₁ à C₄ ou alcoxy en C₁ à C₄ ;
R⁴ est H, un atome d'halogène, un groupe cyano, alkyle en C₁ à C₄, halogénoalkyle en C₁ à C₄, alcoxy en C₁ à C₄, phénoxy, phénylalcoxy en C₁ à C₄, hydroxyle, hydroxyalkyle en C₁ à C₄ ou aminocarbonyle, où le fragment phényle dudit groupe phénoxy ou phénylalcoxy en C₁ à C₄ est facultativement substitué par 1 à 3 substituants, chacun choisi indépendamment parmi un atome d'halogène, les groupes -CF₃, alkyle en C₁ à C₄ et alcoxy en C₁ à C₄ ; et
R⁵ est H, un atome d'halogène, un groupe hydroxyle, -CF₃, hydroxyalkyle en C₁ à C₄, alkyle en C₁ à C₄ ou alcoxy en C₁ à C₄ ; ou
A est un groupe phényle substitué par R⁶, R⁷ et R⁸, ou
R⁶ et R⁷ sont situés sur des atomes adjacents et ensemble avec les atomes auxquels ils sont attachés forment un groupe hétérocyclique de 5 éléments contenant 1, 2 ou 3 hétéroatomes, chacun choisi indépendamment parmi N, O et S, lequel groupe hétérocyclique de 5 éléments est substitué par R⁹ ;
un de R⁸ ou R⁹ est H, un atome d'halogène, un groupe cyano, alkyle en C₁ à C₄, halogénoalkyle en C₁ à C₄, alcoxy en C₁ à C₄, phénoxy, phénylalcoxy en C₁ à C₄, hydroxyle, hydroxyalkyle en C₁ à C₄ ou aminocarbonyle, où le fragment phényle dudit groupe phénoxy ou phénylalcoxy en C₁ à C₄ est facultativement substitué par 1 à 3 substituants, chacun choisi indépendamment parmi un atome d'halogène, le groupes - CF₃, alkyle en C₁ à C₄ et alcoxy en C₁ à C₄ ; et
l'autre de R⁸ ou R⁹ est H, un atome d'halogène, un groupe hydroxyle, -CF₃, hydroxyalkyle en C₁ à C₄, alkyle en C₁ à C₄ ou alcoxy en C₁ à C₄ ; ou
A est un groupe pyrazolyle substitué par R¹⁰ et R¹¹, où :
R¹⁰ et R¹¹ sont situés sur des atomes de carbone adjacents et ensemble avec les atomes auxquels ils sont attachés forment un cycle carbocyclique ou un cycle hétérocyclique de 6 éléments substitué par R¹² et R¹³ ;
où R¹² est H, un atome d'halogène, un groupe cyano, alkyle en C₁ à C₄, halogénoalkyle en C₁ à C₄, alcoxy en C₁ à C₄, phénoxy, phénylalcoxy en C₁ à C₄, hydroxyle, hydroxyalkyle en C₁ à C₄ ou aminocarbonyle, où le fragment phényle dudit groupe phénoxy ou phénylalcoxy en C₁ à C₄ est facultativement substitué par 1 à 3 substituants, chacun choisi indépendamment parmi un atome d'halogène, les groupes -CF₃, alkyle en C₁ à C₄ et alcoxy en C₁ à C₄ ; et
R¹³ est H, un atome d'halogène, un groupe hydroxyle, -CF₃, hydroxyalkyle en C₁ à C₄, alkyle en C₁ à C₄ ou alcoxy en C₁ à C₄.

3. Composé ou sel pharmaceutiquement acceptable selon la revendication 1, dans lequel R¹ est H.

4. Composé ou sel pharmaceutiquement acceptable selon l'une quelconque des revendications 1 à 3, dans lequel R² est -SO₂R^{a}.

5. Composé ou sel pharmaceutiquement acceptable selon l'une quelconque des revendications 1 à 4, dans lequel R^{a} est un groupe alkyle en C₁ à C₆ et ledit groupe alkyle en C₁ à C₆ est facultativement substitué par 1 ou 2 substituants, chacun choisi indépendamment parmi les groupes hydroxyle ; alcoxy en C₁ à C₂ ; (alcoxy en C₁ à C₂)alcoxy en C₂ à C₃ ; amino ; (alkyle en C₁ à C₃)amino ; (alkyle en C₁ à C₃) (alkyle en C₁ à C₂)amino ; phényl(alkyle en C₁ à C₂)amino ; -CO₂(alkyle en C₁ à C₂) ; -CO₂NH₂ ; -SO₂ (alkyle en C₁ à C₂) ; et cycloalkyle en C₃ à C₆, facultativement substitué par un groupe alkyle en C₁ à C₄ ou hydroxyalkyle en C₁ à C₄ ; hétérocycloalkyle de 4 à 6 éléments facultativement substitué par un groupe alkyle en C₁ à C₄ ; hétéroaryle de 5 à 6 éléments, facultativement substitué par un groupe alkyle en C₁ à C₄ ; phényle ; ou hétéroaryle de 9 à 10 éléments.

6. Composé ou sel pharmaceutiquement acceptable selon l'une quelconque des revendications 1 à 4, dans lequel R^{a} est un groupe hétérocycloalkyle de 4 à 6 éléments, hétéroaryle de 5 à 6 éléments ou phényle,
ledit groupe hétérocycloalkyle de 4 à 6 éléments étant substitué par un groupe alkyle en C₁ à C₄, (alcoxy en C₁ à C₄)carbonyle ou benzyle,
ledit groupe hétéroaryle de 5 à 6 éléments étant substitué par un groupe alkyle en C₁ à C₄ ou hydroxyalkyle en C₁ à C₄, et
ledit groupe phényle étant substitué par un groupe amino.

7. Composé ou sel pharmaceutiquement acceptable selon l'une quelconque des revendications 1 à 4, dans lequel R^{a} est -CH₃, -CF₃, -CH₂CH₃, -CH₂CF₃, -CH₂CH₂CH₃, -CH₂CH=CH₂, -CH(CH₃)₂, -CH(CH₃)CH₂CH₃, -C(CH₃)₃, -CH₂CH₂OH, -CH₂CH₂OCH₃, -CH₂CH₂OCH₂CH₂OCH₃, -CH₂CH₂CH₂OH, -CH₂CH₂CH₂CH₂OH, -CH(CH₃)CH₂OH, -C(CH₃)₂CH₂OH, -C(CH₃)₂CO₂H, -C(CH₃)₂CH₂CH₂OH, -C(CH₃)₂CH₂CH₂OCH₃, -CH₂CH₂NH₂, -CH₂CH₂N(CH₃)₂, -CH₂CH₂N(CH₂CH₃)₂, -CH₂CH₂SO₂CH₃, -CH₂CONH₂, -CH₂CH₂CONH₂, -C(CH₃)₂CO₂CH₃, [1-(2-hydroxyéthyl)cyclopropyl]-méthyle, cyclopropyle, cyclopentyle, cyclohexyle, oxétan-3-yle, 3-méthyl-oxétan-3-yle, tétrahydro-2H-pyran-4-yle, tétrahydro-2H-pyran-3-yle, -CH₂-tétrahydro-2H-pyran-4-yle, tétrahydrofuran-3-yle, 2-méthyl-tétrahydrofuran-3-yle, -CH₂-tétrahydrofuran-2-yle, 1H-imidazol-4-yle, 1H-1,2,4-triazol-3-yle, phényle, benzyle, -CH₂CH₂CH₂-phényle, 4-amino-phényle, pyridin-4-yle, -CH₂-(6-méthyl-pyridin-2-yle), pipéridin-4-yle, 1-méthyl-pipéridin-4-yle, 1-((CH₃)₃C-O-CO-pipéridin-4-yle, -CH₂-pipéridin-4-yle, -CH₂CH₂-morpholin-4-yle, -CH₂CH₂CH₂-morpholin-4-yle, pyrimidin-2-yle, -CH₂CH₂-indol-3-yle, 4,5-diméthyl-thiazol-2-yle, (3R)-1-benzyl-pyrrolidin-3-yle, -CH₂CH₂-pyrrolidin-1-yle, -CH₂-benzimidazol-2-yle, -CH₂CH₂CH₂-imidazol-l-yle, -CH₂CH₂-imidazol-4-yle, -CH₂CH₂-(3,5-diméthyl-isoxazol-4-yle), (2S)-1-hydroxy-3-(1H-imidazol-4-yl)prop-2-yle, 3-[méthyl(phényl)amino]prop-1-yle, tétrahydro-2H-thiopyran-4-yle ou 1,1-dioxidotétrahydro-2H-thiopyran-4-yle.

8. Composé ou sel pharmaceutiquement acceptable selon l'une quelconque des revendications 1 à 7, dans lequel A est un groupe phényle ou phénylalkyle en C₁ à C₄, où n'importe quel groupe phényle est substitué par R³, R⁴ et R⁵, où :
R³ est H, un atome d'halogène, un groupe hydroxyle, hydroxyalkyle en C₁ à C₄, alkyle en C₁ à C₄ ou alcoxy en C₁ à C₄ ;
R⁴ est H, un atome d'halogène, un groupe cyano, alkyle en C₁ à C₄, halogénoalkyle en C₁ à C₄, alcoxy en C₁ à C₄, phénoxy, phénylalcoxy en C₁ à C₄, hydroxyle, hydroxyalkyle en C₁ à C₄ ou aminocarbonyle, où le fragment phényle dudit phénoxy ou phénylalcoxy en C₁ à C₄ est facultativement substitué par 1 à 3 substituants, chacun choisi indépendamment parmi un atome d'halogène, le groupes -CF₃, alkyle en C₁ à C₄ et alcoxy en C₁ à C₄ ; et
R⁵ est H, un atome d'halogène, les groupes hydroxyle, hydroxyalkyle en C₁ à C₄, alkyle en C₁ à C₄ ou alcoxy en C₁ à C₄.

9. Composé ou sel pharmaceutiquement acceptable selon l'une quelconque des revendications 1 à 7, dans lequel
A est un groupe phényle, 2-hydroxy-5-chloro-phényle, 2-hydroxy-5-fluoro-phényle, 2-hydroxy-4-fluoro-phényle, 3-hydroxy-4-fluoro-phényle, 3-hydroxy-4-chloro-phényle, 2-méthyl-4-hydroxy-phényle, 2-fluoro-4-hydroxy-phényle, 2-fluoro-5-hydroxy-phényle, 3-fluoro-4-hydroxy-phényle, 2-méthyl-6-hydroxy-phényle, 3-méthyl-5-hydroxy-phényle, 2-chloro-5-hydroxy-phényle, 2-chloro-4-hydroxy-phényle, 3-chloro-4-hydroxy-phényle, 4-hydroxy-phényle, 2-fluoro-phényle, 4-fluoro-phényle, 3-chloro-phényle, 4-chloro-phényle, 2,5-difluoro-phényle, 2,6-difluoro-phényle, 3,5-difluoro-phényle, 3,4,5-trifluoro-phényle, 2,4-dichloro-phényle, 3,4,5-trichloro-phényle, 2,4-diméthyl-phényle, 2-méthyl-5-fluoro-phényle, 2-chloro-3-hydroxy-4-méthyl-phényle, 2-méthyl-phényle, 3-méthoxy-phényle, 2-méthyl-3-hydroxy-phényle, 2-méthyl-5-hydroxy-phényle, 3-méthoxy-4-méthyl-phényle, 2,4-diméthyl-5-hydroxy-phényle, 2,4-diméthyl-5-méthoxy-phényle, 3-hydroxy-4-méthyl-phényle, 2-chloro-5-méthoxy-phényle, 3-méthoxy-4-fluoro-phényle, 3-méthoxy-4-chloro-phényle, 3-méthoxy-4-bromo-phényle, 3-hydroxyméthyl-phényle, 2-méthyl-5-hydroxyméthyl-phényle, 4-phénoxy-phényl ou 4-[(2-chlorophényl)méthoxy]phényle.

10. Composé ou sel pharmaceutiquement acceptable selon l'une quelconque des revendications 1 à 7, dans lequel A est un groupe 1H-pyrazolo[3,4-b]pyridinyle ou [1,3]thiazolo[5,4-b]pyridinyle facultativement substitué par un ou deux substituants, chacun choisi indépendamment parmi un atome d'halogène, les groupes alkyle en C₁ à C₄, halogénoalkyle en C₁ à C₄ et alcoxy en C₁ à C₄.

11. Composé ou sel pharmaceutiquement acceptable selon l'une quelconque des revendications 1 à 8, dans lequel A est un groupe indol-6-yle, indazol-3-yle, indazol-6-yle, 1H-1,2,3-benzotriazol-4-yle, 1H-1,2,3-benzotriazol-5-yle, 1,2,3-benzothiadiazol-5-yle, 2,1,3-benzoxadiazol-5-yle, 1,3-benzodioxol-4-yle, 1,3-benzodioxol-4-yle, 1,3-benzoxathiol-2-on-5-yle, benzofuran-4-yle, benzothiazol-4-yle, benzothiazol-5-yle, benzothiazol-6-yle, benzoxazol-5-yle ou 1,2-benzoisoxazol-6-yle facultativement substitué, où ledit groupe est facultativement substitué par un groupe hydroxyle, un atome de chlore, de fluor, de brome, un groupe -CF₃, cyano, hydroxyméthyle, méthyle, méthoxy ou aminocarbonyle.

12. Composé ou sel pharmaceutiquement acceptable selon l'une quelconque des revendications 1 à 7, dans lequel A est un groupe indol-6-yle, indazol-3-yle, indazol-6-yle, 5-méthoxy-indazol-3-yle, 5-fluoro-indazol-3-yle, 4-chloro-indazol-3-yle, 5-chloro-indazol-3-yle, 6-chloro-indazol-3-yle, 7-trifluorométhyl-indazol-3-yle, 7-chloro-indazol-3-yle, 1-méthyl-indazol-3-yle, 5-cyano-indazol-6-yle, 7-méthyl-indazol-6-yle, 5-aminocarbonyl-indazol-6-yle, 3-fluoro-indazol-6-yle, 3-méthyl-indazol-6-yle, 1H-1,2,3-benzotriazol-4-yle, 1H-1,2,3-benzotriazol-5-yle, 4-méthyl-1H-1,2,3-benzotriazol-6-yle, 5-méthyl-1H-1,2,3-benzotriazol-6-yle, 5-fluoro-1H-1,2,3-benzotriazol-6-yle, 1,2,3-benzothiadiazol-5-yle, 2,1,3-benzoxadiazol-5-yle, 1,3-benzodioxol-4-yle, 5-chloro-1,3-benzodioxol-4-yle, 2-oxo-1,3-benzoxathiol-5-yle, benzofuran-4-yle, benzothiazol-5-yle, benzothiazol-6-yle, 6-méthyl-benzothiazol-5-yle, 6-fluorobenzothiazol-5-yle, 4-méthyl-benzothiazol-5-yle, 4-fluoro-benzothiazol-5-yle, 4-chloro-benzothiazol-5-yle, 4-bromo-benzothiazol-5-yle, benzoxazol-5-yle, 1,2-benzoisoxazol-6-yle, 2,1-benzisothiazol-6-yle, 5-fluoro-1H-pyrazolo[3,4-b]pyridine-3-yle, 5-méthyl-1H-pyrazolo[3,4-b]pyridine-3-yle, 5-fluoro-6-méthyl-1H-pyrazolo[3,4-b]pyridine-3-yle ou [1,3]thiazolo[5,4-b]pyridin-6-yle.

13. Composé selon la revendication 1, qui est :
N-1,3-benzothiazol-5-yl-6-(méthylsulfonyl)-4-quinoléinamine,
N-(2-méthylphényl)-6-(méthylsulfonyl)-4-quinoléinamine,
4-chloro-2-{[6-(méthylsulfonyl)-4-quinoléinyl]amino}phénol,
4-fluoro-2-{[6-(méthylsulfonyl)-4-quinoléinyl]amino}phénol,
N-1H-1,2,3-benzotriazol-5-yl-6-(méthylsulfonyl)-4-quinoléinamine,
3-fluoro-4-{[6-(méthylsulfonyl)-4-quinoléinyl]amino}phénol,
2-chloro-4-{[6-(méthylsulfonyl)-4-quinoléinyl]amino}phénol,
3-méthyl-2-{[6-(méthylsulfonyl)-4-quinoléinyl]amino}phénol,
4-chloro-3-{[6-(méthylsulfonyl)-4-quinoléinyl]amino}phénol,
3-chloro-4-{[6-(méthylsulfonyl)-4-quinoléinyl]amino}phénol,
4-{[6-(méthylsulfonyl)-4-quinoléinyl]amino}phénol,
2-fluoro-4-{[6-(méthylsulfonyl)-4-quinoléinyl]amino}phénol,
N-1H-indol-6-yl-6-(méthylsulfonyl)-4-quinoléinamine,
5-fluoro-2-{[6-(méthylsulfonyl)-4-quinoléinyl]amino}phénol,
2-chloro-6-méthyl-3-{[6-(méthylsulfonyl)-4-quinoléinyl]amino}phénol,
6-(méthylsulfonyl)-N-phényl-4-quinoléinamine,
N-(2-fluorophényl)-6-(méthylsulfonyl)-4-quinoléinamine,
N-(2,5-difluorophényl)-6-(méthylsulfonyl)-4-quinoléinamine,
N-(2,6-difluorophényl)-6-(méthylsulfonyl)-4-quinoléinamine,
N-(2,4-dichlorophényl)-6-(méthylsulfonyl)-4-quinoléinamine,
N-(2,4-diméthylphényl)-6-(méthylsulfonyl)-4-quinoléinamine,
N-(3,5-difluorophényl)-6-(méthylsulfonyl)-4-quinoléinamine,
N-(5-fluoro-2-méthylphényl)-6-(méthylsulfonyl)-4-quinoléinamine,
N-(3-chlorophényl)-6-(méthylsulfonyl)-4-quinoléinamine,
N-(4-fluorophényl)-6-(méthylsulfonyl)-4-quinoléinamine,
N-(4-chlorophényl)-6-(méthylsulfonyl)-4-quinoléinamine,
(4-méthyl-3-{[6-(méthylsulfonyl)-4-quinoléinyl]amino}phényl)méthanol,
N-[4-méthyl-3-(méthyloxy)phényl]-6-(méthylsulfonyl)-4-quinoléinamine,
(3-{[6-(méthylsulfonyl)-4-quinoléinyl]amino}phényl)méthanol,
N-1,3-benzodioxol-4-yl-6-(méthylsulfonyl)-4-quinoléinamine,
N-(4-{[(2-chlorophényl)méthyl]oxy}phényl)-6-(méthylsulfonyl)-4-quinoléinamine,
2-fluoro-5-{[6-(méthylsulfonyl)-4-quinoléinyl]amino}phénol,
4-fluoro-3-{[6-(méthylsulfonyl)-4-quinoléinyl]amino}phénol,
2,4-diméthyl-5-{[6-(méthylsulfonyl)-4-quinoléinyl]amino}phénol,
3-méthyl-5-{[6-(méthylsulfonyl)-4-quinoléinyl]amino}phénol,
N-1H-1,2,3-benzotriazol-4-yl-6-(méthylsulfonyl)-4-quinoléinamine,
5-{[6-(méthylsulfonyl)-4-quinoléinyl]amino}-1,3-benzoxathiol-2-one,
N-(4-méthyl-1H-1,2,3-benzotriazol-6-yl)-6-(méthylsulfonyl)-4-quinoléinamine,
N-(5-fluoro-1H-1,2,3-benzotriazol-6-yl)-6-(méthylsulfonyl)-4-quinoléinamine,
N-(5-méthyl-1H-1,2,3-benzotriazol-6-yl)-6-(méthylsulfonyl)-4-quinoléinamine,
N-1,2,3-benzothiadiazol-5-yl-6-(méthylsulfonyl)-4-quinoléinamine,
N-1,2-benzisoxazol-6-yl-6-(méthylsulfonyl)-4-quinoléinamine,
N-[2,4-diméthyl-5-(méthyloxy)phényl]-6-(méthylsulfonyl)-4-quinoléinamine,
6-(méthylsulfonyl)-N-[4-(phényloxy)phényl]-4-quinoléinamine,
N-[3-(méthyloxy)phényl]-6-(méthylsulfonyl)-4-quinoléinamine,
2-méthyl-3-{[6-(méthylsulfonyl)-4-quinoléinyl]amino}phénol,
2-méthyl-5-{[6-(méthylsulfonyl)-4-quinoléinyl]amino}phénol,
N-[2-chloro-5-(méthyloxy)phényl]-6-(méthylsulfonyl)-4-quinoléinamine,
N-[4-fluoro-3-(méthyloxy)phényl]-6-(méthylsulfonyl)-4-quinoléinamine,
3-méthyl-4-{[6-(méthylsulfonyl)-4-quinoléinyl]amino}phénol,
6-(méthylsulfonyl)-N-phényl-4-quinoléinamine,
N-1-benzofuran-4-yl-6-(méthylsulfonyl)-4-quinoléinamine,
N-[4-bromo-3-(méthyloxy)phényl]-6-(méthylsulfonyl)-4-quinoléinamine,
2-chloro-5-{[6-(méthylsulfonyl)-4-quinoléinyl]amino}phénol,
N-1,3-benzothiazol-6-yl-6-(méthylsulfonyl)-4-quinoléinamine,
N-2H-indazol-3-yl-6-(méthylsulfonyl)-4-quinoléinamine,
N-1,3-benzothiazol-5-yl-6-[(1-méthyléthyl)sulfonyl]-4-quinoléinamine,
N-1H-indazol-6-yl-6-[(1-méthyléthyl)sulfonyl]-4-quinoléinamine,
N-1,3-benzothiazol-5-yl-6-(éthylsulfonyl)-4-quinoléinamine,
6-(éthylsulfonyl)-N-1H-indazol-6-yl-4-quinoléinamine,
6-({6-[(1-méthyléthyl)sulfonyl]-4-quinoléinyl}amino)-1H-indazole-5-carboxamide,
6-[(1-méthyléthyl)sulfonyl]-N-(7-méthyl-1H-indazol-6-yl)-4-quinoléinamine,
N-[4-chloro-3-(méthyloxy)phényl]-6-[(1-méthyléthyl)sulfonyl]-4-quinoléinamine,
6-({6-[(1-méthyléthyl)sulfonyl]-4-quinoléinyl}amino)-1H-indazole-5-carbonitrile,
N-1H-indazol-3-yl-6-[(1-méthyléthyl)sulfonyl]-4-quinoléinamine,
6-[(1-méthyléthyl)sulfonyl]-N-[5-(méthyloxy)-1H-indazol-3-yl]-4-quinoléinamine,
N-(5-fluoro-1H-indazol-3-yl)-6-[(1-méthyléthyl)sulfonyl]-4-quinoléinamine,
N-(3-méthyl-1H-indazol-6-yl)-6-(méthylsulfonyl)-4-quinoléinamine,
N-[4-chloro-3-(méthyloxy)phényl]-6-(méthylsulfonyl)-4-quinoléinamine,
N-(3-fluoro-1H-indazol-6-yl)-6-(méthylsulfonyl)-4-quinoléinamine,
N-(4-chloro-1H-indazol-3-yl)-6-(méthylsulfonyl)-4-quinoléinamine,
N-[5-(méthyloxy)-1H-indazol-3-yl]-6-(méthylsulfonyl)-4-quinoléinamine,
N-(5-fluoro-1H-indazol-3-yl)-6-(méthylsulfonyl)-4-quinoléinamine,
N-(5-chloro-1H-indazol-3-yl)-6-(méthylsulfonyl)-4-quinoléinamine,
N-(6-chloro-1H-indazol-3-yl)-6-(méthylsulfonyl)-4-quinoléinamine,
N-1H-indazol-6-yl-6-(méthylsulfonyl)-4-quinoléinamine,
6-[(1,1-diméthyléthyl)sulfonyl]-N-(5-fluoro-1H-pyrazolo[3,4-b]pyridin-3-yl)-4-quinoléinamine,
N-(5-méthyl-1H-pyrazolo[3,4-b]pyridin-3-yl)-6-(méthylsulfonyl)-4-quinoléinamine,
N-(4-méthyl-1,3-benzothiazol-5-yl)-6-(méthylsulfonyl)-4-quinoléinamine,
N-(4-bromo-1,3-benzothiazol-5-yl)-6-(méthylsulfonyl)-4-quinoléinamine,
N-(4-chloro-1,3-benzothiazol-5-yl)-6-(méthylsulfonyl)-4-quinoléinamine,
6-[(1,1-diméthyléthyl)sulfonyl]-N-(5-fluoro-1H-pyrazolo[3,4-b]pyridin-3-yl)-4-quinoléinamine,
6-[(1,1-diméthyléthyl)sulfonyl]-N-(5-fluoro-6-méthyl-1H-pyrazolo[3,4-b]pyridin-3-yl)-4-quinoléinamine,
6-[(1-méthyléthyl)sulfonyl]-N-(1-méthyl-1H-indazol-3-yl)-4-quinoléinamine,
6-[(1,1-diméthyléthyl)sulfonyl]-N-1H-indazol-6-yl-4-quinoléinamine,
6-[(1,1-diméthyléthyl)sulfonyl]-N-(3-fluoro-1H-indazol-6-yl)-4-quinoléinamine,
6-[(1,1-diméthyléthyl)sulfonyl]-N-(3-méthyl-1H-indazol-6-yl)-4-quinoléinamine,
6-[(1,1-diméthyléthyl)sulfonyl]-N-1H-indazol-3-yl-4-quinoléinamine,
6-[(1,1-diméthyléthyl)sulfonyl]-N-(5-fluoro-1H-indazol-3-yl)-4-quinoléinamine,
N-[4-chloro-3-(méthyloxy)phényl]-6-[(1,1-diméthyléthyl)sulfonyl]-4-quinoléinamine,
N-(3-fluoro-1H-indazol-6-yl)-6-[(1-méthyléthyl)sulfonyl]-4-quinoléinamine,
6-[(1-méthyléthyl)sulfonyl]-N-(3-méthyl-1H-indazol-6-yl)-4-quinoléinamine,
6-[(1-méthyléthyl)sulfonyl]-N-[7-(trifluorométhyl)-1H-indazol-3-yl]-4-quinoléinamine,
N-(5-fluoro-1H-indazol-3-yl)-6-[(trifluorométhyl)sulfonyl]-4-quinoléinamine,
N-(6-(((tétrahydrofuran-2-yl)méthyl)sulfonyl)quinoléin-4-yl)benzo[d]thiazol-5-amine,
4-(1,3-benzothiazol-5-ylamino)-6-[(trifluorométhyl)sulfonyl]quinoléine,
N-(6-(tert-butylsulfonyl)quinoléin-4-yl)thiazolo[5,4-b]pyridin-6-amine,
6-[(1-méthyléthyl)sulfonyl]-N-[1,3]thiazolo[5,4-b]pyridin-6-yl-4-quinoléinamine,
N-2,1,3-benzoxadiazol-5-yl-6-(méthylsulfonyl)-4-quinoléinamine,
3-{[4-(1,3-benzothiazol-5-ylamino)-6-quinoléinyl]sulfonyl}-3-méthyl-1-butanol,
N-1,3-benzothiazol-5-yl-6-(phénylsulfonyl)-4-quinoléinamine,
N-1,3-benzothiazol-5-yl-6-(cyclopropylsulfonyl)-4-quinoléinamine,
6-(méthylsulfonyl)-N-(2-phényléthyl)-4-quinoléinamine,
6-[(4-aminophényl)sulfonyl]-N-1,3-benzothiazol-5-yl-4-quinoléinamine,
N³-méthylidène-4-(méthylthio)-N¹-[6-(propylsulfonyl)-4-quinoléinyl]-1,3-benzenediamine,
N-1,3-benzothiazol-5-yl-6-(cyclohexylsulfonyl)-4-quinoléinamine,
N-1,3-benzothiazol-5-yl-6-(4-pyridinylsulfonyl)-4-quinoléinamine,
3-{[4-(1,3-benzothiazol-5-ylamino)-6-quinoléinyl]thio}-3-méthyl-1-butanol,
N-1,3-benzothiazol-5-yl-6-(éthylsulfonyl)-4-quinoléinamine,
3-{[4-(1,3-benzothiazol-5-ylamino)-6-quinoléinyl]sulfonyl}-1-propanol,
4-{[4-(1,3-benzothiazol-5-ylamino)-6-quinoléinyl]sulfonyl}-1-butanol,
2-{[4-(1,3-benzothiazol-5-ylamino)-6-quinoléinyl]sulfonyl}éthanol,
N-1,3-benzothiazol-5-yl-6-[(1,1-diméthyléthyl)sulfonyl]-4-quinoléinamine,
N-1,3-benzothiazol-5-yl-6-[(1,1-diméthyléthyl)thio]-4-quinoléinamine,
N-1,3-benzothiazol-5-yl-6-[(1-méthylpropyl)thio]-4-quinoléinamine,
N-1,3-benzothiazol-5-yl-6-(éthylthio)-4-quinoléinamine,
6-[(2-aminoéthyl)thio]-N-1,3-benzothiazol-5-yl-4-quinoléinamine,
N-1,3-benzothiazol-5-yl-6-(cyclopentylthio)-4-quinoléinamine,
N-1,3-benzothiazol-5-yl-6-(cyclopentylsulfonyl)-4-quinoléinamine,
3-{[4-(1H-indazol-6-ylamino)-6-quinoléinyl]thio}-3-méthyl-1-butanol,
3-{[4-(1H-indazol-6-ylamino)-6-quinoléinyl]sulfonyl}-3-méthyl-1-butanol,
N-1,3-benzothiazol-5-yl-6-[(1-méthyl-4-pipéridinyl)thio]-4-quinoléinamine,
N-1,3-benzothiazol-5-yl-6-(2-pyrimidinylthio)-4-quinoléinamine,
2-{[4-(1,3-benzothiazol-5-ylamino)-6-quinoléinyl]thio}-1-propanol,
2-{[4-(1,3-benzothiazol-5-ylamino)-6-quinoléinyl]sulfonyl}-1-propanol,
2-[1-({[4-(1,3-benzothiazol-5-ylamino)-6-quinoléinyl]thio}méthyl)cyclopropyl]éthanol,
2-[1-({[4-(1,3-benzothiazol-5-ylamino)-6-quinoléinyl]sulfonyl}méthyl)cyclopropyl]éthanol,
2-{[4-(1,3-benzothiazol-5-ylamino)-6-quinoléinyl]sulfonyl}-2-méthyl-1-propanol,
2-{[4-(1,3-benzothiazol-5-ylamino)-6-quinoléinyl]thio}-2-méthyl-1-propanol,
2-{[4-(1,3-benzothiazol-5-ylamino)-6-quinoléinyl]sulfinyl}-2-méthyl-1-propanol,
2-{[4-(1H-indazol-6-ylamino)-6-quinoléinyl]sulfonyl}-2-méthyl-1-propanol,
2-{[4-(1,3-benzothiazol-5-ylamino)-6-quinoléinyl]thio}-2-méthylpropanoate de méthyle,
2-{[4-(1,3-benzothiazol-5-ylamino)-6-quinoléinyl]sulfonyl}-2-méthylpropanoate de méthyle,
N-1,3-benzothiazol-5-yl-6-{[1,1-diméthyl-3-(méthyloxy)propyl]thio}-4-quinoléinamine,
N-(5-fluoro-1H-indazol-3-yl)-6-(tétrahydro-2H-pyran-4-ylsulfonyl)-4-quinoléinamine,
2-({4-[(5-fluoro-1H-indazol-3-yl)amino]-6-quinoléinyl}sulfonyl)éthanol,
N-1,3-benzothiazol-5-yl-6-(1H-1,2,4-triazol-3-ylsulfonyl)-4-quinoléinamine,
N-(5-fluoro-1H-indazol-3-yl)-6-(1H-imidazol-4-ylsulfonyl)-4-quinoléinamine,
N-(5-fluoro-1H-indazol-3-yl)-6-(1H-1,2,4-triazol-3-ylsulfonyl)-4-quinoléinamine,
N-(5-fluoro-1H-indazol-3-yl)-6-(tétrahydro-3-furanylsulfonyl)-4-quinoléinamine,
N-1,3-benzothiazol-5-yl-6-[(2-méthyltétrahydro-3-furanyl)sulfonyl]-4-quinoléinamine,
N-1,3-benzothiazol-5-yl-6-{[2-(méthyloxy)éthyl]sulfonyl}-4-quinoléinamine,
N-1,3-benzothiazol-5-yl-6-(méthylthio)-4-quinoléinamine,
2-{[4-(1,3-benzothiazol-5-ylamino)-6-quinoléinyl]thio}éthanol,
N-1,3-benzothiazol-5-yl-6-{[2-(diéthylamino)éthyl]thio}-4-quinoléinamine,
N-1,3-benzothiazol-5-yl-6-{[2-(diéthylamino)éthyl]sulfonyl}-4-quinoléinamine,
N-1,3-benzothiazol-5-yl-6-(tétrahydro-3-furanylthio)-4-quinoléinamine,
N-1,3-benzothiazol-5-yl-6-(tétrahydro-3-furanylsulfonyl)-4-quinoléinamine,
N-1,3-benzothiazol-5-yl-6-(tétrahydro-2H-pyran-4-ylthio)-4-quinoléinamine,
N-1,3-benzothiazol-5-yl-6-(tétrahydro-2H-pyran-4-ylsulfonyl)-4-quinoléinamine,
N-1,3-benzothiazol-5-yl-6-(2-propén-1-ylsulfonyl)-4-quinoléinamine,
N-1,3-benzothiazol-5-yl-6-{[2-(4-morpholinyl)éthyl]thio}-4-quinoléinamine,
N-1,3-benzothiazol-5-yl-6-{[2-(3,5-diméthyl-4-isoxazolyl)éthyl]thio}-4-quinoléinamine,
N-1,3-benzothiazol-5-yl-6-{[2-(3,5-diméthyl-4-isoxazolyl)éthyl]sulfonyl}-4-quinoléinamine,
N-(6-((1-méthylpipéridin-4-yl)sulfonyl)quinoléin-4-yl)benzo[d]thiazol-5-amine,
4-{[4-(1,3-benzothiazol-5-ylamino)-6-quinoléinyl]thio}-1-pipéridinecarboxylate de 1,1-diméthyléthyle,
4-{[4-(1,3-benzothiazol-5-ylamino)-6-quinoléinyl]sulfonyl}-1-pipéridinecarboxylate de 1,1-diméthyléthyle,
N-1,3-benzothiazol-5-yl-6-[(2,2,2-trifluoroéthyl)thio]-4-quinoléinamine,
N-1,3-benzothiazol-5-yl-6-[(2,2,2-trifluoroéthyl)sulfonyl]-4-quinoléinamine,
N-1,3-benzothiazol-5-yl-6-(tétrahydro-2H-thiopyran-4-ylthio)-4-quinoléinamine,
2-{[4-(1,3-benzothiazol-5-ylamino)-6-quinoléinyl]sulfinyl}éthanol,
N-(6-(((3S)-tétrahydro-2H-pyran-3-yl)sulfonyl)quinoléin-4-yl)benzo[d]thiazol-5-amine,
N-(6-(((3R)-tétrahydro-2H-pyran-3-yl)sulfonyl)quinoléin-4-yl)benzo[d]thiazol-5-amine,
2-{[4-(1,3-benzothiazol-5-ylamino)-6-quinoléinyl]thio}éthanol,
N-1,3-benzothiazol-5-yl-6-[(1-méthyléthyl)thio]-4-quinoléinamine,
acide 2-{[4-(1,3-benzothiazol-5-ylamino)-6-quinoléinyl]thio}-2-méthylpropanoïque,
acide 2-{[4-(1,3-benzothiazol-5-ylamino)-6-quinoléinyl]sulfonyl}-2-méthylpropanoïque,
N-1,3-benzothiazol-5-yl-6-[(1,1-dioxidotétrahydro-2H-thiopyran-4-yl)sulfonyl]-4-quinoléinamine,
N-1,3-benzothiazol-5-yl-6-(4-pipéridinylsulfonyl)-4-quinoléinamine,
2-{[4-(1,3-benzothiazol-5-ylamino)-6-quinoléinyl]sulfinyl}éthanol,
N-1,3-benzothiazol-5-yl-6-((R)méthylsulfinyl)-4-quinoléinamine,
N-1,3-benzothiazol-5-yl-6-((S)méthylsulfinyl)-4-quinoléinamine,
N-1,3-benzothiazol-5-yl-6-[(1-méthyléthyl)sulfinyl]-4-quinoléinamine,
4-[(5-hydroxy-2-méthylphényl)amino]-N-(phénylméthyl)-6-quinoléinecarboxamide,
N-cyclohexyl-4-[(5-hydroxy-2-méthylphényl)amino]-6-quinoléinecarboxamide,
4-[(5-hydroxy-2-méthylphényl)amino]-N-[3-(4-morpholinyl)propyl]-6-quinoléinecarboxamide,
4-[(5-hydroxy-2-méthylphényl)amino]-N-(tétrahydro-2H-pyran-4-ylméthyl)-6-quinoléinecarboxamide,
4-[(5-hydroxy-2-méthylphényl)amino]-N-(3-phénylpropyl)-6-quinoléinecarboxamide,
4-[(5-hydroxy-2-méthylphényl)amino]-N-[(3R)-1-(phénylméthyl)-3-pyrrolidinyl]-6-quinoléinecarboxamide,
4-[(5-hydroxy-2-méthylphényl)amino]-N-(3-[méthyl(phényl)amino]propyl}-6-quinoléinecarboxamide,
4-[(5-hydroxy-2-méthylphényl)amino]-N-[3-(1H-imidazol-1-yl)propyl]-6-quinoléinecarboxamide,
4-[(5-hydroxy-2-méthylphényl)amino]-N-[2-(1H-imidazol-4-yl)éthyl]-6-quinoléinecarboxamide,
N-[(1S)-2-hydroxy-1-(1H-imidazol-4-ylméthyl)éthyl]-4-[(5-hydroxy-2-méthylphényl)amino]-6-quinoléinecarboxamide,
N-(1H-benzimidazol-2-ylméthyl)-4-[(5-hydroxy-2-méthylphényl)amino]-6-quinoléinecarboxamide,
4-[(5-hydroxy-2-méthylphényl)amino]-N-[2-(1-pyrrolidinyl)éthyl]-6-quinoléinecarboxamide,
4-[(5-hydroxy-2-méthylphényl)amino]-N-[2-(méthylsulfonyl)éthyl]-6-quinoléinecarboxamide,
4-[(5-hydroxy-2-méthylphényl)amino]-N-[2-(1H-indol-3-yl)éthyl]-6-quinoléinecarboxamide,
4-[(5-hydroxy-2-méthylphényl)amino]-N-[(6-méthyl-2-pyridinyl)méthyl]-6-quinoléinecarboxamide,
N-(4,5-diméthyl-1,3-thiazol-2-yl)-4-[(5-hydroxy-2-méthylphényl)amino]-6-quinoléinecarboxamide,
4-(1,3-benzothiazol-5-ylamino)-N-(phénylméthyl)-6-quinoléinecarboxamide,
N-1,3-benzothiazol-5-yl-6-(4-morpholinylsulfonyl)-4-quinoléinamine,
4-(1,3-benzothiazol-5-ylamino)-N-(3-méthyl-3-oxétanyl)-6-quinoléinesulfonamide,
4-(1,3-benzothiazol-5-ylamino)-N-(1-méthyléthyl)-6-quinoléinesulfonamide,
N-1,3-benzothiazol-5-yl-6-(1-pipéridinylsulfonyl)-4-quinoléinamine,
4-(1,3-benzothiazol-5-ylamino)-N,N-diméthyl-6-quinoléinesulfonamide,
4-(1,3-benzothiazol-5-ylamino)-N-méthyl-N-(méthyloxy)-6-quinoléinesulfonamide,
4-(1,3-benzothiazol-5-ylamino)-N-(2-hydroxyéthyl)-N-méthyl-6-quinoléinesulfonamide,
N-(2-hydroxyéthyl)-4-(1H-indazol-6-ylamino)-N-méthyl-6-quinoléinesulfonamide,
4-{[4-chloro-3-(méthyloxy)phényl]amino}-N-(2-hydroxyéthyl)-N-méthyl-6-quinoléinesulfonamide,
4-{[4-chloro-3-(méthyloxy)phényl]amino}-6-quinoléinesulfonamide,
4-(1H-indazol-6-ylamino)-6-quinoléinesulfonamide,
4-(1,3-benzothiazol-5-ylamino)-N-4-pipéridinyl-6-quinoléinesulfonamide,
4-(1,3-benzothiazol-5-ylamino)-N-(4-pipéridinylméthyl)-6-quinoléinesulfonamide,
N-1,3-benzothiazol-5-yl-6-(4-morpholinylsulfonyl)-4-quinoléinamine,
4-(1,3-benzothiazol-5-ylamino)-N-[(6-méthyl-2-pyridinyl)méthyl]-6-quinoléinesulfonamide,
4-(1,3-benzothiazol-5-ylamino)-N-[2-(diméthylamino)éthyl]-6-quinoléinesulfonamide,
4-(1,3-benzothiazol-5-ylamino)-N-[2-(méthyloxy)éthyl]-6-quinoléinesulfonamide,
4-(1,3-benzothiazol-5-ylamino)-N-[3-(4-morpholinyl)propyl]-6-quinoléinesulfonamide,
4-(1,3-benzothiazol-5-ylamino)-N-(tétrahydro-2H-pyran-4-ylméthyl)-6-quinoléinesulfonamide,
4-(1,3-benzothiazol-5-ylamino)-N-(tétrahydro-2H-pyran-4-yl)-6-quinoléinesulfonamide,
4-(1,3-benzothiazol-5-ylamino)-N-cyclohexyl-6-quinoléinesulfonamide,
4-(1,3-benzothiazol-5-ylamino)-N-[2-(méthylsulfonyl)éthyl]-6-quinoléinesulfonamide,
4-(1,3-benzothiazol-5-ylamino)-N-(phénylméthyl)-6-quinoléinesulfonamide,
N~2~-{[4-(1,3-benzothiazol-5-ylamino)-6-quinoléinyl]sulfonyl}glycinamide,
N~3~-{[4-(1,3-benzothiazol-5-ylamino)-6-quinoléinyl]sulfonyl}-bêta-alaninamide,
4-(1,3-benzothiazol-5-ylamino)-N-(2-hydroxyéthyl)-6-quinoléinesulfonamide,
4-(1,3-benzothiazol-5-ylamino)-N-3-oxetanyl-6-quinoléinesulfonamide,
4-(1,3-benzothiazol-5-ylamino)-N-(2-{[2-(méthyloxy)éthyl]oxy}éthyl)-6-quinoléinesulfonamide,
4-(1,3-benzothiazol-5-ylamino)-N-méthyl-N-(1-méthyléthyl)-6-quinoléinesulfonamide,
4-(1H-indazol-6-ylamino)-N,N-diméthyl-6-quinoléinesulfonamide,
4-{[4-chloro-3-(méthyloxy)phényl]amino}-N,N-diméthyl-6-quinoléinesulfonamide,
N-1H-indazol-6-yl-6-(4-morpholinylsulfonyl)-4-quinoléinamine,
N-[4-chloro-3-(méthyloxy)phényl]-6-(4-morpholinylsulfonyl)-4-quinoléinamine,
4-(1H-indazol-6-ylamino)-N-(1-méthyléthyl)-6-quinoléinesulfonamide,
4-{[4-chloro-3-(méthyloxy)phényl]amino}-N-(1-méthyléthyl)-6-quinoléinesulfonamide,
N-1,3-benzothiazol-5-yl-6-(1-pyrrolidinylsulfonyl)-4-quinoléinamine,
1-{[4-(1,3-benzothiazol-5-ylamino)-6-quinoléinyl]sulfonyl}-1-prolinate de méthyle,
(3S,6R)-1-{[4-(1,3-benzothiazol-5-ylamino)-6-quinoléinyl]sulfonyl}-6-méthyl-3-pipéridinecarboxylate de méthyle,
1-{[4-(1,3-benzothiazol-5-ylamino)-6-quinoléinyl]sulfonyl}-3-pyrrolidinol,
N-1,3-benzothiazol-5-yl-6-[(3-méthyl-4-morpholinyl)sulfonyl]-4-quinoléinamine,
N-1,3-benzothiazol-5-yl-6-[(4-méthyl-1-pipérazinyl)sulfonyl]-4-quinoléinamine,
N-1,3-benzothiazol-5-yl-6-(4-thiomorpholinylsulfonyl)-4-quinoléinamine,
N-1,3-benzothiazol-5-yl-6-[(1,1-dioxido-4-thiomorpholinyl)sulfonyl]-4-quinoléinamine,
N-1,3-benzothiazol-5-yl-6-{[(3R)-3-méthyl-4-morpholinyl]sulfonyl}-4-quinoléinamine,
((2S,5R)-4-{[4-(1,3-benzothiazol-5-ylamino)-6-quinoléinyl]sulfonyl}-5-éthyl-2-morpholinyl)méthanol,
N-1,3-benzothiazol-5-yl-6-{[(3S)-3-méthyl-4-morpholinyl]sulfonyl}-4-quinoléinamine,
N-1,3-benzothiazol-5-yl-6-(1-pipérazinylsulfonyl)-4-quinoléinamine,
((2S,5R)-4-{[4-(1,3-benzothiazol-5-ylamino)-6-quinoléinyl]sulfonyl}-5-méthyl-2-morpholinyl)méthanol,
(4-{[4-(1,3-benzothiazol-5-ylamino)-6-quinoléinyl]sulfonyl}-2-morpholinyl)méthanol,
4-(1,3-benzothiazol-5-ylamino)-N-méthyl-N-[2-(méthyloxy)éthyl]-6-quinoléinesulfonamide,
4-(1,3-benzothiazol-5-ylamino)-N-[2-(méthyloxy)éthyl]-6-quinoléinesulfonamide,
N-(5-fluoro-1H-indazol-3-yl)-6-(4-morpholinylsulfonyl)-4-quinoléinamine,
N-1,3-benzothiazol-5-yl-6-[(2,2-diméthyl-4-morpholinyl)sulfonyl]-4-quinoléinamine,
N-1,3-benzothiazol-5-yl-6-[(2-méthyl-4-morpholinyl)sulfonyl]-4-quinoléinamine,
4-[(7-chloro-1H-indazol-3-yl)amino]-N-[2-(méthyloxy)éthyl]-6-quinoléinesulfonamide,
N-1,3-benzothiazol-5-yl-N-[6-(méthylsulfonyl)-4-quinoléinyl]acétamide,
N-1,3-benzothiazol-5-yl-N-[6-(méthylsulfonyl)-4-quinoléinyl]méthanesulfonamide,
N-1,3-benzoxazol-5-yl-6-(méthylsulfonyl)-4-quinoléinamine, N-1,3-benzoxazol-5-yl-6-[(1-méthyléthyl)sulfonyl]-4-quinoléinamine,
N-1,3-benzoxazol-5-yl-6-(4-morpholinylsulfonyl)-4-quinoléinamine,
N-[4-chloro-3-(méthyloxy)phényl]-6-(tétrahydro-2H-pyran-4-ylsulfonyl)-4-quinoléinamine, ou
N-[4-chloro-3-(méthyloxy)phényl]-6-(tétrahydro-2H-pyran-4-ylsulfinyl)-4-quinoléinamine,
ou un sel pharmaceutiquement acceptable de ceux-ci.

14. Composition pharmaceutique comprenant le composé ou sel pharmaceutiquement acceptable selon l'une quelconque des revendications 1 à 13 et un ou plusieurs excipients pharmaceutiquement acceptables.

15. Composé ou sel pharmaceutiquement acceptable selon l'une quelconque des revendications 1 à 14, pour une utilisation en thérapie.
